# EUROPEAN PATENT APPLICATION

(11) **EP 4 015 634 A1**
(43) Date of publication of application: **22.06.2022**
(21) Application number: 20383090.6
(22) Date of filing: 15.12.2020
(51) Int. Cl.: C12N 15/113, A61K 31/713, C12N 15/82, A61P 31/14

(54) **SIRNA AND COMPOSITIONS FOR PROPHYLACTIC AND THERAPEUTIC TREATMENT OF VIRUS DISEASES**

(71) Applicant: SYLENTIS, S.A.U., 28003 Madrid (ES)
(72) Inventor: JIMÉNEZ, Ana Isabel, E-28760 Tres Cantos, Madrid (ES); MARTÍNEZ, Tamara, E-28760 Tres Cantos, Madrid (ES); RAMOS, Facundo Nehuén, E-28760 Tres Cantos, Madrid (ES)
(74) Representative: Williams, Gareth Owen

(57) **Abstract**

The invention relates to siRNA molecules and compositions which modulate the expression of the Eukaryotic Translation Elongation Factor 1 Alpha 2 (EEF1A2) gene, which can be useful in reducing viral replication and managing viral infections or infestations. Such molecules and compositions can be useful in treating or preventing infection with RNA viruses in humans (for example, coronaviruses such as SARS-CoV-2 or influenza viruses such as influenza virus type A) or in agriculturally important plants.

SEQUENCE LISTING

<110> Sylentis S.A.U.

<120> siRNA and compositions for prophylactic and therapeutic treatment of
virus diseases

<130> EEF1A2 PRI EP

<160> 1201

<170> BiSSAP 1.3.6

<210> 1
<211> 19
<212> DNA
<213> Homo sapiens

<220>
<223> cDNA

<400> 1
ccaccaagta ctacatcac                                                    19

<210> 2
<211> 19
<212> DNA
<213> Homo sapiens

<220>
<223> cDNA

<400> 2
agaccaccaa gtactacat                                                    19

<210> 3
<211> 19
<212> DNA
<213> Homo sapiens

<220>
<223> cDNA

<400> 3
gcgcctacat caagaagat                                                    19

<210> 4
<211> 19
<212> DNA
<213> Homo sapiens

<220>
<223> cDNA

<400> 4
accaccaagt actacatca                                                    19

<210> 5
<211> 19
<212> DNA
<213> Homo sapiens

<220>
<223> cDNA

<400> 5
cgcctacatc aagaagatc                                                    19

<210> 6
<211> 19
<212> DNA
<213> Homo sapiens

<220>
<223> cDNA

<400> 6
gcatcaccat cgacatctc                                                    19

<210> 7
<211> 19
<212> DNA
<213> Homo sapiens

<220>
<223> cDNA

<400> 7
catcccaggc ggactgcgc                                                    19

<210> 8
<211> 19
<212> DNA
<213> Homo sapiens

<220>
<223> cDNA

<400> 8
gaccaccaag tactacatc                                                    19

<210> 9
<211> 19
<212> DNA
<213> Homo sapiens

<220>
<223> cDNA

<400> 9
tcagcgccta catcaagaa                                                    19

<210> 10
<211> 19
<212> DNA
<213> Homo sapiens

<220>
<223> cDNA

<400> 10
cagcgcctac atcaagaag                                                    19

<210> 11
<211> 19
<212> DNA
<213> Homo sapiens

<220>
<223> cDNA

<400> 11
agcgcctaca tcaagaaga                                                    19

<210> 12
<211> 19
<212> DNA
<213> Homo sapiens

<220>
<223> cDNA

<400> 12
caagaagatc ggctacaac                                                    19

<210> 13
<211> 19
<212> DNA
<213> Homo sapiens

<220>
<223> cDNA

<400> 13
aagaagatcg gctacaacc                                                    19

<210> 14
<211> 19
<212> DNA
<213> Homo sapiens

<220>

<223> cDNA

<400> 14
gcctacatca agaagatcg                                                    19


<210> 15
<211> 19
<212> DNA
<213> Homo sapiens


<220>
<223> cDNA

<400> 15
agaagatcgg ctacaaccc                                                    19


<210> 16
<211> 19
<212> DNA
<213> Homo sapiens


<220>
<223> cDNA

<400> 16
tcaagaagat cggctacaa                                                    19


<210> 17
<211> 19
<212> DNA
<213> Homo sapiens


<220>
<223> cDNA

<400> 17
atcaagaaga tcggctaca                                                    19


<210> 18
<211> 19
<212> DNA
<213> Homo sapiens


<220>
<223> cDNA

<400> 18
catcaagaag atcggctac                                                    19


<210> 19
<211> 19
<212> DNA
<213> Homo sapiens

<220>
<223> cDNA

<400> 19
caccaagtac tacatcacc                                                    19


<210> 20
<211> 19
<212> DNA
<213> Homo sapiens


<220>
<223> cDNA

<400> 20
accaagtact acatcacca                                                    19


<210> 21
<211> 19
<212> DNA
<213> Homo sapiens


<220>
<223> cDNA

<400> 21
ccaagtacta catcaccat                                                    19


<210> 22
<211> 19
<212> DNA
<213> Homo sapiens


<220>
<223> cDNA

<400> 22
caagtactac atcaccatc                                                    19


<210> 23
<211> 19
<212> DNA
<213> Homo sapiens


<220>
<223> cDNA

<400> 23
aagtactaca tcaccatca                                                    19


<210> 24
<211> 19
<212> DNA

<213> Homo sapiens

<220>
<223> cDNA

<400> 24
agtactacat caccatcat                                                    19

<210> 25
<211> 19
<212> DNA
<213> Homo sapiens

<220>
<223> cDNA

<400> 25
gtactacatc accatcatc                                                    19

<210> 26
<211> 19
<212> DNA
<213> Homo sapiens

<220>
<223> cDNA

<400> 26
tactacatca ccatcatcg                                                    19

<210> 27
<211> 19
<212> DNA
<213> Homo sapiens

<220>
<223> cDNA

<400> 27
acatcaagaa gatcggcta                                                    19

<210> 28
<211> 19
<212> DNA
<213> Homo sapiens

<220>
<223> cDNA

<400> 28
cctacatcaa gaagatcgg                                                    19

<210> 29

<211> 19
<212> DNA
<213> Artificial Sequence

<220>
<223> cDNA

<400> 29
ctacatcaag aagatcggc                                                    19

<210> 30
<211> 19
<212> DNA
<213> Homo sapiens

<220>
<223> cDNA

<400> 30
tacatcaaga agatcggct                                                    19

<210> 31
<211> 19
<212> DNA
<213> Homo sapiens

<220>
<223> cDNA

<400> 31
gaagatcggc tacaacccg                                                    19

<210> 32
<211> 19
<212> DNA
<213> Homo sapiens

<220>
<223> cDNA

<400> 32
catcaccatc gacatctcc                                                    19

<210> 33
<211> 19
<212> DNA
<213> Homo sapiens

<220>
<223> cDNA

<400> 33
atcaccatcg acatctccc                                                    19

<210> 34
<211> 19
<212> DNA
<213> Homo sapiens

<220>
<223> cDNA

<400> 34
tcaccatcga catctccct                                                    19

<210> 35
<211> 19
<212> DNA
<213> Homo sapiens

<220>
<223> cDNA

<400> 35
caccatcgac atctccctc                                                    19

<210> 36
<211> 19
<212> DNA
<213> Homo sapiens

<220>
<223> cDNA

<400> 36
accatcgaca tctccctct                                                    19

<210> 37
<211> 19
<212> DNA
<213> Homo sapiens

<220>
<223> cDNA

<400> 37
ccatcgacat ctccctctg                                                    19

<210> 38
<211> 19
<212> DNA
<213> Homo sapiens

<220>
<223> cDNA

<400> 38

catcgacatc tccctctgg                                                    19

<210> 39
<211> 19
<212> DNA
<213> Homo sapiens

<220>
<223> cDNA

<400> 39
atcgacatct ccctctgga                                                    19

<210> 40
<211> 19
<212> DNA
<213> Homo sapiens

<220>
<223> cDNA

<400> 40
tcgacatctc cctctggaa                                                    19

<210> 41
<211> 19
<212> DNA
<213> Homo sapiens

<220>
<223> cDNA

<400> 41
cgacatctcc ctctggaag                                                    19

<210> 42
<211> 19
<212> DNA
<213> Homo sapiens

<220>
<223> cDNA

<400> 42
gacatctccc tctggaagt                                                    19

<210> 43
<211> 19
<212> DNA
<213> Homo sapiens

<220>
<223> cDNA

<400> 43
acatctccct ctggaagtt                                                    19


<210> 44
<211> 19
<212> DNA
<213> Homo sapiens


<220>
<223> cDNA

<400> 44
catctccctc tggaagttc                                                    19


<210> 45
<211> 19
<212> DNA
<213> Artificial Sequence


<220>
<223> cDNA

<400> 45
atcccaggcg gactgcgcg                                                    19


<210> 46
<211> 19
<212> DNA
<213> Homo sapiens


<220>
<223> cDNA

<400> 46
atgcctgggt gctggacaa                                                    19


<210> 47
<211> 19
<212> DNA
<213> Artificial Sequence


<220>
<223> cDNA

<400> 47
tgcctgggtg ctggacaaa                                                    19


<210> 48
<211> 19
<212> DNA
<213> Homo sapiens

<220>
<223> cDNA

<400> 48
aggcgggcat ctccaagaa                                                    19


<210> 49
<211> 19
<212> DNA
<213> Artificial Sequence


<220>
<223> cDNA

<400> 49
ggcgggcatc tccaagaac                                                    19


<210> 50
<211> 19
<212> DNA
<213> Homo sapiens


<220>
<223> cDNA

<400> 50
cctacagcga gaagcgcta                                                    19


<210> 51
<211> 19
<212> DNA
<213> Homo sapiens


<220>
<223> cDNA

<400> 51
ctacagcgag aagcgctac                                                    19


<210> 52
<211> 19
<212> RNA
<213> Artificial Sequence


<220>
<223> EEF1A2 siRNA antisense strand

<400> 52
gugauguagu acuuggugg                                                    19


<210> 53
<211> 19
<212> RNA
<213> Artificial Sequence

<220>
<223> EEF1A2 siRNA antisense strand

<400> 53
auguaguacu uggugguc u                                                    19

<210> 54
<211> 19
<212> RNA
<213> Artificial Sequence

<220>
<223> EEF1A2 siRNA antisense strand

<400> 54
aucuucuuga uguaggcgc                                                     19

<210> 55
<211> 19
<212> RNA
<213> Artificial Sequence

<220>
<223> EEF1A2 siRNA antisense strand

<400> 55
ugauguagua cuugguggu                                                     19

<210> 56
<211> 19
<212> RNA
<213> Artificial Sequence

<220>
<223> EEF1A2 siRNA antisense strand

<400> 56
gaucuucuug auguaggcg                                                     19

<210> 57
<211> 19
<212> RNA
<213> Artificial Sequence

<220>
<223> EEF1A2 siRNA antisense strand

<400> 57
gagaugucga uggugaugc                                                     19

<210> 58
<211> 19

<212> RNA
<213> Artificial Sequence

<220>
<223> EEF1A2 siRNA antisense strand

<400> 58
gcgcaguccg ccugggaug                                                    19

<210> 59
<211> 19
<212> RNA
<213> Artificial Sequence

<220>
<223> EEF1A2 siRNA antisense strand

<400> 59
gauguaguac uuggugguc                                                    19

<210> 60
<211> 19
<212> RNA
<213> Artificial Sequence

<220>
<223> EEF1A2 siRNA antisense strand

<400> 60
uucuugaugu aggcgcuga                                                    19

<210> 61
<211> 19
<212> RNA
<213> Artificial Sequence

<220>
<223> EEF1A2 siRNA antisense strand

<400> 61
cuucuugaug uaggcgcug                                                    19

<210> 62
<211> 19
<212> RNA
<213> Artificial Sequence

<220>
<223> EEF1A2 siRNA antisense strand

<400> 62
ucuucuugau guaggcgcu                                                    19

<210> 63
<211> 19
<212> RNA
<213> Artificial Sequence

<220>
<223> EEF1A2 siRNA antisense strand

<400> 63
guuguagccg aucuucuug                                                    19

<210> 64
<211> 19
<212> RNA
<213> Artificial Sequence

<220>
<223> EEF1A2 siRNA antisense strand

<400> 64
gguuguagcc gaucuucuu                                                    19

<210> 65
<211> 19
<212> RNA
<213> Artificial Sequence

<220>
<223> EEF1A2 siRNA antisense strand

<400> 65
cgaucuucuu gauguaggc                                                    19

<210> 66
<211> 19
<212> RNA
<213> Artificial Sequence

<220>
<223> EEF1A2 siRNA antisense strand

<400> 66
ggguuguagc cgaucuucu                                                    19

<210> 67
<211> 19
<212> RNA
<213> Artificial Sequence

<220>
<223> EEF1A2 siRNA antisense strand

<400> 67
uuguagccga ucuucuuga                                                    19

<210> 68
<211> 19
<212> RNA
<213> Artificial Sequence

<220>
<223> EEF1A2 siRNA antisense strand

<400> 68
uguagccgau cuucuugau                                                    19

<210> 69
<211> 19
<212> RNA
<213> Artificial Sequence

<220>
<223> EEF1A2 siRNA antisense strand

<400> 69
guagccgauc uucuugaug                                                    19

<210> 70
<211> 19
<212> RNA
<213> Artificial Sequence

<220>
<223> EEF1A2 siRNA antisense strand

<400> 70
ggugauguag uacuuggug                                                    19

<210> 71
<211> 19
<212> RNA
<213> Artificial Sequence

<220>
<223> EEF1A2 siRNA antisense strand

<400> 71
uggugaugua guacuuggu                                                    19

<210> 72
<211> 19
<212> RNA
<213> Artificial Sequence

<220>
<223> EEF1A2 siRNA antisense strand

<400> 72
auggugaugu aguacuugg                                                    19

<210> 73
<211> 19
<212> RNA
<213> Artificial Sequence

<220>
<223> EEF1A2 siRNA antisense strand

<400> 73
gauggugaug uaguacuug                                                    19

<210> 74
<211> 19
<212> RNA
<213> Artificial Sequence

<220>
<223> EEF1A2 siRNA antisense strand

<400> 74
ugauggugau guaguacuu                                                    19

<210> 75
<211> 19
<212> RNA
<213> Artificial Sequence

<220>
<223> EEF1A2 siRNA antisense strand

<400> 75
augaugguga uguaguacu                                                    19

<210> 76
<211> 19
<212> RNA
<213> Artificial Sequence

<220>
<223> EEF1A2 siRNA antisense strand

<400> 76
gaugauggug auguaguac                                                    19

<210> 77
<211> 19
<212> RNA
<213> Artificial Sequence

<220>

<223> EEF1A2 siRNA antisense strand

<400> 77
cgaugauggu gauguagua                                                    19


<210> 78
<211> 19
<212> RNA
<213> Artificial Sequence


<220>
<223> EEF1A2 siRNA antisense strand

<400> 78
uagccgaucu ucuugaugu                                                    19


<210> 79
<211> 19
<212> RNA
<213> Artificial Sequence


<220>
<223> EEF1A2 siRNA antisense strand

<400> 79
ccgaucuucu ugauguagg                                                    19


<210> 80
<211> 19
<212> RNA
<213> Artificial Sequence


<220>
<223> EEF1A2 siRNA antisense strand

<400> 80
gccgaucuuc uugauguag                                                    19


<210> 81
<211> 19
<212> RNA
<213> Artificial Sequence


<220>
<223> EEF1A2 siRNA antisense strand

<400> 81
agccgaucuu cuugaugua                                                    19


<210> 82
<211> 19
<212> RNA
<213> Artificial Sequence

<220>
<223> EEF1A2 siRNA antisense strand

<400> 82
cggguuguag ccgaucuuc                                                    19


<210> 83
<211> 19
<212> RNA
<213> Artificial Sequence


<220>
<223> EEF1A2 siRNA antisense strand

<400> 83
ggagaugucg auggugaug                                                    19


<210> 84
<211> 19
<212> RNA
<213> Artificial Sequence


<220>
<223> EEF1A2 siRNA antisense strand

<400> 84
gggagauguc gauggugau                                                    19


<210> 85
<211> 19
<212> RNA
<213> Artificial Sequence


<220>
<223> EEF1A2 siRNA antisense strand

<400> 85
agggagaugu cgaugguga                                                    19


<210> 86
<211> 19
<212> RNA
<213> Artificial Sequence


<220>
<223> EEF1A2 siRNA antisense strand

<400> 86
gagggagaug ucgauggug                                                    19


<210> 87
<211> 19
<212> RNA

<213> Artificial Sequence

<220>
<223> EEF1A2 siRNA antisense strand

<400> 87
agagggagau gucgauggu                                                    19

<210> 88
<211> 19
<212> RNA
<213> Artificial Sequence

<220>
<223> EEF1A2 siRNA antisense strand

<400> 88
cagagggaga ugucgaugg                                                    19

<210> 89
<211> 19
<212> RNA
<213> Artificial Sequence

<220>
<223> EEF1A2 siRNA antisense strand

<400> 89
ccagagggag augucgaug                                                    19

<210> 90
<211> 19
<212> RNA
<213> Artificial Sequence

<220>
<223> EEF1A2 siRNA antisense strand

<400> 90
uccagaggga gaugucgau                                                    19

<210> 91
<211> 19
<212> RNA
<213> Artificial Sequence

<220>
<223> EEF1A2 siRNA antisense strand

<400> 91
uuccagaggg agaugucga                                                    19

<210> 92

<211> 19
<212> RNA
<213> Artificial Sequence

<220>
<223> EEF1A2 siRNA antisense strand

<400> 92
cuuccagagg gagaugucg                                                    19

<210> 93
<211> 19
<212> RNA
<213> Artificial Sequence

<220>
<223> EEF1A2 siRNA antisense strand

<400> 93
acuuccagag ggagauguc                                                    19

<210> 94
<211> 19
<212> RNA
<213> Artificial Sequence

<220>
<223> EEF1A2 siRNA antisense strand

<400> 94
aacuuccaga gggagaugu                                                    19

<210> 95
<211> 19
<212> RNA
<213> Artificial Sequence

<220>
<223> EEF1A2 siRNA antisense strand

<400> 95
gaacuuccag agggagaug                                                    19

<210> 96
<211> 19
<212> RNA
<213> Artificial Sequence

<220>
<223> EEF1A2 siRNA antisense strand

<400> 96
cgcgcagucc gccugggau                                                    19

<210> 97
<211> 19
<212> RNA
<213> Artificial Sequence

<220>
<223> EEF1A2 siRNA antisense strand

<400> 97
uuguccagca cccaggcau                                                    19

<210> 98
<211> 19
<212> RNA
<213> Artificial Sequence

<220>
<223> EEF1A2 siRNA antisense strand

<400> 98
uuuguccagc acccaggca                                                    19

<210> 99
<211> 19
<212> RNA
<213> Artificial Sequence

<220>
<223> EEF1A2 siRNA antisense strand

<400> 99
uucuuggaga ugcccgccu                                                    19

<210> 100
<211> 19
<212> RNA
<213> Artificial Sequence

<220>
<223> EEF1A2 siRNA antisense strand

<400> 100
guucuuggag augcccgcc                                                    19

<210> 101
<211> 19
<212> RNA
<213> Artificial Sequence

<220>
<223> EEF1A2 siRNA antisense strand

<400> 101

uagcgcuucu cgcuguagg                                                    19

<210> 102
<211> 19
<212> RNA
<213> Artificial Sequence

<220>
<223> EEF1A2 siRNA antisense strand

<400> 102
guagcgcuuc ucgcuguag                                                    19

<210> 103
<211> 19
<212> RNA
<213> Artificial Sequence

<220>
<223> EEF1A2 siRNA sense strand

<400> 103
ccaccaagua cuacaucac                                                    19

<210> 104
<211> 19
<212> RNA
<213> Artificial Sequence

<220>
<223> EEF1A2 siRNA sense strand

<400> 104
agaccaccaa guacuacau                                                    19

<210> 105
<211> 19
<212> RNA
<213> Artificial Sequence

<220>
<223> EEF1A2 siRNA sense strand

<400> 105
gcgccuacau caagaagau                                                    19

<210> 106
<211> 19
<212> RNA
<213> Artificial Sequence

<220>
<223> EEF1A2 siRNA sense strand

<400> 106
accaccaagu acuacauca                                                    19

<210> 107
<211> 19
<212> RNA
<213> Artificial Sequence

<220>
<223> EEF1A2 siRNA sense strand

<400> 107
cgccuacauc aagaagauc                                                    19

<210> 108
<211> 19
<212> RNA
<213> Artificial Sequence

<220>
<223> EEF1A2 siRNA sense strand

<400> 108
gcaucaccau cgacaucuc                                                    19

<210> 109
<211> 19
<212> RNA
<213> Artificial Sequence

<220>
<223> EEF1A2 siRNA sense strand

<400> 109
caucccaggc ggacugcgc                                                    19

<210> 110
<211> 19
<212> RNA
<213> Artificial Sequence

<220>
<223> EEF1A2 siRNA sense strand

<400> 110
gaccaccaag uacuacauc                                                    19

<210> 111
<211> 19
<212> RNA
<213> Artificial Sequence

<220>
<223> EEF1A2 siRNA sense strand

<400> 111
ucagcgccua caucaagaa                                                    19


<210> 112
<211> 19
<212> RNA
<213> Artificial Sequence


<220>
<223> EEF1A2 siRNA sense strand

<400> 112
cagcgccuac aucaagaag                                                    19


<210> 113
<211> 19
<212> RNA
<213> Artificial Sequence


<220>
<223> EEF1A2 siRNA sense strand

<400> 113
agcgccuaca ucaagaaga                                                    19


<210> 114
<211> 19
<212> RNA
<213> Artificial Sequence


<220>
<223> EEF1A2 siRNA sense strand

<400> 114
caagaagauc ggcuacaac                                                    19


<210> 115
<211> 19
<212> RNA
<213> Artificial Sequence


<220>
<223> EEF1A2 siRNA sense strand

<400> 115
aagaagaucg gcuacaacc                                                    19


<210> 116
<211> 19
<212> RNA
<213> Artificial Sequence

<220>
<223> EEF1A2 siRNA sense strand

<400> 116
gccuacauca agaagaucg                                                    19


<210> 117
<211> 19
<212> RNA
<213> Artificial Sequence


<220>
<223> EEF1A2 siRNA sense strand

<400> 117
agaagaucgg cuacaaccc                                                    19


<210> 118
<211> 19
<212> RNA
<213> Artificial Sequence


<220>
<223> EEF1A2 siRNA sense strand

<400> 118
ucaagaagau cggcuacaa                                                    19


<210> 119
<211> 19
<212> RNA
<213> Artificial Sequence


<220>
<223> EEF1A2 siRNA sense strand

<400> 119
aucaagaaga ucggcuaca                                                    19


<210> 120
<211> 19
<212> RNA
<213> Artificial Sequence


<220>
<223> EEF1A2 siRNA sense strand

<400> 120
caucaagaag aucggcuac                                                    19


<210> 121
<211> 19

<212> RNA
<213> Artificial Sequence

<220>
<223> EEF1A2 siRNA sense strand

<400> 121
caccaaguac uacaucacc                                                    19

<210> 122
<211> 19
<212> RNA
<213> Artificial Sequence

<220>
<223> EEF1A2 siRNA sense strand

<400> 122
accaaguacu acaucacca                                                    19

<210> 123
<211> 19
<212> RNA
<213> Artificial Sequence

<220>
<223> EEF1A2 siRNA sense strand

<400> 123
ccaaguacua caucaccau                                                    19

<210> 124
<211> 19
<212> RNA
<213> Artificial Sequence

<220>
<223> EEF1A2 siRNA sense strand

<400> 124
caaguacuac aucaccauc                                                    19

<210> 125
<211> 19
<212> RNA
<213> Artificial Sequence

<220>
<223> EEF1A2 siRNA sense strand

<400> 125
aaguacuaca ucaccauca                                                    19

<210> 126
<211> 19
<212> RNA
<213> Artificial Sequence

<220>
<223> EEF1A2 siRNA sense strand

<400> 126
aguacuacau caccaucau                                                    19

<210> 127
<211> 19
<212> RNA
<213> Artificial Sequence

<220>
<223> EEF1A2 siRNA sense strand

<400> 127
guacuacauc accaucauc                                                    19

<210> 128
<211> 19
<212> RNA
<213> Artificial Sequence

<220>
<223> EEF1A2 siRNA sense strand

<400> 128
uacuacauca ccaucaucg                                                    19

<210> 129
<211> 19
<212> RNA
<213> Artificial Sequence

<220>
<223> EEF1A2 siRNA sense strand

<400> 129
acaucaagaa gaucggcua                                                    19

<210> 130
<211> 19
<212> RNA
<213> Artificial Sequence

<220>
<223> EEF1A2 siRNA sense strand

<400> 130
ccuacaucaa gaagaucgg                                                    19

<210> 131
<211> 19
<212> RNA
<213> Artificial Sequence

<220>
<223> EEF1A2 siRNA sense strand

<400> 131
cuacaucaag aagaucggc                                                    19

<210> 132
<211> 19
<212> RNA
<213> Artificial Sequence

<220>
<223> EEF1A2 siRNA sense strand

<400> 132
uacaucaaga agaucggcu                                                    19

<210> 133
<211> 19
<212> RNA
<213> Artificial Sequence

<220>
<223> EEF1A2 siRNA sense strand

<400> 133
gaagaucggc uacaacccg                                                    19

<210> 134
<211> 19
<212> RNA
<213> Artificial Sequence

<220>
<223> EEF1A2 siRNA sense strand

<400> 134
caucaccauc gacaucucc                                                    19

<210> 135
<211> 19
<212> RNA
<213> Artificial Sequence

<220>
<223> EEF1A2 siRNA sense strand

<400> 135
aucaccaucg acaucuccc                                                    19

<210> 136
<211> 19
<212> RNA
<213> Artificial Sequence

<220>
<223> EEF1A2 siRNA sense strand

<400> 136
ucaccaucga caucucccu                                                    19

<210> 137
<211> 19
<212> RNA
<213> Artificial Sequence

<220>
<223> EEF1A2 siRNA sense strand

<400> 137
caccaucgac aucucccuc                                                    19

<210> 138
<211> 19
<212> RNA
<213> Artificial Sequence

<220>
<223> EEF1A2 siRNA sense strand

<400> 138
accaucgaca ucucccucu                                                    19

<210> 139
<211> 19
<212> RNA
<213> Artificial Sequence

<220>
<223> EEF1A2 siRNA sense strand

<400> 139
ccaucgacau cucccucug                                                    19

<210> 140
<211> 19
<212> RNA
<213> Artificial Sequence

<220>

<223> EEF1A2 siRNA sense strand

<400> 140
caucgacauc ucccucugg                                                    19


<210> 141
<211> 19
<212> RNA
<213> Artificial Sequence


<220>
<223> EEF1A2 siRNA sense strand

<400> 141
aucgacaucu cccucugga                                                    19


<210> 142
<211> 19
<212> RNA
<213> Artificial Sequence


<220>
<223> EEF1A2 siRNA sense strand

<400> 142
ucgacaucuc ccucuggaa                                                    19


<210> 143
<211> 19
<212> RNA
<213> Artificial Sequence


<220>
<223> EEF1A2 siRNA sense strand

<400> 143
cgacaucucc cucuggaag                                                    19


<210> 144
<211> 19
<212> RNA
<213> Artificial Sequence


<220>
<223> EEF1A2 siRNA sense strand

<400> 144
gacaucuccc ucuggaagu                                                    19


<210> 145
<211> 19
<212> RNA
<213> Artificial Sequence

<220>
<223> EEF1A2 siRNA sense strand

<400> 145
acaucucccu cuggaaguu                                                    19


<210> 146
<211> 19
<212> RNA
<213> Artificial Sequence


<220>
<223> EEF1A2 siRNA sense strand

<400> 146
caucucccuc uggaaguuc                                                    19


<210> 147
<211> 19
<212> RNA
<213> Artificial Sequence


<220>
<223> EEF1A2 siRNA sense strand

<400> 147
aucccaggcg gacugcgcg                                                    19


<210> 148
<211> 19
<212> RNA
<213> Artificial Sequence


<220>
<223> EEF1A2 siRNA sense strand

<400> 148
augccugggu gcuggacaa                                                    19


<210> 149
<211> 19
<212> RNA
<213> Artificial Sequence


<220>
<223> EEF1A2 siRNA sense strand

<400> 149
ugccugggug cuggacaaa                                                    19


<210> 150
<211> 19
<212> RNA

<213> Artificial Sequence

<220>
<223> EEF1A2 siRNA sense strand

<400> 150
aggcgggcau cuccaagaa                                                    19

<210> 151
<211> 19
<212> RNA
<213> Artificial Sequence

<220>
<223> EEF1A2 siRNA sense strand

<400> 151
ggcgggcauc uccaagaac                                                    19

<210> 152
<211> 19
<212> RNA
<213> Artificial Sequence

<220>
<223> EEF1A2 siRNA sense strand

<400> 152
ccuacagcga gaagcgcua                                                    19

<210> 153
<211> 19
<212> RNA
<213> Artificial Sequence

<220>
<223> EEF1A2 siRNA sense strand

<400> 153
cuacagcgag aagcgcuac                                                    19

<210> 154
<211> 21
<212> RNA
<213> Artificial Sequence

<220>
<223> EEF1A2 siRNA antisense strand

<400> 154
gugauguagu acuugguggu u                                                 21

<210> 155

<211> 21
<212> RNA
<213> Artificial Sequence

<220>
<223> EEF1A2 siRNA antisense strand

<400> 155
   gugauguagu acuugguggu u                                                21

<210> 156
<211> 21
<212> RNA
<213> Artificial Sequence

<220>
<223> EEF1A2 siRNA antisense strand

<400> 156
   gugauguagu acuugguggu u                                                21

<210> 157
<211> 21
<212> RNA
<213> Artificial Sequence

<220>
<223> EEF1A2 siRNA antisense strand

<400> 157
   gugauguagu acuugguggu u                                                21

<210> 158
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 158
   gugauguagu acuugguggn n                                                21

<210> 159
<211> 21

<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 159
gugauguagu acuugguggn n                                                21

<210> 160
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 160
gugauguagu acuugguggn n                                                21

<210> 161
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 161
gugauguagu acuugguggn n                                                21

<210> 162
<211> 21
<212> RNA
<213> Artificial Sequence

<220>
<223> EEF1A2 siRNA antisense strand

<400> 162
aucuucuuga uguaggcgcu u                                                21

<210> 163
<211> 21
<212> RNA
<213> Artificial Sequence

<220>
<223> EEF1A2 siRNA antisense strand

<400> 163
aucuucuuga uguaggcgcu u                                                21

<210> 164
<211> 21
<212> RNA
<213> Artificial Sequence

<220>
<223> EEF1A2 siRNA antisense strand

<400> 164
aucuucuuga uguaggcgcu u                                                21

<210> 165
<211> 21
<212> RNA
<213> Artificial Sequence

<220>
<223> EEF1A2 siRNA antisense strand

<400> 165
aucuucuuga uguaggcgcu u                                                21

<210> 166
<211> 21
<212> RNA
<213> Artificial Sequence

<220>

<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 166
aucuucuuga uguaggcgcn n                                                21


<210> 167
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 167
aucuucuuga uguaggcgcn n                                                21


<210> 168
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 168
aucuucuuga uguaggcgcn n                                                21


<210> 169

<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 169
aucuucuuga uguaggcgcn n                                                21

<210> 170
<211> 21
<212> RNA
<213> Artificial Sequence

<220>
<223> EEF1A2 siRNA antisense strand

<400> 170
ugauguagua cuuggugguu u                                                21

<210> 171
<211> 21
<212> RNA
<213> Artificial Sequence

<220>
<223> EEF1A2 siRNA antisense strand

<400> 171
ugauguagua cuuggugguu u                                                21

<210> 172
<211> 21
<212> RNA
<213> Artificial Sequence

<220>
<223> EEF1A2 siRNA antisense strand

<400> 172
ugauguagua cuuggugguu u                                                21

<210> 173
<211> 21

<212> RNA
<213> Artificial Sequence

<220>
<223> EEF1A2 siRNA antisense strand

<400> 173
ugauguagua cuuggugguu u                                                21

<210> 174
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 174
   ugauguagua cuugguggun n                                                21

<210> 175
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 175
   ugauguagua cuugguggun n                                                21

<210> 176
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 176
ugauguagua cuugguggun n                                                21


<210> 177
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 177
ugauguagua cuugguggun n                                                21


<210> 178
<211> 21
<212> RNA
<213> Artificial Sequence


<220>
<223> EEF1A2 siRNA antisense strand

<400> 178
gaucuucuug auguaggcgu u                                                21


<210> 179
<211> 21
<212> RNA
<213> Artificial Sequence


<220>
<223> EEF1A2 siRNA antisense strand

<400> 179

gaucuucuug auguaggcgu u                                                21

<210> 180
<211> 21
<212> RNA
<213> Artificial Sequence

<220>
<223> EEF1A2 siRNA antisense strand

<400> 180
gaucuucuug auguaggcgu u                                                21

<210> 181
<211> 21
<212> RNA
<213> Artificial Sequence

<220>
<223> EEF1A2 siRNA antisense strand

<400> 181
gaucuucuug auguaggcgu u                                                21

<210> 182
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 182
gaucuucuug auguaggcgn n                                                21

<210> 183
<211> 21
<212> RNA
<213> Artificial Sequence

<220>
<223> EEF1A2 siRNA antisense strand

<220>
<221> misc_feature

<222> 20
<223> /note="n is dT (deoxythymine or 2'H thymine)"

<220>
<221> misc_feature
<222> 21
<223> /note="n is dT (deoxythymine or 2'H thymine)"

<400> 183
gaucuucuug auguaggcgn n                                                21


<210> 184
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 184
gaucuucuug auguaggcgn n                                                21


<210> 185
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 185
gaucuucuug auguaggcgn n                                                21


<210> 186
<211> 21
<212> RNA
<213> Artificial Sequence

<220>
<223> EEF1A2 siRNA antisense strand

<400> 186
ucuucuugau guaggcgcuu u                                                21


<210> 187
<211> 21
<212> RNA
<213> Artificial Sequence


<220>
<223> EEF1A2 siRNA antisense strand

<400> 187
ucuucuugau guaggcgcuu u                                                21


<210> 188
<211> 21
<212> RNA
<213> Artificial Sequence


<220>
<223> EEF1A2 siRNA antisense strand

<400> 188
ucuucuugau guaggcgcuu u                                                21


<210> 189
<211> 21
<212> RNA
<213> Artificial Sequence


<220>
<223> EEF1A2 siRNA antisense strand

<400> 189
ucuucuugau guaggcgcuu u                                                21


<210> 190
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature

<222> 21
<223> /note="n is dT (deoxythymine or 2'H thymine)"

<400> 190
ucuucuugau guaggcgcun n                                                21


<210> 191
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 191
ucuucuugau guaggcgcun n                                                21


<210> 192
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 192
ucuucuugau guaggcgcun n                                                21


<210> 193
<211> 21
<212> RNA
<213> Artificial Sequence


<220>
<223> EEF1A2 siRNA antisense strand

<220>

<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 193
ucuucuugau guaggcgcun n                                                21

<210> 194
<211> 21
<212> RNA
<213> Artificial Sequence

<220>
<223> EEF1A2 siRNA antisense strand

<400> 194
guuguagccg aucuucuugu u                                                21

<210> 195
<211> 21
<212> RNA
<213> Artificial Sequence

<220>
<223> EEF1A2 siRNA antisense strand

<400> 195
guuguagccg aucuucuugu u                                                21

<210> 196
<211> 21
<212> RNA
<213> Artificial Sequence

<220>
<223> EEF1A2 siRNA antisense strand

<400> 196
guuguagccg aucuucuugu u                                                21

<210> 197
<211> 21
<212> RNA
<213> Artificial Sequence

<220>
<223> EEF1A2 siRNA antisense strand

<400> 197
guuguagccg aucuucuugu u                                                21

<210> 198
<211> 21
<212> RNA
<213> Artificial Sequence

<220>
<223> EEF1A2 siRNA antisense strand

<220>
<221> misc_feature
<222> 20
<223> /note="n is dT (deoxythymine or 2′H thymine)"

<220>
<221> misc_feature
<222> 21
<223> /note="n is dT (deoxythymine or 2′H thymine)"

<400> 198
guuguagccg aucuucuugn n                                                21

<210> 199
<211> 21
<212> RNA
<213> Artificial Sequence

<220>
<223> EEF1A2 siRNA antisense strand

<220>
<221> misc_feature
<222> 20
<223> /note="n is dT (deoxythymine or 2′H thymine)"

<220>
<221> misc_feature
<222> 21
<223> /note="n is dT (deoxythymine or 2′H thymine)"

<400> 199
guuguagccg aucuucuugn n                                                21

<210> 200
<211> 21
<212> RNA
<213> Artificial Sequence

<220>
<223> EEF1A2 siRNA antisense strand

<220>
<221> misc_feature
<222> 20
<223> /note="n is dT (deoxythymine or 2′H thymine)"

<220>

<221> misc_feature
<222> 21
<223> /note="n is dT (deoxythymine or 2'H thymine)"

<400> 200
guuguagccg aucuucuugn n                                                21


<210> 201
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 201
guuguagccg aucuucuugn n                                                21


<210> 202
<211> 21
<212> RNA
<213> Artificial Sequence


<220>
<223> EEF1A2 siRNA antisense strand

<400> 202
gguuguagcc gaucuucuuu u                                                21


<210> 203
<211> 21
<212> RNA
<213> Artificial Sequence


<220>
<223> EEF1A2 siRNA antisense strand

<400> 203
gguuguagcc gaucuucuuu u                                                21


<210> 204
<211> 21
<212> RNA
<213> Artificial Sequence

<220>
<223> EEF1A2 siRNA antisense strand

<400> 204
gguuguagcc gaucuucuuu u                                                21


<210> 205
<211> 21
<212> RNA
<213> Artificial Sequence


<220>
<223> EEF1A2 siRNA antisense strand

<400> 205
gguuguagcc gaucuucuuu u                                                21


<210> 206
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 206
gguuguagcc gaucuucuun n                                                21


<210> 207
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 207

gguuguagcc gaucuucuun n                                                21

<210> 208
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 208
gguuguagcc gaucuucuun n                                                21

<210> 209
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 209
gguuguagcc gaucuucuun n                                                21

<210> 210
<211> 21
<212> RNA
<213> Artificial Sequence

<220>
<223> EEF1A2 siRNA antisense strand

<400> 210
cgaucuucuu gauguaggcu u                                                21

<210> 211

<211> 21
<212> RNA
<213> Artificial Sequence

<220>
<223> EEF1A2 siRNA antisense strand

<400> 211
cgaucuucuu gauguaggcu u                                                21

<210> 212
<211> 21
<212> RNA
<213> Artificial Sequence

<220>
<223> EEF1A2 siRNA antisense strand

<400> 212
cgaucuucuu gauguaggcu u                                                21

<210> 213
<211> 21
<212> RNA
<213> Artificial Sequence

<220>
<223> EEF1A2 siRNA antisense strand

<400> 213
cgaucuucuu gauguaggcu u                                                21

<210> 214
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 214
cgaucuucuu gauguaggcn n                                                21

<210> 215
<211> 21

<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 215
cgaucuucuu gauguaggcn n                                                21

<210> 216
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 216
cgaucuucuu gauguaggcn n                                                21

<210> 217
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 217
cgaucuucuu gauguaggcn n                                                21

<210> 218
<211> 21
<212> RNA
<213> Artificial Sequence

<220>
<223> EEF1A2 siRNA antisense strand

<400> 218
ggguuguagc cgaucuucuu u                                                21

<210> 219
<211> 21
<212> RNA
<213> Artificial Sequence

<220>
<223> EEF1A2 siRNA antisense strand

<400> 219
ggguuguagc cgaucuucuu u                                                21

<210> 220
<211> 21
<212> RNA
<213> Artificial Sequence

<220>
<223> EEF1A2 siRNA antisense strand

<400> 220
ggguuguagc cgaucuucuu u                                                21

<210> 221
<211> 21
<212> RNA
<213> Artificial Sequence

<220>
<223> EEF1A2 siRNA antisense strand

<400> 221
ggguuguagc cgaucuucuu u                                                21

<210> 222
<211> 21
<212> RNA
<213> Artificial Sequence

<220>

<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 222
ggguuguagc cgaucuucun n                                                21


<210> 223
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 223
ggguuguagc cgaucuucun n                                                21


<210> 224
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 224
ggguuguagc cgaucuucun n                                                21


<210> 225

<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 225
ggguuguagc cgaucuucun n                                                21

<210> 226
<211> 21
<212> RNA
<213> Artificial Sequence

<220>
<223> EEF1A2 siRNA antisense strand

<400> 226
uuguagccga ucuucuugau u                                                21

<210> 227
<211> 21
<212> RNA
<213> Artificial Sequence

<220>
<223> EEF1A2 siRNA antisense strand

<400> 227
uuguagccga ucuucuugau u                                                21

<210> 228
<211> 21
<212> RNA
<213> Artificial Sequence

<220>
<223> EEF1A2 siRNA antisense strand

<400> 228
uuguagccga ucuucuugau u                                                21

<210> 229
<211> 21

<212> RNA
<213> Artificial Sequence

<220>
<223> EEF1A2 siRNA antisense strand

<400> 229
uuguagccga ucuucuugau u                                                21

<210> 230
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 230
uuguagccga ucuucuugan n                                                21

<210> 231
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 231
uuguagccga ucuucuugan n                                                21

<210> 232
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 232
uuguagccga ucuucuugan n                                                21


<210> 233
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 233
uuguagccga ucuucuugan n                                                21


<210> 234
<211> 21
<212> RNA
<213> Artificial Sequence


<220>
<223> EEF1A2 siRNA antisense strand

<400> 234
uguagccgau cuucuugauu u                                                21


<210> 235
<211> 21
<212> RNA
<213> Artificial Sequence


<220>
<223> EEF1A2 siRNA antisense strand

<400> 235

uguagccgau cuucuugauu u                                                21

<210> 236
<211> 21
<212> RNA
<213> Artificial Sequence

<220>
<223> EEF1A2 siRNA antisense strand

<400> 236
uguagccgau cuucuugauu u                                                21

<210> 237
<211> 21
<212> RNA
<213> Artificial Sequence

<220>
<223> EEF1A2 siRNA antisense strand

<400> 237
uguagccgau cuucuugauu u                                                21

<210> 238
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 238
uguagccgau cuucuugaun n                                                21

<210> 239
<211> 21
<212> RNA
<213> Artificial Sequence

<220>
<223> EEF1A2 siRNA antisense strand

<220>
<221> misc_feature

<222> 20
<223> /note="n is dT (deoxythymine or 2'H thymine)"

<220>
<221> misc_feature
<222> 21
<223> /note="n is dT (deoxythymine or 2'H thymine)"

<400> 239
uguagccgau cuucuugaun n                                                21


<210> 240
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 240
uguagccgau cuucuugaun n                                                21


<210> 241
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 241
uguagccgau cuucuugaun n                                                21


<210> 242
<211> 21
<212> RNA
<213> Artificial Sequence

<220>
<223> EEF1A2 siRNA antisense strand

<400> 242
guagccgauc uucuugaugu u                                                21


<210> 243
<211> 21
<212> RNA
<213> Artificial Sequence


<220>
<223> EEF1A2 siRNA antisense strand

<400> 243
guagccgauc uucuugaugu u                                                21


<210> 244
<211> 21
<212> RNA
<213> Artificial Sequence


<220>
<223> EEF1A2 siRNA antisense strand

<400> 244
guagccgauc uucuugaugu u                                                21


<210> 245
<211> 21
<212> RNA
<213> Artificial Sequence


<220>
<223> EEF1A2 siRNA antisense strand

<400> 245
guagccgauc uucuugaugu u                                                21


<210> 246
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature

<222> 21
<223> /note="n is dT (deoxythymine or 2'H thymine)"

<400> 246
guagccgauc uucuugaugn n                                                21

<210> 247
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 247
guagccgauc uucuugaugn n                                                21

<210> 248
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 248
guagccgauc uucuugaugn n                                                21

<210> 249
<211> 21
<212> RNA
<213> Artificial Sequence

<220>
<223> EEF1A2 siRNA antisense strand

<220>

<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 249
guagccgauc uucuugaugn n                                                21


<210> 250
<211> 21
<212> RNA
<213> Artificial Sequence


<220>
<223> EEF1A2 siRNA antisense strand

<400> 250
ggugauguag uacuuggugu u                                                21


<210> 251
<211> 21
<212> RNA
<213> Artificial Sequence


<220>
<223> EEF1A2 siRNA antisense strand

<400> 251
ggugauguag uacuuggugu u                                                21


<210> 252
<211> 21
<212> RNA
<213> Artificial Sequence


<220>
<223> EEF1A2 siRNA antisense strand

<400> 252
ggugauguag uacuuggugu u                                                21


<210> 253
<211> 21
<212> RNA
<213> Artificial Sequence


<220>
<223> EEF1A2 siRNA antisense strand

<400> 253
ggugauguag uacuuggugu u                                                21

<210> 254
<211> 21
<212> RNA
<213> Artificial Sequence

<220>
<223> EEF1A2 siRNA antisense strand

<220>
<221> misc_feature
<222> 20
<223> /note="n is dT (deoxythymine or 2′H thymine)"

<220>
<221> misc_feature
<222> 21
<223> /note="n is dT (deoxythymine or 2′H thymine)"

<400> 254
ggugauguag uacuuggugn n                                                21

<210> 255
<211> 21
<212> RNA
<213> Artificial Sequence

<220>
<223> EEF1A2 siRNA antisense strand

<220>
<221> misc_feature
<222> 20
<223> /note="n is dT (deoxythymine or 2′H thymine)"

<220>
<221> misc_feature
<222> 21
<223> /note="n is dT (deoxythymine or 2′H thymine)"

<400> 255
ggugauguag uacuuggugn n                                                21

<210> 256
<211> 21
<212> RNA
<213> Artificial Sequence

<220>
<223> EEF1A2 siRNA antisense strand

<220>
<221> misc_feature
<222> 20
<223> /note="n is dT (deoxythymine or 2′H thymine)"

<220>

<221> misc_feature
<222> 21
<223> /note="n is dT (deoxythymine or 2'H thymine)"

<400> 256
ggugauguag uacuuggugn n                                                21


<210> 257
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 257
ggugauguag uacuuggugn n                                                21


<210> 258
<211> 21
<212> RNA
<213> Artificial Sequence


<220>
<223> EEF1A2 siRNA antisense strand

<400> 258
uggugaugua guacuugguu u                                                21


<210> 259
<211> 21
<212> RNA
<213> Artificial Sequence


<220>
<223> EEF1A2 siRNA antisense strand

<400> 259
uggugaugua guacuugguu u                                                21


<210> 260
<211> 21
<212> RNA
<213> Artificial Sequence

<220>
<223> EEF1A2 siRNA antisense strand

<400> 260
uggugaugua guacuugguu u                                                21


<210> 261
<211> 21
<212> RNA
<213> Artificial Sequence


<220>
<223> EEF1A2 siRNA antisense strand

<400> 261
uggugaugua guacuugguu u                                                21


<210> 262
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 262
uggugaugua guacuuggun n                                                21


<210> 263
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 263

uggugaugua guacuuggun n                                                21

<210> 264
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 264
uggugaugua guacuuggun n                                                21

<210> 265
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 265
uggugaugua guacuuggun n                                                21

<210> 266
<211> 21
<212> RNA
<213> Artificial Sequence

<220>
<223> EEF1A2 siRNA antisense strand

<400> 266
auggugaugu aguacuuggu u                                                21

<210> 267

<211> 21
<212> RNA
<213> Artificial Sequence

<220>
<223> EEF1A2 siRNA antisense strand

<400> 267
auggugaugu aguacuuggu u                                                21

<210> 268
<211> 21
<212> RNA
<213> Artificial Sequence

<220>
<223> EEF1A2 siRNA antisense strand

<400> 268
auggugaugu aguacuuggu u                                                21

<210> 269
<211> 21
<212> RNA
<213> Artificial Sequence

<220>
<223> EEF1A2 siRNA antisense strand

<400> 269
auggugaugu aguacuuggu u                                                21

<210> 270
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 270
auggugaugu aguacuuggn n                                                21

<210> 271
<211> 21

<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 271
auggugaugu aguacuuggn n                                                21

<210> 272
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 272
auggugaugu aguacuuggn n                                                21

<210> 273
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 273
auggugaugu aguacuuggn n                                                21

<210> 274
<211> 21
<212> RNA
<213> Artificial Sequence

<220>
<223> EEF1A2 siRNA antisense strand

<400> 274
gauggugaug uaguacuugu u                                                21

<210> 275
<211> 21
<212> RNA
<213> Artificial Sequence

<220>
<223> EEF1A2 siRNA antisense strand

<400> 275
gauggugaug uaguacuugu u                                                21

<210> 276
<211> 21
<212> RNA
<213> Artificial Sequence

<220>
<223> EEF1A2 siRNA antisense strand

<400> 276
gauggugaug uaguacuugu u                                                21

<210> 277
<211> 21
<212> RNA
<213> Artificial Sequence

<220>
<223> EEF1A2 siRNA antisense strand

<400> 277
gauggugaug uaguacuugu u                                                21

<210> 278
<211> 21
<212> RNA
<213> Artificial Sequence

<220>

<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 278
gauggugaug uaguacuugn n                                                21


<210> 279
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 279
gauggugaug uaguacuugn n                                                21


<210> 280
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 280
gauggugaug uaguacuugn n                                                21


<210> 281

<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 281
gauggugaug uaguacuugn n                                                21

<210> 282
<211> 21
<212> RNA
<213> Artificial Sequence

<220>
<223> EEF1A2 siRNA antisense strand

<400> 282
ugauggugau guaguacuuu u                                                21

<210> 283
<211> 21
<212> RNA
<213> Artificial Sequence

<220>
<223> EEF1A2 siRNA antisense strand

<400> 283
ugauggugau guaguacuuu u                                                21

<210> 284
<211> 21
<212> RNA
<213> Artificial Sequence

<220>
<223> EEF1A2 siRNA antisense strand

<400> 284
ugauggugau guaguacuuu u                                                21

<210> 285
<211> 21

<212> RNA
<213> Artificial Sequence

<220>
<223> EEF1A2 siRNA antisense strand

<400> 285
ugauggugau guaguacuuu u                                                21

<210> 286
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 286
ugauggugau guaguacuun n                                                21

<210> 287
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 287
ugauggugau guaguacuun n                                                21

<210> 288
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 288
ugauggugau guaguacuun n                                                21


<210> 289
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 289
ugauggugau guaguacuun n                                                21


<210> 290
<211> 21
<212> RNA
<213> Artificial Sequence


<220>
<223> EEF1A2 siRNA antisense strand

<400> 290
augaugguga uguaguacuu u                                                21


<210> 291
<211> 21
<212> RNA
<213> Artificial Sequence


<220>
<223> EEF1A2 siRNA antisense strand

<400> 291

augaugguga uguaguacuu u                                                21

<210> 292
<211> 21
<212> RNA
<213> Artificial Sequence

<220>
<223> EEF1A2 siRNA antisense strand

<400> 292
augaugguga uguaguacuu u                                                21

<210> 293
<211> 21
<212> RNA
<213> Artificial Sequence

<220>
<223> EEF1A2 siRNA antisense strand

<400> 293
augaugguga uguaguacuu u                                                21

<210> 294
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 294
augaugguga uguaguacun n                                                21

<210> 295
<211> 21
<212> RNA
<213> Artificial Sequence

<220>
<223> EEF1A2 siRNA antisense strand

<220>
<221> misc_feature

<222> 20
<223> /note="n is dT (deoxythymine or 2′H thymine)"

<220>
<221> misc_feature
<222> 21
<223> /note="n is dT (deoxythymine or 2′H thymine)"

<400> 295
augaugguga uguaguacun n                                                21


<210> 296
<211> 21
<212> RNA
<213> Artificial Sequence


<220>
<223> EEF1A2 siRNA antisense strand

<220>
<221> misc_feature
<222> 20
<223> /note="n is dT (deoxythymine or 2′H thymine)"

<220>
<221> misc_feature
<222> 21
<223> /note="n is dT (deoxythymine or 2′H thymine)"

<400> 296
augaugguga uguaguacun n                                                21


<210> 297
<211> 21
<212> RNA
<213> Artificial Sequence


<220>
<223> EEF1A2 siRNA antisense strand

<220>
<221> misc_feature
<222> 20
<223> /note="n is dT (deoxythymine or 2′H thymine)"

<220>
<221> misc_feature
<222> 21
<223> /note="n is dT (deoxythymine or 2′H thymine)"

<400> 297
augaugguga uguaguacun n                                                21


<210> 298
<211> 21
<212> RNA
<213> Artificial Sequence

<220>
<223> EEF1A2 siRNA antisense strand

<400> 298
gaugauggug auguaguacu u                                                21


<210> 299
<211> 21
<212> RNA
<213> Artificial Sequence


<220>
<223> EEF1A2 siRNA antisense strand

<400> 299
gaugauggug auguaguacu u                                                21


<210> 300
<211> 21
<212> RNA
<213> Artificial Sequence


<220>
<223> EEF1A2 siRNA antisense strand

<400> 300
gaugauggug auguaguacu u                                                21


<210> 301
<211> 21
<212> RNA
<213> Artificial Sequence


<220>
<223> EEF1A2 siRNA antisense strand

<400> 301
gaugauggug auguaguacu u                                                21


<210> 302
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature

<222> 21
<223> /note="n is dT (deoxythymine or 2'H thymine)"

<400> 302
gaugauggug auguaguacn n                                                21


<210> 303
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 303
gaugauggug auguaguacn n                                                21


<210> 304
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 304
gaugauggug auguaguacn n                                                21


<210> 305
<211> 21
<212> RNA
<213> Artificial Sequence


<220>
<223> EEF1A2 siRNA antisense strand

<220>

<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 305
gaugauggug auguaguacn n                                                21


<210> 306
<211> 21
<212> RNA
<213> Artificial Sequence


<220>
<223> EEF1A2 siRNA antisense strand

<400> 306
cgaugauggu gauguaguau u                                                21


<210> 307
<211> 21
<212> RNA
<213> Artificial Sequence


<220>
<223> EEF1A2 siRNA antisense strand

<400> 307
cgaugauggu gauguaguau u                                                21


<210> 308
<211> 21
<212> RNA
<213> Artificial Sequence


<220>
<223> EEF1A2 siRNA antisense strand

<400> 308
cgaugauggu gauguaguau u                                                21


<210> 309
<211> 21
<212> RNA
<213> Artificial Sequence


<220>
<223> EEF1A2 siRNA antisense strand

<400> 309
cgaugauggu gauguaguau u                                                21

<210> 310
<211> 21
<212> RNA
<213> Artificial Sequence

<220>
<223> EEF1A2 siRNA antisense strand

<220>
<221> misc_feature
<222> 20
<223> /note="n is dT (deoxythymine or 2′H thymine)"

<220>
<221> misc_feature
<222> 21
<223> /note="n is dT (deoxythymine or 2′H thymine)"

<400> 310
cgaugauggu gauguaguan n                                                21

<210> 311
<211> 21
<212> RNA
<213> Artificial Sequence

<220>
<223> EEF1A2 siRNA antisense strand

<220>
<221> misc_feature
<222> 20
<223> /note="n is dT (deoxythymine or 2′H thymine)"

<220>
<221> misc_feature
<222> 21
<223> /note="n is dT (deoxythymine or 2′H thymine)"

<400> 311
cgaugauggu gauguaguan n                                                21

<210> 312
<211> 21
<212> RNA
<213> Artificial Sequence

<220>
<223> EEF1A2 siRNA antisense strand

<220>
<221> misc_feature
<222> 20
<223> /note="n is dT (deoxythymine or 2′H thymine)"

<220>

<221> misc_feature
<222> 21
<223> /note="n is dT (deoxythymine or 2'H thymine)"

<400> 312
cgaugauggu gauguaguan n                                                21


<210> 313
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 313
cgaugauggu gauguaguan n                                                21


<210> 314
<211> 21
<212> RNA
<213> Artificial Sequence


<220>
<223> EEF1A2 siRNA antisense strand

<400> 314
uagccgaucu ucuugauguu u                                                21


<210> 315
<211> 21
<212> RNA
<213> Artificial Sequence


<220>
<223> EEF1A2 siRNA antisense strand

<400> 315
uagccgaucu ucuugauguu u                                                21


<210> 316
<211> 21
<212> RNA
<213> Artificial Sequence

<220>
<223> EEF1A2 siRNA antisense strand

<400> 316
uagccgaucu ucuugauguu u                                                21


<210> 317
<211> 21
<212> RNA
<213> Artificial Sequence


<220>
<223> EEF1A2 siRNA antisense strand

<400> 317
uagccgaucu ucuugauguu u                                                21


<210> 318
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 318
uagccgaucu ucuugaugun n                                                21


<210> 319
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 319

uagccgaucu ucuugaugun n 21

<210> 320
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 320
uagccgaucu ucuugaugun n 21

<210> 321
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 321
uagccgaucu ucuugaugun n 21

<210> 322
<211> 21
<212> RNA
<213> Artificial Sequence

<220>
<223> EEF1A2 siRNA antisense strand

<400> 322
ccgaucuucu ugauguaggu u 21

<210> 323

<211> 21
<212> RNA
<213> Artificial Sequence

<220>
<223> EEF1A2 siRNA antisense strand

<400> 323
ccgaucuucu ugauguaggu u                                                21

<210> 324
<211> 21
<212> RNA
<213> Artificial Sequence

<220>
<223> EEF1A2 siRNA antisense strand

<400> 324
ccgaucuucu ugauguaggu u                                                21

<210> 325
<211> 21
<212> RNA
<213> Artificial Sequence

<220>
<223> EEF1A2 siRNA antisense strand

<400> 325
ccgaucuucu ugauguaggu u                                                21

<210> 326
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 326
ccgaucuucu ugauguaggn n                                                21

<210> 327
<211> 21

<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 327
ccgaucuucu ugauguaggn n                                                21

<210> 328
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 328
ccgaucuucu ugauguaggn n                                                21

<210> 329
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 329
ccgaucuucu ugauguaggn n                                                21

<210> 330
<211> 21
<212> RNA
<213> Artificial Sequence

<220>
<223> EEF1A2 siRNA antisense strand

<400> 330
gccgaucuuc uugauguagu u                                                21

<210> 331
<211> 21
<212> RNA
<213> Artificial Sequence

<220>
<223> EEF1A2 siRNA antisense strand

<400> 331
gccgaucuuc uugauguagu u                                                21

<210> 332
<211> 21
<212> RNA
<213> Artificial Sequence

<220>
<223> EEF1A2 siRNA antisense strand

<400> 332
gccgaucuuc uugauguagu u                                                21

<210> 333
<211> 21
<212> RNA
<213> Artificial Sequence

<220>
<223> EEF1A2 siRNA antisense strand

<400> 333
gccgaucuuc uugauguagu u                                                21

<210> 334
<211> 21
<212> RNA
<213> Artificial Sequence

<220>

<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 334
gccgaucuuc uugauguagn n                                                21


<210> 335
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 335
gccgaucuuc uugauguagn n                                                21


<210> 336
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 336
gccgaucuuc uugauguagn n                                                21


<210> 337

<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 337
gccgaucuuc uugauguagn n                                                21

<210> 338
<211> 21
<212> RNA
<213> Artificial Sequence

<220>
<223> EEF1A2 siRNA antisense strand

<400> 338
agccgaucuu cuugauguau u                                                21

<210> 339
<211> 21
<212> RNA
<213> Artificial Sequence

<220>
<223> EEF1A2 siRNA antisense strand

<400> 339
agccgaucuu cuugauguau u                                                21

<210> 340
<211> 21
<212> RNA
<213> Artificial Sequence

<220>
<223> EEF1A2 siRNA antisense strand

<400> 340
agccgaucuu cuugauguau u                                                21

<210> 341
<211> 21

<212> RNA
<213> Artificial Sequence

<220>
<223> EEF1A2 siRNA antisense strand

<400> 341
agccgaucuu cuugauguau u                                                21

<210> 342
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 342
agccgaucuu cuugauguan n                                                21

<210> 343
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 343
agccgaucuu cuugauguan n                                                21

<210> 344
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 344
agccgaucuu cuugauguan n                                                21


<210> 345
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 345
agccgaucuu cuugauguan n                                                21


<210> 346
<211> 21
<212> RNA
<213> Artificial Sequence


<220>
<223> EEF1A2 siRNA antisense strand

<400> 346
cggguuguag ccgaucuucu u                                                21


<210> 347
<211> 21
<212> RNA
<213> Artificial Sequence


<220>
<223> EEF1A2 siRNA antisense strand

<400> 347

cggguuguag ccgaucuucu u                                                21

<210> 348
<211> 21
<212> RNA
<213> Artificial Sequence

<220>
<223> EEF1A2 siRNA antisense strand

<400> 348
cggguuguag ccgaucuucu u                                                21

<210> 349
<211> 21
<212> RNA
<213> Artificial Sequence

<220>
<223> EEF1A2 siRNA antisense strand

<400> 349
cggguuguag ccgaucuucu u                                                21

<210> 350
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 350
cggguuguag ccgaucuucn n                                                21

<210> 351
<211> 21
<212> RNA
<213> Artificial Sequence

<220>
<223> EEF1A2 siRNA antisense strand

<220>
<221> misc_feature

<222> 20
<223> /note="n is dT (deoxythymine or 2'H thymine)"

<220>
<221> misc_feature
<222> 21
<223> /note="n is dT (deoxythymine or 2'H thymine)"

<400> 351
cggguuguag ccgaucuucn n                                                21


<210> 352
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 352
cggguuguag ccgaucuucn n                                                21


<210> 353
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 353
cggguuguag ccgaucuucn n                                                21


<210> 354
<211> 21
<212> RNA
<213> Artificial Sequence

<220>
<223> EEF1A2 siRNA antisense strand

<400> 354
gggagauguc gauggugauu u                                                21


<210> 355
<211> 21
<212> RNA
<213> Artificial Sequence


<220>
<223> EEF1A2 siRNA antisense strand

<400> 355
gggagauguc gauggugauu u                                                21


<210> 356
<211> 21
<212> RNA
<213> Artificial Sequence


<220>
<223> EEF1A2 siRNA antisense strand

<400> 356
gggagauguc gauggugauu u                                                21


<210> 357
<211> 21
<212> RNA
<213> Artificial Sequence


<220>
<223> EEF1A2 siRNA antisense strand

<400> 357
gggagauguc gauggugauu u                                                21


<210> 358
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature

<222> 21
<223> /note="n is dT (deoxythymine or 2'H thymine)"

<400> 358
gggagauguc gauggugaun n                                                21


<210> 359
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 359
gggagauguc gauggugaun n                                                21


<210> 360
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 360
gggagauguc gauggugaun n                                                21


<210> 361
<211> 21
<212> RNA
<213> Artificial Sequence


<220>
<223> EEF1A2 siRNA antisense strand

<220>

<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 361
gggagauguc gauggugaun n                                                21


<210> 362
<211> 21
<212> RNA
<213> Artificial Sequence


<220>
<223> EEF1A2 siRNA antisense strand

<400> 362
agggagaugu cgauggugau u                                                21


<210> 363
<211> 21
<212> RNA
<213> Artificial Sequence


<220>
<223> EEF1A2 siRNA antisense strand

<400> 363
agggagaugu cgauggugau u                                                21


<210> 364
<211> 21
<212> RNA
<213> Artificial Sequence


<220>
<223> EEF1A2 siRNA antisense strand

<400> 364
agggagaugu cgauggugau u                                                21


<210> 365
<211> 21
<212> RNA
<213> Artificial Sequence


<220>
<223> EEF1A2 siRNA antisense strand

<400> 365
agggagaugu cgauggugau u                                                21

<210> 366
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 366
agggagaugu cgauggugan n                                                21

<210> 367
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 367
agggagaugu cgauggugan n                                                21

<210> 368
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>

<221> misc_feature
<222> 21
<223> /note="n is dT (deoxythymine or 2'H thymine)"

<400> 368
agggagaugu cgauggugan n                                                21


<210> 369
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 369
agggagaugu cgauggugan n                                                21


<210> 370
<211> 21
<212> RNA
<213> Artificial Sequence


<220>
<223> EEF1A2 siRNA antisense strand

<400> 370
gagggagaug ucgauggugu u                                                21


<210> 371
<211> 21
<212> RNA
<213> Artificial Sequence


<220>
<223> EEF1A2 siRNA antisense strand

<400> 371
gagggagaug ucgauggugu u                                                21


<210> 372
<211> 21
<212> RNA
<213> Artificial Sequence

<220>
<223> EEF1A2 siRNA antisense strand

<400> 372
gagggagaug ucgauggugu u                                                21


<210> 373
<211> 21
<212> RNA
<213> Artificial Sequence


<220>
<223> EEF1A2 siRNA antisense strand

<400> 373
gagggagaug ucgauggugu u                                                21


<210> 374
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 374
gagggagaug ucgauggugn n                                                21


<210> 375
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 375

gagggagaug ucgauggugn n                                                21

<210> 376
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 376
gagggagaug ucgauggugn n                                                21

<210> 377
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 377
gagggagaug ucgauggugn n                                                21

<210> 378
<211> 21
<212> RNA
<213> Artificial Sequence

<220>
<223> EEF1A2 siRNA antisense strand

<400> 378
   agagggagau gucgaugguu u                                                21

<210> 379

<211> 21
<212> RNA
<213> Artificial Sequence

<220>
<223> EEF1A2 siRNA antisense strand

<400> 379
agagggagau gucgaugguu u                                                21

<210> 380
<211> 21
<212> RNA
<213> Artificial Sequence

<220>
<223> EEF1A2 siRNA antisense strand

<400> 380
agagggagau gucgaugguu u                                                21

<210> 381
<211> 21
<212> RNA
<213> Artificial Sequence

<220>
<223> EEF1A2 siRNA antisense strand

<400> 381
agagggagau gucgaugguu u                                                21

<210> 382
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 382
agagggagau gucgauggun n                                                21

<210> 383
<211> 21

<212> RNA
<213> Artificial Sequence

<220>
<223> EEF1A2 siRNA antisense strand

<220>
<221> misc_feature
<222> 20
<223> /note="n is dT (deoxythymine or 2′H thymine)"

<220>
<221> misc_feature
<222> 21
<223> /note="n is dT (deoxythymine or 2′H thymine)"

<400> 383
agagggagau gucgauggun n                                                21

<210> 384
<211> 21
<212> RNA
<213> Artificial Sequence

<220>
<223> EEF1A2 siRNA antisense strand

<220>
<221> misc_feature
<222> 20
<223> /note="n is dT (deoxythymine or 2′H thymine)"

<220>
<221> misc_feature
<222> 21
<223> /note="n is dT (deoxythymine or 2′H thymine)"

<400> 384
agagggagau gucgauggun n                                                21

<210> 385
<211> 21
<212> RNA
<213> Artificial Sequence

<220>
<223> EEF1A2 siRNA antisense strand

<220>
<221> misc_feature
<222> 20
<223> /note="n is dT (deoxythymine or 2′H thymine)"

<220>
<221> misc_feature
<222> 21
<223> /note="n is dT (deoxythymine or 2′H thymine)"

<400> 385
agagggagau gucgauggun n                                                21

<210> 386
<211> 21
<212> RNA
<213> Artificial Sequence

<220>
<223> EEF1A2 siRNA antisense strand

<400> 386
cagagggaga ugucgauggu u                                                21

<210> 387
<211> 21
<212> RNA
<213> Artificial Sequence

<220>
<223> EEF1A2 siRNA antisense strand

<400> 387
cagagggaga ugucgauggu u                                                21

<210> 388
<211> 21
<212> RNA
<213> Artificial Sequence

<220>
<223> EEF1A2 siRNA antisense strand

<400> 388
cagagggaga ugucgauggu u                                                21

<210> 389
<211> 21
<212> RNA
<213> Artificial Sequence

<220>
<223> EEF1A2 siRNA antisense strand

<400> 389
cagagggaga ugucgauggu u                                                21

<210> 390
<211> 21
<212> RNA
<213> Artificial Sequence

<220>

<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 390
cagagggaga ugucgauggn n                                                21


<210> 391
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 391
cagagggaga ugucgauggn n                                                21


<210> 392
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 392
cagagggaga ugucgauggn n                                                21


<210> 393

<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 393
cagagggaga ugucgauggn n                                                21

<210> 394
<211> 21
<212> RNA
<213> Artificial Sequence

<220>
<223> EEF1A2 siRNA antisense strand

<400> 394
ccagagggag augucgaugu u                                                21

<210> 395
<211> 21
<212> RNA
<213> Artificial Sequence

<220>
<223> EEF1A2 siRNA antisense strand

<400> 395
ccagagggag augucgaugu u                                                21

<210> 396
<211> 21
<212> RNA
<213> Artificial Sequence

<220>
<223> EEF1A2 siRNA antisense strand

<400> 396
ccagagggag augucgaugu u                                                21

<210> 397
<211> 21

<212> RNA
<213> Artificial Sequence

<220>
<223> EEF1A2 siRNA antisense strand

<400> 397
ccagagggag augucgaugu u                                                21

<210> 398
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 398
ccagagggag augucgaugn n                                                21

<210> 399
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 399
ccagagggag augucgaugn n                                                21

<210> 400
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 400
ccagagggag augucgaugn n                                                21

<210> 401
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 401
ccagagggag augucgaugn n                                                21

<210> 402
<211> 21
<212> RNA
<213> Artificial Sequence

<220>
<223> EEF1A2 siRNA antisense strand

<400> 402
uccagaggga gaugucgauu u                                                21

<210> 403
<211> 21
<212> RNA
<213> Artificial Sequence

<220>
<223> EEF1A2 siRNA antisense strand

<400> 403

uccagaggga gaugucgauu u                                                21

<210> 404
<211> 21
<212> RNA
<213> Artificial Sequence

<220>
<223> EEF1A2 siRNA antisense strand

<400> 404
uccagaggga gaugucgauu u                                                21

<210> 405
<211> 21
<212> RNA
<213> Artificial Sequence

<220>
<223> EEF1A2 siRNA antisense strand

<400> 405
uccagaggga gaugucgauu u                                                21

<210> 406
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 406
uccagaggga gaugucgaun n                                                21

<210> 407
<211> 21
<212> RNA
<213> Artificial Sequence

<220>
<223> EEF1A2 siRNA antisense strand

<220>
<221> misc_feature

<222> 20
<223> /note="n is dT (deoxythymine or 2'H thymine)"

<220>
<221> misc_feature
<222> 21
<223> /note="n is dT (deoxythymine or 2'H thymine)"

<400> 407
uccagaggga gaugucgaun n                                                21


<210> 408
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 408
uccagaggga gaugucgaun n                                                21


<210> 409
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 409
uccagaggga gaugucgaun n                                                21


<210> 410
<211> 21
<212> RNA
<213> Artificial Sequence

<220>
<223> EEF1A2 siRNA antisense strand

<400> 410
uuccagaggg agaugucgau u                                                21


<210> 411
<211> 21
<212> RNA
<213> Artificial Sequence


<220>
<223> EEF1A2 siRNA antisense strand

<400> 411
uuccagaggg agaugucgau u                                                21


<210> 412
<211> 21
<212> RNA
<213> Artificial Sequence


<220>
<223> EEF1A2 siRNA antisense strand

<400> 412
uuccagaggg agaugucgau u                                                21


<210> 413
<211> 21
<212> RNA
<213> Artificial Sequence


<220>
<223> EEF1A2 siRNA antisense strand

<400> 413
uuccagaggg agaugucgau u                                                21


<210> 414
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature

<222> 21
<223> /note="n is dT (deoxythymine or 2'H thymine)"

<400> 414
uuccagaggg agaugucgan n                                                21


<210> 415
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 415
uuccagaggg agaugucgan n                                                21


<210> 416
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 416
uuccagaggg agaugucgan n                                                21


<210> 417
<211> 21
<212> RNA
<213> Artificial Sequence


<220>
<223> EEF1A2 siRNA antisense strand

<220>

<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 417
uuccagaggg agaugucgan n                                                21


<210> 418
<211> 21
<212> RNA
<213> Artificial Sequence


<220>
<223> EEF1A2 siRNA antisense strand

<400> 418
cuuccagagg gagaugucgu u                                                21


<210> 419
<211> 21
<212> RNA
<213> Artificial Sequence


<220>
<223> EEF1A2 siRNA antisense strand

<400> 419
cuuccagagg gagaugucgu u                                                21


<210> 420
<211> 21
<212> RNA
<213> Artificial Sequence


<220>
<223> EEF1A2 siRNA antisense strand

<400> 420
cuuccagagg gagaugucgu u                                                21


<210> 421
<211> 21
<212> RNA
<213> Artificial Sequence


<220>
<223> EEF1A2 siRNA antisense strand

<400> 421
cuuccagagg gagaugucgu u                                                21

<210> 422
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 422
cuuccagagg gagaugucgn n                                                21

<210> 423
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 423
cuuccagagg gagaugucgn n                                                21

<210> 424
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>

<221> misc_feature
<222> 21
<223> /note="n is dT (deoxythymine or 2'H thymine)"

<400> 424
cuuccagagg gagaugucgn n                                                21


<210> 425
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 425
cuuccagagg gagaugucgn n                                                21


<210> 426
<211> 21
<212> RNA
<213> Artificial Sequence


<220>
<223> EEF1A2 siRNA antisense strand

<400> 426
acuuccagag ggagaugucu u                                                21


<210> 427
<211> 21
<212> RNA
<213> Artificial Sequence


<220>
<223> EEF1A2 siRNA antisense strand

<400> 427
acuuccagag ggagaugucu u                                                21


<210> 428
<211> 21
<212> RNA
<213> Artificial Sequence

<220>
<223> EEF1A2 siRNA antisense strand

<400> 428
acuuccagag ggagaugucu u                                                21


<210> 429
<211> 21
<212> RNA
<213> Artificial Sequence


<220>
<223> EEF1A2 siRNA antisense strand

<400> 429
acuuccagag ggagaugucu u                                                21


<210> 430
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 430
acuuccagag ggagaugucn n                                                21


<210> 431
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 431

acuuccagag ggagaugucn n                                                21

<210> 432
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 432
acuuccagag ggagaugucn n                                                21

<210> 433
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 433
acuuccagag ggagaugucn n                                                21

<210> 434
<211> 21
<212> RNA
<213> Artificial Sequence

<220>
<223> EEF1A2 siRNA antisense strand

<400> 434
aacuuccaga gggagauguu u                                                21

<210> 435

<211> 21
<212> RNA
<213> Artificial Sequence

<220>
<223> EEF1A2 siRNA antisense strand

<400> 435
aacuuccaga gggagauguu u                                                21

<210> 436
<211> 21
<212> RNA
<213> Artificial Sequence

<220>
<223> EEF1A2 siRNA antisense strand

<400> 436
aacuuccaga gggagauguu u                                                21

<210> 437
<211> 21
<212> RNA
<213> Artificial Sequence

<220>
<223> EEF1A2 siRNA antisense strand

<400> 437
aacuuccaga gggagauguu u                                                21

<210> 438
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 438
aacuuccaga gggagaugun n                                                21

<210> 439
<211> 21

<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 439
aacuuccaga gggagaugun n                                                21

<210> 440
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 440
aacuuccaga gggagaugun n                                                21

<210> 441
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 441
aacuuccaga gggagaugun n                                                21

<210> 442
<211> 21
<212> RNA
<213> Artificial Sequence

<220>
<223> EEF1A2 siRNA antisense strand

<400> 442
gaacuuccag agggagaugu u                                                21

<210> 443
<211> 21
<212> RNA
<213> Artificial Sequence

<220>
<223> EEF1A2 siRNA antisense strand

<400> 443
gaacuuccag agggagaugu u                                                21

<210> 444
<211> 21
<212> RNA
<213> Artificial Sequence

<220>
<223> EEF1A2 siRNA antisense strand

<400> 444
gaacuuccag agggagaugu u                                                21

<210> 445
<211> 21
<212> RNA
<213> Artificial Sequence

<220>
<223> EEF1A2 siRNA antisense strand

<400> 445
gaacuuccag agggagaugu u                                                21

<210> 446
<211> 21
<212> RNA
<213> Artificial Sequence

<220>

<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 446
gaacuuccag agggagaugn n                                                21


<210> 447
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 447
gaacuuccag agggagaugn n                                                21


<210> 448
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 448
gaacuuccag agggagaugn n                                                21


<210> 449

<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 449
gaacuuccag agggagaugn n                                                21

<210> 450
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 450
gugauguagu acuugguggn                                                  20

<210> 451
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 451
gugauguagu acuugguggn n                                                21

<210> 452
<211> 21
<212> RNA
<213> Artificial Sequence

<220>
<223> EEF1A2 siRNA antisense strand

<400> 452
gugauguagu acuugguggu u                                                21

<210> 453
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 453
aucuucuuga uguaggcgcn                                                  20

<210> 454
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 454
aucuucuuga uguaggcgcn n                                                21

<210> 455
<211> 21
<212> RNA
<213> Artificial Sequence

<220>
<223> EEF1A2 siRNA antisense strand

<400> 455
aucuucuuga uguaggcgcu u                                                21

<210> 456
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 456
   ugauguagua cuugguggun                                                 20

<210> 457
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 457
   ugauguagua cuugguggun n                                               21

<210> 458
<211> 21
<212> RNA
<213> Artificial Sequence

<220>
<223> EEF1A2 siRNA antisense strand

<400> 458
ugauguagua cuuggugguu u                                                21

<210> 459
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 459
gaucuucuug auguaggcgn                                                    20


<210> 460
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 460
gaucuucuug auguaggcgn n                                                  21


<210> 461
<211> 21
<212> RNA
<213> Artificial Sequence


<220>
<223> EEF1A2 siRNA antisense strand

<400> 461
gaucuucuug auguaggcgu u                                                  21


<210> 462
<211> 20
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<400> 462
   gauguaguac uugguggucn                                                    20

<210> 463
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 463
cuucuugaug uaggcgcugn                                                    20

<210> 464
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 464
ucuucuugau guaggcgcun                                                    20

<210> 465
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 465
ucuucuugau guaggcgcun n                                                  21

<210> 466
<211> 21
<212> RNA
<213> Artificial Sequence

<220>
<223> EEF1A2 siRNA antisense strand

<400> 466
ucuucuugau guaggcgcuu u                                                 21

<210> 467
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 467
guuguagccg aucuucuugn                                                   20

<210> 468
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 468
guuguagccg aucuucuugn n                                                 21

<210> 469
<211> 21
<212> RNA
<213> Artificial Sequence

<220>
<223> EEF1A2 siRNA antisense strand

<400> 469
guuguagccg aucuucuugu u                                                21

<210> 470
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 470
gguuguagcc gaucuucuun                                                  20

<210> 471
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 471
gguuguagcc gaucuucuun n                                                21

<210> 472
<211> 21
<212> RNA
<213> Artificial Sequence

<220>
<223> EEF1A2 siRNA antisense strand

<400> 472
gguuguagcc gaucuucuuu u                                                21

<210> 473
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 473
cgaucuucuu gauguaggcn                                                    20


<210> 474
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 474
cgaucuucuu gauguaggcn n                                                  21


<210> 475
<211> 21
<212> RNA
<213> Artificial Sequence


<220>
<223> EEF1A2 siRNA antisense strand

<400> 475
cgaucuucuu gauguaggcu u                                                  21


<210> 476
<211> 20
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<400> 476
ggguuguagc cgaucuucun                                                    20


<210> 477
<211> 20
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<400> 477
uuguagccga ucuucuugan                                                    20


<210> 478
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 478
uuguagccga ucuucuugan n                                                  21


<210> 479
<211> 21
<212> RNA
<213> Artificial Sequence


<220>
<223> EEF1A2 siRNA antisense strand

<400> 479
uuguagccga ucuucuugau u                                                  21


<210> 480
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 480
uguagccgau cuucuugaun                                                20


<210> 481
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 481
uguagccgau cuucuugaun n                                              21


<210> 482
<211> 21
<212> RNA
<213> Artificial Sequence


<220>
<223> EEF1A2 siRNA antisense strand

<400> 482
uguagccgau cuucuugauu u                                              21


<210> 483
<211> 20
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<400> 483
guagccgauc uucuugaugn                                                20

<210> 484
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 484
guagccgauc uucuugaugn n                                              21

<210> 485
<211> 21
<212> RNA
<213> Artificial Sequence

<220>
<223> EEF1A2 siRNA antisense strand

<400> 485
guagccgauc uucuugaugu u                                              21

<210> 486
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 486
ggugauguag uacuuggugn                                                20

<210> 487
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 487
ggugauguag uacuuggugn n                                                21


<210> 488
<211> 21
<212> RNA
<213> Artificial Sequence


<220>
<223> EEF1A2 siRNA antisense strand

<400> 488
ggugauguag uacuuggugu u                                                21


<210> 489
<211> 20
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<400> 489
uggugaugua guacuuggun                                                  20


<210> 490
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 490
uggugaugua guacuuggun n                                                21

<210> 491
<211> 21
<212> RNA
<213> Artificial Sequence

<220>
<223> EEF1A2 siRNA antisense strand

<400> 491
uggugaugua guacuugguu u                                                21

<210> 492
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 492
auggugaugu aguacuuggn                                                  20

<210> 493
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 493
auggugaugu aguacuuggn n                                                21

<210> 494
<211> 21
<212> RNA
<213> Artificial Sequence

<220>
<223> EEF1A2 siRNA antisense strand

<400> 494
auggugaugu aguacuuggu u                                                21

<210> 495
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 495
gauggugaug uaguacuugn                                                  20

<210> 496
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 496
gauggugaug uaguacuugn n                                                21

<210> 497
<211> 21
<212> RNA
<213> Artificial Sequence

<220>
<223> EEF1A2 siRNA antisense strand

<400> 497
gauggugaug uaguacuugu u                                                21

<210> 498
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 498
ugauggugau guaguacuun                                                  20

<210> 499
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 499
ugauggugau guaguacuun n                                                21

<210> 500
<211> 21
<212> RNA
<213> Artificial Sequence

<220>
<223> EEF1A2 siRNA antisense strand

<400> 500
ugauggugau guaguacuuu u                                                21

<210> 501
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 501
augaugguga uguaguacun                                                    20


<210> 502
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 502
augaugguga uguaguacun n                                                  21


<210> 503
<211> 21
<212> RNA
<213> Artificial Sequence


<220>
<223> EEF1A2 siRNA antisense strand

<400> 503
augaugguga uguaguacuu u                                                  21


<210> 504
<211> 20
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<400> 504
gaugauggug auguaguacn                                                20

<210> 505
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 505
gaugauggug auguaguacn n                                              21

<210> 506
<211> 21
<212> RNA
<213> Artificial Sequence

<220>
<223> EEF1A2 siRNA antisense strand

<400> 506
gaugauggug auguaguacu u                                              21

<210> 507
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 507
cgaugauggu gauguaguan                                                20

<210> 508
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 508
cgaugauggu gauguaguan n                                                21


<210> 509
<211> 21
<212> RNA
<213> Artificial Sequence


<220>
<223> EEF1A2 siRNA antisense strand

<400> 509
cgaugauggu gauguaguau u                                                21


<210> 510
<211> 20
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<400> 510
uagccgaucu ucuugaugun                                                  20


<210> 511
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 511
uagccgaucu ucuugaugun n                                                21


<210> 512
<211> 21
<212> RNA
<213> Artificial Sequence


<220>
<223> EEF1A2 siRNA antisense strand

<400> 512
uagccgaucu ucuugauguu u                                                21


<210> 513
<211> 20
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<400> 513
ccgaucuucu ugauguaggn                                                  20


<210> 514
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 514
ccgaucuucu ugauguaggn n                                                21

<210> 515
<211> 21
<212> RNA
<213> Artificial Sequence

<220>
<223> EEF1A2 siRNA antisense strand

<400> 515
ccgaucuucu ugauguaggu u                                                21

<210> 516
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 516
gccgaucuuc uugauguagn                                                  20

<210> 517
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 517
gccgaucuuc uugauguagn n                                                21

<210> 518
<211> 21
<212> RNA
<213> Artificial Sequence

<220>
<223> EEF1A2 siRNA antisense strand

<400> 518
gccgaucuuc uugauguagu u                                                21

<210> 519
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 519
agccgaucuu cuugauguan                                                  20

<210> 520
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 520
agccgaucuu cuugauguan n                                                21

<210> 521
<211> 21
<212> RNA
<213> Artificial Sequence

<220>
<223> EEF1A2 siRNA antisense strand

<400> 521
agccgaucuu cuugauguau u                                                21

<210> 522
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 522
ggagaugucg auggugaugn                                                    20


<210> 523
<211> 20
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<400> 523
gggagauguc gauggugaun                                                    20


<210> 524
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 524
gggagauguc gauggugaun n                                                  21


<210> 525
<211> 21
<212> RNA
<213> Artificial Sequence


<220>
<223> EEF1A2 siRNA antisense strand

<400> 525
gggagauguc gauggugauu u                                                21

<210> 526
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 526
agggagaugu cgauggugan                                                  20

<210> 527
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 527
agggagaugu cgauggugan n                                                21

<210> 528
<211> 21
<212> RNA
<213> Artificial Sequence

<220>
<223> EEF1A2 siRNA antisense strand

<400> 528
agggagaugu cgauggugau u                                                21

<210> 529
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 529
gagggagaug ucgauggugn                                                    20


<210> 530
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 530
gagggagaug ucgauggugn n                                                  21


<210> 531
<211> 21
<212> RNA
<213> Artificial Sequence


<220>
<223> EEF1A2 siRNA antisense strand

<400> 531
gagggagaug ucgauggugu u                                                  21


<210> 532
<211> 20
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<400> 532
agagggagau gucgauggun                                                    20

<210> 533
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 533
agagggagau gucgauggun n                                                  21

<210> 534
<211> 21
<212> RNA
<213> Artificial Sequence

<220>
<223> EEF1A2 siRNA antisense strand

<400> 534
agagggagau gucgaugguu u                                                  21

<210> 535
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 535
cagagggaga ugucgauggn                                                    20

<210> 536
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 536
cagagggaga ugucgauggn n                                                21

<210> 537
<211> 21
<212> RNA
<213> Artificial Sequence

<220>
<223> EEF1A2 siRNA antisense strand

<400> 537
cagagggaga ugucgauggu u                                                21

<210> 538
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 538
ccagagggag augucgaugn                                                  20

<210> 539
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 539
ccagagggag augucgaugn n                                                21


<210> 540
<211> 21
<212> RNA
<213> Artificial Sequence


<220>
<223> EEF1A2 siRNA antisense strand

<400> 540
ccagagggag augucgaugu u                                                21


<210> 541
<211> 20
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<400> 541
uccagaggga gaugucgaun                                                  20


<210> 542
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 542
uccagaggga gaugucgaun n                                                21

<210> 543
<211> 21
<212> RNA
<213> Artificial Sequence

<220>
<223> EEF1A2 siRNA antisense strand

<400> 543
uccagaggga gaugucgauu u                                                21

<210> 544
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 544
uuccagaggg agaugucgan                                                  20

<210> 545
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 545
uuccagaggg agaugucgan n                                                21

<210> 546
<211> 21
<212> RNA
<213> Artificial Sequence

<220>
<223> EEF1A2 siRNA antisense strand

<400> 546
uuccagaggg agaugucgau u                                                21

<210> 547
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 547
cuuccagagg gagaugucgn                                                  20

<210> 548
<211> 21
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 548
cuuccagagg gagaugucgn n                                                21

<210> 549
<211> 21
<212> RNA
<213> Artificial Sequence

<220>
<223> EEF1A2 siRNA antisense strand

<400> 549
cuuccagagg gagaugucgu u                                                21

<210> 550
<211> 20
<212> RNA
<213> Artificial Sequence

<220>
<223> EEF1A2 siRNA antisense strand

<220>
<221> misc_feature
<222> 20
<223> /note="n is dT (deoxythymine or 2'H thymine)"

<400> 550
acuuccagag ggagaugucn                                                    20


<210> 551
<211> 21
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 551
acuuccagag ggagaugucn n                                                  21


<210> 552
<211> 21
<212> RNA
<213> Artificial Sequence


<220>
<223> EEF1A2 siRNA antisense strand

<400> 552
acuuccagag ggagaugucu u                                                  21


<210> 553
<211> 20
<212> RNA
<213> Artificial Sequence


<220>
<223> EEF1A2 siRNA antisense strand

<220>
<221> misc_feature
<222> 20
<223> /note="n is dT (deoxythymine or 2'H thymine)"

<400> 553
aacuuccaga gggagaugun                                                    20

<210> 554
<211> 20
<212> RNA
<213> Artificial Sequence

<220>
<223> EEF1A2 siRNA antisense strand

<400> 554
   gugauguagu acuugguggu                                                    20

<210> 555
<211> 21
<212> RNA
<213> Artificial Sequence

<220>
<223> EEF1A2 siRNA antisense strand

<400> 555
   gugauguagu acuugguggu c                                                  21

<210> 556
<211> 20
<212> RNA
<213> Artificial Sequence

<220>
<223> EEF1A2 siRNA antisense strand

<400> 556
aucuucuuga uguaggcgcu                                                    20

<210> 557
<211> 21
<212> RNA
<213> Artificial Sequence

<220>
<223> EEF1A2 siRNA antisense strand

<400> 557
aucuucuuga uguaggcgcu g                                                  21

<210> 558
<211> 20
<212> RNA
<213> Artificial Sequence

<220>

<223> EEF1A2 siRNA antisense strand

<400> 558
ugauguagua cuuggugguc                                                    20


<210> 559
<211> 21
<212> RNA
<213> Artificial Sequence


<220>
<223> EEF1A2 siRNA antisense strand

<400> 559
ugauguagua cuuggugguc u                                                  21


<210> 560
<211> 20
<212> RNA
<213> Artificial Sequence


<220>
<223> EEF1A2 siRNA antisense strand

<400> 560
gaucuucuug auguaggcgc                                                    20


<210> 561
<211> 21
<212> RNA
<213> Artificial Sequence


<220>
<223> EEF1A2 siRNA antisense strand

<400> 561
gaucuucuug auguaggcgc u                                                  21


<210> 562
<211> 20
<212> RNA
<213> Artificial Sequence


<220>
<223> EEF1A2 siRNA antisense strand

<400> 562
gauguaguac uuggugguc u                                                   20


<210> 563
<211> 20
<212> RNA
<213> Artificial Sequence

<220>
<223> EEF1A2 siRNA antisense strand

<400> 563
cuucuugaug uaggcgcuga                                                    20


<210> 564
<211> 20
<212> RNA
<213> Artificial Sequence


<220>
<223> EEF1A2 siRNA antisense strand

<400> 564
ucuucuugau guaggcgcug                                                    20


<210> 565
<211> 21
<212> RNA
<213> Artificial Sequence


<220>
<223> EEF1A2 siRNA antisense strand

<400> 565
ucuucuugau guaggcgcug a                                                  21


<210> 566
<211> 20
<212> RNA
<213> Artificial Sequence


<220>
<223> EEF1A2 siRNA antisense strand

<400> 566
guuguagccg aucuucuuga                                                    20


<210> 567
<211> 21
<212> RNA
<213> Artificial Sequence


<220>
<223> EEF1A2 siRNA antisense strand

<400> 567
guuguagccg aucuucuuga u                                                  21


<210> 568
<211> 20
<212> RNA

<213> Artificial Sequence

<220>
<223> EEF1A2 siRNA antisense strand

<400> 568
gguuguagcc gaucuucuug                                                    20

<210> 569
<211> 21
<212> RNA
<213> Artificial Sequence

<220>
<223> EEF1A2 siRNA antisense strand

<400> 569
gguuguagcc gaucuucuug a                                                  21

<210> 570
<211> 20
<212> RNA
<213> Artificial Sequence

<220>
<223> EEF1A2 siRNA antisense strand

<400> 570
cgaucuucuu gauguaggcg                                                    20

<210> 571
<211> 21
<212> RNA
<213> Artificial Sequence

<220>
<223> EEF1A2 siRNA antisense strand

<400> 571
cgaucuucuu gauguaggcg c                                                  21

<210> 572
<211> 20
<212> RNA
<213> Artificial Sequence

<220>
<223> EEF1A2 siRNA antisense strand

<400> 572
ggguuguagc cgaucuucuu                                                    20

<210> 573

<211> 20
<212> RNA
<213> Artificial Sequence

<220>
<223> EEF1A2 siRNA antisense strand

<400> 573
uuguagccga ucuucuugau                                                    20

<210> 574
<211> 21
<212> RNA
<213> Artificial Sequence

<220>
<223> EEF1A2 siRNA antisense strand

<400> 574
uuguagccga ucuucuugau g                                                  21

<210> 575
<211> 20
<212> RNA
<213> Artificial Sequence

<220>
<223> EEF1A2 siRNA antisense strand

<400> 575
uguagccgau cuucuugaug                                                    20

<210> 576
<211> 21
<212> RNA
<213> Artificial Sequence

<220>
<223> EEF1A2 siRNA antisense strand

<400> 576
uguagccgau cuucuugaug u                                                  21

<210> 577
<211> 20
<212> RNA
<213> Artificial Sequence

<220>
<223> EEF1A2 siRNA antisense strand

<400> 577
guagccgauc uucuugaugu                                                    20

<210> 578
<211> 21
<212> RNA
<213> Artificial Sequence

<220>
<223> EEF1A2 siRNA antisense strand

<400> 578
guagccgauc uucuugaugu a                                                21

<210> 579
<211> 20
<212> RNA
<213> Artificial Sequence

<220>
<223> EEF1A2 siRNA antisense strand

<400> 579
ggugauguag uacuuggugg                                                  20

<210> 580
<211> 21
<212> RNA
<213> Artificial Sequence

<220>
<223> EEF1A2 siRNA antisense strand

<400> 580
ggugauguag uacuuggugg u                                                21

<210> 581
<211> 20
<212> RNA
<213> Artificial Sequence

<220>
<223> EEF1A2 siRNA antisense strand

<400> 581
uggugaugua guacuuggug                                                  20

<210> 582
<211> 21
<212> RNA
<213> Artificial Sequence

<220>
<223> EEF1A2 siRNA antisense strand

<400> 582

uggugaugua guacuuggug g                                                21

<210> 583
<211> 20
<212> RNA
<213> Artificial Sequence

<220>
<223> EEF1A2 siRNA antisense strand

<400> 583
auggugaugu aguacuuggu                                                  20

<210> 584
<211> 21
<212> RNA
<213> Artificial Sequence

<220>
<223> EEF1A2 siRNA antisense strand

<400> 584
auggugaugu aguacuuggu g                                                21

<210> 585
<211> 20
<212> RNA
<213> Artificial Sequence

<220>
<223> EEF1A2 siRNA antisense strand

<400> 585
gauggugaug uaguacuugg                                                  20

<210> 586
<211> 21
<212> RNA
<213> Artificial Sequence

<220>
<223> EEF1A2 siRNA antisense strand

<400> 586
gauggugaug uaguacuugg u                                                21

<210> 587
<211> 20
<212> RNA
<213> Artificial Sequence

<220>
<223> EEF1A2 siRNA antisense strand

<400> 587
ugauggugau guaguacuug                                                    20


<210> 588
<211> 21
<212> RNA
<213> Artificial Sequence


<220>
<223> EEF1A2 siRNA antisense strand

<400> 588
ugauggugau guaguacuug g                                                  21


<210> 589
<211> 20
<212> RNA
<213> Artificial Sequence


<220>
<223> EEF1A2 siRNA antisense strand

<400> 589
augaugguga uguaguacuu                                                    20


<210> 590
<211> 21
<212> RNA
<213> Artificial Sequence


<220>
<223> EEF1A2 siRNA antisense strand

<400> 590
augaugguga uguaguacuu g                                                  21


<210> 591
<211> 20
<212> RNA
<213> Artificial Sequence


<220>
<223> EEF1A2 siRNA antisense strand

<400> 591
gaugauggug auguaguacu                                                    20


<210> 592
<211> 21
<212> RNA
<213> Artificial Sequence

<220>
<223> EEF1A2 siRNA antisense strand

<400> 592
gaugauggug auguaguacu u                                                21


<210> 593
<211> 20
<212> RNA
<213> Artificial Sequence


<220>
<223> EEF1A2 siRNA antisense strand

<400> 593
cgaugauggu gauguaguac                                                  20


<210> 594
<211> 21
<212> RNA
<213> Artificial Sequence


<220>
<223> EEF1A2 siRNA antisense strand

<400> 594
cgaugauggu gauguaguac u                                                21


<210> 595
<211> 20
<212> RNA
<213> Artificial Sequence


<220>
<223> EEF1A2 siRNA antisense strand

<400> 595
uagccgaucu ucuugaugua                                                  20


<210> 596
<211> 21
<212> RNA
<213> Artificial Sequence


<220>
<223> EEF1A2 siRNA antisense strand

<400> 596
uagccgaucu ucuugaugua g                                                21


<210> 597
<211> 20
<212> RNA
<213> Artificial Sequence

<220>
<223> EEF1A2 siRNA antisense strand

<400> 597
ccgaucuucu ugauguaggc                                                    20


<210> 598
<211> 21
<212> RNA
<213> Artificial Sequence


<220>
<223> EEF1A2 siRNA antisense strand

<400> 598
ccgaucuucu ugauguaggc g                                                  21


<210> 599
<211> 20
<212> RNA
<213> Artificial Sequence


<220>
<223> EEF1A2 siRNA antisense strand

<400> 599
gccgaucuuc uugauguagg                                                    20


<210> 600
<211> 21
<212> RNA
<213> Artificial Sequence


<220>
<223> EEF1A2 siRNA antisense strand

<400> 600
gccgaucuuc uugauguagg c                                                  21


<210> 601
<211> 20
<212> RNA
<213> Artificial Sequence


<220>
<223> EEF1A2 siRNA antisense strand

<400> 601
agccgaucuu cuugauguag                                                    20


<210> 602
<211> 21

<212> RNA
<213> Artificial Sequence

<220>
<223> EEF1A2 siRNA antisense strand

<400> 602
agccgaucuu cuugauguag g                                                21

<210> 603
<211> 20
<212> RNA
<213> Artificial Sequence

<220>
<223> EEF1A2 siRNA antisense strand

<400> 603
ggagaugucg auggugaugc                                                  20

<210> 604
<211> 20
<212> RNA
<213> Artificial Sequence

<220>
<223> EEF1A2 siRNA antisense strand

<400> 604
gggagauguc gauggugaug                                                  20

<210> 605
<211> 21
<212> RNA
<213> Artificial Sequence

<220>
<223> EEF1A2 siRNA antisense strand

<400> 605
gggagauguc gauggugaug c                                                21

<210> 606
<211> 20
<212> RNA
<213> Artificial Sequence

<220>
<223> EEF1A2 siRNA antisense strand

<400> 606
agggagaugu cgauggugau                                                  20

<210> 607
<211> 21
<212> RNA
<213> Artificial Sequence

<220>
<223> EEF1A2 siRNA antisense strand

<400> 607
agggagaugu cgauggugau g                                                21

<210> 608
<211> 20
<212> RNA
<213> Artificial Sequence

<220>
<223> EEF1A2 siRNA antisense strand

<400> 608
gagggagaug ucgaugguga                                                  20

<210> 609
<211> 21
<212> RNA
<213> Artificial Sequence

<220>
<223> EEF1A2 siRNA antisense strand

<400> 609
gagggagaug ucgaugguga u                                                21

<210> 610
<211> 20
<212> RNA
<213> Artificial Sequence

<220>
<223> EEF1A2 siRNA antisense strand

<400> 610
agagggagau gucgauggug                                                  20

<210> 611
<211> 21
<212> RNA
<213> Artificial Sequence

<220>
<223> EEF1A2 siRNA antisense strand

<400> 611
agagggagau gucgauggug a                                                21

<210> 612
<211> 20
<212> RNA
<213> Artificial Sequence

<220>
<223> EEF1A2 siRNA antisense strand

<400> 612
cagagggaga ugucgauggu                                                    20

<210> 613
<211> 21
<212> RNA
<213> Artificial Sequence

<220>
<223> EEF1A2 siRNA antisense strand

<400> 613
cagagggaga ugucgauggu g                                                  21

<210> 614
<211> 20
<212> RNA
<213> Artificial Sequence

<220>
<223> EEF1A2 siRNA antisense strand

<400> 614
ccagagggag augucgaugg                                                    20

<210> 615
<211> 21
<212> RNA
<213> Artificial Sequence

<220>
<223> EEF1A2 siRNA antisense strand

<400> 615
ccagagggag augucgaugg u                                                  21

<210> 616
<211> 20
<212> RNA
<213> Artificial Sequence

<220>
<223> EEF1A2 siRNA antisense strand

<400> 616
uccagaggga gaugucgaug                                                   20

<210> 617
<211> 21
<212> RNA
<213> Artificial Sequence

<220>
<223> EEF1A2 siRNA antisense strand

<400> 617
uccagaggga gaugucgaug g                                                 21

<210> 618
<211> 20
<212> RNA
<213> Artificial Sequence

<220>
<223> EEF1A2 siRNA antisense strand

<400> 618
uuccagaggg agaugucgau                                                   20

<210> 619
<211> 21
<212> RNA
<213> Artificial Sequence

<220>
<223> EEF1A2 siRNA antisense strand

<400> 619
uuccagaggg agaugucgau g                                                 21

<210> 620
<211> 20
<212> RNA
<213> Artificial Sequence

<220>
<223> EEF1A2 siRNA antisense strand

<400> 620
cuuccagagg gagaugucga                                                   20

<210> 621
<211> 21
<212> RNA
<213> Artificial Sequence

<220>

<223> EEF1A2 siRNA antisense strand

<400> 621
cuuccagagg gagaugucga u                                                21

<210> 622
<211> 20
<212> RNA
<213> Artificial Sequence

<220>
<223> EEF1A2 siRNA antisense strand

<400> 622
acuuccagag ggagaugucg                                                  20

<210> 623
<211> 21
<212> RNA
<213> Artificial Sequence

<220>
<223> EEF1A2 siRNA antisense strand

<400> 623
acuuccagag ggagaugucg a                                                21

<210> 624
<211> 20
<212> RNA
<213> Artificial Sequence

<220>
<223> EEF1A2 siRNA antisense strand

<400> 624
aacuuccaga gggagauguc                                                  20

<210> 625
<211> 19
<212> RNA
<213> Artificial Sequence

<220>
<223> EEF1A2 siRNA sense strand

<400> 625
ccaccaagua cuacaucag                                                   19

<210> 626
<211> 19
<212> RNA
<213> Artificial Sequence

<220>
<223> EEF1A2 siRNA sense strand

<400> 626
ccaccaagua cuacaucug                                                    19


<210> 627
<211> 19
<212> RNA
<213> Artificial Sequence


<220>
<223> EEF1A2 siRNA sense strand

<400> 627
ccaccaagua cuacaugug                                                    19


<210> 628
<211> 19
<212> RNA
<213> Artificial Sequence


<220>
<223> EEF1A2 siRNA sense strand

<400> 628
ccaccaagua cuacaagug                                                    19


<210> 629
<211> 19
<212> RNA
<213> Artificial Sequence


<220>
<223> EEF1A2 siRNA sense strand

<400> 629
ccaccaagua cuacaucag                                                    19


<210> 630
<211> 19
<212> RNA
<213> Artificial Sequence


<220>
<223> EEF1A2 siRNA sense strand

<400> 630
ccaccaagua cuacaucug                                                    19


<210> 631
<211> 19
<212> RNA

<213> Artificial Sequence

<220>
<223> EEF1A2 siRNA sense strand

<400> 631
ccaccaagua cuacaugug                                                    19

<210> 632
<211> 19
<212> RNA
<213> Artificial Sequence

<220>
<223> EEF1A2 siRNA sense strand

<400> 632
ccaccaagua cuacaagug                                                    19

<210> 633
<211> 19
<212> RNA
<213> Artificial Sequence

<220>
<223> EEF1A2 siRNA sense strand

<400> 633
gcgccuacau caagaagaa                                                    19

<210> 634
<211> 19
<212> RNA
<213> Artificial Sequence

<220>
<223> EEF1A2 siRNA sense strand

<400> 634
gcgccuacau caagaagua                                                    19

<210> 635
<211> 19
<212> RNA
<213> Artificial Sequence

<220>
<223> EEF1A2 siRNA sense strand

<400> 635
gcgccuacau caagaacua                                                    19

<210> 636

<211> 19
<212> RNA
<213> Artificial Sequence

<220>
<223> EEF1A2 siRNA sense strand

<400> 636
gcgccuacau caagaucua                                                    19

<210> 637
<211> 19
<212> RNA
<213> Artificial Sequence

<220>
<223> EEF1A2 siRNA sense strand

<400> 637
gcgccuacau caagaagaa                                                    19

<210> 638
<211> 19
<212> RNA
<213> Artificial Sequence

<220>
<223> EEF1A2 siRNA sense strand

<400> 638
gcgccuacau caagaagua                                                    19

<210> 639
<211> 19
<212> RNA
<213> Artificial Sequence

<220>
<223> EEF1A2 siRNA sense strand

<400> 639
gcgccuacau caagaacua                                                    19

<210> 640
<211> 19
<212> RNA
<213> Artificial Sequence

<220>
<223> EEF1A2 siRNA sense strand

<400> 640
gcgccuacau caagaucua                                                    19

<210> 641
<211> 19
<212> RNA
<213> Artificial Sequence

<220>
<223> EEF1A2 siRNA sense strand

<400> 641
accaccaagu acuacaucu                                                    19

<210> 642
<211> 19
<212> RNA
<213> Artificial Sequence

<220>
<223> EEF1A2 siRNA sense strand

<400> 642
accaccaagu acuacaugu                                                    19

<210> 643
<211> 19
<212> RNA
<213> Artificial Sequence

<220>
<223> EEF1A2 siRNA sense strand

<400> 643
accaccaagu acuacaagu                                                    19

<210> 644
<211> 19
<212> RNA
<213> Artificial Sequence

<220>
<223> EEF1A2 siRNA sense strand

<400> 644
accaccaagu acuacuagu                                                    19

<210> 645
<211> 19
<212> RNA
<213> Artificial Sequence

<220>
<223> EEF1A2 siRNA sense strand

<400> 645

accaccaagu acuacaucu                                                    19

<210> 646
<211> 19
<212> RNA
<213> Artificial Sequence

<220>
<223> EEF1A2 siRNA sense strand

<400> 646
accaccaagu acuacaugu                                                    19

<210> 647
<211> 19
<212> RNA
<213> Artificial Sequence

<220>
<223> EEF1A2 siRNA sense strand

<400> 647
accaccaagu acuacaagu                                                    19

<210> 648
<211> 19
<212> RNA
<213> Artificial Sequence

<220>
<223> EEF1A2 siRNA sense strand

<400> 648
accaccaagu acuacuagu                                                    19

<210> 649
<211> 19
<212> RNA
<213> Artificial Sequence

<220>
<223> EEF1A2 siRNA sense strand

<400> 649
cgccuacauc aagaagaug                                                    19

<210> 650
<211> 19
<212> RNA
<213> Artificial Sequence

<220>
<223> EEF1A2 siRNA sense strand

<400> 650
cgccuacauc aagaagaag                                                    19


<210> 651
<211> 19
<212> RNA
<213> Artificial Sequence


<220>
<223> EEF1A2 siRNA sense strand

<400> 651
cgccuacauc aagaaguag                                                    19


<210> 652
<211> 19
<212> RNA
<213> Artificial Sequence


<220>
<223> EEF1A2 siRNA sense strand

<400> 652
cgccuacauc aagaacuag                                                    19


<210> 653
<211> 19
<212> RNA
<213> Artificial Sequence


<220>
<223> EEF1A2 siRNA sense strand

<400> 653
cgccuacauc aagaagaug                                                    19


<210> 654
<211> 19
<212> RNA
<213> Artificial Sequence


<220>
<223> EEF1A2 siRNA sense strand

<400> 654
cgccuacauc aagaagaag                                                    19


<210> 655
<211> 19
<212> RNA
<213> Artificial Sequence

<220>
<223> EEF1A2 siRNA sense strand

<400> 655
cgccuacauc aagaaguag                                                    19


<210> 656
<211> 19
<212> RNA
<213> Artificial Sequence


<220>
<223> EEF1A2 siRNA sense strand

<400> 656
cgccuacauc aagaacuag                                                    19


<210> 657
<211> 19
<212> RNA
<213> Artificial Sequence


<220>
<223> EEF1A2 siRNA sense strand

<400> 657
agcgccuaca ucaagaagu                                                    19


<210> 658
<211> 19
<212> RNA
<213> Artificial Sequence


<220>
<223> EEF1A2 siRNA sense strand

<400> 658
agcgccuaca ucaagaacu                                                    19


<210> 659
<211> 19
<212> RNA
<213> Artificial Sequence


<220>
<223> EEF1A2 siRNA sense strand

<400> 659
agcgccuaca ucaagaucu                                                    19


<210> 660
<211> 19
<212> RNA
<213> Artificial Sequence

<220>
<223> EEF1A2 siRNA sense strand

<400> 660
agcgccuaca ucaaguucu                                                    19


<210> 661
<211> 19
<212> RNA
<213> Artificial Sequence


<220>
<223> EEF1A2 siRNA sense strand

<400> 661
agcgccuaca ucaagaagu                                                    19


<210> 662
<211> 19
<212> RNA
<213> Artificial Sequence


<220>
<223> EEF1A2 siRNA sense strand

<400> 662
agcgccuaca ucaagaacu                                                    19


<210> 663
<211> 19
<212> RNA
<213> Artificial Sequence


<220>
<223> EEF1A2 siRNA sense strand

<400> 663
agcgccuaca ucaagaucu                                                    19


<210> 664
<211> 19
<212> RNA
<213> Artificial Sequence


<220>
<223> EEF1A2 siRNA sense strand

<400> 664
agcgccuaca ucaaguucu                                                    19


<210> 665
<211> 19

<212> RNA
<213> Artificial Sequence

<220>
<223> EEF1A2 siRNA sense strand

<400> 665
caagaagauc ggcuacaag                                                    19

<210> 666
<211> 19
<212> RNA
<213> Artificial Sequence

<220>
<223> EEF1A2 siRNA sense strand

<400> 666
caagaagauc ggcuacaug                                                    19

<210> 667
<211> 19
<212> RNA
<213> Artificial Sequence

<220>
<223> EEF1A2 siRNA sense strand

<400> 667
caagaagauc ggcuacuug                                                    19

<210> 668
<211> 19
<212> RNA
<213> Artificial Sequence

<220>
<223> EEF1A2 siRNA sense strand

<400> 668
caagaagauc ggcuaguug                                                    19

<210> 669
<211> 19
<212> RNA
<213> Artificial Sequence

<220>
<223> EEF1A2 siRNA sense strand

<400> 669
caagaagauc ggcuacaag                                                    19

<210> 670
<211> 19
<212> RNA
<213> Artificial Sequence

<220>
<223> EEF1A2 siRNA sense strand

<400> 670
caagaagauc ggcuacaug                                                    19

<210> 671
<211> 19
<212> RNA
<213> Artificial Sequence

<220>
<223> EEF1A2 siRNA sense strand

<400> 671
caagaagauc ggcuacuug                                                    19

<210> 672
<211> 19
<212> RNA
<213> Artificial Sequence

<220>
<223> EEF1A2 siRNA sense strand

<400> 672
caagaagauc ggcuaguug                                                    19

<210> 673
<211> 19
<212> RNA
<213> Artificial Sequence

<220>
<223> EEF1A2 siRNA sense strand

<400> 673
aagaagaucg gcuacaacg                                                    19

<210> 674
<211> 19
<212> RNA
<213> Artificial Sequence

<220>
<223> EEF1A2 siRNA sense strand

<400> 674
aagaagaucg gcuacaagg                                                    19

<210> 675
<211> 19
<212> RNA
<213> Artificial Sequence

<220>
<223> EEF1A2 siRNA sense strand

<400> 675
aagaagaucg gcuacaugg                                                    19

<210> 676
<211> 19
<212> RNA
<213> Artificial Sequence

<220>
<223> EEF1A2 siRNA sense strand

<400> 676
aagaagaucg gcuacuugg                                                    19

<210> 677
<211> 19
<212> RNA
<213> Artificial Sequence

<220>
<223> EEF1A2 siRNA sense strand

<400> 677
aagaagaucg gcuacaacg                                                    19

<210> 678
<211> 19
<212> RNA
<213> Artificial Sequence

<220>
<223> EEF1A2 siRNA sense strand

<400> 678
aagaagaucg gcuacaagg                                                    19

<210> 679
<211> 19
<212> RNA
<213> Artificial Sequence

<220>
<223> EEF1A2 siRNA sense strand

<400> 679
aagaagaucg gcuacaugg                                                    19

<210> 680
<211> 19
<212> RNA
<213> Artificial Sequence

<220>
<223> EEF1A2 siRNA sense strand

<400> 680
aagaagaucg gcuacuugg                                                    19

<210> 681
<211> 19
<212> RNA
<213> Artificial Sequence

<220>
<223> EEF1A2 siRNA sense strand

<400> 681
gccuacauca agaagaucc                                                    19

<210> 682
<211> 19
<212> RNA
<213> Artificial Sequence

<220>
<223> EEF1A2 siRNA sense strand

<400> 682
gccuacauca agaagaugc                                                    19

<210> 683
<211> 19
<212> RNA
<213> Artificial Sequence

<220>
<223> EEF1A2 siRNA sense strand

<400> 683
gccuacauca agaagaagc                                                    19

<210> 684
<211> 19
<212> RNA
<213> Artificial Sequence

<220>

<223> EEF1A2 siRNA sense strand

<400> 684
gccuacauca agaaguagc                                                    19


<210> 685
<211> 19
<212> RNA
<213> Artificial Sequence


<220>
<223> EEF1A2 siRNA sense strand

<400> 685
gccuacauca agaagaucc                                                    19


<210> 686
<211> 19
<212> RNA
<213> Artificial Sequence


<220>
<223> EEF1A2 siRNA sense strand

<400> 686
gccuacauca agaagaugc                                                    19


<210> 687
<211> 19
<212> RNA
<213> Artificial Sequence


<220>
<223> EEF1A2 siRNA sense strand

<400> 687
gccuacauca agaagaagc                                                    19


<210> 688
<211> 19
<212> RNA
<213> Artificial Sequence


<220>
<223> EEF1A2 siRNA sense strand

<400> 688
gccuacauca agaaguagc                                                    19


<210> 689
<211> 19
<212> RNA
<213> Artificial Sequence

<220>
<223> EEF1A2 siRNA sense strand

<400> 689
agaagaucgg cuacaaccg                                                    19


<210> 690
<211> 19
<212> RNA
<213> Artificial Sequence


<220>
<223> EEF1A2 siRNA sense strand

<400> 690
agaagaucgg cuacaacgg                                                    19


<210> 691
<211> 19
<212> RNA
<213> Artificial Sequence


<220>
<223> EEF1A2 siRNA sense strand

<400> 691
agaagaucgg cuacaaggg                                                    19


<210> 692
<211> 19
<212> RNA
<213> Artificial Sequence


<220>
<223> EEF1A2 siRNA sense strand

<400> 692
agaagaucgg cuacauggg                                                    19


<210> 693
<211> 19
<212> RNA
<213> Artificial Sequence


<220>
<223> EEF1A2 siRNA sense strand

<400> 693
agaagaucgg cuacaaccg                                                    19


<210> 694
<211> 19
<212> RNA

<213> Artificial Sequence

<220>
<223> EEF1A2 siRNA sense strand

<400> 694
agaagaucgg cuacaacgg                                                    19

<210> 695
<211> 19
<212> RNA
<213> Artificial Sequence

<220>
<223> EEF1A2 siRNA sense strand

<400> 695
agaagaucgg cuacaaggg                                                    19

<210> 696
<211> 19
<212> RNA
<213> Artificial Sequence

<220>
<223> EEF1A2 siRNA sense strand

<400> 696
agaagaucgg cuacauggg                                                    19

<210> 697
<211> 19
<212> RNA
<213> Artificial Sequence

<220>
<223> EEF1A2 siRNA sense strand

<400> 697
ucaagaagau cggcuacau                                                    19

<210> 698
<211> 19
<212> RNA
<213> Artificial Sequence

<220>
<223> EEF1A2 siRNA sense strand

<400> 698
ucaagaagau cggcuacuu                                                    19

<210> 699

<211> 19
<212> RNA
<213> Artificial Sequence

<220>
<223> EEF1A2 siRNA sense strand

<400> 699
ucaagaagau cggcuaguu                                                    19

<210> 700
<211> 19
<212> RNA
<213> Artificial Sequence

<220>
<223> EEF1A2 siRNA sense strand

<400> 700
ucaagaagau cggcuuguu                                                    19

<210> 701
<211> 19
<212> RNA
<213> Artificial Sequence

<220>
<223> EEF1A2 siRNA sense strand

<400> 701
ucaagaagau cggcuacau                                                    19

<210> 702
<211> 19
<212> RNA
<213> Artificial Sequence

<220>
<223> EEF1A2 siRNA sense strand

<400> 702
ucaagaagau cggcuacuu                                                    19

<210> 703
<211> 19
<212> RNA
<213> Artificial Sequence

<220>
<223> EEF1A2 siRNA sense strand

<400> 703
ucaagaagau cggcuaguu                                                    19

<210> 704
<211> 19
<212> RNA
<213> Artificial Sequence

<220>
<223> EEF1A2 siRNA sense strand

<400> 704
ucaagaagau cggcuuguu                                                    19

<210> 705
<211> 19
<212> RNA
<213> Artificial Sequence

<220>
<223> EEF1A2 siRNA sense strand

<400> 705
aucaagaaga ucggcuacu                                                    19

<210> 706
<211> 19
<212> RNA
<213> Artificial Sequence

<220>
<223> EEF1A2 siRNA sense strand

<400> 706
aucaagaaga ucggcuagu                                                    19

<210> 707
<211> 19
<212> RNA
<213> Artificial Sequence

<220>
<223> EEF1A2 siRNA sense strand

<400> 707
aucaagaaga ucggcuugu                                                    19

<210> 708
<211> 19
<212> RNA
<213> Artificial Sequence

<220>
<223> EEF1A2 siRNA sense strand

<400> 708

aucaagaaga ucggcaugu                                                    19

<210> 709
<211> 19
<212> RNA
<213> Artificial Sequence

<220>
<223> EEF1A2 siRNA sense strand

<400> 709
aucaagaaga ucggcuacu                                                    19

<210> 710
<211> 19
<212> RNA
<213> Artificial Sequence

<220>
<223> EEF1A2 siRNA sense strand

<400> 710
aucaagaaga ucggcuagu                                                    19

<210> 711
<211> 19
<212> RNA
<213> Artificial Sequence

<220>
<223> EEF1A2 siRNA sense strand

<400> 711
aucaagaaga ucggcuugu                                                    19

<210> 712
<211> 19
<212> RNA
<213> Artificial Sequence

<220>
<223> EEF1A2 siRNA sense strand

<400> 712
aucaagaaga ucggcaugu                                                    19

<210> 713
<211> 19
<212> RNA
<213> Artificial Sequence

<220>
<223> EEF1A2 siRNA sense strand

<400> 713
caucaagaag aucggcuag                                                    19

<210> 714
<211> 19
<212> RNA
<213> Artificial Sequence

<220>
<223> EEF1A2 siRNA sense strand

<400> 714
caucaagaag aucggcuug                                                    19

<210> 715
<211> 19
<212> RNA
<213> Artificial Sequence

<220>
<223> EEF1A2 siRNA sense strand

<400> 715
caucaagaag aucggcaug                                                    19

<210> 716
<211> 19
<212> RNA
<213> Artificial Sequence

<220>
<223> EEF1A2 siRNA sense strand

<400> 716
caucaagaag aucgggaug                                                    19

<210> 717
<211> 19
<212> RNA
<213> Artificial Sequence

<220>
<223> EEF1A2 siRNA sense strand

<400> 717
caucaagaag aucggcuag                                                    19

<210> 718
<211> 19
<212> RNA
<213> Artificial Sequence

<220>
<223> EEF1A2 siRNA sense strand

<400> 718
caucaagaag aucggcuug                                                    19


<210> 719
<211> 19
<212> RNA
<213> Artificial Sequence


<220>
<223> EEF1A2 siRNA sense strand

<400> 719
caucaagaag aucggcaug                                                    19


<210> 720
<211> 19
<212> RNA
<213> Artificial Sequence


<220>
<223> EEF1A2 siRNA sense strand

<400> 720
caucaagaag aucgggaug                                                    19


<210> 721
<211> 19
<212> RNA
<213> Artificial Sequence


<220>
<223> EEF1A2 siRNA sense strand

<400> 721
caccaaguac uacaucacg                                                    19


<210> 722
<211> 19
<212> RNA
<213> Artificial Sequence


<220>
<223> EEF1A2 siRNA sense strand

<400> 722
caccaaguac uacaucagg                                                    19


<210> 723
<211> 19
<212> RNA
<213> Artificial Sequence

<220>
<223> EEF1A2 siRNA sense strand

<400> 723
caccaaguac uacaucugg                                                    19


<210> 724
<211> 19
<212> RNA
<213> Artificial Sequence


<220>
<223> EEF1A2 siRNA sense strand

<400> 724
caccaaguac uacaugugg                                                    19


<210> 725
<211> 19
<212> RNA
<213> Artificial Sequence


<220>
<223> EEF1A2 siRNA sense strand

<400> 725
caccaaguac uacaucacg                                                    19


<210> 726
<211> 19
<212> RNA
<213> Artificial Sequence


<220>
<223> EEF1A2 siRNA sense strand

<400> 726
caccaaguac uacaucagg                                                    19


<210> 727
<211> 19
<212> RNA
<213> Artificial Sequence


<220>
<223> EEF1A2 siRNA sense strand

<400> 727
caccaaguac uacaucugg                                                    19


<210> 728
<211> 19

<212> RNA
<213> Artificial Sequence

<220>
<223> EEF1A2 siRNA sense strand

<400> 728
caccaaguac uacaugugg                                                    19

<210> 729
<211> 19
<212> RNA
<213> Artificial Sequence

<220>
<223> EEF1A2 siRNA sense strand

<400> 729
accaaguacu acaucaccu                                                    19

<210> 730
<211> 19
<212> RNA
<213> Artificial Sequence

<220>
<223> EEF1A2 siRNA sense strand

<400> 730
accaaguacu acaucacgu                                                    19

<210> 731
<211> 19
<212> RNA
<213> Artificial Sequence

<220>
<223> EEF1A2 siRNA sense strand

<400> 731
accaaguacu acaucaggu                                                    19

<210> 732
<211> 19
<212> RNA
<213> Artificial Sequence

<220>
<223> EEF1A2 siRNA sense strand

<400> 732
accaaguacu acaucuggu                                                    19

<210> 733
<211> 19
<212> RNA
<213> Artificial Sequence

<220>
<223> EEF1A2 siRNA sense strand

<400> 733
accaaguacu acaucaccu                                                    19

<210> 734
<211> 19
<212> RNA
<213> Artificial Sequence

<220>
<223> EEF1A2 siRNA sense strand

<400> 734
accaaguacu acaucacgu                                                    19

<210> 735
<211> 19
<212> RNA
<213> Artificial Sequence

<220>
<223> EEF1A2 siRNA sense strand

<400> 735
accaaguacu acaucaggu                                                    19

<210> 736
<211> 19
<212> RNA
<213> Artificial Sequence

<220>
<223> EEF1A2 siRNA sense strand

<400> 736
accaaguacu acaucuggu                                                    19

<210> 737
<211> 19
<212> RNA
<213> Artificial Sequence

<220>
<223> EEF1A2 siRNA sense strand

<400> 737
ccaaguacua caucaccaa                                                    19

<210> 738
<211> 19
<212> RNA
<213> Artificial Sequence

<220>
<223> EEF1A2 siRNA sense strand

<400> 738
ccaaguacua caucaccua                                                    19

<210> 739
<211> 19
<212> RNA
<213> Artificial Sequence

<220>
<223> EEF1A2 siRNA sense strand

<400> 739
ccaaguacua caucacgua                                                    19

<210> 740
<211> 19
<212> RNA
<213> Artificial Sequence

<220>
<223> EEF1A2 siRNA sense strand

<400> 740
ccaaguacua caucaggua                                                    19

<210> 741
<211> 19
<212> RNA
<213> Artificial Sequence

<220>
<223> EEF1A2 siRNA sense strand

<400> 741
ccaaguacua caucaccaa                                                    19

<210> 742
<211> 19
<212> RNA
<213> Artificial Sequence

<220>
<223> EEF1A2 siRNA sense strand

<400> 742
ccaaguacua caucaccua                                                    19

<210> 743
<211> 19
<212> RNA
<213> Artificial Sequence

<220>
<223> EEF1A2 siRNA sense strand

<400> 743
ccaaguacua caucacgua                                                    19

<210> 744
<211> 19
<212> RNA
<213> Artificial Sequence

<220>
<223> EEF1A2 siRNA sense strand

<400> 744
ccaaguacua caucaggua                                                    19

<210> 745
<211> 19
<212> RNA
<213> Artificial Sequence

<220>
<223> EEF1A2 siRNA sense strand

<400> 745
caaguacuac aucaccaug                                                    19

<210> 746
<211> 19
<212> RNA
<213> Artificial Sequence

<220>
<223> EEF1A2 siRNA sense strand

<400> 746
caaguacuac aucaccaag                                                    19

<210> 747
<211> 19
<212> RNA
<213> Artificial Sequence

<220>

<223> EEF1A2 siRNA sense strand

<400> 747
caaguacuac aucaccuag                                                    19


<210> 748
<211> 19
<212> RNA
<213> Artificial Sequence


<220>
<223> EEF1A2 siRNA sense strand

<400> 748
caaguacuac aucacguag                                                    19


<210> 749
<211> 19
<212> RNA
<213> Artificial Sequence


<220>
<223> EEF1A2 siRNA sense strand

<400> 749
caaguacuac aucaccaug                                                    19


<210> 750
<211> 19
<212> RNA
<213> Artificial Sequence


<220>
<223> EEF1A2 siRNA sense strand

<400> 750
caaguacuac aucaccaag                                                    19


<210> 751
<211> 19
<212> RNA
<213> Artificial Sequence


<220>
<223> EEF1A2 siRNA sense strand

<400> 751
caaguacuac aucaccuag                                                    19


<210> 752
<211> 19
<212> RNA
<213> Artificial Sequence

<220>
<223> EEF1A2 siRNA sense strand

<400> 752
caaguacuac aucacguag                                                    19


<210> 753
<211> 19
<212> RNA
<213> Artificial Sequence


<220>
<223> EEF1A2 siRNA sense strand

<400> 753
aaguacuaca ucaccaucu                                                    19


<210> 754
<211> 19
<212> RNA
<213> Artificial Sequence


<220>
<223> EEF1A2 siRNA sense strand

<400> 754
aaguacuaca ucaccaugu                                                    19


<210> 755
<211> 19
<212> RNA
<213> Artificial Sequence


<220>
<223> EEF1A2 siRNA sense strand

<400> 755
aaguacuaca ucaccaagu                                                    19


<210> 756
<211> 19
<212> RNA
<213> Artificial Sequence


<220>
<223> EEF1A2 siRNA sense strand

<400> 756
aaguacuaca ucaccuagu                                                    19


<210> 757
<211> 19
<212> RNA

<213> Artificial Sequence

<220>
<223> EEF1A2 siRNA sense strand

<400> 757
aaguacuaca ucaccaucu                                                    19

<210> 758
<211> 19
<212> RNA
<213> Artificial Sequence

<220>
<223> EEF1A2 siRNA sense strand

<400> 758
aaguacuaca ucaccaugu                                                    19

<210> 759
<211> 19
<212> RNA
<213> Artificial Sequence

<220>
<223> EEF1A2 siRNA sense strand

<400> 759
aaguacuaca ucaccaagu                                                    19

<210> 760
<211> 19
<212> RNA
<213> Artificial Sequence

<220>
<223> EEF1A2 siRNA sense strand

<400> 760
aaguacuaca ucaccuagu                                                    19

<210> 761
<211> 19
<212> RNA
<213> Artificial Sequence

<220>
<223> EEF1A2 siRNA sense strand

<400> 761
aguacuacau caccaucaa                                                    19

<210> 762

<211> 19
<212> RNA
<213> Artificial Sequence

<220>
<223> EEF1A2 siRNA sense strand

<400> 762
aguacuacau caccaucua                                                    19

<210> 763
<211> 19
<212> RNA
<213> Artificial Sequence

<220>
<223> EEF1A2 siRNA sense strand

<400> 763
aguacuacau caccaugua                                                    19

<210> 764
<211> 19
<212> RNA
<213> Artificial Sequence

<220>
<223> EEF1A2 siRNA sense strand

<400> 764
aguacuacau caccaagua                                                    19

<210> 765
<211> 19
<212> RNA
<213> Artificial Sequence

<220>
<223> EEF1A2 siRNA sense strand

<400> 765
aguacuacau caccaucaa                                                    19

<210> 766
<211> 19
<212> RNA
<213> Artificial Sequence

<220>
<223> EEF1A2 siRNA sense strand

<400> 766
aguacuacau caccaucua                                                    19

<210> 767
<211> 19
<212> RNA
<213> Artificial Sequence

<220>
<223> EEF1A2 siRNA sense strand

<400> 767
aguacuacau caccaugua                                                    19

<210> 768
<211> 19
<212> RNA
<213> Artificial Sequence

<220>
<223> EEF1A2 siRNA sense strand

<400> 768
aguacuacau caccaagua                                                    19

<210> 769
<211> 19
<212> RNA
<213> Artificial Sequence

<220>
<223> EEF1A2 siRNA sense strand

<400> 769
guacuacauc accaucaug                                                    19

<210> 770
<211> 19
<212> RNA
<213> Artificial Sequence

<220>
<223> EEF1A2 siRNA sense strand

<400> 770
guacuacauc accaucaag                                                    19

<210> 771
<211> 19
<212> RNA
<213> Artificial Sequence

<220>
<223> EEF1A2 siRNA sense strand

<400> 771

guacuacauc accaucuag                                                    19

<210> 772
<211> 19
<212> RNA
<213> Artificial Sequence

<220>
<223> EEF1A2 siRNA sense strand

<400> 772
guacuacauc accauguag                                                    19

<210> 773
<211> 19
<212> RNA
<213> Artificial Sequence

<220>
<223> EEF1A2 siRNA sense strand

<400> 773
guacuacauc accaucaug                                                    19

<210> 774
<211> 19
<212> RNA
<213> Artificial Sequence

<220>
<223> EEF1A2 siRNA sense strand

<400> 774
guacuacauc accaucaag                                                    19

<210> 775
<211> 19
<212> RNA
<213> Artificial Sequence

<220>
<223> EEF1A2 siRNA sense strand

<400> 775
guacuacauc accaucuag                                                    19

<210> 776
<211> 19
<212> RNA
<213> Artificial Sequence

<220>
<223> EEF1A2 siRNA sense strand

<400> 776
guacuacauc accauguag                                                    19


<210> 777
<211> 19
<212> RNA
<213> Artificial Sequence


<220>
<223> EEF1A2 siRNA sense strand

<400> 777
uacuacauca ccaucaucc                                                    19


<210> 778
<211> 19
<212> RNA
<213> Artificial Sequence


<220>
<223> EEF1A2 siRNA sense strand

<400> 778
uacuacauca ccaucaugc                                                    19


<210> 779
<211> 19
<212> RNA
<213> Artificial Sequence


<220>
<223> EEF1A2 siRNA sense strand

<400> 779
uacuacauca ccaucaagc                                                    19


<210> 780
<211> 19
<212> RNA
<213> Artificial Sequence


<220>
<223> EEF1A2 siRNA sense strand

<400> 780
uacuacauca ccaucuagc                                                    19


<210> 781
<211> 19
<212> RNA
<213> Artificial Sequence

<220>
<223> EEF1A2 siRNA sense strand

<400> 781
uacuacauca ccaucaucc                                                    19


<210> 782
<211> 19
<212> RNA
<213> Artificial Sequence


<220>
<223> EEF1A2 siRNA sense strand

<400> 782
uacuacauca ccaucaugc                                                    19


<210> 783
<211> 19
<212> RNA
<213> Artificial Sequence


<220>
<223> EEF1A2 siRNA sense strand

<400> 783
uacuacauca ccaucaagc                                                    19


<210> 784
<211> 19
<212> RNA
<213> Artificial Sequence


<220>
<223> EEF1A2 siRNA sense strand

<400> 784
uacuacauca ccaucuagc                                                    19


<210> 785
<211> 19
<212> RNA
<213> Artificial Sequence


<220>
<223> EEF1A2 siRNA sense strand

<400> 785
acaucaagaa gaucggcuu                                                    19


<210> 786
<211> 19
<212> RNA
<213> Artificial Sequence

<220>
<223> EEF1A2 siRNA sense strand

<400> 786
acaucaagaa gaucggcau                                                    19


<210> 787
<211> 19
<212> RNA
<213> Artificial Sequence


<220>
<223> EEF1A2 siRNA sense strand

<400> 787
acaucaagaa gaucgggau                                                    19


<210> 788
<211> 19
<212> RNA
<213> Artificial Sequence


<220>
<223> EEF1A2 siRNA sense strand

<400> 788
acaucaagaa gaucgcgau                                                    19


<210> 789
<211> 19
<212> RNA
<213> Artificial Sequence


<220>
<223> EEF1A2 siRNA sense strand

<400> 789
acaucaagaa gaucggcuu                                                    19


<210> 790
<211> 19
<212> RNA
<213> Artificial Sequence


<220>
<223> EEF1A2 siRNA sense strand

<400> 790
acaucaagaa gaucggcau                                                    19


<210> 791
<211> 19

<212> RNA
<213> Artificial Sequence

<220>
<223> EEF1A2 siRNA sense strand

<400> 791
acaucaagaa gaucgggau                                                    19

<210> 792
<211> 19
<212> RNA
<213> Artificial Sequence

<220>
<223> EEF1A2 siRNA sense strand

<400> 792
acaucaagaa gaucgcgau                                                    19

<210> 793
<211> 19
<212> RNA
<213> Artificial Sequence

<220>
<223> EEF1A2 siRNA sense strand

<400> 793
ccuacaucaa gaagaucgc                                                    19

<210> 794
<211> 19
<212> RNA
<213> Artificial Sequence

<220>
<223> EEF1A2 siRNA sense strand

<400> 794
ccuacaucaa gaagauccc                                                    19

<210> 795
<211> 19
<212> RNA
<213> Artificial Sequence

<220>
<223> EEF1A2 siRNA sense strand

<400> 795
ccuacaucaa gaagaugcc                                                    19

<210> 796
<211> 19
<212> RNA
<213> Artificial Sequence

<220>
<223> EEF1A2 siRNA sense strand

<400> 796
ccuacaucaa gaagaagcc 19

<210> 797
<211> 19
<212> RNA
<213> Artificial Sequence

<220>
<223> EEF1A2 siRNA sense strand

<400> 797
ccuacaucaa gaagaucgc 19

<210> 798
<211> 19
<212> RNA
<213> Artificial Sequence

<220>
<223> EEF1A2 siRNA sense strand

<400> 798
ccuacaucaa gaagauccc 19

<210> 799
<211> 19
<212> RNA
<213> Artificial Sequence

<220>
<223> EEF1A2 siRNA sense strand

<400> 799
ccuacaucaa gaagaugcc 19

<210> 800
<211> 19
<212> RNA
<213> Artificial Sequence

<220>
<223> EEF1A2 siRNA sense strand

<400> 800
ccuacaucaa gaagaagcc 19

<210> 801
<211> 19
<212> RNA
<213> Artificial Sequence

<220>
<223> EEF1A2 siRNA sense strand

<400> 801
cuacaucaag aagaucggg                                                    19

<210> 802
<211> 19
<212> RNA
<213> Artificial Sequence

<220>
<223> EEF1A2 siRNA sense strand

<400> 802
cuacaucaag aagaucgcg                                                    19

<210> 803
<211> 19
<212> RNA
<213> Artificial Sequence

<220>
<223> EEF1A2 siRNA sense strand

<400> 803
cuacaucaag aagaucccg                                                    19

<210> 804
<211> 19
<212> RNA
<213> Artificial Sequence

<220>
<223> EEF1A2 siRNA sense strand

<400> 804
cuacaucaag aagaugccg                                                    19

<210> 805
<211> 19
<212> RNA
<213> Artificial Sequence

<220>
<223> EEF1A2 siRNA sense strand

<400> 805
cuacaucaag aagaucggg                                                    19

<210> 806
<211> 19
<212> RNA
<213> Artificial Sequence

<220>
<223> EEF1A2 siRNA sense strand

<400> 806
cuacaucaag aagaucgcg                                                    19

<210> 807
<211> 19
<212> RNA
<213> Artificial Sequence

<220>
<223> EEF1A2 siRNA sense strand

<400> 807
cuacaucaag aagaucccg                                                    19

<210> 808
<211> 19
<212> RNA
<213> Artificial Sequence

<220>
<223> EEF1A2 siRNA sense strand

<400> 808
cuacaucaag aagaugccg                                                    19

<210> 809
<211> 19
<212> RNA
<213> Artificial Sequence

<220>
<223> EEF1A2 siRNA sense strand

<400> 809
uacaucaaga agaucggca                                                    19

<210> 810
<211> 19
<212> RNA
<213> Artificial Sequence

<220>

<223> EEF1A2 siRNA sense strand

<400> 810
uacaucaaga agaucggga                                                    19


<210> 811
<211> 19
<212> RNA
<213> Artificial Sequence


<220>
<223> EEF1A2 siRNA sense strand

<400> 811
uacaucaaga agaucgcga                                                    19


<210> 812
<211> 19
<212> RNA
<213> Artificial Sequence


<220>
<223> EEF1A2 siRNA sense strand

<400> 812
uacaucaaga agaucccga                                                    19


<210> 813
<211> 19
<212> RNA
<213> Artificial Sequence


<220>
<223> EEF1A2 siRNA sense strand

<400> 813
uacaucaaga agaucggca                                                    19


<210> 814
<211> 19
<212> RNA
<213> Artificial Sequence


<220>
<223> EEF1A2 siRNA sense strand

<400> 814
uacaucaaga agaucggga                                                    19


<210> 815
<211> 19
<212> RNA
<213> Artificial Sequence

<220>
<223> EEF1A2 siRNA sense strand

<400> 815
uacaucaaga agaucgcga                                                    19


<210> 816
<211> 19
<212> RNA
<213> Artificial Sequence


<220>
<223> EEF1A2 siRNA sense strand

<400> 816
uacaucaaga agaucccga                                                    19


<210> 817
<211> 19
<212> RNA
<213> Artificial Sequence


<220>
<223> EEF1A2 siRNA sense strand

<400> 817
gaagaucggc uacaacccc                                                    19


<210> 818
<211> 19
<212> RNA
<213> Artificial Sequence


<220>
<223> EEF1A2 siRNA sense strand

<400> 818
gaagaucggc uacaaccgc                                                    19


<210> 819
<211> 19
<212> RNA
<213> Artificial Sequence


<220>
<223> EEF1A2 siRNA sense strand

<400> 819
gaagaucggc uacaacggc                                                    19


<210> 820
<211> 19
<212> RNA

<213> Artificial Sequence

<220>
<223> EEF1A2 siRNA sense strand

<400> 820
gaagaucggc uacaagggc                                                    19

<210> 821
<211> 19
<212> RNA
<213> Artificial Sequence

<220>
<223> EEF1A2 siRNA sense strand

<400> 821
gaagaucggc uacaacccc                                                    19

<210> 822
<211> 19
<212> RNA
<213> Artificial Sequence

<220>
<223> EEF1A2 siRNA sense strand

<400> 822
gaagaucggc uacaaccgc                                                    19

<210> 823
<211> 19
<212> RNA
<213> Artificial Sequence

<220>
<223> EEF1A2 siRNA sense strand

<400> 823
gaagaucggc uacaacggc                                                    19

<210> 824
<211> 19
<212> RNA
<213> Artificial Sequence

<220>
<223> EEF1A2 siRNA sense strand

<400> 824
gaagaucggc uacaagggc                                                    19

<210> 825

<211> 19
<212> RNA
<213> Artificial Sequence

<220>
<223> EEF1A2 siRNA sense strand

<400> 825
aucaccaucg acaucuccg                                                    19

<210> 826
<211> 19
<212> RNA
<213> Artificial Sequence

<220>
<223> EEF1A2 siRNA sense strand

<400> 826
aucaccaucg acaucucgg                                                    19

<210> 827
<211> 19
<212> RNA
<213> Artificial Sequence

<220>
<223> EEF1A2 siRNA sense strand

<400> 827
aucaccaucg acaucuggg                                                    19

<210> 828
<211> 19
<212> RNA
<213> Artificial Sequence

<220>
<223> EEF1A2 siRNA sense strand

<400> 828
aucaccaucg acaucaggg                                                    19

<210> 829
<211> 19
<212> RNA
<213> Artificial Sequence

<220>
<223> EEF1A2 siRNA sense strand

<400> 829
aucaccaucg acaucuccg                                                    19

<210> 830
<211> 19
<212> RNA
<213> Artificial Sequence

<220>
<223> EEF1A2 siRNA sense strand

<400> 830
aucaccaucg acaucucgg 19

<210> 831
<211> 19
<212> RNA
<213> Artificial Sequence

<220>
<223> EEF1A2 siRNA sense strand

<400> 831
aucaccaucg acaucuggg 19

<210> 832
<211> 19
<212> RNA
<213> Artificial Sequence

<220>
<223> EEF1A2 siRNA sense strand

<400> 832
aucaccaucg acaucaggg 19

<210> 833
<211> 19
<212> RNA
<213> Artificial Sequence

<220>
<223> EEF1A2 siRNA sense strand

<400> 833
ucaccaucga caucuccca 19

<210> 834
<211> 19
<212> RNA
<213> Artificial Sequence

<220>
<223> EEF1A2 siRNA sense strand

<400> 834

ucaccaucga caucuccga                                                    19

<210> 835
<211> 19
<212> RNA
<213> Artificial Sequence

<220>
<223> EEF1A2 siRNA sense strand

<400> 835
ucaccaucga caucucgga                                                    19

<210> 836
<211> 19
<212> RNA
<213> Artificial Sequence

<220>
<223> EEF1A2 siRNA sense strand

<400> 836
ucaccaucga caucuggga                                                    19

<210> 837
<211> 19
<212> RNA
<213> Artificial Sequence

<220>
<223> EEF1A2 siRNA sense strand

<400> 837
ucaccaucga caucuccca                                                    19

<210> 838
<211> 19
<212> RNA
<213> Artificial Sequence

<220>
<223> EEF1A2 siRNA sense strand

<400> 838
ucaccaucga caucuccga                                                    19

<210> 839
<211> 19
<212> RNA
<213> Artificial Sequence

<220>
<223> EEF1A2 siRNA sense strand

<400> 839
ucaccaucga caucucgga                                                    19


<210> 840
<211> 19
<212> RNA
<213> Artificial Sequence


<220>
<223> EEF1A2 siRNA sense strand

<400> 840
ucaccaucga caucuggga                                                    19


<210> 841
<211> 19
<212> RNA
<213> Artificial Sequence


<220>
<223> EEF1A2 siRNA sense strand

<400> 841
caccaucgac aucucccug                                                    19


<210> 842
<211> 19
<212> RNA
<213> Artificial Sequence


<220>
<223> EEF1A2 siRNA sense strand

<400> 842
caccaucgac aucucccag                                                    19


<210> 843
<211> 19
<212> RNA
<213> Artificial Sequence


<220>
<223> EEF1A2 siRNA sense strand

<400> 843
caccaucgac aucuccgag                                                    19


<210> 844
<211> 19
<212> RNA
<213> Artificial Sequence

<220>
<223> EEF1A2 siRNA sense strand

<400> 844
caccaucgac aucucggag                                                    19


<210> 845
<211> 19
<212> RNA
<213> Artificial Sequence


<220>
<223> EEF1A2 siRNA sense strand

<400> 845
caccaucgac aucucccug                                                    19


<210> 846
<211> 19
<212> RNA
<213> Artificial Sequence


<220>
<223> EEF1A2 siRNA sense strand

<400> 846
caccaucgac aucucccag                                                    19


<210> 847
<211> 19
<212> RNA
<213> Artificial Sequence


<220>
<223> EEF1A2 siRNA sense strand

<400> 847
caccaucgac aucuccgag                                                    19


<210> 848
<211> 19
<212> RNA
<213> Artificial Sequence


<220>
<223> EEF1A2 siRNA sense strand

<400> 848
caccaucgac aucucggag                                                    19


<210> 849
<211> 19
<212> RNA
<213> Artificial Sequence

<220>
<223> EEF1A2 siRNA sense strand

<400> 849
accaucgaca ucucccuca                                                    19


<210> 850
<211> 19
<212> RNA
<213> Artificial Sequence


<220>
<223> EEF1A2 siRNA sense strand

<400> 850
accaucgaca ucucccuga                                                    19


<210> 851
<211> 19
<212> RNA
<213> Artificial Sequence


<220>
<223> EEF1A2 siRNA sense strand

<400> 851
accaucgaca ucucccaga                                                    19


<210> 852
<211> 19
<212> RNA
<213> Artificial Sequence


<220>
<223> EEF1A2 siRNA sense strand

<400> 852
accaucgaca ucuccgaga                                                    19


<210> 853
<211> 19
<212> RNA
<213> Artificial Sequence


<220>
<223> EEF1A2 siRNA sense strand

<400> 853
accaucgaca ucucccuca                                                    19


<210> 854
<211> 19

<212> RNA
<213> Artificial Sequence

<220>
<223> EEF1A2 siRNA sense strand

<400> 854
accaucgaca ucucccuga                                                    19

<210> 855
<211> 19
<212> RNA
<213> Artificial Sequence

<220>
<223> EEF1A2 siRNA sense strand

<400> 855
accaucgaca ucucccaga                                                    19

<210> 856
<211> 19
<212> RNA
<213> Artificial Sequence

<220>
<223> EEF1A2 siRNA sense strand

<400> 856
accaucgaca ucuccgaga                                                    19

<210> 857
<211> 19
<212> RNA
<213> Artificial Sequence

<220>
<223> EEF1A2 siRNA sense strand

<400> 857
ccaucgacau cucccucuc                                                    19

<210> 858
<211> 19
<212> RNA
<213> Artificial Sequence

<220>
<223> EEF1A2 siRNA sense strand

<400> 858
ccaucgacau cucccucac                                                    19

<210> 859
<211> 19
<212> RNA
<213> Artificial Sequence

<220>
<223> EEF1A2 siRNA sense strand

<400> 859
ccaucgacau cucccugac                                                    19

<210> 860
<211> 19
<212> RNA
<213> Artificial Sequence

<220>
<223> EEF1A2 siRNA sense strand

<400> 860
ccaucgacau cucccagac                                                    19

<210> 861
<211> 19
<212> RNA
<213> Artificial Sequence

<220>
<223> EEF1A2 siRNA sense strand

<400> 861
ccaucgacau cucccucuc                                                    19

<210> 862
<211> 19
<212> RNA
<213> Artificial Sequence

<220>
<223> EEF1A2 siRNA sense strand

<400> 862
ccaucgacau cucccucac                                                    19

<210> 863
<211> 19
<212> RNA
<213> Artificial Sequence

<220>
<223> EEF1A2 siRNA sense strand

<400> 863
ccaucgacau cucccugac                                                    19

<210> 864
<211> 19
<212> RNA
<213> Artificial Sequence

<220>
<223> EEF1A2 siRNA sense strand

<400> 864
ccaucgacau cucccagac                                                    19

<210> 865
<211> 19
<212> RNA
<213> Artificial Sequence

<220>
<223> EEF1A2 siRNA sense strand

<400> 865
caucgacauc ucccucugc                                                    19

<210> 866
<211> 19
<212> RNA
<213> Artificial Sequence

<220>
<223> EEF1A2 siRNA sense strand

<400> 866
caucgacauc ucccucucc                                                    19

<210> 867
<211> 19
<212> RNA
<213> Artificial Sequence

<220>
<223> EEF1A2 siRNA sense strand

<400> 867
caucgacauc ucccucacc                                                    19

<210> 868
<211> 19
<212> RNA
<213> Artificial Sequence

<220>
<223> EEF1A2 siRNA sense strand

<400> 868
caucgacauc ucccugacc                                                    19

<210> 869
<211> 19
<212> RNA
<213> Artificial Sequence

<220>
<223> EEF1A2 siRNA sense strand

<400> 869
caucgacauc ucccucugc                                                    19

<210> 870
<211> 19
<212> RNA
<213> Artificial Sequence

<220>
<223> EEF1A2 siRNA sense strand

<400> 870
caucgacauc ucccucucc                                                    19

<210> 871
<211> 19
<212> RNA
<213> Artificial Sequence

<220>
<223> EEF1A2 siRNA sense strand

<400> 871
caucgacauc ucccucacc                                                    19

<210> 872
<211> 19
<212> RNA
<213> Artificial Sequence

<220>
<223> EEF1A2 siRNA sense strand

<400> 872
caucgacauc ucccugacc                                                    19

<210> 873
<211> 19
<212> RNA
<213> Artificial Sequence

<220>

<223> EEF1A2 siRNA sense strand

<400> 873
aucgacaucu cccucuggu                                                    19


<210> 874
<211> 19
<212> RNA
<213> Artificial Sequence


<220>
<223> EEF1A2 siRNA sense strand

<400> 874
aucgacaucu cccucugcu                                                    19


<210> 875
<211> 19
<212> RNA
<213> Artificial Sequence


<220>
<223> EEF1A2 siRNA sense strand

<400> 875
aucgacaucu cccucuccu                                                    19


<210> 876
<211> 19
<212> RNA
<213> Artificial Sequence


<220>
<223> EEF1A2 siRNA sense strand

<400> 876
aucgacaucu cccucaccu                                                    19


<210> 877
<211> 19
<212> RNA
<213> Artificial Sequence


<220>
<223> EEF1A2 siRNA sense strand

<400> 877
aucgacaucu cccucuggu                                                    19


<210> 878
<211> 19
<212> RNA
<213> Artificial Sequence

<220>
<223> EEF1A2 siRNA sense strand

<400> 878
aucgacaucu cccucugcu                                                    19


<210> 879
<211> 19
<212> RNA
<213> Artificial Sequence


<220>
<223> EEF1A2 siRNA sense strand

<400> 879
aucgacaucu cccucuccu                                                    19


<210> 880
<211> 19
<212> RNA
<213> Artificial Sequence


<220>
<223> EEF1A2 siRNA sense strand

<400> 880
aucgacaucu cccucaccu                                                    19


<210> 881
<211> 19
<212> RNA
<213> Artificial Sequence


<220>
<223> EEF1A2 siRNA sense strand

<400> 881
ucgacaucuc ccucuggau                                                    19


<210> 882
<211> 19
<212> RNA
<213> Artificial Sequence


<220>
<223> EEF1A2 siRNA sense strand

<400> 882
ucgacaucuc ccucugguu                                                    19


<210> 883
<211> 19
<212> RNA

<213> Artificial Sequence

<220>
<223> EEF1A2 siRNA sense strand

<400> 883
ucgacaucuc ccucugcuu                                                    19

<210> 884
<211> 19
<212> RNA
<213> Artificial Sequence

<220>
<223> EEF1A2 siRNA sense strand

<400> 884
ucgacaucuc ccucuccuu                                                    19

<210> 885
<211> 19
<212> RNA
<213> Artificial Sequence

<220>
<223> EEF1A2 siRNA sense strand

<400> 885
ucgacaucuc ccucuggau                                                    19

<210> 886
<211> 19
<212> RNA
<213> Artificial Sequence

<220>
<223> EEF1A2 siRNA sense strand

<400> 886
ucgacaucuc ccucugguu                                                    19

<210> 887
<211> 19
<212> RNA
<213> Artificial Sequence

<220>
<223> EEF1A2 siRNA sense strand

<400> 887
ucgacaucuc ccucugcuu                                                    19

<210> 888

<211> 19
<212> RNA
<213> Artificial Sequence

<220>
<223> EEF1A2 siRNA sense strand

<400> 888
ucgacaucuc ccucuccuu                                                    19

<210> 889
<211> 19
<212> RNA
<213> Artificial Sequence

<220>
<223> EEF1A2 siRNA sense strand

<400> 889
cgacaucucc cucuggaac                                                    19

<210> 890
<211> 19
<212> RNA
<213> Artificial Sequence

<220>
<223> EEF1A2 siRNA sense strand

<400> 890
cgacaucucc cucuggauc                                                    19

<210> 891
<211> 19
<212> RNA
<213> Artificial Sequence

<220>
<223> EEF1A2 siRNA sense strand

<400> 891
cgacaucucc cucugguuc                                                    19

<210> 892
<211> 19
<212> RNA
<213> Artificial Sequence

<220>
<223> EEF1A2 siRNA sense strand

<400> 892
cgacaucucc cucugcuuc                                                    19

<210> 893
<211> 19
<212> RNA
<213> Artificial Sequence

<220>
<223> EEF1A2 siRNA sense strand

<400> 893
cgacaucucc cucuggaac 19

<210> 894
<211> 19
<212> RNA
<213> Artificial Sequence

<220>
<223> EEF1A2 siRNA sense strand

<400> 894
cgacaucucc cucuggauc 19

<210> 895
<211> 19
<212> RNA
<213> Artificial Sequence

<220>
<223> EEF1A2 siRNA sense strand

<400> 895
cgacaucucc cucugguuc 19

<210> 896
<211> 19
<212> RNA
<213> Artificial Sequence

<220>
<223> EEF1A2 siRNA sense strand

<400> 896
cgacaucucc cucugcuuc 19

<210> 897
<211> 19
<212> RNA
<213> Artificial Sequence

<220>
<223> EEF1A2 siRNA sense strand

<400> 897

gacaucuccc ucuggaaga                                                    19

<210> 898
<211> 19
<212> RNA
<213> Artificial Sequence

<220>
<223> EEF1A2 siRNA sense strand

<400> 898
gacaucuccc ucuggaaca                                                    19

<210> 899
<211> 19
<212> RNA
<213> Artificial Sequence

<220>
<223> EEF1A2 siRNA sense strand

<400> 899
gacaucuccc ucuggauca                                                    19

<210> 900
<211> 19
<212> RNA
<213> Artificial Sequence

<220>
<223> EEF1A2 siRNA sense strand

<400> 900
gacaucuccc ucugguuca                                                    19

<210> 901
<211> 19
<212> RNA
<213> Artificial Sequence

<220>
<223> EEF1A2 siRNA sense strand

<400> 901
gacaucuccc ucuggaaga                                                    19

<210> 902
<211> 19
<212> RNA
<213> Artificial Sequence

<220>
<223> EEF1A2 siRNA sense strand

<400> 902
gacaucuccc ucuggaaca                                                    19


<210> 903
<211> 19
<212> RNA
<213> Artificial Sequence


<220>
<223> EEF1A2 siRNA sense strand

<400> 903
gacaucuccc ucuggauca                                                    19


<210> 904
<211> 19
<212> RNA
<213> Artificial Sequence


<220>
<223> EEF1A2 siRNA sense strand

<400> 904
gacaucuccc ucugguuca                                                    19


<210> 905
<211> 19
<212> RNA
<213> Artificial Sequence


<220>
<223> EEF1A2 siRNA sense strand

<400> 905
acaucucccu cuggaagua                                                    19


<210> 906
<211> 19
<212> RNA
<213> Artificial Sequence


<220>
<223> EEF1A2 siRNA sense strand

<400> 906
acaucucccu cuggaagaa                                                    19


<210> 907
<211> 19
<212> RNA
<213> Artificial Sequence

<220>
<223> EEF1A2 siRNA sense strand

<400> 907
acaucucccu cuggaacaa                                                    19


<210> 908
<211> 19
<212> RNA
<213> Artificial Sequence


<220>
<223> EEF1A2 siRNA sense strand

<400> 908
acaucucccu cuggaucaa                                                    19


<210> 909
<211> 19
<212> RNA
<213> Artificial Sequence


<220>
<223> EEF1A2 siRNA sense strand

<400> 909
acaucucccu cuggaagua                                                    19


<210> 910
<211> 19
<212> RNA
<213> Artificial Sequence


<220>
<223> EEF1A2 siRNA sense strand

<400> 910
acaucucccu cuggaagaa                                                    19


<210> 911
<211> 19
<212> RNA
<213> Artificial Sequence


<220>
<223> EEF1A2 siRNA sense strand

<400> 911
acaucucccu cuggaacaa                                                    19


<210> 912
<211> 19
<212> RNA
<213> Artificial Sequence

<220>
<223> EEF1A2 siRNA sense strand

<400> 912
acaucucccu cuggaucaa                                                    19


<210> 913
<211> 19
<212> RNA
<213> Artificial Sequence


<220>
<223> EEF1A2 siRNA sense strand

<400> 913
caucucccuc uggaaguug                                                    19


<210> 914
<211> 19
<212> RNA
<213> Artificial Sequence


<220>
<223> EEF1A2 siRNA sense strand

<400> 914
caucucccuc uggaaguag                                                    19


<210> 915
<211> 19
<212> RNA
<213> Artificial Sequence


<220>
<223> EEF1A2 siRNA sense strand

<400> 915
caucucccuc uggaagaag                                                    19


<210> 916
<211> 19
<212> RNA
<213> Artificial Sequence


<220>
<223> EEF1A2 siRNA sense strand

<400> 916
caucucccuc uggaacaag                                                    19


<210> 917
<211> 19

<212> RNA
<213> Artificial Sequence

<220>
<223> EEF1A2 siRNA sense strand

<400> 917
caucucccuc uggaaguug                                                    19

<210> 918
<211> 19
<212> RNA
<213> Artificial Sequence

<220>
<223> EEF1A2 siRNA sense strand

<400> 918
caucucccuc uggaaguag                                                    19

<210> 919
<211> 19
<212> RNA
<213> Artificial Sequence

<220>
<223> EEF1A2 siRNA sense strand

<400> 919
caucucccuc uggaagaag                                                    19

<210> 920
<211> 19
<212> RNA
<213> Artificial Sequence

<220>
<223> EEF1A2 siRNA sense strand

<400> 920
caucucccuc uggaacaag                                                    19

<210> 921
<211> 20
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<400> 921
ccaccaagua cuacaucacn                                                    20

<210> 922
<211> 21
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 922
ccaccaagua cuacaucacn n                                                  21

<210> 923
<211> 21
<212> RNA
<213> Artificial Sequence

<220>
<223> EEF1A2 siRNA sense strand

<400> 923
ccaccaagua cuacaucacu u                                                  21

<210> 924
<211> 20
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<400> 924
gcgccuacau caagaagaun                                                    20

<210> 925
<211> 21
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 925
gcgccuacau caagaagaun n                                                21


<210> 926
<211> 21
<212> RNA
<213> Artificial Sequence


<220>
<223> EEF1A2 siRNA sense strand

<400> 926
gcgccuacau caagaagauu u                                                21


<210> 927
<211> 20
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<400> 927
accaccaagu acuacaucan                                                  20


<210> 928
<211> 21
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 928
accaccaagu acuacaucan n                                                21


<210> 929
<211> 21
<212> RNA
<213> Artificial Sequence


<220>
<223> EEF1A2 siRNA sense strand

<400> 929
accaccaagu acuacaucau u                                                21


<210> 930
<211> 20
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<400> 930
cgccuacauc aagaagaucn                                                  20


<210> 931
<211> 21
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 931
cgccuacauc aagaagaucn n                                                21

<210> 932
<211> 21
<212> RNA
<213> Artificial Sequence

<220>
<223> EEF1A2 siRNA sense strand

<400> 932
cgccuacauc aagaagaucu u                                                21

<210> 933
<211> 20
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<400> 933
gaccaccaag uacuacaucn                                                  20

<210> 934
<211> 20
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<400> 934
cagcgccuac aucaagaagn                                                  20

<210> 935
<211> 20
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<400> 935
agcgccuaca ucaagaagan                                                20

<210> 936
<211> 21
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 936
agcgccuaca ucaagaagan n                                              21

<210> 937
<211> 21
<212> RNA
<213> Artificial Sequence

<220>
<223> EEF1A2 siRNA sense strand

<400> 937
agcgccuaca ucaagaagau u                                              21

<210> 938
<211> 20
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<400> 938
caagaagauc ggcuacaacn                                                20

<210> 939
<211> 21
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 939
caagaagauc ggcuacaacn n                                                21


<210> 940
<211> 21
<212> RNA
<213> Artificial Sequence


<220>
<223> EEF1A2 siRNA sense strand

<400> 940
caagaagauc ggcuacaacu u                                                21


<210> 941
<211> 20
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<400> 941
aagaagaucg gcuacaaccn                                                  20


<210> 942
<211> 21
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 942
aagaagaucg gcuacaaccn n                                                21


<210> 943
<211> 21
<212> RNA
<213> Artificial Sequence


<220>
<223> EEF1A2 siRNA sense strand

<400> 943
aagaagaucg gcuacaaccu u                                                21


<210> 944
<211> 20
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<400> 944
gccuacauca agaagaucgn                                                  20


<210> 945
<211> 21
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 945
gccuacauca agaagaucgn n                                                21

<210> 946
<211> 21
<212> RNA
<213> Artificial Sequence

<220>
<223> EEF1A2 siRNA sense strand

<400> 946
gccuacauca agaagaucgu u                                                21

<210> 947
<211> 20
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<400> 947
agaagaucgg cuacaacccn                                                  20

<210> 948
<211> 20
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<400> 948
ucaagaagau cggcuacaan                                                  20

<210> 949
<211> 21
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 949
ucaagaagau cggcuacaan n                                                21


<210> 950
<211> 21
<212> RNA
<213> Artificial Sequence


<220>
<223> EEF1A2 siRNA sense strand

<400> 950
ucaagaagau cggcuacaau u                                                21


<210> 951
<211> 20
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<400> 951
aucaagaaga ucggcuacan                                                  20


<210> 952
<211> 21
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 952
aucaagaaga ucggcuacan n                                                21

<210> 953
<211> 21
<212> RNA
<213> Artificial Sequence

<220>
<223> EEF1A2 siRNA sense strand

<400> 953
aucaagaaga ucggcuacau u                                                21

<210> 954
<211> 20
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<400> 954
caucaagaag aucggcuacn                                                  20

<210> 955
<211> 21
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 955
caucaagaag aucggcuacn n                                                21

<210> 956
<211> 21
<212> RNA
<213> Artificial Sequence

<220>
<223> EEF1A2 siRNA sense strand

<400> 956
caucaagaag aucggcuacu u                                                21

<210> 957
<211> 20
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<400> 957
caccaaguac uacaucaccn                                                  20

<210> 958
<211> 21
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 958
caccaaguac uacaucaccn n                                                21

<210> 959
<211> 21
<212> RNA
<213> Artificial Sequence

<220>
<223> EEF1A2 siRNA sense strand

<400> 959
caccaaguac uacaucaccu u                                                21

<210> 960
<211> 20
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<400> 960
accaaguacu acaucaccan                                                    20


<210> 961
<211> 21
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 961
accaaguacu acaucaccan n                                                  21


<210> 962
<211> 21
<212> RNA
<213> Artificial Sequence


<220>
<223> EEF1A2 siRNA sense strand

<400> 962
accaaguacu acaucaccau u                                                  21


<210> 963
<211> 20
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<400> 963
ccaaguacua caucaccaun                                                20

<210> 964
<211> 21
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 964
ccaaguacua caucaccaun n                                              21

<210> 965
<211> 21
<212> RNA
<213> Artificial Sequence

<220>
<223> EEF1A2 siRNA sense strand

<400> 965
ccaaguacua caucaccauu u                                              21

<210> 966
<211> 20
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<400> 966
caaguacuac aucaccaucn                                                20

<210> 967
<211> 21
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 967
caaguacuac aucaccaucn n                                                21


<210> 968
<211> 21
<212> RNA
<213> Artificial Sequence


<220>
<223> EEF1A2 siRNA sense strand

<400> 968
caaguacuac aucaccaucu u                                                21


<210> 969
<211> 20
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<400> 969
aaguacuaca ucaccaucan                                                  20


<210> 970
<211> 21
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 970
aaguacuaca ucaccaucan n                                                21


<210> 971
<211> 21
<212> RNA
<213> Artificial Sequence


<220>
<223> EEF1A2 siRNA sense strand

<400> 971
aaguacuaca ucaccaucau u                                                21


<210> 972
<211> 20
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<400> 972
aguacuacau caccaucaun                                                  20


<210> 973
<211> 21
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 973
aguacuacau caccaucaun n                                                21

<210> 974
<211> 21
<212> RNA
<213> Artificial Sequence

<220>
<223> EEF1A2 siRNA sense strand

<400> 974
aguacuacau caccaucauu u                                                21

<210> 975
<211> 20
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<400> 975
guacuacauc accaucaucn                                                  20

<210> 976
<211> 21
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 976
guacuacauc accaucaucn n                                                21

<210> 977
<211> 21
<212> RNA
<213> Artificial Sequence

<220>
<223> EEF1A2 siRNA sense strand

<400> 977
guacuacauc accaucaucu u                                                21

<210> 978
<211> 20
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<400> 978
uacuacauca ccaucaucgn                                                  20

<210> 979
<211> 21
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 979
uacuacauca ccaucaucgn n                                                21

<210> 980
<211> 21
<212> RNA
<213> Artificial Sequence

<220>
<223> EEF1A2 siRNA sense strand

<400> 980
uacuacauca ccaucaucgu u                                                21

<210> 981
<211> 20
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<400> 981
acaucaagaa gaucggcuan                                                    20


<210> 982
<211> 21
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 982
acaucaagaa gaucggcuan n                                                  21


<210> 983
<211> 21
<212> RNA
<213> Artificial Sequence


<220>
<223> EEF1A2 siRNA sense strand

<400> 983
acaucaagaa gaucggcuau u                                                  21


<210> 984
<211> 20
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<400> 984
ccuacaucaa gaagaucggn                                                    20

<210> 985
<211> 21
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 985
ccuacaucaa gaagaucggn n                                                  21

<210> 986
<211> 21
<212> RNA
<213> Artificial Sequence

<220>
<223> EEF1A2 siRNA sense strand

<400> 986
ccuacaucaa gaagaucggu u                                                  21

<210> 987
<211> 20
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<400> 987
cuacaucaag aagaucggcn                                                    20

<210> 988
<211> 21
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 988
cuacaucaag aagaucggcn n                                                21


<210> 989
<211> 21
<212> RNA
<213> Artificial Sequence


<220>
<223> EEF1A2 siRNA sense strand

<400> 989
cuacaucaag aagaucggcu u                                                21


<210> 990
<211> 20
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<400> 990
uacaucaaga agaucggcun                                                  20


<210> 991
<211> 21
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 991
uacaucaaga agaucggcun n                                                21


<210> 992
<211> 21
<212> RNA
<213> Artificial Sequence


<220>
<223> EEF1A2 siRNA sense strand

<400> 992
uacaucaaga agaucggcuu u                                                21


<210> 993
<211> 20
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<400> 993
caucaccauc gacaucuccn                                                  20


<210> 994
<211> 20
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<400> 994
aucaccaucg acaucucccn                                                  20


<210> 995
<211> 21
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 995
aucaccaucg acaucucccn n                                                21


<210> 996
<211> 21
<212> RNA
<213> Artificial Sequence


<220>
<223> EEF1A2 siRNA sense strand

<400> 996
aucaccaucg acaucucccu u                                                21


<210> 997
<211> 20
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<400> 997
ucaccaucga caucucccun                                                  20


<210> 998
<211> 21
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 998
ucaccaucga caucucccun n                                                21


<210> 999
<211> 21
<212> RNA
<213> Artificial Sequence


<220>
<223> EEF1A2 siRNA sense strand

<400> 999
ucaccaucga caucucccuu u                                                21


<210> 1000
<211> 20
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<400> 1000
caccaucgac aucucccucn                                                  20


<210> 1001
<211> 21
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 1001
caccaucgac aucucccucn n                                                21

<210> 1002
<211> 21
<212> RNA
<213> Artificial Sequence

<220>
<223> EEF1A2 siRNA sense strand

<400> 1002
caccaucgac aucucccucu u                                                21

<210> 1003
<211> 20
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<400> 1003
accaucgaca ucucccucun                                                  20

<210> 1004
<211> 21
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 1004
accaucgaca ucucccucun n                                                21

<210> 1005
<211> 21
<212> RNA
<213> Artificial Sequence

<220>
<223> EEF1A2 siRNA sense strand

<400> 1005
accaucgaca ucucccucuu u                                                21

<210> 1006
<211> 20
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<400> 1006
ccaucgacau cucccucugn                                                  20

<210> 1007
<211> 21
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 1007
ccaucgacau cucccucugn n                                                21

<210> 1008
<211> 21
<212> RNA
<213> Artificial Sequence

<220>
<223> EEF1A2 siRNA sense strand

<400> 1008
ccaucgacau cucccucugu u                                                21

<210> 1009
<211> 20
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<400> 1009
caucgacauc ucccucuggn                                                    20


<210> 1010
<211> 21
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 1010
caucgacauc ucccucuggn n                                                  21


<210> 1011
<211> 21
<212> RNA
<213> Artificial Sequence


<220>
<223> EEF1A2 siRNA sense strand

<400> 1011
caucgacauc ucccucuggu u                                                  21


<210> 1012
<211> 20
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<400> 1012
aucgacaucu cccucuggan                                                    20


<210> 1013
<211> 21
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 1013
aucgacaucu cccucuggan n                                                  21


<210> 1014
<211> 21
<212> RNA
<213> Artificial Sequence


<220>
<223> EEF1A2 siRNA sense strand

<400> 1014
aucgacaucu cccucuggau u                                                  21


<210> 1015
<211> 20
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<400> 1015
ucgacaucuc ccucuggaan                                                    20


<210> 1016
<211> 21
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 1016
ucgacaucuc ccucuggaan n                                                21


<210> 1017
<211> 21
<212> RNA
<213> Artificial Sequence


<220>
<223> EEF1A2 siRNA sense strand

<400> 1017
ucgacaucuc ccucuggaau u                                                21


<210> 1018
<211> 20
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<400> 1018
cgacaucucc cucuggaagn                                                  20


<210> 1019
<211> 21
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 1019
cgacaucucc cucuggaagn n                                                21


<210> 1020
<211> 21
<212> RNA
<213> Artificial Sequence


<220>
<223> EEF1A2 siRNA sense strand

<400> 1020
cgacaucucc cucuggaagu u                                                21


<210> 1021
<211> 20
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<400> 1021
gacaucuccc ucuggaagun                                                  20


<210> 1022
<211> 21
<212> RNA
<213> Artificial Sequence


<220>
<223> EEF1A2 siRNA sense strand

<220>
<221> misc_feature
<222> 20
<223> /note="n is dT (deoxythymine or 2'H thymine)"

<220>
<221> misc_feature
<222> 21
<223> /note="n is dT (deoxythymine or 2'H thymine)"

<400> 1022
gacaucuccc ucuggaagun n                                                21

<210> 1023
<211> 21
<212> RNA
<213> Artificial Sequence

<220>
<223> EEF1A2 siRNA sense strand

<400> 1023
gacaucuccc ucuggaaguu u                                                21

<210> 1024
<211> 20
<212> RNA
<213> Artificial Sequence

<220>
<223> EEF1A2 siRNA sense strand

<220>
<221> misc_feature
<222> 20
<223> /note="n is dT (deoxythymine or 2'H thymine)"

<400> 1024
acaucucccu cuggaaguun                                                  20

<210> 1025
<211> 20
<212> RNA
<213> Artificial Sequence

<220>
<223> EEF1A2 siRNA sense strand

<400> 1025
ccaccaagua cuacaucacc                                                  20

<210> 1026
<211> 21
<212> RNA
<213> Artificial Sequence

<220>
<223> EEF1A2 siRNA sense strand

<400> 1026
ccaccaagua cuacaucacc a                                                21

<210> 1027
<211> 20
<212> RNA
<213> Artificial Sequence

<220>
<223> EEF1A2 siRNA sense strand

<400> 1027
gcgccuacau caagaagauc                                                    20


<210> 1028
<211> 21
<212> RNA
<213> Artificial Sequence


<220>
<223> EEF1A2 siRNA sense strand

<400> 1028
gcgccuacau caagaagauc g                                                  21


<210> 1029
<211> 20
<212> RNA
<213> Artificial Sequence


<220>
<223> EEF1A2 siRNA sense strand

<400> 1029
accaccaagu acuacaucac                                                    20


<210> 1030
<211> 21
<212> RNA
<213> Artificial Sequence


<220>
<223> EEF1A2 siRNA sense strand

<400> 1030
accaccaagu acuacaucac c                                                  21


<210> 1031
<211> 20
<212> RNA
<213> Artificial Sequence


<220>
<223> EEF1A2 siRNA sense strand

<400> 1031
cgccuacauc aagaagaucg                                                    20


<210> 1032
<211> 21
<212> RNA

<213> Artificial Sequence

<220>
<223> EEF1A2 siRNA sense strand

<400> 1032
cgccuacauc aagaagaucg g                                                21

<210> 1033
<211> 20
<212> RNA
<213> Artificial Sequence

<220>
<223> EEF1A2 siRNA sense strand

<400> 1033
gaccaccaag uacuacauca                                                  20

<210> 1034
<211> 20
<212> RNA
<213> Artificial Sequence

<220>
<223> EEF1A2 siRNA sense strand

<400> 1034
cagcgccuac aucaagaaga                                                  20

<210> 1035
<211> 20
<212> RNA
<213> Artificial Sequence

<220>
<223> EEF1A2 siRNA sense strand

<400> 1035
agcgccuaca ucaagaagau                                                  20

<210> 1036
<211> 21
<212> RNA
<213> Artificial Sequence

<220>
<223> EEF1A2 siRNA sense strand

<400> 1036
agcgccuaca ucaagaagau c                                                21

<210> 1037

<211> 20
<212> RNA
<213> Artificial Sequence

<220>
<223> EEF1A2 siRNA sense strand

<400> 1037
caagaagauc ggcuacaacc                                                    20

<210> 1038
<211> 21
<212> RNA
<213> Artificial Sequence

<220>
<223> EEF1A2 siRNA sense strand

<400> 1038
caagaagauc ggcuacaacc c                                                  21

<210> 1039
<211> 20
<212> RNA
<213> Artificial Sequence

<220>
<223> EEF1A2 siRNA sense strand

<400> 1039
aagaagaucg gcuacaaccc                                                    20

<210> 1040
<211> 21
<212> RNA
<213> Artificial Sequence

<220>
<223> EEF1A2 siRNA sense strand

<400> 1040
aagaagaucg gcuacaaccc g                                                  21

<210> 1041
<211> 20
<212> RNA
<213> Artificial Sequence

<220>
<223> EEF1A2 siRNA sense strand

<400> 1041
gccuacauca agaagaucgg                                                    20

<210> 1042
<211> 21
<212> RNA
<213> Artificial Sequence

<220>
<223> EEF1A2 siRNA sense strand

<400> 1042
gccuacauca agaagaucgg c                                                21

<210> 1043
<211> 20
<212> RNA
<213> Artificial Sequence

<220>
<223> EEF1A2 siRNA sense strand

<400> 1043
agaagaucgg cuacaacccg                                                  20

<210> 1044
<211> 20
<212> RNA
<213> Artificial Sequence

<220>
<223> EEF1A2 siRNA sense strand

<400> 1044
ucaagaagau cggcuacaac                                                  20

<210> 1045
<211> 21
<212> RNA
<213> Artificial Sequence

<220>
<223> EEF1A2 siRNA sense strand

<400> 1045
ucaagaagau cggcuacaac c                                                21

<210> 1046
<211> 20
<212> RNA
<213> Artificial Sequence

<220>
<223> EEF1A2 siRNA sense strand

<400> 1046

aucaagaaga ucggcuacaa                                                    20

<210> 1047
<211> 21
<212> RNA
<213> Artificial Sequence

<220>
<223> EEF1A2 siRNA sense strand

<400> 1047
aucaagaaga ucggcuacaa c                                                  21

<210> 1048
<211> 20
<212> RNA
<213> Artificial Sequence

<220>
<223> EEF1A2 siRNA sense strand

<400> 1048
caucaagaag aucggcuaca                                                    20

<210> 1049
<211> 21
<212> RNA
<213> Artificial Sequence

<220>
<223> EEF1A2 siRNA sense strand

<400> 1049
caucaagaag aucggcuaca a                                                  21

<210> 1050
<211> 20
<212> RNA
<213> Artificial Sequence

<220>
<223> EEF1A2 siRNA sense strand

<400> 1050
caccaaguac uacaucacca                                                    20

<210> 1051
<211> 21
<212> RNA
<213> Artificial Sequence

<220>
<223> EEF1A2 siRNA sense strand

<400> 1051
caccaaguac uacaucacca u                                                21

<210> 1052
<211> 20
<212> RNA
<213> Artificial Sequence

<220>
<223> EEF1A2 siRNA sense strand

<400> 1052
accaaguacu acaucaccau                                                  20

<210> 1053
<211> 21
<212> RNA
<213> Artificial Sequence

<220>
<223> EEF1A2 siRNA sense strand

<400> 1053
accaaguacu acaucaccau c                                                21

<210> 1054
<211> 20
<212> RNA
<213> Artificial Sequence

<220>
<223> EEF1A2 siRNA sense strand

<400> 1054
ccaaguacua caucaccauc                                                  20

<210> 1055
<211> 21
<212> RNA
<213> Artificial Sequence

<220>
<223> EEF1A2 siRNA sense strand

<400> 1055
ccaaguacua caucaccauc a                                                21

<210> 1056
<211> 20
<212> RNA
<213> Artificial Sequence

<220>
<223> EEF1A2 siRNA sense strand

<400> 1056
caaguacuac aucaccauca                                                    20


<210> 1057
<211> 21
<212> RNA
<213> Artificial Sequence


<220>
<223> EEF1A2 siRNA sense strand

<400> 1057
caaguacuac aucaccauca u                                                  21


<210> 1058
<211> 20
<212> RNA
<213> Artificial Sequence


<220>
<223> EEF1A2 siRNA sense strand

<400> 1058
aaguacuaca ucaccaucau                                                    20


<210> 1059
<211> 21
<212> RNA
<213> Artificial Sequence


<220>
<223> EEF1A2 siRNA sense strand

<400> 1059
aaguacuaca ucaccaucau c                                                  21


<210> 1060
<211> 20
<212> RNA
<213> Artificial Sequence


<220>
<223> EEF1A2 siRNA sense strand

<400> 1060
aguacuacau caccaucauc                                                    20


<210> 1061
<211> 21
<212> RNA
<213> Artificial Sequence

<220>
<223> EEF1A2 siRNA sense strand

<400> 1061
aguacuacau caccaucauc g                                                21


<210> 1062
<211> 20
<212> RNA
<213> Artificial Sequence


<220>
<223> EEF1A2 siRNA sense strand

<400> 1062
guacuacauc accaucaucg                                                  20


<210> 1063
<211> 21
<212> RNA
<213> Artificial Sequence


<220>
<223> EEF1A2 siRNA sense strand

<400> 1063
guacuacauc accaucaucg a                                                21


<210> 1064
<211> 20
<212> RNA
<213> Artificial Sequence


<220>
<223> EEF1A2 siRNA sense strand

<400> 1064
uacuacauca ccaucaucga                                                  20


<210> 1065
<211> 21
<212> RNA
<213> Artificial Sequence


<220>
<223> EEF1A2 siRNA sense strand

<400> 1065
uacuacauca ccaucaucga c                                                21


<210> 1066
<211> 20

<212> RNA
<213> Artificial Sequence

<220>
<223> EEF1A2 siRNA sense strand

<400> 1066
acaucaagaa gaucggcuac                                                   20

<210> 1067
<211> 21
<212> RNA
<213> Artificial Sequence

<220>
<223> EEF1A2 siRNA sense strand

<400> 1067
acaucaagaa gaucggcuac a                                                 21

<210> 1068
<211> 20
<212> RNA
<213> Artificial Sequence

<220>
<223> EEF1A2 siRNA sense strand

<400> 1068
ccuacaucaa gaagaucggc                                                   20

<210> 1069
<211> 21
<212> RNA
<213> Artificial Sequence

<220>
<223> EEF1A2 siRNA sense strand

<400> 1069
ccuacaucaa gaagaucggc u                                                 21

<210> 1070
<211> 20
<212> RNA
<213> Artificial Sequence

<220>
<223> EEF1A2 siRNA sense strand

<400> 1070
cuacaucaag aagaucggcu                                                   20

<210> 1071
<211> 21
<212> RNA
<213> Artificial Sequence

<220>
<223> EEF1A2 siRNA sense strand

<400> 1071
cuacaucaag aagaucggcu a                                                21

<210> 1072
<211> 20
<212> RNA
<213> Artificial Sequence

<220>
<223> EEF1A2 siRNA sense strand

<400> 1072
uacaucaaga agaucggcua                                                  20

<210> 1073
<211> 21
<212> RNA
<213> Artificial Sequence

<220>
<223> EEF1A2 siRNA sense strand

<400> 1073
uacaucaaga agaucggcua c                                                21

<210> 1074
<211> 20
<212> RNA
<213> Artificial Sequence

<220>
<223> EEF1A2 siRNA sense strand

<400> 1074
caucaccauc gacaucuccc                                                  20

<210> 1075
<211> 20
<212> RNA
<213> Artificial Sequence

<220>
<223> EEF1A2 siRNA sense strand

<400> 1075
aucaccaucg acaucucccu                                                  20

<210> 1076
<211> 21
<212> RNA
<213> Artificial Sequence

<220>
<223> EEF1A2 siRNA sense strand

<400> 1076
aucaccaucg acaucucccu c                                                21

<210> 1077
<211> 20
<212> RNA
<213> Artificial Sequence

<220>
<223> EEF1A2 siRNA sense strand

<400> 1077
ucaccaucga caucucccuc                                                  20

<210> 1078
<211> 21
<212> RNA
<213> Artificial Sequence

<220>
<223> EEF1A2 siRNA sense strand

<400> 1078
ucaccaucga caucucccuc u                                                21

<210> 1079
<211> 20
<212> RNA
<213> Artificial Sequence

<220>
<223> EEF1A2 siRNA sense strand

<400> 1079
caccaucgac aucucccucu                                                  20

<210> 1080
<211> 21
<212> RNA
<213> Artificial Sequence

<220>
<223> EEF1A2 siRNA sense strand

<400> 1080
caccaucgac aucucccucu g                                                21

<210> 1081
<211> 20
<212> RNA
<213> Artificial Sequence

<220>
<223> EEF1A2 siRNA sense strand

<400> 1081
accaucgaca ucucccucug                                                  20

<210> 1082
<211> 21
<212> RNA
<213> Artificial Sequence

<220>
<223> EEF1A2 siRNA sense strand

<400> 1082
accaucgaca ucucccucug g                                                21

<210> 1083
<211> 20
<212> RNA
<213> Artificial Sequence

<220>
<223> EEF1A2 siRNA sense strand

<400> 1083
ccaucgacau cucccucugg                                                  20

<210> 1084
<211> 21
<212> RNA
<213> Artificial Sequence

<220>
<223> EEF1A2 siRNA sense strand

<400> 1084
ccaucgacau cucccucugg a                                                21

<210> 1085
<211> 20
<212> RNA
<213> Artificial Sequence

<220>

<223> EEF1A2 siRNA sense strand

<400> 1085
caucgacauc ucccucugga                                                    20


<210> 1086
<211> 21
<212> RNA
<213> Artificial Sequence


<220>
<223> EEF1A2 siRNA sense strand

<400> 1086
caucgacauc ucccucugga a                                                  21


<210> 1087
<211> 20
<212> RNA
<213> Artificial Sequence


<220>
<223> EEF1A2 siRNA sense strand

<400> 1087
aucgacaucu cccucuggaa                                                    20


<210> 1088
<211> 21
<212> RNA
<213> Artificial Sequence


<220>
<223> EEF1A2 siRNA sense strand

<400> 1088
aucgacaucu cccucuggaa g                                                  21


<210> 1089
<211> 20
<212> RNA
<213> Artificial Sequence


<220>
<223> EEF1A2 siRNA sense strand

<400> 1089
ucgacaucuc ccucuggaag                                                    20


<210> 1090
<211> 21
<212> RNA
<213> Artificial Sequence

<220>
<223> EEF1A2 siRNA sense strand

<400> 1090
ucgacaucuc ccucuggaag u                                                21


<210> 1091
<211> 20
<212> RNA
<213> Artificial Sequence


<220>
<223> EEF1A2 siRNA sense strand

<400> 1091
cgacaucucc cucuggaagu                                                  20


<210> 1092
<211> 21
<212> RNA
<213> Artificial Sequence


<220>
<223> EEF1A2 siRNA sense strand

<400> 1092
cgacaucucc cucuggaagu u                                                21


<210> 1093
<211> 20
<212> RNA
<213> Artificial Sequence


<220>
<223> EEF1A2 siRNA sense strand

<400> 1093
gacaucuccc ucuggaaguu                                                  20


<210> 1094
<211> 21
<212> RNA
<213> Artificial Sequence


<220>
<223> EEF1A2 siRNA sense strand

<400> 1094
gacaucuccc ucuggaaguu c                                                21


<210> 1095
<211> 20
<212> RNA

<213> Artificial Sequence

<220>
<223> EEF1A2 siRNA sense strand

<400> 1095
acaucucccu cuggaaguuc                                                    20

<210> 1096
<211> 19
<212> RNA
<213> Artificial Sequence

<220>
<223> Modified EEF1A2 siRNA antisense strand

<220>
<221> modified_base
<222> 7
<223> /mod_base=OTHER
      /note="2'-O-methyluridine"

<220>
<221> modified_base
<222> 10
<223> /mod_base=OTHER
      /note="2'-O-methyluridine"

<400> 1096
gugauguagu acuuggugg                                                     19

<210> 1097
<211> 19
<212> RNA
<213> Artificial Sequence

<220>
<223> Modified EEF1A2 siRNA antisense strand

<220>
<221> modified_base
<222> 1
<223> /mod_base=OTHER
      /note="2'-fluoroguanosine"

<220>
<221> modified_base
<222> 2
<223> /mod_base=OTHER
      /note="2'-fluorouridine"

<220>
<221> modified_base
<222> 3
<223> /mod_base=OTHER
      /note="2'-O-methylguanosine"

<220>

<221> modified_base
<222> 4
<223> /mod_base=OTHER
      /note="2'-fluoroadenosine"

<220>
<221> modified_base
<222> 5
<223> /mod_base=OTHER
      /note="2'-O-methyluridine"

<220>
<221> modified_base
<222> 6
<223> /mod_base=OTHER
      /note="2'-fluoroguanosine"

<220>
<221> modified_base
<222> 7
<223> /mod_base=OTHER
      /note="2'-O-methyluridine"

<220>
<221> modified_base
<222> 8
<223> /mod_base=OTHER
      /note="2'-fluoroadenosine"

<220>
<221> modified_base
<222> 9
<223> /mod_base=OTHER
      /note="2'-O-methylguanosine"

<220>
<221> modified_base
<222> 10
<223> /mod_base=OTHER
      /note="2'-fluorouridine"

<220>
<221> modified_base
<222> 11
<223> /mod_base=OTHER
      /note="2'-O-methyladenosine"

<220>
<221> modified_base
<222> 12
<223> /mod_base=OTHER
      /note="2'-fluorocytidine"

<220>
<221> modified_base
<222> 13
<223> /mod_base=OTHER
      /note="2'-O-methyluridine"

<220>
<221> modified_base
<222> 14
<223> /mod_base=OTHER

/note="2'-fluorouridine"

<220>
<221> modified_base
<222> 15
<223> /mod_base=OTHER
      /note="2'-O-methylguanosine"

<220>
<221> modified_base
<222> 16
<223> /mod_base=OTHER
      /note="2'-fluoroguanosine"

<220>
<221> modified_base
<222> 17
<223> /mod_base=OTHER
      /note="2'-O-methyluridine"

<220>
<221> modified_base
<222> 18
<223> /mod_base=OTHER
      /note="2'-fluoroguanosine"

<220>
<221> modified_base
<222> 19
<223> /mod_base=OTHER
      /note="2'-O-methylguanosine"

<400> 1097
gugauguagu acuuggugg                                                    19


<210> 1098
<211> 19
<212> RNA
<213> Artificial Sequence


<220>
<223> Modified EEF1A2 siRNA antisense strand

<220>
<221> modified_base
<222> 4
<223> /mod_base=OTHER
      /note="2'-O-methyluridine"

<220>
<221> modified_base
<222> 7
<223> /mod_base=OTHER
      /note="2'-O-methyluridine"

<400> 1098
auguaguacu ugguggucu                                                    19


<210> 1099
<211> 19

<212> RNA
<213> Artificial Sequence

<220>
<223> Modified EEF1A2 siRNA antisense strand

<220>
<221> modified_base
<222> 1
<223> /mod_base=OTHER
      /note="2'-fluoroadenosine"

<220>
<221> modified_base
<222> 2
<223> /mod_base=OTHER
      /note="2'-fluorouridine"

<220>
<221> modified_base
<222> 3
<223> /mod_base=OTHER
      /note="2'-O-methylguanosine"

<220>
<221> modified_base
<222> 4
<223> /mod_base=OTHER
      /note="2'-fluorouridine"

<220>
<221> modified_base
<222> 5
<223> /mod_base=OTHER
      /note="2'-O-methyladenosine"

<220>
<221> modified_base
<222> 6
<223> /mod_base=OTHER
      /note="2'-fluoroguanosine"

<220>
<221> modified_base
<222> 7
<223> /mod_base=OTHER
      /note="2'-O-methyluridine"

<220>
<221> modified_base
<222> 8
<223> /mod_base=OTHER
      /note="2'-fluoroadenosine"

<220>
<221> modified_base
<222> 9
<223> /mod_base=OTHER
      /note="2'-O-methylcytidine"

<220>
<221> modified_base

<222> 10
<223> /mod_base=OTHER
      /note="2'-fluorouridine"

<220>
<221> modified_base
<222> 11
<223> /mod_base=OTHER
      /note="2'-O-methyluridine"

<220>
<221> modified_base
<222> 12
<223> /mod_base=OTHER
      /note="2'-fluoroguanosine"

<220>
<221> modified_base
<222> 13
<223> /mod_base=OTHER
      /note="2'-O-methylguanosine"

<220>
<221> modified_base
<222> 14
<223> /mod_base=OTHER
      /note="2'-fluorouridine"

<220>
<221> modified_base
<222> 15
<223> /mod_base=OTHER
      /note="2'-O-methylguanosine"

<220>
<221> modified_base
<222> 16
<223> /mod_base=OTHER
      /note="2'-fluoroguanosine"

<220>
<221> modified_base
<222> 17
<223> /mod_base=OTHER
      /note="2'-O-methyluridine"

<220>
<221> modified_base
<222> 18
<223> /mod_base=OTHER
      /note="2'-fluorocytidine"

<220>
<221> modified_base
<222> 19
<223> /mod_base=OTHER
      /note="2'-O-methyluridine"

<400> 1099
auguaguacu uggugggucu                                                    19

<210> 1100

<211> 19
<212> RNA
<213> Artificial Sequence

<220>
<223> Modified EEF1A2 siRNA antisense strand

<220>
<221> modified_base
<222> 13
<223> /mod_base=OTHER
      /note="2'-O-methyluridine"

<400> 1100
aucuucuuga uguaggcgc                                                    19

<210> 1101
<211> 19
<212> RNA
<213> Artificial Sequence

<220>
<223> Modified EEF1A2 siRNA antisense strand

<220>
<221> modified_base
<222> 1
<223> /mod_base=OTHER
      /note="2'-fluoroadenosine"

<220>
<221> modified_base
<222> 2
<223> /mod_base=OTHER
      /note="2'-fluorouridine"

<220>
<221> modified_base
<222> 3
<223> /mod_base=OTHER
      /note="2'-O-methylcytidine"

<220>
<221> modified_base
<222> 4
<223> /mod_base=OTHER
      /note="2'-fluorouridine"

<220>
<221> modified_base
<222> 5
<223> /mod_base=OTHER
      /note="2'-O-methyluridine"

<220>
<221> modified_base
<222> 6
<223> /mod_base=OTHER
      /note="2'-fluorocytidine"

<220>
<221> modified_base
<222> 7
<223> /mod_base=OTHER
      /note="2'-O-methyluridine"

<220>
<221> modified_base
<222> 8
<223> /mod_base=OTHER
      /note="2'-fluorouridine"

<220>
<221> modified_base
<222> 9
<223> /mod_base=OTHER
      /note="2'-O-methylguanosine"

<220>
<221> modified_base
<222> 10
<223> /mod_base=OTHER
      /note="2'-fluoroadenosine"

<220>
<221> modified_base
<222> 11
<223> /mod_base=OTHER
      /note="2'-O-methyluridine"

<220>
<221> modified_base
<222> 12
<223> /mod_base=OTHER
      /note="2'-fluoroguanosine"

<220>
<221> modified_base
<222> 13
<223> /mod_base=OTHER
      /note="2'-O-methyluridine"

<220>
<221> modified_base
<222> 14
<223> /mod_base=OTHER
      /note="2'-fluoroadenosine"

<220>
<221> modified_base
<222> 15
<223> /mod_base=OTHER
      /note="2'-O-methylguanosine"

<220>
<221> modified_base
<222> 16
<223> /mod_base=OTHER
      /note="2'-fluoroguanosine"

<220>
<221> modified_base
<222> 17

<223> /mod_base=OTHER
      /note="2'-O-methylcytidine"

<220>
<221> modified_base
<222> 18
<223> /mod_base=OTHER
      /note="2'-fluoroguanosine"

<220>
<221> modified_base
<222> 19
<223> /mod_base=OTHER
      /note="2'-O-methylcytidine"

<400> 1101
aucuucuuga uguaggcgc                                                    19


<210> 1102
<211> 19
<212> RNA
<213> Artificial Sequence


<220>
<223> Modified EEF1A2 siRNA antisense strand

<220>
<221> modified_base
<222> 6
<223> /mod_base=OTHER
      /note="2'-O-methyluridine"

<220>
<221> modified_base
<222> 9
<223> /mod_base=OTHER
      /note="2'-O-methyluridine"

<400> 1102
ugauguagua cuugguggu                                                    19


<210> 1103
<211> 19
<212> RNA
<213> Artificial Sequence


<220>
<223> Modified EEF1A2 siRNA antisense strand

<220>
<221> modified_base
<222> 1
<223> /mod_base=OTHER
      /note="2'-fluorouridine"

<220>
<221> modified_base
<222> 2
<223> /mod_base=OTHER

/note="2'-fluoroguanosine"

<220>
<221> modified_base
<222> 3
<223> /mod_base=OTHER
/note="2'-O-methyladenosine"

<220>
<221> modified_base
<222> 4
<223> /mod_base=OTHER
/note="2'-fluorouridine"

<220>
<221> modified_base
<222> 5
<223> /mod_base=OTHER
/note="2'-O-methylguanosine"

<220>
<221> modified_base
<222> 6
<223> /mod_base=OTHER
/note="2'-fluorouridine"

<220>
<221> modified_base
<222> 7
<223> /mod_base=OTHER
/note="2'-O-methyladenosine"

<220>
<221> modified_base
<222> 8
<223> /mod_base=OTHER
/note="2'-fluoroguanosine"

<220>
<221> modified_base
<222> 9
<223> /mod_base=OTHER
/note="2'-O-methyluridine"

<220>
<221> modified_base
<222> 10
<223> /mod_base=OTHER
/note="2'-fluoroadenosine"

<220>
<221> modified_base
<222> 11
<223> /mod_base=OTHER
/note="2'-O-methylcytidine"

<220>
<221> modified_base
<222> 12
<223> /mod_base=OTHER
/note="2'-fluorouridine"

<220>

<221> modified_base
<222> 13
<223> /mod_base=OTHER
      /note="2'-O-methyluridine"

<220>
<221> modified_base
<222> 14
<223> /mod_base=OTHER
      /note="2'-fluoroguanosine"

<220>
<221> modified_base
<222> 15
<223> /mod_base=OTHER
      /note="2'-O-methylguanosine"

<220>
<221> modified_base
<222> 16
<223> /mod_base=OTHER
      /note="2'-fluorouridine"

<220>
<221> modified_base
<222> 17
<223> /mod_base=OTHER
      /note="2'-O-methylguanosine"

<220>
<221> modified_base
<222> 18
<223> /mod_base=OTHER
      /note="2'-fluoroguanosine"

<220>
<221> modified_base
<222> 19
<223> /mod_base=OTHER
      /note="2'-O-methyluridine"

<400> 1103
ugauguagua cuugguggu                                                    19


<210> 1104
<211> 19
<212> RNA
<213> Artificial Sequence


<220>
<223> Modified EEF1A2 siRNA antisense strand

<400> 1104
gagaugucga uggugaugc                                                    19


<210> 1105
<211> 19
<212> RNA
<213> Artificial Sequence

<220>
<223> Modified EEF1A2 siRNA antisense strand

<220>
<221> modified_base
<222> 1
<223> /mod_base=OTHER
      /note="2'-fluoroguanosine"

<220>
<221> modified_base
<222> 2
<223> /mod_base=OTHER
      /note="2'-fluoroadenosine"

<220>
<221> modified_base
<222> 3
<223> /mod_base=OTHER
      /note="2'-O-methylguanosine"

<220>
<221> modified_base
<222> 4
<223> /mod_base=OTHER
      /note="2'-fluoroadenosine"

<220>
<221> modified_base
<222> 5
<223> /mod_base=OTHER
      /note="2'-O-methyluridine"

<220>
<221> modified_base
<222> 6
<223> /mod_base=OTHER
      /note="2'-fluoroguanosine"

<220>
<221> modified_base
<222> 7
<223> /mod_base=OTHER
      /note="2'-O-methyluridine"

<220>
<221> modified_base
<222> 8
<223> /mod_base=OTHER
      /note="2'-fluorocytidine"

<220>
<221> modified_base
<222> 9
<223> /mod_base=OTHER
      /note="2'-O-methylguanosine"

<220>
<221> modified_base
<222> 10
<223> /mod_base=OTHER
      /note="2'-fluoroadenosine"

<220>
<221> modified_base
<222> 11
<223> /mod_base=OTHER
      /note="2'-O-methyluridine"

<220>
<221> modified_base
<222> 12
<223> /mod_base=OTHER
      /note="2'-fluoroguanosine"

<220>
<221> modified_base
<222> 13
<223> /mod_base=OTHER
      /note="2'-O-methylguanosine"

<220>
<221> modified_base
<222> 14
<223> /mod_base=OTHER
      /note="2'-fluorouridine"

<220>
<221> modified_base
<222> 15
<223> /mod_base=OTHER
      /note="2'-O-methylguanosine"

<220>
<221> modified_base
<222> 16
<223> /mod_base=OTHER
      /note="2'-fluoroadenosine"

<220>
<221> modified_base
<222> 17
<223> /mod_base=OTHER
      /note="2'-O-methyluridine"

<220>
<221> modified_base
<222> 18
<223> /mod_base=OTHER
      /note="2'-fluoroguanosine"

<220>
<221> modified_base
<222> 19
<223> /mod_base=OTHER
      /note="2'-O-methylcytidine"

<400> 1105
gagaugucga uggugaugc                                                    19


<210> 1106
<211> 19
<212> RNA
<213> Artificial Sequence

<220>
<223> Modified EEF1A2 siRNA antisense strand

<220>
<221> modified_base
<222> 14
<223> /mod_base=OTHER
      /note="2'-O-methyluridine"

<400> 1106
gaucuucuug auguaggcg                                                    19


<210> 1107
<211> 19
<212> RNA
<213> Artificial Sequence


<220>
<223> Modified EEF1A2 siRNA antisense strand

<220>
<221> modified_base
<222> 1
<223> /mod_base=OTHER
      /note="2'-fluoroguanosine"

<220>
<221> modified_base
<222> 2
<223> /mod_base=OTHER
      /note="2'-fluoroadenosine"

<220>
<221> modified_base
<222> 3
<223> /mod_base=OTHER
      /note="2'-O-methyluridine"

<220>
<221> modified_base
<222> 4
<223> /mod_base=OTHER
      /note="2'-fluorocytidine"

<220>
<221> modified_base
<222> 5
<223> /mod_base=OTHER
      /note="2'-O-methyluridine"

<220>
<221> modified_base
<222> 6
<223> /mod_base=OTHER
      /note="2'-fluorouridine"

<220>
<221> modified_base
<222> 7

<223> /mod_base=OTHER
      /note="2'-O-methylcytidine"

<220>
<221> modified_base
<222> 8
<223> /mod_base=OTHER
      /note="2'-fluorouridine"

<220>
<221> modified_base
<222> 9
<223> /mod_base=OTHER
      /note="2'-O-methyluridine"

<220>
<221> modified_base
<222> 10
<223> /mod_base=OTHER
      /note="2'-fluoroguanosine"

<220>
<221> modified_base
<222> 11
<223> /mod_base=OTHER
      /note="2'-O-methyladenosine"

<220>
<221> modified_base
<222> 12
<223> /mod_base=OTHER
      /note="2'-fluorouridine"

<220>
<221> modified_base
<222> 13
<223> /mod_base=OTHER
      /note="2'-O-methylguanosine"

<220>
<221> modified_base
<222> 14
<223> /mod_base=OTHER
      /note="2'-fluorouridine"

<220>
<221> modified_base
<222> 15
<223> /mod_base=OTHER
      /note="2'-O-methyladenosine"

<220>
<221> modified_base
<222> 16
<223> /mod_base=OTHER
      /note="2'-fluoroguanosine"

<220>
<221> modified_base
<222> 17
<223> /mod_base=OTHER
      /note="2'-O-methylguanosine"

<220>
<221> modified_base
<222> 18
<223> /mod_base=OTHER
      /note="2'-fluorocytidine"

<220>
<221> modified_base
<222> 19
<223> /mod_base=OTHER
      /note="2'-O-methylguanosine"

<400> 1107
gaucuucuug auguaggcg                                                    19


<210> 1108
<211> 19
<212> RNA
<213> Artificial Sequence


<220>
<223> Modified EEF1A2 siRNA antisense strand

<220>
<221> modified_base
<222> 4
<223> /mod_base=OTHER
      /note="2'-O-methylcytidine"

<400> 1108
gcgcaguccg ccugggaug                                                    19


<210> 1109
<211> 19
<212> RNA
<213> Artificial Sequence


<220>
<223> Modified EEF1A2 siRNA antisense strand

<220>
<221> modified_base
<222> 1
<223> /mod_base=OTHER
      /note="2'-fluoroguanosine"

<220>
<221> modified_base
<222> 2
<223> /mod_base=OTHER
      /note="2'-fluorocytidine"

<220>
<221> modified_base
<222> 3
<223> /mod_base=OTHER
      /note="2'-O-methylguanosine"

<220>

<221> modified_base
<222> 4
<223> /mod_base=OTHER
      /note="2'-fluorocytidine"

<220>
<221> modified_base
<222> 5
<223> /mod_base=OTHER
      /note="2'-O-methyladenosine"

<220>
<221> modified_base
<222> 6
<223> /mod_base=OTHER
      /note="2'-fluoroguanosine"

<220>
<221> modified_base
<222> 7
<223> /mod_base=OTHER
      /note="2'-O-methyluridine"

<220>
<221> modified_base
<222> 8
<223> /mod_base=OTHER
      /note="2'-fluorocytidine"

<220>
<221> modified_base
<222> 9
<223> /mod_base=OTHER
      /note="2'-O-methylcytidine"

<220>
<221> modified_base
<222> 10
<223> /mod_base=OTHER
      /note="2'-fluoroguanosine"

<220>
<221> modified_base
<222> 11
<223> /mod_base=OTHER
      /note="2'-O-methylcytidine"

<220>
<221> modified_base
<222> 12
<223> /mod_base=OTHER
      /note="2'-fluorocytidine"

<220>
<221> modified_base
<222> 13
<223> /mod_base=OTHER
      /note="2'-O-methyluridine"

<220>
<221> modified_base
<222> 14
<223> /mod_base=OTHER

/note="2'-fluoroguanosine"

<220>
<221> modified_base
<222> 15
<223> /mod_base=OTHER
/note="2'-O-methylguanosine"

<220>
<221> modified_base
<222> 16
<223> /mod_base=OTHER
/note="2'-fluoroguanosine"

<220>
<221> modified_base
<222> 17
<223> /mod_base=OTHER
/note="2'-O-methyladenosine"

<220>
<221> modified_base
<222> 18
<223> /mod_base=OTHER
/note="2'-fluorouridine"

<220>
<221> modified_base
<222> 19
<223> /mod_base=OTHER
/note="2'-O-methylguanosine"

<400> 1109
gcgcaguccg ccugggaug 19

<210> 1110
<211> 19
<212> RNA
<213> Artificial Sequence

<220>
<223> Modified EEF1A2 siRNA sense strand

<220>
<221> modified_base
<222> 1
<223> /mod_base=OTHER
/note="2'-O-methylcytidine"

<220>
<221> modified_base
<222> 2
<223> /mod_base=OTHER
/note="2'-O-methylcytidine"

<220>
<221> modified_base
<222> 4
<223> /mod_base=OTHER
/note="2'-O-methylcytidine"

<220>
<221> modified_base
<222> 5
<223> /mod_base=OTHER
      /note="2'-O-methylcytidine"

<220>
<221> modified_base
<222> 9
<223> /mod_base=OTHER
      /note="2'-O-methyluridine"

<220>
<221> modified_base
<222> 11
<223> /mod_base=OTHER
      /note="2'-O-methylcytidine"

<220>
<221> modified_base
<222> 12
<223> /mod_base=OTHER
      /note="2'-O-methyluridine"

<220>
<221> modified_base
<222> 14
<223> /mod_base=OTHER
      /note="2'-O-methylcytidine"

<220>
<221> modified_base
<222> 16
<223> /mod_base=OTHER
      /note="2'-O-methyluridine"

<220>
<221> modified_base
<222> 17
<223> /mod_base=OTHER
      /note="2'-O-methylcytidine"

<220>
<221> modified_base
<222> 19
<223> /mod_base=OTHER
      /note="2'-O-methylcytidine"

<400> 1110
ccaccaagua cuacaucac                                                    19


<210> 1111
<211> 19
<212> RNA
<213> Artificial Sequence


<220>
<223> Modified EEF1A2 siRNA sense strand

<220>
<221> modified_base

<222> 1
<223> /mod_base=OTHER
      /note="2'-O-methylcytidine"

<220>
<221> modified_base
<222> 2
<223> /mod_base=OTHER
      /note="2'-O-methylcytidine"

<220>
<221> modified_base
<222> 3
<223> /mod_base=OTHER
      /note="2'-fluoroadenosine"

<220>
<221> modified_base
<222> 4
<223> /mod_base=OTHER
      /note="2'-O-methylcytidine"

<220>
<221> modified_base
<222> 5
<223> /mod_base=OTHER
      /note="2'-fluorocytidine"

<220>
<221> modified_base
<222> 6
<223> /mod_base=OTHER
      /note="2'-O-methyladenosine"

<220>
<221> modified_base
<222> 7
<223> /mod_base=OTHER
      /note="2'-fluoroadenosine"

<220>
<221> modified_base
<222> 8
<223> /mod_base=OTHER
      /note="2'-O-methylguanosine"

<220>
<221> modified_base
<222> 9
<223> /mod_base=OTHER
      /note="2'-fluorouridine"

<220>
<221> modified_base
<222> 10
<223> /mod_base=OTHER
      /note="2'-O-methyladenosine"

<220>
<221> modified_base
<222> 11
<223> /mod_base=OTHER
      /note="2'-fluorocytidine"

<220>
<221> modified_base
<222> 12
<223> /mod_base=OTHER
      /note="2'-O-methyluridine"

<220>
<221> modified_base
<222> 13
<223> /mod_base=OTHER
      /note="2'-fluoroadenosine"

<220>
<221> modified_base
<222> 14
<223> /mod_base=OTHER
      /note="2'-O-methylcytidine"

<220>
<221> modified_base
<222> 15
<223> /mod_base=OTHER
      /note="2'-fluoroadenosine"

<220>
<221> modified_base
<222> 16
<223> /mod_base=OTHER
      /note="2'-O-methyluridine"

<220>
<221> modified_base
<222> 17
<223> /mod_base=OTHER
      /note="2'-fluorocytidine"

<220>
<221> modified_base
<222> 18
<223> /mod_base=OTHER
      /note="2'-O-methyladenosine"

<220>
<221> modified_base
<222> 19
<223> /mod_base="OTHER"
      /note="2'-fluorocytidine"

<400> 1111
ccaccaagua cuacaucac                                                    19


<210> 1112
<211> 19
<212> RNA
<213> Artificial Sequence


<220>
<223> Modified EEF1A2 siRNA sense strand

<220>

<221> modified_base
<222> 1
<223> /mod_base=OTHER
      /note="2'-O-methyladenosine"

<220>
<221> modified_base
<222> 4
<223> /mod_base=OTHER
      /note="2'-O-methylcytidine"

<220>
<221> modified_base
<222> 5
<223> /mod_base=OTHER
      /note="2'-O-methylcytidine"

<220>
<221> modified_base
<222> 7
<223> /mod_base=OTHER
      /note="2'-O-methylcytidine"

<220>
<221> modified_base
<222> 8
<223> /mod_base=OTHER
      /note="2'-O-methylcytidine"

<220>
<221> modified_base
<222> 12
<223> /mod_base=OTHER
      /note="2'-O-methyluridine"

<220>
<221> modified_base
<222> 14
<223> /mod_base=OTHER
      /note="2'-O-methylcytidine"

<220>
<221> modified_base
<222> 15
<223> /mod_base=OTHER
      /note="2'-O-methyluridine"

<220>
<221> modified_base
<222> 17
<223> /mod_base=OTHER
      /note="2'-O-methylcytidine"

<220>
<221> modified_base
<222> 19
<223> /mod_base=OTHER
      /note="2'-O-methyluridine"

<400> 1112
agaccaccaa guacuacau                                                    19

<210> 1113
<211> 19
<212> RNA
<213> Artificial Sequence

<220>
<223> Modified EEF1A2 siRNA sense strand

<220>
<221> modified_base
<222> 1
<223> /mod_base=OTHER
      /note="2'-O-methyladenosine"

<220>
<221> modified_base
<222> 2
<223> /mod_base=OTHER
      /note="2'-O-methylguanosine"

<220>
<221> modified_base
<222> 3
<223> /mod_base=OTHER
      /note="2'-fluoroadenosine"

<220>
<221> modified_base
<222> 4
<223> /mod_base=OTHER
      /note="2'-O-methylcytidine"

<220>
<221> modified_base
<222> 5
<223> /mod_base=OTHER
      /note="2'-fluorocytidine"

<220>
<221> modified_base
<222> 6
<223> /mod_base=OTHER
      /note="2'-O-methyladenosine"

<220>
<221> modified_base
<222> 7
<223> /mod_base=OTHER
      /note="2'-fluorocytidine"

<220>
<221> modified_base
<222> 8
<223> /mod_base=OTHER
      /note="2'-O-methylcytidine"

<220>
<221> modified_base
<222> 9
<223> /mod_base=OTHER
      /note="2'-fluoroadenosine"

<220>
<221> modified_base
<222> 10
<223> /mod_base=OTHER
      /note="2'-O-methyladenosine"

<220>
<221> modified_base
<222> 11
<223> /mod_base=OTHER
      /note="2'-fluoroguanosine"

<220>
<221> modified_base
<222> 12
<223> /mod_base=OTHER
      /note="2'-O-methyluridine"

<220>
<221> modified_base
<222> 13
<223> /mod_base=OTHER
      /note="2'-fluoroadenosine"

<220>
<221> modified_base
<222> 14
<223> /mod_base=OTHER
      /note="2'-O-methylcytidine"

<220>
<221> modified_base
<222> 15
<223> /mod_base=OTHER
      /note="2'-fluorouridine"

<220>
<221> modified_base
<222> 16
<223> /mod_base=OTHER
      /note="2'-O-methyladenosine"

<220>
<221> modified_base
<222> 17
<223> /mod_base=OTHER
      /note="2'-fluorocytidine"

<220>
<221> modified_base
<222> 18
<223> /mod_base=OTHER
      /note="2'-O-methyladenosine"

<220>
<221> modified_base
<222> 19
<223> /mod_base="OTHER"
      /note="2'-fluorouridine"

<400> 1113
agaccaccaa guacuacau                                                    19

<210> 1114
<211> 19
<212> RNA
<213> Artificial Sequence

<220>
<223> Modified EEF1A2 siRNA sense strand

<220>
<221> modified_base
<222> 1
<223> /mod_base=OTHER
      /note="2'-O-methylguanosine"

<220>
<221> modified_base
<222> 2
<223> /mod_base=OTHER
      /note="2'-O-methylcytidine"

<220>
<221> modified_base
<222> 4
<223> /mod_base=OTHER
      /note="2'-O-methylcytidine"

<220>
<221> modified_base
<222> 5
<223> /mod_base=OTHER
      /note="2'-O-methylcytidine"

<220>
<221> modified_base
<222> 6
<223> /mod_base=OTHER
      /note="2'-O-methyluridine"

<220>
<221> modified_base
<222> 8
<223> /mod_base=OTHER
      /note="2'-O-methylcytidine"

<220>
<221> modified_base
<222> 10
<223> /mod_base=OTHER
      /note="2'-O-methyluridine"

<220>
<221> modified_base
<222> 11
<223> /mod_base=OTHER
      /note="2'-O-methylcytidine"

<220>
<221> modified_base
<222> 14
<223> /mod_base=OTHER
      /note="2'-O-methylguanosine"

<220>
<221> modified_base
<222> 19
<223> /mod_base=OTHER
      /note="2'-O-methyluridine"

<400> 1114
gcgccuacau caagaagau                                                    19


<210> 1115
<211> 19
<212> RNA
<213> Artificial Sequence


<220>
<223> Modified EEF1A2 siRNA sense strand

<220>
<221> modified_base
<222> 1
<223> /mod_base=OTHER
      /note="2'-O-methylguanosine"

<220>
<221> modified_base
<222> 2
<223> /mod_base=OTHER
      /note="2'-O-methylcytidine"

<220>
<221> modified_base
<222> 3
<223> /mod_base=OTHER
      /note="2'-fluoroguanosine"

<220>
<221> modified_base
<222> 4
<223> /mod_base=OTHER
      /note="2'-O-methylcytidine"

<220>
<221> modified_base
<222> 5
<223> /mod_base=OTHER
      /note="2'-fluorocytidine"

<220>
<221> modified_base
<222> 6
<223> /mod_base=OTHER
      /note="2'-O-methyluridine"

<220>
<221> modified_base
<222> 7
<223> /mod_base=OTHER
      /note="2'-fluoroadenosine"

<220>

<221> modified_base
<222> 8
<223> /mod_base=OTHER
      /note="2'-O-methylcytidine"

<220>
<221> modified_base
<222> 9
<223> /mod_base=OTHER
      /note="2'-fluoroadenosine"

<220>
<221> modified_base
<222> 10
<223> /mod_base=OTHER
      /note="2'-O-methyluridine"

<220>
<221> modified_base
<222> 11
<223> /mod_base=OTHER
      /note="2'-fluorocytidine"

<220>
<221> modified_base
<222> 12
<223> /mod_base=OTHER
      /note="2'-O-methyladenosine"

<220>
<221> modified_base
<222> 13
<223> /mod_base=OTHER
      /note="2'-fluoroadenosine"

<220>
<221> modified_base
<222> 14
<223> /mod_base=OTHER
      /note="2'-O-methylguanosine"

<220>
<221> modified_base
<222> 15
<223> /mod_base=OTHER
      /note="2'-fluoroadenosine"

<220>
<221> modified_base
<222> 16
<223> /mod_base=OTHER
      /note="2'-O-methyladenosine"

<220>
<221> modified_base
<222> 17
<223> /mod_base=OTHER
      /note="2'-fluoroguanosine"

<220>
<221> modified_base
<222> 18
<223> /mod_base=OTHER

/note="2'-O-methyladenosine"

<220>
<221> modified_base
<222> 19
<223> /mod_base="OTHER"
/note="2'-fluorouridine"

<400> 1115
gcgccuacau caagaagau 19

<210> 1116
<211> 19
<212> RNA
<213> Artificial Sequence

<220>
<223> Modified EEF1A2 siRNA sense strand

<220>
<221> modified_base
<222> 1
<223> /mod_base=OTHER
/note="2'-O-methyladenosine"

<220>
<221> modified_base
<222> 2
<223> /mod_base=OTHER
/note="2'-O-methylcytidine"

<220>
<221> modified_base
<222> 3
<223> /mod_base=OTHER
/note="2'-O-methylcytidine"

<220>
<221> modified_base
<222> 5
<223> /mod_base=OTHER
/note="2'-O-methylcytidine"

<220>
<221> modified_base
<222> 6
<223> /mod_base=OTHER
/note="2'-O-methylcytidine"

<220>
<221> modified_base
<222> 10
<223> /mod_base=OTHER
/note="2'-O-methyluridine"

<220>
<221> modified_base
<222> 12
<223> /mod_base=OTHER
/note="2'-O-methylcytidine"

<220>
<221> modified_base
<222> 13
<223> /mod_base=OTHER
      /note="2'-O-methyluridine"

<220>
<221> modified_base
<222> 14
<223> /mod_base=OTHER
      /note="2'-O-methyladenosine"

<220>
<221> modified_base
<222> 15
<223> /mod_base=OTHER
      /note="2'-O-methylcytidine"

<220>
<221> modified_base
<222> 17
<223> /mod_base=OTHER
      /note="2'-O-methyluridine"

<220>
<221> modified_base
<222> 18
<223> /mod_base=OTHER
      /note="2'-O-methylcytidine"

<400> 1116
accaccaagu acuacauca                                                    19


<210> 1117
<211> 19
<212> RNA
<213> Artificial Sequence


<220>
<223> Modified EEF1A2 siRNA sense strand

<220>
<221> modified_base
<222> 1
<223> /mod_base=OTHER
      /note="2'-O-methyladenosine"

<220>
<221> modified_base
<222> 2
<223> /mod_base=OTHER
      /note="2'-O-methylcytidine"

<220>
<221> modified_base
<222> 3
<223> /mod_base=OTHER
      /note="2'-fluorocytidine"

<220>
<221> modified_base

<222> 4
<223> /mod_base=OTHER
      /note="2'-O-methyladenosine"

<220>
<221> modified_base
<222> 5
<223> /mod_base=OTHER
      /note="2'-fluorocytidine"

<220>
<221> modified_base
<222> 6
<223> /mod_base=OTHER
      /note="2'-O-methylcytidine"

<220>
<221> modified_base
<222> 7
<223> /mod_base=OTHER
      /note="2'-fluoroadenosine"

<220>
<221> modified_base
<222> 8
<223> /mod_base=OTHER
      /note="2'-O-methyladenosine"

<220>
<221> modified_base
<222> 9
<223> /mod_base=OTHER
      /note="2'-fluoroguanosine"

<220>
<221> modified_base
<222> 10
<223> /mod_base=OTHER
      /note="2'-O-methyluridine"

<220>
<221> modified_base
<222> 11
<223> /mod_base=OTHER
      /note="2'-fluoroadenosine"

<220>
<221> modified_base
<222> 12
<223> /mod_base=OTHER
      /note="2'-O-methylcytidine"

<220>
<221> modified_base
<222> 13
<223> /mod_base=OTHER
      /note="2'-fluorouridine"

<220>
<221> modified_base
<222> 14
<223> /mod_base=OTHER
      /note="2'-O-methyladenosine"

<220>
<221> modified_base
<222> 15
<223> /mod_base=OTHER
      /note="2'-fluorocytidine"

<220>
<221> modified_base
<222> 16
<223> /mod_base=OTHER
      /note="2'-O-methyladenosine"

<220>
<221> modified_base
<222> 17
<223> /mod_base=OTHER
      /note="2'-fluorouridine"

<220>
<221> modified_base
<222> 18
<223> /mod_base=OTHER
      /note="2'-O-methylcytidine"

<220>
<221> modified_base
<222> 19
<223> /mod_base="OTHER"
      /note="2'-fluoroadenosine"

<400> 1117
accaccaagu acuacauca                                                    19


<210> 1118
<211> 19
<212> RNA
<213> Artificial Sequence


<220>
<223> Modified EEF1A2 siRNA sense strand

<220>
<221> modified_base
<222> 1
<223> /mod_base=OTHER
      /note="2'-O-methylguanosine"

<220>
<221> modified_base
<222> 2
<223> /mod_base=OTHER
      /note="2'-O-methylcytidine"

<220>
<221> modified_base
<222> 4
<223> /mod_base=OTHER
      /note="2'-O-methyluridine"

<220>

<221> modified_base
<222> 5
<223> /mod_base=OTHER
      /note="2'-O-methylcytidine"

<220>
<221> modified_base
<222> 7
<223> /mod_base=OTHER
      /note="2'-O-methylcytidine"

<220>
<221> modified_base
<222> 8
<223> /mod_base=OTHER
      /note="2'-O-methylcytidine"

<220>
<221> modified_base
<222> 10
<223> /mod_base=OTHER
      /note="2'-O-methyluridine"

<220>
<221> modified_base
<222> 11
<223> /mod_base=OTHER
      /note="2'-O-methylcytidine"

<220>
<221> modified_base
<222> 14
<223> /mod_base=OTHER
      /note="2'-O-methylcytidine"

<220>
<221> modified_base
<222> 16
<223> /mod_base=OTHER
      /note="2'-O-methyluridine"

<220>
<221> modified_base
<222> 17
<223> /mod_base=OTHER
      /note="2'-O-methylcytidine"

<220>
<221> modified_base
<222> 18
<223> /mod_base=OTHER
      /note="2'-O-methyluridine"

<220>
<221> modified_base
<222> 19
<223> /mod_base=OTHER
      /note="2'-O-methylcytidine"

<400> 1118
gcaucaccau cgacaucuc                                                    19

<210> 1119
<211> 19
<212> RNA
<213> Artificial Sequence

<220>
<223> Modified EEF1A2 siRNA sense strand

<220>
<221> modified_base
<222> 1
<223> /mod_base=OTHER
      /note="2'-O-methylguanosine"

<220>
<221> modified_base
<222> 2
<223> /mod_base=OTHER
      /note="2'-O-methylcytidine"

<220>
<221> modified_base
<222> 3
<223> /mod_base=OTHER
      /note="2'-fluoroadenosine"

<220>
<221> modified_base
<222> 4
<223> /mod_base=OTHER
      /note="2'-O-methyluridine"

<220>
<221> modified_base
<222> 5
<223> /mod_base=OTHER
      /note="2'-fluorocytidine"

<220>
<221> modified_base
<222> 6
<223> /mod_base=OTHER
      /note="2'-O-methyladenosine"

<220>
<221> modified_base
<222> 7
<223> /mod_base=OTHER
      /note="2'-fluorocytidine"

<220>
<221> modified_base
<222> 8
<223> /mod_base=OTHER
      /note="2'-O-methylcytidine"

<220>
<221> modified_base
<222> 9
<223> /mod_base=OTHER
      /note="2'-fluoroadenosine"

<220>
<221> modified_base
<222> 10
<223> /mod_base=OTHER
      /note="2'-O-methyluridine"

<220>
<221> modified_base
<222> 11
<223> /mod_base=OTHER
      /note="2'-fluorocytidine"

<220>
<221> modified_base
<222> 12
<223> /mod_base=OTHER
      /note="2'-O-methylguanosine"

<220>
<221> modified_base
<222> 13
<223> /mod_base=OTHER
      /note="2'-fluoroadenosine"

<220>
<221> modified_base
<222> 14
<223> /mod_base=OTHER
      /note="2'-O-methylcytidine"

<220>
<221> modified_base
<222> 15
<223> /mod_base=OTHER
      /note="2'-fluoroadenosine"

<220>
<221> modified_base
<222> 16
<223> /mod_base=OTHER
      /note="2'-O-methyluridine"

<220>
<221> modified_base
<222> 17
<223> /mod_base=OTHER
      /note="2'-fluorocytidine"

<220>
<221> modified_base
<222> 18
<223> /mod_base=OTHER
      /note="2'-O-methyluridine"

<220>
<221> modified_base
<222> 19
<223> /mod_base="OTHER"
      /note="2'-fluorocytidine"

<400> 1119
gcaucaccau cgacaucuc                                                    19

<210> 1120
<211> 19
<212> RNA
<213> Artificial Sequence

<220>
<223> Modified EEF1A2 siRNA sense strand

<220>
<221> modified_base
<222> 1
<223> /mod_base=OTHER
      /note="2'-O-methylcytidine"

<220>
<221> modified_base
<222> 3
<223> /mod_base=OTHER
      /note="2'-O-methylcytidine"

<220>
<221> modified_base
<222> 4
<223> /mod_base=OTHER
      /note="2'-O-methylcytidine"

<220>
<221> modified_base
<222> 5
<223> /mod_base=OTHER
      /note="2'-O-methyluridine"

<220>
<221> modified_base
<222> 7
<223> /mod_base=OTHER
      /note="2'-O-methylcytidine"

<220>
<221> modified_base
<222> 9
<223> /mod_base=OTHER
      /note="2'-O-methyluridine"

<220>
<221> modified_base
<222> 10
<223> /mod_base=OTHER
      /note="2'-O-methylcytidine"

<220>
<221> modified_base
<222> 14
<223> /mod_base=OTHER
      /note="2'-O-methyladenosine"

<220>
<221> modified_base
<222> 18
<223> /mod_base=OTHER
      /note="2'-O-methyluridine"

<220>
<221> modified_base
<222> 19
<223> /mod_base=OTHER
      /note="2'-O-methylcytidine"

<400> 1120
cgccuacauc aagaagauc                                                    19


<210> 1121
<211> 19
<212> RNA
<213> Artificial Sequence


<220>
<223> Modified EEF1A2 siRNA sense strand

<220>
<221> modified_base
<222> 1
<223> /mod_base=OTHER
      /note="2'-O-methylcytidine"

<220>
<221> modified_base
<222> 2
<223> /mod_base=OTHER
      /note="2'-O-methylguanosine"

<220>
<221> modified_base
<222> 3
<223> /mod_base=OTHER
      /note="2'-fluorocytidine"

<220>
<221> modified_base
<222> 4
<223> /mod_base=OTHER
      /note="2'-O-methylcytidine"

<220>
<221> modified_base
<222> 5
<223> /mod_base=OTHER
      /note="2'-fluorouridine"

<220>
<221> modified_base
<222> 6
<223> /mod_base=OTHER
      /note="2'-O-methyladenosine"

<220>
<221> modified_base
<222> 7
<223> /mod_base=OTHER
      /note="2'-fluorocytidine"

<220>

<221> modified_base
<222> 8
<223> /mod_base=OTHER
      /note="2'-O-methyladenosine"

<220>
<221> modified_base
<222> 9
<223> /mod_base=OTHER
      /note="2'-fluorouridine"

<220>
<221> modified_base
<222> 10
<223> /mod_base=OTHER
      /note="2'-O-methylcytidine"

<220>
<221> modified_base
<222> 11
<223> /mod_base=OTHER
      /note="2'-fluoroadenosine"

<220>
<221> modified_base
<222> 12
<223> /mod_base=OTHER
      /note="2'-O-methyladenosine"

<220>
<221> modified_base
<222> 13
<223> /mod_base=OTHER
      /note="2'-fluoroguanosine"

<220>
<221> modified_base
<222> 14
<223> /mod_base=OTHER
      /note="2'-O-methyladenosine"

<220>
<221> modified_base
<222> 15
<223> /mod_base=OTHER
      /note="2'-fluoroadenosine"

<220>
<221> modified_base
<222> 16
<223> /mod_base=OTHER
      /note="2'-O-methylguanosine"

<220>
<221> modified_base
<222> 17
<223> /mod_base=OTHER
      /note="2'-fluoroadenosine"

<220>
<221> modified_base
<222> 18
<223> /mod_base=OTHER

/note="2'-O-methyluridine"

<220>
<221> modified_base
<222> 19
<223> /mod_base="OTHER"
/note="2'-fluorocytidine"

<400> 1121
cgccuacauc aagaagauc 19

<210> 1122
<211> 19
<212> RNA
<213> Artificial Sequence

<220>
<223> Modified EEF1A2 siRNA sense strand

<220>
<221> modified_base
<222> 1
<223> /mod_base=OTHER
/note="2'-O-methylcytidine"

<220>
<221> modified_base
<222> 3
<223> /mod_base=OTHER
/note="2'-O-methyluridine"

<220>
<221> modified_base
<222> 4
<223> /mod_base=OTHER
/note="2'-O-methylcytidine"

<220>
<221> modified_base
<222> 5
<223> /mod_base=OTHER
/note="2'-O-methylcytidine"

<220>
<221> modified_base
<222> 6
<223> /mod_base=OTHER
/note="2'-O-methylcytidine"

<220>
<221> modified_base
<222> 10
<223> /mod_base=OTHER
/note="2'-O-methylcytidine"

<220>
<221> modified_base
<222> 14
<223> /mod_base=OTHER
/note="2'-O-methylcytidine"

<220>
<221> modified_base
<222> 15
<223> /mod_base=OTHER
      /note="2'-O-methyluridine"

<220>
<221> modified_base
<222> 17
<223> /mod_base=OTHER
      /note="2'-O-methylcytidine"

<220>
<221> modified_base
<222> 19
<223> /mod_base=OTHER
      /note="2'-O-methylcytidine"

<400> 1122
caucccaggc ggacugcgc                                                    19


<210> 1123
<211> 19
<212> RNA
<213> Artificial Sequence


<220>
<223> Modified EEF1A2 siRNA sense strand

<220>
<221> modified_base
<222> 1
<223> /mod_base=OTHER
      /note="2'-O-methylcytidine"

<220>
<221> modified_base
<222> 2
<223> /mod_base=OTHER
      /note="2'-O-methyladenosine"

<220>
<221> modified_base
<222> 3
<223> /mod_base=OTHER
      /note="2'-fluorouridine"

<220>
<221> modified_base
<222> 4
<223> /mod_base=OTHER
      /note="2'-O-methylcytidine"

<220>
<221> modified_base
<222> 5
<223> /mod_base=OTHER
      /note="2'-fluorocytidine"

<220>
<221> modified_base

<222> 6
<223> /mod_base=OTHER
      /note="2'-O-methylcytidine"

<220>
<221> modified_base
<222> 7
<223> /mod_base=OTHER
      /note="2'-fluoroadenosine"

<220>
<221> modified_base
<222> 8
<223> /mod_base=OTHER
      /note="2'-O-methylguanosine"

<220>
<221> modified_base
<222> 9
<223> /mod_base=OTHER
      /note="2'-fluoroguanosine"

<220>
<221> modified_base
<222> 10
<223> /mod_base=OTHER
      /note="2'-O-methylcytidine"

<220>
<221> modified_base
<222> 11
<223> /mod_base=OTHER
      /note="2'-fluoroguanosine"

<220>
<221> modified_base
<222> 12
<223> /mod_base=OTHER
      /note="2'-O-methylguanosine"

<220>
<221> modified_base
<222> 13
<223> /mod_base=OTHER
      /note="2'-fluoroadenosine"

<220>
<221> modified_base
<222> 14
<223> /mod_base=OTHER
      /note="2'-O-methylcytidine"

<220>
<221> modified_base
<222> 15
<223> /mod_base=OTHER
      /note="2'-fluorouridine"

<220>
<221> modified_base
<222> 16
<223> /mod_base=OTHER
      /note="2'-O-methylguanosine"

<220>
<221> modified_base
<222> 17
<223> /mod_base=OTHER
      /note="2'-fluorocytidine"

<220>
<221> modified_base
<222> 18
<223> /mod_base=OTHER
      /note="2'-O-methylguanosine"

<220>
<221> modified_base
<222> 19
<223> /mod_base="OTHER"
      /note="2'-fluorocytidine"

<400> 1123
caucccaggc ggacugcgc                                                    19


<210> 1124
<211> 30
<212> RNA
<213> Artificial Sequence


<220>
<223> Common region for siRNA antisense strands

<400> 1124
cgaugauggu gauguaguac uugguggucu                                       30


<210> 1125
<211> 35
<212> RNA
<213> Artificial Sequence


<220>
<223> Common region for siRNA antisense strands

<400> 1125
cggguuguag ccgaucuucu ugauguaggc gcuga                                  35


<210> 1126
<211> 32
<212> RNA
<213> Artificial Sequence


<220>
<223> Common region for siRNA antisense strands

<400> 1126
gaacuuccag agggagaugu cgauggugau gc                                     32


<210> 1127

<211> 20
<212> RNA
<213> Artificial Sequence

<220>
<223> Common region for siRNA antisense strands

<220>
<221> misc_feature
<222> 1
<223> /note="y is u or c, and preferably is c"

<400> 1127
ygcgcagucc gccugggaug                                                    20

<210> 1128
<211> 20
<212> RNA
<213> Artificial Sequence

<220>
<223> Common region for siRNA antisense strands

<220>
<221> misc_feature
<222> 1
<223> /note="y is u or c, and preferably is u"

<400> 1128
yuuguccagc acccaggcau                                                    20

<210> 1129
<211> 20
<212> RNA
<213> Artificial Sequence

<220>
<223> Common region for siRNA antisense strands

<220>
<221> misc_feature
<222> 1
<223> /note="r is g or a, and preferably is g"

<400> 1129
ruucuuggag augcccgccu                                                    20

<210> 1130
<211> 20
<212> RNA
<213> Artificial Sequence

<220>
<223> Common region for siRNA antisense strands

<400> 1130

guagcgcuuc ucgcuguagg                                                    20

<210> 1131
<211> 22
<212> RNA
<213> Artificial Sequence

<220>
<223> Common region for siRNA antisense strands

<400> 1131
   gugauguagu acuugguggu cu                                                 22

<210> 1132
<211> 20
<212> RNA
<213> Artificial Sequence

<220>
<223> Common region for siRNA antisense strands

<400> 1132
gaucuucuug auguaggcgc                                                    20

<210> 1133
<211> 32
<212> RNA
<213> Artificial Sequence

<220>
<223> Common region for siRNA sense strands

<220>
<221> misc_feature
<222> 32
<223> /note="y is u or c, and preferably is c"

<400> 1133
agaccaccaa guacuacauc accaucaucg ay                                      32

<210> 1134
<211> 35
<212> RNA
<213> Artificial Sequence

<220>
<223> Common region for siRNA sense strands

<400> 1134
ucagcgccua caucaagaag aucggcuaca acccg                                   35

<210> 1135
<211> 32
<212> RNA

<213> Artificial Sequence

<220>
<223> Common region for siRNA sense strands

<400> 1135
gcaucaccau cgacaucucc cucuggaagu uc                                    32

<210> 1136
<211> 20
<212> RNA
<213> Artificial Sequence

<220>
<223> Common region for siRNA sense strands

<220>
<221> misc_feature
<222> 20
<223> /note="r is g or a, and preferably is g"

<400> 1136
caucccaggc ggacugcgcr                                                  20

<210> 1137
<211> 20
<212> RNA
<213> Artificial Sequence

<220>
<223> Common region for siRNA sense strands

<220>
<221> misc_feature
<222> 20
<223> /note="r is g or a, and preferably is a"

<400> 1137
augccugggu gcuggacaar                                                  20

<210> 1138
<211> 20
<212> RNA
<213> Artificial Sequence

<220>
<223> Common region for siRNA sense strands

<220>
<221> misc_feature
<222> 20
<223> /note="y is u or c, and preferably is c"

<400> 1138
aggcgggcau cuccaagaay                                                  20

<210> 1139
<211> 20
<212> RNA
<213> Artificial Sequence

<220>
<223> Common region for siRNA sense strands

<400> 1139
ccuacagcga gaagcgcuac                                                    20

<210> 1140
<211> 22
<212> RNA
<213> Artificial Sequence

<220>
<223> Common region for siRNA sense strands

<400> 1140
agaccaccaa guacuacauc ac                                                 22

<210> 1141
<211> 20
<212> RNA
<213> Artificial Sequence

<220>
<223> Common region for siRNA sense strands

<400> 1141
gcgccuacau caagaagauc                                                    20

<210> 1142
<211> 19
<212> RNA
<213> Artificial Sequence

<220>
<223> Antisense sequence of siRNAs targeting SEQ ID NO:1

<220>
<221> misc_feature
<222> 19
<223> /note="n is G, GU, GUC, GUU, GdT or GdTdT, and preferably is G or
      GUU"

<400> 1142
gugauguagu acuuggugn                                                     19

<210> 1143
<211> 19
<212> RNA
<213> Artificial Sequence

<220>
<223> Antisense sequences targeting SEQ ID NO: 2

<220>
<221> misc_feature
<222> 19
<223> /note="n is U"

<400> 1143
auguaguacu ugguggucn                                                    19


<210> 1144
<211> 19
<212> RNA
<213> Artificial Sequence


<220>
<223> Antisense sequences targeting SEQ ID NO:1

<220>
<221> misc_feature
<222> 19
<223> /note="n is C, CU, CUG, CUU, CdT or CdTdT, and preferably is C"

<400> 1144
aucuucuuga uguaggcgn                                                    19


<210> 1145
<211> 19
<212> RNA
<213> Artificial Sequence


<220>
<223> Antisense sequences targeting SEQ ID NO:4

<220>
<221> misc_feature
<222> 19
<223> /note="n is U, UC, UCU, UUU, UdT or UdTdT, and preferably is U or
      UUU"

<400> 1145
ugauguagua cuugguggn                                                    19


<210> 1146
<211> 19
<212> RNA
<213> Artificial Sequence


<220>
<223> Antisense sequences targeting SEQ ID NO:5

<220>
<221> misc_feature
<222> 19

<223> /note="n is G, GC, GCU, GUU, GdT or GdTdT, and preferably is G"

<400> 1146
gaucuucuug auguaggcn                                                    19


<210> 1147
<211> 19
<212> RNA
<213> Artificial Sequence


<220>
<223> Antisense sequences targeting SEQ ID NO:6

<220>
<221> misc_feature
<222> 19
<223> /note="n is C"

<400> 1147
gagaugucga uggugaugn                                                    19


<210> 1148
<211> 19
<212> RNA
<213> Artificial Sequence


<220>
<223> Antisense sequences targeting SEQ ID NO:7

<220>
<221> misc_feature
<222> 19
<223> /note="n is G"

<400> 1148
gcgcaguccg ccugggaun                                                    19


<210> 1149
<211> 19
<212> RNA
<213> Artificial Sequence


<220>
<223> Antisense sequences targeting SEQ ID NO:8

<220>
<221> misc_feature
<222> 19
<223> /note="n is C, CU or CdT, and preferably is CU"

<400> 1149
   gauguaguac uugguggun                                                    19


<210> 1150
<211> 16
<212> RNA

<213> Artificial Sequence

<220>
<223> Sense sequences of siRNAs targeting SEQ ID NO:1

<220>
<221> misc_feature
<222> 16
<223> /note="n is UCAC, UCACC, UCACCA, UCACUU, UCACdT, UCACdTdT, UCAG,
      UCUG, UGUG or AGUG, and preferably is UCAC, UCAG, UCUG or UGUG "

<400> 1150
ccaccaagua cuacan                                                    16

<210> 1151
<211> 16
<212> RNA
<213> Artificial Sequence

<220>
<223> Sense sequences of siRNAs targeting SEQ ID NO:2

<220>
<221> misc_feature
<222> 16
<223> /note="n is ACAU"

<400> 1151
agaccaccaa guacun                                                    16

<210> 1152
<211> 16
<212> RNA
<213> Artificial Sequence

<220>
<223> Sense sequence of siRNAs targeting SEQ ID NO:3

<220>
<221> misc_feature
<222> 16
<223> /note="n is AGAU, AGAUC, AGAUCG, AGAUUU, AGAUdT, AGAUdTdT, AGAA,
      AGUA, ACUA or UCUA, and preferably is AGAU"

<400> 1152
gcgccuacau caagan                                                    16

<210> 1153
<211> 16
<212> RNA
<213> Artificial Sequence

<220>
<223> Sense sequences of siRNAs targeting SEQ ID NO:4

<220>

<221> misc_feature
<222> 16
<223> /note="n is AUCA, AUCAC, AUCACC, AUCAUU, AUCAdT, AUCAdTdT, AUCU,
      AUGU, AAGU or UAGU, and preferably is AUCA, AUGU, AAGU, or UAGU"

<400> 1153
accaccaagu acuacn                                                    16


<210> 1154
<211> 16
<212> RNA
<213> Artificial Sequence


<220>
<223> Sense sequences of siRNAs targeting SEQ ID NO:5

<220>
<221> misc_feature
<222> 16
<223> /note="n is GAUC, GAUCG, GAUCGG, GAUCUU, GAUCdT, GAUCdTdT, GAUG,
      GAAG, GUAG or CUAG, and preferably is GAUC"

<400> 1154
cgccuacauc aagaan                                                    16


<210> 1155
<211> 16
<212> RNA
<213> Artificial Sequence


<220>
<223> Sense sequence of siRNAs targeting SEQ ID NO:6

<220>
<221> misc_feature
<222> 16
<223> /note="n is UCUC"

<400> 1155
gcaucaccau cgacan                                                    16


<210> 1156
<211> 16
<212> RNA
<213> Artificial Sequence


<220>
<223> Sense sequences of siRNAs targeting SEQ ID NO:7

<220>
<221> misc_feature
<222> 16
<223> /note="n is GCGC"

<400> 1156
caucccaggc ggacun                                                    16

<210> 1157
<211> 16
<212> RNA
<213> Artificial Sequence

<220>
<223> Sense sequences of siRNAs targeting SEQ ID NO:8

<220>
<221> misc_feature
<222> 16
<223> /note="n is CAUC, CAUCA or CAUCdT, and preferably is CAUC or
      CAUCA"

<400> 1157
gaccaccaag uacuan                                                    16

<210> 1158
<211> 19
<212> RNA
<213> Artificial Sequence

<220>
<223> Antisense sequence of siRNAs targeting SEQ ID NO:1

<220>
<221> misc_feature
<222> 19
<223> /note="n is G or GUU"

<400> 1158
gugauguagu acuuggugn                                                 19

<210> 1159
<211> 19
<212> RNA
<213> Artificial Sequence

<220>
<223> Antisense sequences targeting SEQ ID NO:3

<220>
<221> misc_feature
<222> 19
<223> /note="n is C"

<400> 1159
aucuucuuga uguaggcgn                                                 19

<210> 1160
<211> 19
<212> RNA
<213> Artificial Sequence

<220>

<223> Antisense sequence of siRNAs targeting SEQ ID NO:4

<220>
<221> misc_feature
<222> 19
<223> /note="n is U or UUU"

<400> 1160
   ugauguagua cuugguggn                                                    19


<210> 1161
<211> 19
<212> RNA
<213> Artificial Sequence


<220>
<223> Antisense sequence of siRNAs targeting SEQ ID NO:5

<220>
<221> misc_feature
<222> 19
<223> /note="n is G"

<400> 1161
gaucuucuug auguaggcn                                                   19


<210> 1162
<211> 19
<212> RNA
<213> Artificial Sequence


<220>
<223> Antisense sequence of siRNAs targeting SEQ ID NO:8

<220>
<221> misc_feature
<222> 19
<223> /note="n is C or CU, and preferably is C"

<400> 1162
   gauguaguac uugguggun                                                     19


<210> 1163
<211> 16
<212> RNA
<213> Artificial Sequence


<220>
<223> Sense sequence of siRNAs targeting SEQ ID NO:1

<220>
<221> misc_feature
<222> 16
<223> /note="n is UCAC, UCAG, UCUG or UGUG"

<400> 1163
ccaccaagua cuacan                                                      16

<210> 1164
<211> 16
<212> RNA
<213> Artificial Sequence

<220>
<223> Sense sequence of siRNAs targeting SEQ ID NO:3

<220>
<221> misc_feature
<222> 16
<223> /note="n is AGAU"

<400> 1164
gcgccuacau caagan                                                    16

<210> 1165
<211> 16
<212> RNA
<213> Artificial Sequence

<220>
<223> Sense sequence of siRNAs targeting SEQ ID NO:4

<220>
<221> misc_feature
<222> 16
<223> /note="n is AUCA, AUGU, AAGU or UAGU"

<400> 1165
accaccaagu acuacn                                                    16

<210> 1166
<211> 16
<212> RNA
<213> Artificial Sequence

<220>
<223> Sense sequence of siRNAs targeting SEQ ID NO:5

<220>
<221> misc_feature
<222> 16
<223> /note="n is GAUC"

<400> 1166
cgccuacauc aagaan                                                    16

<210> 1167
<211> 16
<212> RNA
<213> Artificial Sequence

<220>

<223> Sense sequence of siRNAs targeting SEQ ID NO:8

<220>
<221> misc_feature
<222> 16
<223> /note="n is CAUC or CAUCA, and preferably is CAUCA"

<400> 1167
gaccaccaag uacuan                                                    16


<210> 1168
<211> 19
<212> DNA
<213> Homo sapiens


<220>
<223> cDNA

<400> 1168
tccttcaagt atgcctggg                                                 19


<210> 1169
<211> 19
<212> DNA
<213> Homo sapiens


<220>
<223> cDNA

<400> 1169
ccttcaagta tgcctgggt                                                 19


<210> 1170
<211> 19
<212> DNA
<213> Homo sapiens


<220>
<223> cDNA

<400> 1170
atctccaaga atgggcaga                                                 19


<210> 1171
<211> 19
<212> DNA
<213> Homo sapiens


<220>
<223> cDNA

<400> 1171
tctccaagaa tgggcagac                                                 19

<210> 1172
<211> 19
<212> DNA
<213> Homo sapiens

<220>
<223> cDNA

<400> 1172
ggtgacaaca tgctggagc                                                    19

<210> 1173
<211> 19
<212> DNA
<213> Homo sapiens

<220>
<223> cDNA

<400> 1173
gtgacaacat gctggagcc                                                    19

<210> 1174
<211> 19
<212> RNA
<213> Artificial Sequence

<220>
<223> antisense strand of short dsRNA complementary to human EEF1A gene
      paralogs

<400> 1174
cccaggcaua cuugaagga                                                    19

<210> 1175
<211> 19
<212> RNA
<213> Artificial Sequence

<220>
<223> antisense strand of short dsRNA complementary to human EEF1A gene
      paralogs

<400> 1175
acccaggcau acuugaagg                                                    19

<210> 1176
<211> 19
<212> RNA
<213> Artificial Sequence

<220>
<223> antisense strand of short dsRNA complementary to human EEF1A gene
      paralogs

<400> 1176
ucugcccauu cuuggagau                                                    19

<210> 1177
<211> 19
<212> RNA
<213> Artificial Sequence

<220>
<223> antisense strand of short dsRNA complementary to human EEF1A gene
      paralogs

<400> 1177
gucugcccau ucuuggaga                                                    19

<210> 1178
<211> 19
<212> RNA
<213> Artificial Sequence

<220>
<223> antisense strand of short dsRNA complementary to human EEF1A gene
      paralogs

<400> 1178
gcuccagcau guugucacc                                                    19

<210> 1179
<211> 19
<212> RNA
<213> Artificial Sequence

<220>
<223> antisense strand of short dsRNA complementary to human EEF1A gene
      paralogs

<400> 1179
ggcuccagca uguugucac                                                    19

<210> 1180
<211> 19
<212> RNA
<213> Artificial Sequence

<220>
<223> antisense strand of short dsRNA complementary to human EEF1A gene
      paralogs

<400> 1180
ggcuccagca uguugucac                                                    19

<210> 1181
<211> 19

<212> RNA
<213> Artificial Sequence

<220>
<223> sense strand of short dsRNA complementary to SEQ ID NO: 1174

<400> 1181
uccuucaagu augccuggg                                                    19

<210> 1182
<211> 19
<212> RNA
<213> Artificial Sequence

<220>
<223> sense strand of short dsRNA complementary to SEQ ID NO: 1175

<400> 1182
ccuucaagua ugccugggu                                                    19

<210> 1183
<211> 19
<212> RNA
<213> Artificial Sequence

<220>
<223> sense strand of short dsRNA complementary to SEQ ID NO: 1176

<400> 1183
aucuccaaga augggcaga                                                    19

<210> 1184
<211> 19
<212> RNA
<213> Artificial Sequence

<220>
<223> sense strand of short dsRNA complementary to SEQ ID NO: 1177

<400> 1184
ucuccaagaa ugggcagac                                                    19

<210> 1185
<211> 19
<212> RNA
<213> Artificial Sequence

<220>
<223> sense strand of short dsRNA complementary to SEQ ID NO: 1178

<400> 1185
ggugacaaca ugcuggagc                                                    19

<210> 1186
<211> 19
<212> RNA
<213> Artificial Sequence

<220>
<223> sense strand of short dsRNA complementary to SEQ ID NO: 1179

<400> 1186
gtgacaacat gctggagcc                                                    19

<210> 1187
<211> 19
<212> RNA
<213> Artificial Sequence

<220>
<223> sense strand of short dsRNA complementary to SEQ ID NO: 1180

<400> 1187
gugacaacau gcuggagcc                                                    19

<210> 1188
<211> 20
<212> RNA
<213> Artificial Sequence

<220>
<223> Common region for dsRNA antisense strands of EEF1A paralogs

<400> 1188
acccaggcau acuugaagga                                                   20

<210> 1189
<211> 20
<212> RNA
<213> Artificial Sequence

<220>
<223> Common region for dsRNA antisense strands of EEF1A paralogs

<400> 1189
gucugcccau ucuuggagau                                                   20

<210> 1190
<211> 20
<212> RNA
<213> Artificial Sequence

<220>
<223> Common region for dsRNA antisense strands of EEF1A paralogs

<400> 1190
ggcuccagca uguugucacc                                                   20

<210> 1191
<211> 20
<212> RNA
<213> Artificial Sequence

<220>
<223> Common region for dsRNA sense strands of EEF1A paralogs

<400> 1191
uccuucaagu augccugggu                                                    20

<210> 1192
<211> 20
<212> RNA
<213> Artificial Sequence

<220>
<223> Common region for dsRNA sense strands of EEF1A paralogs

<400> 1192
aucuccaaga augggcagac                                                    20

<210> 1193
<211> 20
<212> RNA
<213> Artificial Sequence

<220>
<223> Common region for dsRNA sense strands of EEF1A paralogs

<220>
<221> misc_feature
<222> 3
<223> /note="n is U, T or dT (deoxythymine or 2'H thymine), and
      preferably U"

<220>
<221> misc_feature
<222> 11
<223> /note="n is U, T or dT (deoxythymine or 2'H thymine), and
      preferably U"

<220>
<221> misc_feature
<222> 14
<223> /note="n is U, T or dT (deoxythymine or 2'H thymine), and
      preferably U"

<400> 1193
ggngacaaca ngcnggagcc                                                    20

<210> 1194
<211> 19
<212> RNA
<213> Artificial Sequence

<220>
<223> dsRNA antisense strand of EEF1A paralogs

<400> 1194
ggcuccagca uguugucac                                                    19


<210> 1195
<211> 19
<212> RNA
<213> Artificial Sequence


<220>
<223> dsRNA sense strand of EEF1A paralogs

<220>
<221> misc_feature
<222> 2
<223> /note="n is U, T or dT (deoxythymine or 2'H thymine), and
      preferably U"

<220>
<221> misc_feature
<222> 10
<223> /note="n is U, T or dT (deoxythymine or 2'H thymine), and
      preferably U"

<220>
<221> misc_feature
<222> 13
<223> /note="n is U, T or dT (deoxythymine or 2'H thymine), and
      preferably U"

<400> 1195
gngacaacan gcnggagcc                                                    19


<210> 1196
<211> 18
<212> RNA
<213> Artificial Sequence


<220>
<223> dsRNA antisense strands targeting sequences SEQ ID NO. 1168 or
      SEQ ID NO. 1169 of both human EEF1A gene paralogs

<220>
<221> misc_feature
<222> 1
<223> /note="n is AC or C"

<220>
<221> misc_feature
<222> 18
<223> /note="n is G or GA"

<400> 1196
nccaggcaua cuugaagn                                                     18

<210> 1197
<211> 18
<212> RNA
<213> Artificial Sequence

<220>
<223> dsRNA antisense strands targeting sequences SEQ ID NO. 1170 or
      SEQ ID NO. 1171 of both human EEF1A gene paralogs

<220>
<221> misc_feature
<222> 1
<223> /note="n is GU or U"

<220>
<221> misc_feature
<222> 18
<223> /note="n is A or AU"

<400> 1197
ncugcccauu cuuggagn                                                    18

<210> 1198
<211> 18
<212> RNA
<213> Artificial Sequence

<220>
<223> dsRNA antisense strands targeting sequences SEQ ID NO. 1172 or
      SEQ ID NO. 1173 of both human EEF1A gene paralogs

<220>
<221> misc_feature
<222> 1
<223> /note="n is GG or G"

<220>
<221> misc_feature
<222> 18
<223> /note="n is C or CC"

<400> 1198
ncuccagcau guugucan                                                    18

<210> 1199
<211> 18
<212> RNA
<213> Artificial Sequence

<220>
<223> sense strands complementary to SEQ ID NO. 1196

<220>
<221> misc_feature
<222> 1
<223> /note="n is UC or C"

<220>
<221> misc_feature
<222> 18
<223> /note="n is G or GU"

<400> 1199
ncuucaagua ugccuggn                                                    18


<210> 1200
<211> 18
<212> RNA
<213> Artificial Sequence


<220>
<223> sense strands complementary to SEQ ID NO. 1197

<220>
<221> misc_feature
<222> 1
<223> /note="n is AU or U"

<220>
<221> misc_feature
<222> 18
<223> /note="n is A or AC"

<400> 1200
ncuccaagaa ugggcagn                                                    18


<210> 1201
<211> 18
<212> RNA
<213> Artificial Sequence


<220>
<223> sense strands complementary to SEQ ID NO. 1198

<220>
<221> misc_feature
<222> 1
<223> /note="n is GG or G"

<220>
<221> misc_feature
<222> 2
<223> /note="n is U, T or dT, and preferably is U"

<220>
<221> misc_feature
<222> 10
<223> /note="n is U, T or dT, and preferably is U"

<220>
<221> misc_feature
<222> 13
<223> /note="n is U, T or dT, and preferably is U"

<220>
<221> misc_feature

<222> 18
<223> /note="n is C or CC"

<400> 1201
nngacaacan gcnggagn                                                    18

## Description

### FIELD OF THE INVENTION

The present invention provides compositions and methods for modulating the expression of a gene encoding for an eukaryotic translation elongation factor, such as the Eukaryotic Translation Elongation Factor 1 Alpha 2 (EEF1A2) gene, which can be useful in reducing viral replication and managing viral infections or infestations. In particular, this invention relates to oligonucleotide compounds, for example small interfering RNA (siRNA) compounds, which in some embodiments hybridize with nucleic acid molecules encoding EEF1A2, an abundant, multifunctional host protein which has been subverted by some viruses and seems to be involved in viral replication. Such compounds are shown herein to reduce the expression of EEF1A2 in cells, which can be useful for diminishing the replication of viruses, mainly RNA viruses including those causing infection in the human population, for example, coronaviruses such as SARS-CoV-2 or influenza viruses such as influenza virus type A, and can be also useful for reducing, preventing and treating such viral infections or their related diseases.

### BACKGROUND OF THE INVENTION

Viruses are obligate intracellular parasites. They have small genomes compared to the host genome and require host cellular machineries such as those for protein translation, mRNA transcription, membrane biogenesis, and many more functions to complete the virus life cycle.

RNA viruses can be highly pathogenic to humans, animals, plants, and even bacteria. Indeed, notable human diseases such as the common cold, influenza, severe acute respiratory syndrome (SARS), coronavirus disease 2019 (COVID-19), hepatitis C, hepatitis E, West Nile fever, Ebola virus disease, rabies, polio and measles are caused by RNA viruses. These kind of viruses have RNA (ribonucleic acid) genomes which may be either a double strand or single strand of RNA, and in case of single stranded genomes, the RNA strand may be of the same polarity as mRNA (plus- or positive-sense) and will encode the genes, or complementary to mRNA (minus- or negative-sense) and must be transcribed into positive-sense RNA by an RNA polymerase before translation. Positive-sense viral RNA is similar to mRNA and thus can be immediately translated by the host cell and can directly cause infection. Negative-sense viral RNA is complementary to mRNA and must be converted to positive-sense RNA by an RNA-dependent RNA polymerase before translation. In contrast, purified RNA of a negative-sense virus is not infectious by itself as it needs to be transcribed into positive-sense RNA. We can also find ambisense RNA viruses, which can use their genomes in both positive-sense and negative-sense directions and some even contain both positive-sense and negative-sense sections (Knipe and Howley, 2013).

The genomes of RNA viruses are usually smaller than the genomes of DNA viruses, and RNA viruses are therefore more reliant on subverting host-cell proteins, and cellular structures and functions to facilitate virus replication (Li, Wei et al. 2013). For example, several RNA viruses have subverted cellular the eukaryotic translation elongation factor 1 alpha (eEF1A) for viral replication and genome synthesis (Snape et al. 2018).

Translation elongation in eukaryotes requires a set of non-ribosomal proteins called eukaryotic elongation factors (eEFs), with eEF1A being one of the most abundant proteins in eukaryotic cells (Hwang et al. 2015). The protein is involved in delivering aminoacyl-tRNAs to ribosomes for polypeptide synthesis. In addition to its canonical role in translational elongation by ribosomes, eEF1A binds to RNA and actin in non-canonical cellular roles outside protein synthesis, including protein degradation, cellular apoptosis, nucleocytoplasmic trafficking, heat shock responses, and cytoskeletal regulation (Wei et al. 2014). Some different mechanisms that viral pathogens use to usurp host eEF1A proteins for their replication can be found in the review of Li, Wei et al. 2013 and references included therein. For example, eEF1A can bind aminoacylated tRNAs and some viruses use this RNA binding ability to regulate their life cycle. eEF1A1 was shown to bind tRNA-like structures in the 3' untranslated regions (3'UTRs) of several plant viral genomes, including turnip yellow mosaic virus (TYMV), tobacco mosaic virus (TMV), and brome mosaic virus (BMV), which can be aminoacylated and have important roles in virus replication, such as the enhancement of virus protein translation, *in vitro* repression of virus minus-strand RNA synthesis and facilitation of virus RNA encapsidation. In tomato bushy stunt virus (TBSV), a plus-strand RNA virus member of the *Tombusvirus* genus in the *Tombusviridae* family, evidence suggests that eEF1A interacts with a 5-nucleotide-long sequence located within an internal stem-loop (SL) region of the 3'UTR of viral genomic RNA to stimulate minus-strand RNA synthesis during viral replication. In West Nile virus (WNV), a mosquito-borne member of the *Flavivirus* genus within the family *Flaviviridae* having a single-stranded positive-sense RNA genome, it was seen that a conserved SL structure at the 3'UTR binds eEF1A specifically, and two lines of evidence suggest it facilitates viral minus-strand synthesis. This role played by eEF1A in minus-strand synthesis has been also suggested in *Flavivirus* replication, as eEF1A was shown to bind with similar efficiency to the 3'-terminal SL RNAs of four divergent *Flavivirus* members, including tick-borne encephalitis virus, dengue virus, and yellow fever virus.

The eEF1A subunits of the eukaryotic elongation factor 1 interact with viral RNA-dependent RNA polymerases (RdRps) and other replication complex proteins from a variety of plant and animal viruses, such as TMV, TBSV, WNV, and the vesicular stomatitis virus (VSV), a non-segmented negative-strand RNA virus of the *Vesiculovirus* genus in the family *Rhabdoviridae,* and some lines of evidence have shown that it has an important role in the genomic viral replication and/or viral transcription of such viruses. Other studies have shown that eEF1A associates with reverse transcriptase (RT) of human immunodeficiency virus 1 (HIV-1) and that HIV-1 reverse transcription is inhibited when the target cells were pre-treated with small interfering RNA (siRNA) targeting eEF1A (Warren et al. 2012). Moreover, several structural and non-structural proteins of different viruses have shown to interact also with eEF1A. For example, eEF1A has been identified as interacting with non-structural protein 5A (NS5A) of bovine viral diarrhea virus (BVDV), with NS4A of hepatitis C virus (HCV), with the precursor of the mature 3C protease and 3D poliovirus 3CDpro, with HIV-1 proteins such as integrase, Gag protein, and Nef protein, with the viral oncoproteins E6 and E7 of cutaneous human papillomavirus (HPV) type 38, and with the X protein of hepatitis B virus (HBV). Additionally, eEF1A has been found in purified particles of different viruses, including vesicular stomatitis virus (Rhabdoviridae), vaccinia virus (Poxviridae), cytomegalovirus (Herpesviridae), severe acute respiratory syndrome coronavirus (SARS-CoV) (Coronaviridae), and HIV-1 (Retroviridae). But it is not clear if encapsidation is biologically important for the replication of these viruses or if encapsidation is simply a consequence of the relative abundance of eEF1A.

There are two isoforms of eEF1A, eEF1A1 and eEF1A2, which share 92% amino acid identity and are encoded by the two single copy gene paralogs EEF1A1 and EEF1A2, respectively (Li, Wei et al. 2013, Li, Rawle et al. 2019). However, despite the high identity at the amino acid level, the sequence identity at the nucleotide level is actually somewhat lower, which has been described to be 75% in the case of the human paralogs (Abbas et al. 2015). The EEF1A paralogs are differentially expressed depending on the cell type. Whereas paralog EEF1A1 is almost ubiquitously expressed in many cell types, EEF1A2 is expressed in terminally differentiated muscle, heart, neuronal tissue and in some specialized cells of pancreatic islet and gut tissues (Li, Rawle et al. 2019). Nevertheless, certain tumor cell types and cell lines also express both eEF1A isoforms (Abbas et al. 2015). This might suggest that the cellular expression pattern of these genes in cells could be altered or could change under pathological or specific environmental conditions.

As disturbing the viral subversion of eEF1A without affecting the crucial cellular role in protein synthesis is difficult, it is recognized that targeting EEF1A genes or their encoded proteins as an antiviral strategy is challenging due to their different functional profiles and post-transcriptional and post-translational regulation mechanisms. Despite sharing 92% sequence identity, paralogous human translation elongation factor 1 alpha-1 (eEF1A1) and elongation factor 1 alpha-2 (eEF1A2) have different but overlapping functional profiles. This may reflect the differential requirements of the cell-types in which they are expressed and is consistent with complex roles for these proteins that extend beyond delivery of tRNA to the ribosome. These differences are related to interactions with residues previously inferred to be directly involved in binding GTP/GDP, eEF1 Balpha and aminoacyl-tRNA, and also related to different phosphorylation patterns (Soares et al. 2009) and their combination to other EF1 complex members in different species (Sasikumar et al. 2012).

However, precise targeting of eEF1A specific domains has been suggested as a promising therapeutic avenue for further research (Li et al. 2013). This might encourage therapeutic approaches based on, for example, the use of antibodies, aptamers or inhibitors that partially block the activity or functionality of the desired domain, but not based on compounds that may almost completely or fully suppress EEF1A activity or its expression, as in case of RNAi molecules.

The fact that eEF1A is the common denominator in the infection processes of different RNA viruses and given the relevance of eEF1A in the control of the different cellular mechanisms, it seems reasonable to act on this protein complex as a strategy to prevent the replication and the infection produced by the RNA viruses responsible for different human and animal and plant diseases. eEF1A can be positioned as a molecular target that will allow common antiviral approaches as a pan therapeutic alternative for RNA virus-induced diseases. This approach would have an impact on viral diseases affecting human health such as seasonal influenza infections and the current health crisis of COVID-19, both without an effective health response, and responsible for thousands of deaths, or even on viruses affecting the production of various vegetable and ornamental crops, such as *Tobamovirus* tomato brown rugose fruit virus (ToBRFV), which are responsible for millions of dollars of losses in agriculture. Some approaches based on eEF1A inhibitors have already begun to be made in the current COVID-19 crisis with drugs such as plitidepsin (Aplidin^{®}). Plitidepsin is a cyclic depsipeptide that targets eEF1A2 administered by intravenous (IV) infusion that is currently approved in Australia in combination with dexamethasone, for the treatment of patients with relapsed and refractory multiple myeloma. In tumor cells, plitidepsin induces a potent oxidative stress which finally activates a caspase-dependent apoptotic cell death pathway (Losada et al. 2016). More recently, it has been described that this compound has demonstrated *in vitro* efficacy against SARS-CoV-2 and other types of coronaviruses, showing that the product reduced SARS-CoV-2 viral load at nanomolar concentrations in the *in vitro* studies. In addition, this product has recently met the primary safety endpoint in a phase I clinical trial carried out in patients with COVID-19 who required hospitalization, and also met preliminary secondary endpoints of efficacy by achieving a substantial reduction in viral load and the C-reactive protein (CRP) in treated patients.

Several members of the family *Coronaviridae* usually cause mild respiratory disease in humans (Corman et al., 2019). However, other family members such as the severe acute respiratory syndrome coronavirus (SARS-CoV) and the Middle East respiratory syndrome coronavirus (MERS-CoV) are transmitted from animals to humans and cause severe respiratory diseases in afflicted individuals, SARS and MERS, respectively (Fehr et al., 2017). More recently, a new infectious respiratory disease has emerged which is termed coronavirus disease 2019 (COVID-19) and is caused by severe acute respiratory syndrome coronavirus 2 (SARS-CoV-2), a novel coronavirus closely related to SARS-CoV identified at the end of the year 2019 (Hoffmann et al., 2020), previously known as "2019 novel coronavirus" or 2019-nCoV. COVID-19 can seriously damage the respiratory system, with some patients developing acute respiratory infection symptoms, and even acute respiratory distress syndrome (ARDS), acute respiratory failure and other complications, such as in the cardiovascular system (Guzik et al 2020) and central nervous system (ladecola et al 2020) which in some cases even lead to death.

In fact, as of November 2020, SARS-CoV-2 has been widely spread worldwide in more than 200 countries, with more than 50,000,000 people suffering from the disease and over 1,250,000 deaths, making this virus a major threat to global public health.

Annual deaths due to respiratory conditions associated with seasonal flu (influenza) are also not negligible, thus, up to 650,000 deaths annually are associated with respiratory diseases from seasonal influenza, according to new estimates by the United States Centers for Disease Control and Prevention (US-CDC), the World Health Organization and global health partners.

Vaccination is still the most efficacious method of preventing viral infections (otherthan avoiding exposure); however, this is still a challenge for many viruses, especially in highly mutating viruses, such as RNA viruses (a group which includes the Coronaviruses and other respiratory viruses). Among them we can find the influenza virus, that requires annual vaccine re-formulation and prediction of likely prevalent strains each year and only reduces the risk of flu illness by between 40% and 60% among the overall population during seasons when most circulating flu viruses are well-matched to the flu vaccine. In the case of viruses such as respiratory syncytial virus (RSV), vaccine development has major challenges such as the inadequate response to vaccination during immunization of very young infants and the circulation of two antigenically distinct RSV groups (A and B).

Unfortunately, there are currently not enough effective and specific therapies that have been registered for treating such coronavirus and influenza infections. Besides, vaccination against influenza viruses is not fully effective and potential vaccines for the prevention of COVID-19 are still in development. Unlike vaccines, which may not be available during the early stages of a pandemic, antiviral medicines can be used for the treatment of infected patients from the onset of the pandemic, and having broad spectrum antiviral products ready for use can play an important role in the management of a pandemic caused by any of these type of viruses or in any other future viral outbreaks. Consequently, there is an urgent need for agents capable of treating or preventing virus infections causing devastating diseases and/or pandemics, mainly coronavirus and flu infections, and/or that to some extent are capable of reducing the viral load, the symptoms or complications related to such viral infections.

Although some viruses that affect plants have double-stranded (ds) RNA, single-stranded (ss) DNA or dsDNA genomes, most plant viruses have an RNA genome, which is usually small and single stranded, similarly to many viruses causing pandemics in humans.

Damages caused by pests and diseases have a considerable negative economic impact in agriculture, with emergent viral diseases being particularly important. Unlike bacteria or fungi that can be treated with antibacterial or antifungal agents, respectively, curing plants once they have been infected by a virus is not feasible. Eradication or control of virus diseases is difficult given the complex and dynamic nature of virus epidemics and the great evolvability of viruses, and currently, disease management in agriculture is based on two approaches: immunization to get resistant plants to viral infections and prophylactic measures to restrain virus dispersion (Rubio et al. 2020, and references cited therein). However, resistance is not available for most crops and viruses, and sometimes just provides a reduction of virus accumulation (i.e. relative or partial resistance) or a reduction of virus damage without affecting virus multiplication (i.e. tolerance). In addition, resistance achieved by genetic engineering faces heavy opposition in some countries due to the potential impact of transgenic plants. Indeed, disease management for most plant virus diseases mainly relies on preventing introduction of new viruses by border control and certification of virus-free propagative material (e.g., seeds) and preventing virus dispersal in the field at the present time (Rubio et al. 2020). Thus, there is also need for the development of new antiviral compounds that can be useful for preventing and/or treating viral infections in plants and crops.

Tobamoviruses are among the most important plant viruses causing severe damages in agriculture, especially to vegetable and ornamental crops around the world. Tobamoviruses are easily transmitted by mechanical means while there is no evidence of a natural vector, as well as through seed transmission. Tobamoviruses are generally characterized by a rod-shaped particle of about 300 nm, their structure consists of a single stranded, positive RNA genome encoding four proteins, encapsidated by 17KDa coat protein (CP) molecules. According to the most recent ICTV Master Species List from the International Committee on Taxonomy of Viruses (ICTV), Tobamovirus genus currently comprises the following virus species: Bell pepper mottle virus, Brugmansia mild mottle virus, Cactus mild mottle virus, Clitoria yellow mottle virus, Cucumber fruit mottle mosaic virus, Cucumber green mottle mosaic virus, Cucumber mottle virus, Frangipani mosaic virus, Hibiscus latent Fort Pierce virus, Hibiscus latent Singapore virus, Kyuri green mottle mosaic virus, Maracuja mosaic virus, Obuda pepper virus, Odontoglossum ringspot virus, Opuntia chlorotic ringspot virus, Paprika mild mottle virus, Passion fruit mosaic virus, Pepper mild mottle virus, Plumeria mosaic virus, Rattail cactus necrosis-associated virus, Rehmannia mosaic virus, Ribgrass mosaic virus, Streptocarpus flower break virus, Sunn-hemp mosaic virus, Tobacco latent virus, Tobacco mild green mosaic virus, Tobacco mosaic virus, Tomato brown rugose fruit virus, Tomato mosaic virus, Tomato mottle mosaic virus, Tropical soda apple mosaic virus, Turnip vein-clearing virus, Ullucus mild mottle virus, Wasabi mottle virus, Yellow tailflower mild mottle virus, Youcai mosaic virus, and Zucchini green mottle mosaic virus.

Tomato (*Solanum lycopersicum*) in the Solanaceae family has become one of the most important and widely grown crops, with a global production of over 45 million tons between 2007 and 2017 (FAO 2017). In tomatoes, tobacco mosaic virus (TMV) and tomato mosaic virus (ToMV) are feared by growers worldwide as they can severely damage crop production, for example through irregular ripening (fruits having yellowish patches on the surface and brownish spots beneath the surface).

During 2014-2015, a severe outbreak of virus affected tomato production areas in the middle east, such as in Jordan and Israel. Most of the tomato varieties affected were considered TMV and/or ToMV resistant, but were still severely affected and showed typical TMV/ToMV like symptoms: while the foliar ones were quite similar to the TMV/ToMV symptoms, the fruit symptoms were much more frequent and severe than the usual symptoms from such viruses with fruits lesions and deformations. Later, a new virus was identified which was proposed to be named Tomato Brown Rugose Fruit virus (TBRFV or ToBRFV). Since its identification in 2015, ToBRFV outbreaks have recently occurred in Italy, Mexico, Turkey, China, the United Kingdom, the Netherlands, Cyprus, Greece, Germany, the Czech Republic, Belgium, Hungary, Spain and France, where the virus is of great concern for tomato and pepper growers. Tomato (*Solanum lycopersicum*) and pepper (*Capsicum sp*.) are the main hosts of ToBRFV. However, inoculation experiments showed that *Nicotiana bentamiana, N. glutinosa, N. sylvestris, N. tabacum* (tobacco) develop symptoms and that weeds such as *Chenopodiastrum murale* and *Solanum nigrum* can act as reservoirs for ToBRFV.

As Tobamoviruses are not easily controlled other than through genetic improvement by the identification and use in breeding of resistance genes, and as the resistance genes currently available to control TMV and/or ToMV are useless against the damage and propagation from the new Tomato Brown Rugose Fruit virus (ToBRFV), there is an urgent need to identify antiviral agents against this new Tobamovirus, to prevent its dissemination and spreading in the plant and crops.

When there is no good vaccine or effective treatment, current drug research and development can offer new specific biologically active molecules designed against targets that are responsible for virus disease infection and progression. In these approaches, we can consider proteins such as eEF1A proteins (i.e. eEF1A2, eEF1A1 or both), and/or other proteins such as receptors relevant in viruses' entry and progression of infection processes in different tissues. Relevant receptors include angiotensin converting enzyme 2 (ACE2), the transmembrane Protease Serine 2 (TMPRSS2) and (AT1R) (Hoffmann et al., 2020) highly expressed in relevant tissues for coronaviruses infection such as nasopharynx, lungs, cardiovascular and central nervous system among others (Harmer et al. 2002, Hamming et al. 2004, Sun et al. 2020). In the case of Influenza viruses we find similar receptors on which we could act, such as HA receptor-binding site with α-2,3 or α-2,6 -linked SAs residues, or even acting on proteins involved in endolysosomal fusion processes such as dynamine, Epsin 1 and M2 ion channel (Dou et al 2018). The action on this type of molecular targets could efficiently block viral replication in infected individuals and therefore serve as promising therapeutic interventions against several viral infections. In this regard, nucleic acid-based molecules have shown tremendous potential to block gene expression either during the transcriptional or post-transcriptional phases (Asha et al., 2019).

Therefore, inhibiting or reducing the expression levels of any of these eEF1A proteins (eEF1A2 or eEF1A1) or both, alone or in combination with other targets such as those mentioned above could be a useful approach for managing the prevention or treatment of SARS-CoV-2 infection or COVID-19, as well as the prevention or treatment of other virus infections and their related diseases where the virus requires eEF1A proteins expressed in host cells for completing the viral cycle and spreading in the infected organism.

Among the technologies available to address these types of molecular targets, a good alternative to EEF1A2 inhibitor drugs for the treatment of such virus diseases are RNA interference (RNAi) based drugs. Since RNAi therapies target RNA, these are very advantageous over conventional small molecule drugs used for therapy against respiratory diseases.

RNAi is a naturally occurring post-transcriptional regulatory mechanism present in most eukaryotic cells that uses small double stranded RNA (dsRNA) molecules to direct homology-dependent gene silencing. Its discovery by Fire and Mello in the worm *C. elegans* (Fire et al 1998) was awarded the Nobel Prize in 2006. Shortly after its first description, RNAi was also shown to occur in mammalian cells by means of double-stranded small interfering RNAs (siRNAs) 21 nucleotides long (Elbashir et al 2001).

The process of RNA interference is thought to be an evolutionarily-conserved cellular defence mechanism used to prevent the expression of foreign genes and is commonly shared by diverse phyla and flora, where it is called post-transcriptional gene silencing. Since the discovery of the RNAi mechanism there has been an explosion of research to uncover new compounds that can selectively alter gene expression as a new way to treat human disease by addressing targets that are otherwise "undruggable" with traditional pharmaceutical approaches involving small molecules or proteins.

According to current knowledge, the mechanism of RNAi is initiated when long double stranded RNAs are processed by an RNase III-like protein known as Dicer. The protein Dicer typically contains an N-terminal RNA helicase domain, an RNA-binding so-called Piwi/Argonaute/Zwille (PAZ) domain, two RNase III domains and a double-stranded RNA binding domain (dsRBD) (Collins et al 2005) and its activity leads to the processing of the long double stranded RNAs into 21-24 nucleotide double stranded siRNAs with 2 base 3' overhangs and a 5' phosphate and 3' hydroxyl group. The resulting siRNA duplexes are then incorporated into the effector complex known as RNA-induced silencing complex (RISC), where the antisense or guide strand of the siRNA guides RISC to recognize and cleave target mRNA sequences (Elbashir et al 2001) upon adenosine-triphosphate (ATP)-dependent unwinding of the double-stranded siRNA molecule through an RNA helicase activity (Nykanen et al 2001). The catalytic activity of RISC, which leads to mRNA degradation, is mediated by the endonuclease Argonaute 2 (AGO2) (Liu et al 2004; Song et al 2004). AGO2 belongs to the highly conserved Argonaute family of proteins. Argonaute proteins are ∼100 KDa highly basic proteins that contain two common domains, namely PIWI and PAZ domains (Cerutti et al 2000). The PIWI domain is crucial for the interaction with Dicer and contains the nuclease activity responsible for the cleavage of mRNAs. AGO2 uses one strand of the siRNA duplex as a guide to find messenger RNAs containing complementary sequences and cleaves the phosphodiester backbone between bases 10 and 11 relative to the guide strand's 5' end (Elbashir et al 2001). An important step during the activation of RISC is the cleavage of the sense or passenger strand by AGO2, removing this strand from the complex (Rand et al 2005). Crystallography studies analyzing the interaction between the siRNA guide strand and the PIWI domain reveal that it is only nucleotides 2 to 8 that constitute a "seed sequence" that directs target mRNA recognition by RISC, and that a mismatch of a single nucleotide in this sequence may drastically affect silencing capability of the molecule (Ma et al 2005; Doench et al 2004; Lewis et al 2003). Once the mRNA has been cleaved, due to the presence of unprotected RNA ends in the fragments the mRNA is further cleaved and degraded by intracellular nucleases and will no longer be translated into proteins (Orban et al 2005) while RISC will be recycled for subsequent rounds (Hutvagner et al 2002). This constitutes a catalytic process leading to the selective reduction of specific mRNA molecules and the corresponding proteins. It is possible to exploit this native mechanism for gene silencing with the purpose of regulating any gene(s) of choice by directly delivering siRNA effectors into the cells or tissues, where they will activate RISC and produce a potent and specific silencing of the targeted mRNA. RNAi has been applied in biomedical research such as treatment for HIV, viral hepatitis, cardiovascular and cerebrovascular diseases, metabolic disease, neurodegenerative disorders and cancer (Angaji SA et al 2010) and it has also emerged as a viable therapy with two RNAi based therapies being approved in the last two years (Godinho and Khvorova, 2019). In 2018, the first formulation-based RNAi therapeutic, Patisiran (Onpattro^{™}), was approved by the Food and Drug Administration (FDA) and the European Medicines Agency (EMA), and approximately a year later, another breakthrough was achieved with the approval of Givosiran (GIVLAARI^{™}), the first siRNA using the conjugate-mediated approach for targeted delivery to hepatocytes.

Many studies have been published describing the ideal features a siRNA should have to achieve maximum effectiveness, regarding length, structure, chemical composition, and sequence. Initial parameters for siRNA design were set out by Tuschl and co-workers in WO02/44321, although many subsequent studies, algorithms and/or improvements have been published since then. siRNA selection approaches have become more sophisticated as mechanistic details have emerged; in addition, further analysis of existing and new data can provide additional insights into further refinement of these approaches (Walton SP et al 2010). Alternatively, several recent studies reported the design and analysis of novel RNAi-triggering structures distinct from the classical 19+2 siRNA structure and which do not conform to the key features of classical siRNA in terms of overhang, length, or symmetry, discussing the flexibility of the RNAi machinery in mammalian cells (Chang CI et al 2011).

Also, a lot of efforts have been put into enhancing siRNA stability as this is perceived as one of the potential obstacles for therapy based on siRNA, given the ubiquitous nature of RNAses in biological fluids. Another inherent problem of siRNA molecules is their immunogenicity, whereby siRNAs have been found to induce unspecific activation of the innate immune system. The knockdown of unintended genes (mRNAs) is a well-known side effect of siRNA-mediated gene silencing. It is caused as a result of partial complementarity between the siRNA and mRNAs other than the intended target and causes off-target effects (OTEs) from genes having sequence complementarity to either siRNA strand. One of the main strategies followed for stability enhancement and OTE reduction has been the use of modified nucleotides such as 2'-O-methyl nucleotides, 2'-amino nucleotides, or nucleotides containing 2'-O or 4'-C methylene bridges. Also, the modification of the ribonucleotide backbone connecting adjacent nucleotides has been described, mainly by the introduction of phosphorothioate modified nucleotides. It seems that enhanced stability and/or reduction of immunogenicity are often inversely proportional to efficacy (Parrish, 2000), and only a certain number, positions and/or combinations of modified nucleotides may result in a stable and non-immunogenic silencing compound. As this is an important hurdle for siRNA-based treatments, different studies have been published which describe certain modification patterns showing good results, examples of such include EP1527176, WO2008/050329, WO2008/104978 or WO2009/044392, although many more may be found in the literature (Sanghvi YS. 2011; Deleavey et al 2012).

Other important hurdles for RNAi therapies include the requirement for the siRNA drugs to be delivered to the intended targeted cells and tissues, and to be internalized into the specific cells, where the compound further will need to be released from the endosomal compartment. These problems are not yet completely resolved. Indeed, many efforts have been also made to find optimal delivery strategies for in vivo applications of RNAi drugs, including formulation and conjugation, two approaches widely used and demonstrated to be useful in the clinic, as demonstrated by the two RNAi therapeutic products currently approved for use. Formulations can be employed with or without modifications to the siRNA molecule itself, whereas the conjugation approach relies heavily on chemical modifications to protect the oligonucleotide from nuclease attack. An overview of the strategies for delivery with formulation and conjugation of RNAi compounds, together with some examples of materials used as non-viral vectors, nanocarriers or in formulated nanosystems, as well as some ligands used for conjugation can be found in Godinho and Khvorova 2019 and in references included therein. Regarding formulations, examples of biomaterials that enhance biodistribution and promote cellular uptake of nucleic acids include cationic lipids (e.g. D-Lin-MC3-DMA), polymers (e.g. cyclodextrin-based polymers and biocollagen), polypeptides, and exosomes. These materials usually interact with nucleic acids and self-assemble into nanoparticles that protect the cargo from enzymatic degradation, and using cationic vectors (including for example cationic lipids and/or cationic nanoparticles) can confer a positive surface charge to the complex that facilitates the interaction of the positively charged complex surface with negatively-charged cellular membrane and endocytosis. Endosomolytic moieties (e.g. melittin-like peptides) or fusogenic lipids (e.g. DOPE) can also be included in nanosystems engineered to promote release from the endosomal compartment and enable immediate release of siRNAs to the cytoplasm, which can reduce dose requirements for *in vivo* administration. Also useful can be materials that can mask positive charges of the nanosystem, such as polyethyleneglycol (PEG)-lipid layer, and reduce *in vivo* aggregation, which together with the subsequent deposition in small capillary beds, such as alveolar capillaries in the lung, has been associated with toxicities of some nanosystems. Regarding conjugation of ligands to improve bioavailability of RNAi drugs, ligands have been successfully attached to the 5'- and/or 3'-end of the sense strand without affecting RISC loading. Examples of available ligands that have been investigated for such purposes include lipids (for example, cholesterol, docosahexanoic acid), carbohydrates (for example, N-acetylgalactosamine (GaINAc)), glycoproteins, and peptides or peptide derivatives (for example, transferrin, tat peptide, GLP1), folate, and vitamin E. Broad functional delivery to many different tissues can be achieved with lipid bioconjugates such as cholesterol and docosahexaenoic acid (DHA), which preferably are selected depending on their specific distribution profile and the desired tissue intended to be targeted. For example, conjugation to cholesterol has been suggested to extend the duration, but not the magnitude of siRNA-mediated knockdown on the target mRNA in mouse lung (Moschos et al. 2007). Other ligands, however, enable targeted delivery to specific cell types, such as GalNAc which enables targeted delivery of conjugated siRNAs to the liver, since the conjugates bind to the asialoglycoprotein receptor (ASGPR), which is highly expressed by hepatocytes.

The lung is a well-suited organ for the uptake of small, hydrophobic drug molecules due to the vast surface area and strong perfusion, although the air-blood barrier is only permeable to a limited extent for large, hydrophilic molecules, such as therapeutic nucleic acids. Besides, the lung imposes intrinsic hurdles to efficient siRNA delivery, including the reticulate pulmonary architecture ranging from the trachea to the alveoli, active clearance processes, such as mucociliary clearance and cough clearance, and effective immune responses, mainly mediated by macrophages and the influx of polymorphic neutrophils (PMNs) that prevent the invasion of foreign material into the lung, as well as the presence of respiratory mucus in the upper airways and airway surface liquid (surfactant) in the lower airways that constitute major physical and chemical barriers entrapping substances and slowing down their transport velocities. However, some obstacles encountered in systemic delivery of siRNA can be overcome by inhalation or aerosol delivery, which provides some advantages such as local targeting, immediate availability, decreased systemic side effects, and non-invasive application for the treatment of pulmonary diseases that affect lung epithelial cells such as cystic fibrosis, chronic obstructive pulmonary disease (COPD), asthma, and pulmonary fibrosis, as well as for preventing or treating infection of various viruses that infect the lung epithelium, including respiratory syncytial virus (RSV), parainfluenza virus (PIV), influenza, rhinoviruses, and severe acute respiratory syndrome (SARS) coronaviruses SARS-CoV and SARS-CoV-2. (Merkel et al. 2012).

Numerous studies conducted in experimental animal models as well as in cell culture systems have evaluated the efficiency and efficacy of nucleic acid-based therapeutics in post-transcriptional silencing of crucial viral or cellular genes to obstruct protein translation and inhibit viral replication. Some of these strategies have already proceeded to clinical trials. Indeed, since the first time RNAi was used against the respiratory syncytial virus (RSV), there has been an increase in the number of studies related to the potential of RNAi technology in the treatment of other respiratory viral infections including influenza, severe acute respiratory syndrome (SARS), and adenoviruses known to cause respiratory and gastrointestinal infections (Asha et al. 2019, and references included therein).

Despite all barriers, local therapy in the lung with intranasal siRNA has been achieved using naked siRNA, or after administration with isotonic dextrose solution, saline or phosphate buffered saline (PBS) as carrier, or in complex with transfection reagents. For example, intranasal administration of siRNAs designed against viral genes of respiratory syncytial virus (RSV) and parainfluenza virus (PIV) was shown to prevent and inhibit the individual or combined virus infection in mice, both in the presence or the absence of transfection agents (Barik and Lu, 2015). Barik and Lu further discloses that intranasal siRNA complexed with oligofectamine or polyethylenimine (PEI) and targeted to essential viral genes of influenza was also protective against influenza A viruses in mice. However, the authors remark the transient activity of these siRNAs, which only lasts a few days, and the continuing goal of enhancing the intra- and extracellular stability of the synthetic siRNAs while their silencing activities are increased or maintained for their therapeutic translation. Furthermore, the presence of pulmonary surfactants in the airway has been suggested as a natural carrier that facilitates the cellular uptake of siRNA lacking any delivery system.

However, the presence of respiratory mucus in the upper airways and airway surface liquid (surfactant) in the lower airways can constitute also major physical and chemical barriers hampering the cellular delivery of siRNA by entrapping siRNA loaded nanoparticles and by slowing down the velocity of their transport, and appropriate siRNA carrier systems might be necessary to improve the siRNA delivery to its target region or tissue and also improve its cellular uptake by the specific target cell (Merkel et al. 2012). Some obstacles to pulmonary siRNA delivery are discussed in Merkel et al. 2012 and several successful examples of formulations and administration routes can be found there and in cited references therein.

Different oligonucleotides that modulate expression of EEF1A genes and some of their applications have been already described. For example, WO03/104488 discloses antisense oligonucleotides for inhibiting specifically expression of eEF1A2 protein in tumor cells positive for this gene which are useful for the treatment of cancer, such as ovarian, breast and colorectal cancer. Likewise, the therapeutic utility in cancer treatment has been also approached by means of a lentivirus EEF1A2 shRNA which is able to reduce its expression in human hepatocellular carcinoma cells with detected levels of EEF1A2 and results in reduced cell proliferation, migration and invasion, increased apoptosis and induced cell cycle arrest (Qiu et al. 2016). In addition, the inactivation of EEF1A2 using a siRNA against this gene in human breast cancer cells has been shown to result in a reduction in filopodia formation, but also in a reduced phosphorylation of Akt, through which eEF1A2 could promote tumor development (Amiri et al. 2007).

Besides, it has been demonstrated that knockdown of endogenous eEF1A in HEK293T cells by siRNA treatment impairs HIV-1 reverse transcription efficiency (Warren et al. 2012). However, the siRNA used by Warren et al. specifically targets the paralog EEF1A1, which has been reported to be important for the replication of many viruses, but not EEF1A2, which paralog no study has specifically linked to virus replication (Li et al. 2019). Other examples of EEF1A1 siRNAs already described have shown that downregulation of EEF1A1 in human epithelial carcinoma cells Hep-2 can restrict the expression of viral genomic RNA and release of respiratory syncytial virus (RSV) (Wei et al. 2014), and can also reduce the formation of cellular filopodia induced by the virus (Snape et al. 2018).

Identifying new molecules that interfere or block virus replication more efficiently is of great importance in the development of new prophylactic and therapeutic agents against virus infections, especially in the case of viruses which cause epidemics and pandemics, or which are responsible for new outbreaks, such as in the case of many influenza A viruses, or in the case of the new pandemic coronavirus SARS-CoV-2 for which currently there is no approved prophylactic vaccine or specific therapeutic treatment.

In addition, in the particular case of the COVID-19 pandemic, it is believed that vaccines against SARS-CoV-2 might not be enough, and that COVID-19 countermeasures also need to include therapies that can treat the disease. In this regard, remdesivir and the anti-inflammatory steroid dexamethasone have both shown some benefit in patients with advanced COVID-19, but therapies that can address early stages of COVID-19 are still needed.

### SUMMARY OF THE INVENTION

The present invention provides improved products, in particular siRNA compounds, which are targeted to an Eukaryotic Translation Elongation Factor 1 Alpha (EEF1A) gene, in particular the eukaryotic translation elongation factor 1 alpha 2 (EEF1A2), and which modulate or reduce the expression and/or activity of EEF1A2 and/or the expression and/or activity of eukaryotic translation elongation factor 1 alpha 1 (EEF1A1), which are useful for preventing or treating virus infections which involve or require the proteins encoded by EEF1A2 and/or EEF1A1, preferably the protein encoded by EEF1A2 gene, in any step of the viral cycle, for example during the elongation process of the viral RNA translation into proteins within the host cell, and which consequently are useful for interfering with or reducing virus replication.

One of the advantages of using siRNA products for managing such virus infections versus traditional EEF1A inhibitors is that they are highly specific for their target and are optimized for minimum off-target effect. Thus, their side effects are expected to be negligible. In addition, treatments based on siRNA will have a longer-lasting effect. For example, the siRNA compounds targeted to an EEF1A gene (referred to as "EEF1A siRNAs") of the present invention have high and sustained silencing effects after 90 hours, and even 120 hours, post-transfection. This feature is due to the fact that siRNAs block the synthesis of the target protein. When treatment is suspended the cell will have to synthesise new target proteins from scratch; whereas traditional treatments would leave the target protein intact, ready to be active again once the inhibitor is no longer present. Another advantage is that their potency for preventing or treating the virus infection can be increased by using a combination of different siRNAs or a combination of EEF1A siRNAs with other therapeutic agents, which could be achieved by combining siRNAs targeting EEF1A2 with other modulators or inhibitors of EEF1A2 (for example, plitidepsin) and/or with modulators or inhibitors of other molecular proteins or effectors required for the virus replication, including other host proteins or essential viral proteins for virus recognition and entry in host cells, replication and formation of the virion, including among others antisense oligonucleotides or siRNAs targeting the EEF1A1 paralog. Such additional targets may include protein receptors and/or proteases used for virus entry in the cells, or other known viral structural proteins of the nucleocapsid, envelop or membrane.

Examples of additional targets for coronaviruses can be the host receptor angiotensin converting enzyme 2 (ACE2), the host protease TMPRSS2 or the viral structural protein spike (S) essential for entry of SARS-CoV-2; or other known viral structural proteins, such as envelope (E) and membrane (M) proteins that together with S protein create the viral envelope of SARS-CoV-2; or the nucleocapsid (N) protein that holds the RNA genome of virus SARS-CoV-2.

Proteins encoded by SARS-CoV-2 genes that can be used as potential targets against SARS-CoV-2 are described and analysed in Wu et al. 2020. Regarding therapies acting on the coronavirus, viral genes or proteins that can be useful to be targeted include Nsps which are involved in RNA transcription, translation, protein synthesis processing and modification, virus replication and infection of the host, such as Nsp3b, Nsp3e, Nsp7_Nsp8 complex, Nsp9, Nsp10, Nsp14, NSp15, Nsp16, 3C-like main protease (3CLpro), papain-like proteinase (PLpro), RNA-dependent RNA polymerase (RdRp) and helicase; virus structural proteins such as spike (S), envelope (E) or E-channel, nucleocapsid (N) and membrane (M) proteins; and some coronavirus virulence factors related to interfering with the host's innate immunity and assisting coronavirus immune escape, such as Nsp1, Nsp3c and ORF7a. Wu et al. also describe examples of drug molecules that can be potentially used for inhibiting any of these viral target proteins, and also of known molecules which are know that bind host target proteins involved during the viral infection such as ACE2 or TMPRSS2. Equivalent viral genes in other strains of coronavirus may also be potential targets for treatment or prevention of said other coronaviruses.

Examples of additional targets for influenza viruses can be, for instance, any of the or known viral membrane proteins, such as the viral envelope glycoproteins hemagglutinin (HA) or neuraminidase (NA), or the matrix 2 (M2) protein, or any of the eight viral ribonucleoproteins (vRNPs) along with the matrix protein M1 that supports the viral envelope of influenza A and influenza B viruses, or one of the host surface glycoconjugates that contain terminal sialic acid residue that can attach to the HA receptor-binding site for virus entry. Alternatively, or in addition, siRNA may be combined with other therapeutic agents (for example, antibodies or other immunoglobulins; small molecule therapeutics) which target EEF1A2 and/or the additional targets referred to herein. The viral and cellular mechanisms for cell entry, replication, virion assembly and intercellular movement of influenza A virus is reviewed in (Dou et al. 2018), which shows some examples of viral proteins encoded by influenza A and influenza B viruses, as well as some host proteins involved that can be used as potential targets against influenza viruses.

In some embodiments, the siRNA compounds of the invention comprise two complementary oligonucleotide strands, wherein the antisense strand has from 19 to 21 nucleobases and is complementary to at least 19 consecutive nucleotides of the EEF1A2 mRNA, and the sense strand has from 19 to 21 nucleobases with at least 15 consecutive nucleotides from the 5'-end being complementary to at least 15 consecutive nucleotides of the antisense strand, preferably from 15 to about 16, 17, 18 or 19 nucleobases. In some embodiments, the compound comprises at least one modified nucleobase, modified sugar moiety or modified internucleoside linkage. In some embodiments, the compound comprises at least one 2'-O-methoxy modification and/or at least one 2'-fluoro modification and/or at least one phosphorothioate linkage.

In some embodiments, the siRNA is blunt ended; that is, each strand is the same length, and there are no overhangs. Other embodiments may also include 3' mononucleotide or dinucleotide overhangs (for example, U, UU, dT or dTdT).

In other embodiments the siRNA compounds may be short hairpin oligonucleotides, being a single strand which forms a hairpin such that a double stranded region is formed wherein a first portion of the oligonucleotide has from 19 to 21 nucleobases and is complementary to at least 19 consecutive nucleotides of the EEF1A2 mRNA, and a second portion of the oligonucleotide has from 19 to 21 nucleobases with at least 15 consecutive nucleotides from the 5'-end being complementary to at least 15 consecutive nucleotides of the first portion, preferably from 15 to about 16, 17, 18 or 19 nucleobases; and said double stranded region comprises said at least 15 consecutive nucleobases of said second portion.

Also provided are kits, compositions, including pharmaceutical compositions, and medicaments comprising compounds of the invention.

Also provided are siRNA compounds, which are targeted to a nucleic acid encoding EEF1A2, and which modulate the expression of EEF1A2, or both EEF1A2 and EEF1A1, for use as an antiviral therapeutic or in preventing or treating virus infections. These compounds can be useful against viruses which require a host eEF1A protein (eEF1A2 and/or eEF1A1) in any step of the viral cycle from the virus binding to the cell and its cell entry to the virus assembly and release from the cell required for normal virus replication, and consequently can be useful for interfering with or reducing the virus replication. This may include viruses that use the host translation machinery depending on eEF1A for the synthesis of viral proteins, such as in the case of viruses having positive sense single-stranded RNA genome (e.g. viruses from the families *Coronaviridae,* including SARS-CoV-2, SARS-CoV-1 and MERS-CoV; *Picornaviridae,* including hepatoviruses such as hepatitis A virus, cardioviruses, enteroviruses, polioviruses, aphthoviruses, parechoviruses, rhinoviruses, erboviruses, kobuviruses, coxsackie and teschoviruses; *Flaviviridae,* including West Nile virus, Dengue virus, Zika virus, yellow fever virus, and hepatitis C virus; *Hepeviridae,* such as hepatitis E virus; *Tombusviridae,* such as the tomato bushy stunt virus; *Tymoviridae,* such as the turnip yellow mosaic virus; *Virgaviridae,* such as the tobacco mosaic virus (TMV) and the tomato brown rugose fruit virus (ToBRFV); and *Bromoviridae,* such as the brome mosaic virus), but it may also include other DNA and RNA viruses having proteins or genomes that interact with this elongation factor of the host cell (e.g. animal viruses from the families *Coronaviridae,* such as SARS-CoV-1; *Flaviviridae,* such as West Nile virus (WNV), Dengue virus, yellow fever virus (YFV) and tick-borne encephalitis virus (TBEV); *Rhabdoviridae,* such as the vesicular stomatitis virus; *Retroviridae,* such as HIV-1; *Poxviridae,* such as vaccinia virus; and *Herpesviridae,* such as cytomegalovirus; and also fitovirus such as the tomato bushy stunt virus (TBSV), the turnip yellow mosaic virus (TYMV), the tobacco mosaic virus (TMV), and the brome mosaic virus (BMV)), and other viruses from *Orthomyxoviridae* family, such as the influenza A virus. These compounds can be also useful for ameliorating or reducing the signs or symptoms of the disease caused by any or some of these viruses. The use of these compounds is particularly preferred against RNA viruses causing devastating diseases in humans or in agriculture and livestock species, including viruses from the *Coronaviridae* and *Orthomyxoviridae* families related to human epidemics or pandemics, including SARS-CoV-2, SARS-CoV-1 and MERS-CoV, or different influenza A virus types.

Also provided is an antiviral composition comprising at least an siRNA molecule or compound targeted to a nucleic acid encoding EEF1A2 and an acceptable carrier.

Also provided is the use of an siRNA compound in the manufacture of a medicament for preventing or treating an infection of a virus which requires the host eEF1A2 and/or eEF1A1 protein in any step of the viral cycle, wherein the siRNA compound is targeted to a nucleic acid encoding EEF1A2 and reduces the expression and/or activity of EEF1A2 or the levels of eEF1A2 protein, or reduces the expression and/or activity of EEF1A2 and EEF1A1 or the levels of eEF1A2 and eEF1A1 proteins. Preferably the virus infection is a coronavirus infection, more preferably a coronavirus infection causing a respiratory syndrome (including MERS or SARS), and most preferably a SARS-CoV-2 infection, or an infection by an influenzavirus, more preferably by an influenza virus A. Yet further provided is the use of an siRNA compound in the manufacture of a medicament for preventing or treating a coronavirus infection or an infection by influenza virus, wherein the siRNA compound is targeted to a nucleic acid encoding EEF1A2 and reduces the expression of EEF1A2, or reduces the expression of both EEF1A2 and EEF1A1. Still further provided is the use of an siRNA compound in the manufacture of a medicament for preventing or treating COVID-19 or influenza ("flu"), wherein the siRNA compound is targeted to a nucleic acid encoding EEF1A2 and reduces the expression of EEF1A2, or reduces the expression of both EEF1A2 and EEF1A1.

Further provided are methods of modulating the expression of EEF1A2 in one or more cells or tissues, comprising contacting the cell(s) or tissue(s) with one or more compounds or compositions of the invention. The method may be in vivo, ex vivo, or in vitro. Methods of treating a subject, particularly a human, suspected of having or being prone to a disease or condition associated with expression of EEF1A, preferably EEF1A2 and/or EEF1A1, or in need of treatment therefore, are also set forth herein; as are methods of treating a subject, particularly a human, suspected of having or being prone to an infection of a virus which requires the host eEF1A2 and/or eEF1A1 protein in any step of the viral cycle. Such methods comprise, for example, administering a therapeutically or prophylactically effective amount of one or more of the compounds or compositions of the invention to the subject being treated. In some embodiments, the subject to be treated has been diagnosed with having a disease or condition associated with expression of EEF1A2, such as a coronavirus infection or its related disease, preferably a SARS-CoV-2 infection or its related disease COVID-19, or an infection caused by an influenza virus or the influenza itself, and preferably caused by influenza A virus.

The present invention also provides methods of inhibiting a coronavirus, including preferably SARS-CoV-2, but also SARS-CoV-1 and MERS-CoV, in one or more cells or tissues comprising, for example, contacting the cell(s) or tissue(s) with one or more compounds of the invention. The method may be in vivo, ex vivo, or in vitro. The present invention also provides methods of treating a subject, particularly a human, having a disease or condition associated with a coronavirus, or in need of treatment therefor, the virus preferably being SARS-CoV-2, but also SARS-CoV-1 and MERS-CoV, comprising administering to the subject a therapeutically or prophylactically effective amount of a compound described herein so that expression of EEF1A2 is inhibited. Such methods comprise administering a therapeutically or prophylactically effective amount of one or more of the compounds or compositions of the invention to the subject being treated, which can be administered in a combined treatment with an eEF1A inhibitor, such as plitidepsin, to improve the therapeutic effect. The components of a combined treatment may be administered simultaneously or sequentially. In some embodiments, the animal or person being treated has been diagnosed with having a disease or condition associated with a coronavirus.

The present invention also provides methods of inhibiting an influenza virus, preferably influenza A virus, in one or more cells or tissues comprising, for example, contacting the cell(s) or tissue(s) with one or more compounds of the invention. The method may be in vivo, ex vivo, or in vitro. The present invention also provides methods of treating a subject, particularly a human, having a disease or condition associated with an influenza virus, preferably an influenza A virus, or in need of treatment therefore, comprising administering to the subject a therapeutically or prophylactically effective amount of a compound described herein so that expression of EEF1A2 is inhibited. Such methods comprise administering a therapeutically or prophylactically effective amount of one or more of the compounds or compositions of the invention to the subject being treated, which can be administered in a combined treatment with an eEF1A inhibitor, such as plitidepsin, to improve the therapeutic effect. The components of a combined treatment may be administered simultaneously or sequentially. In some embodiments, the animal or person being treated has been diagnosed with having a disease or condition associated with an influenza virus.

### DESCRIPTION OF THE DRAWINGS

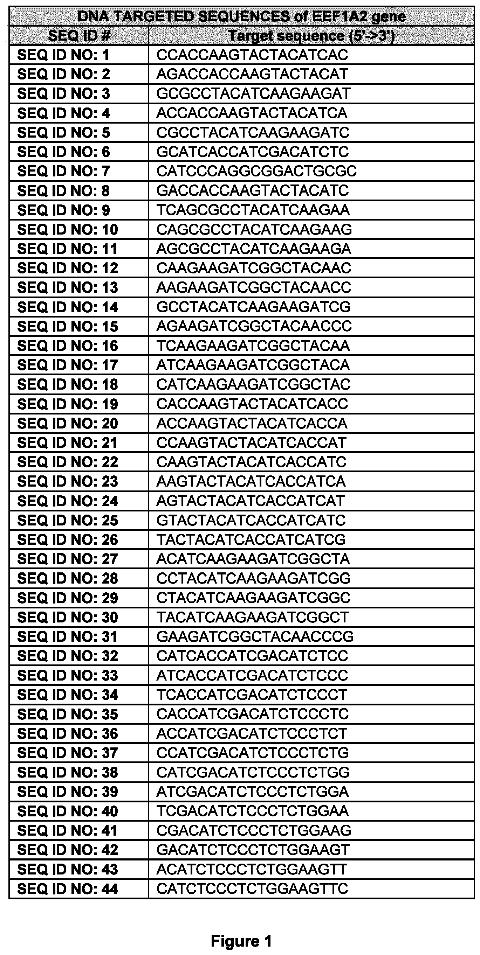

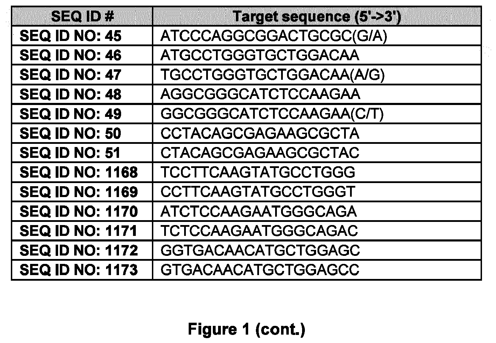

**Figure 1****:** shows the short fragments of the target gene sequence of EEF1A2 chosen as the target sequences for the siRNAs of the present invention. Nucleotide sequences SEQ ID NO:1168 to SEQ ID NO:1173 correspond to fragments of the gene sequences of both human EEF1A gene paralogues (i.e. EEF1A2 and EEF1A1) which have 19 consecutive nucleotides and share 100% identity.

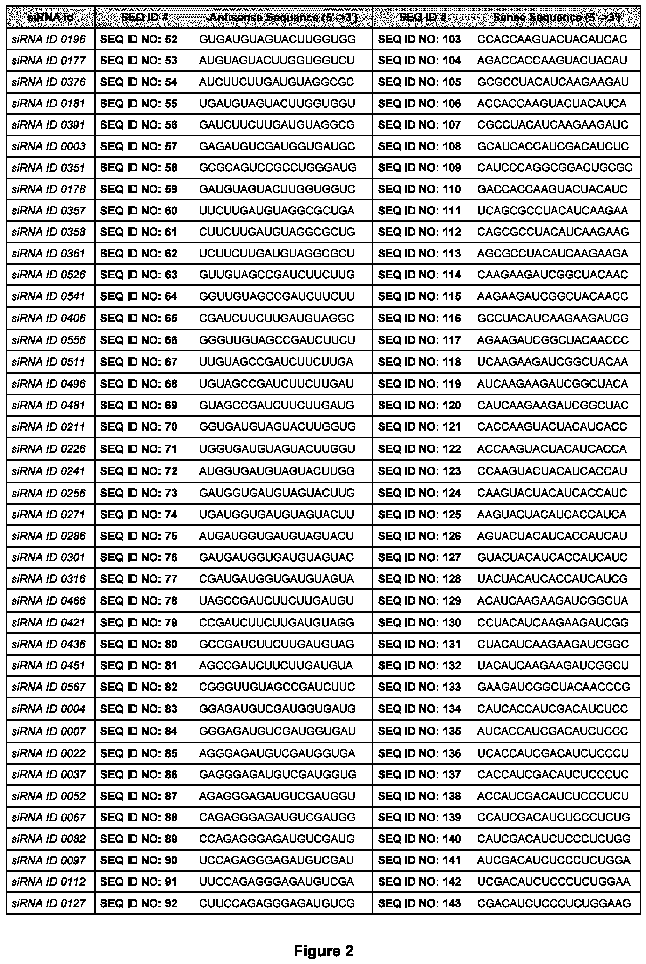

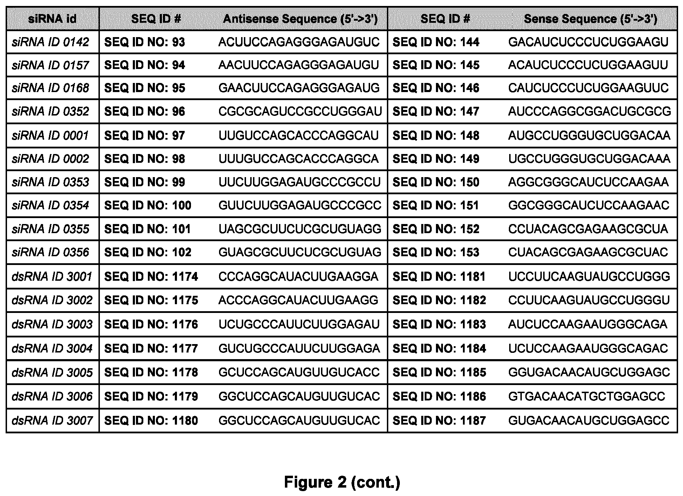

**Figure 2****:** shows the oligonucleotide sequences for the blunt ended siRNA molecules of the present invention targeting EEF1A2 encompassed by the present invention. The SEQ ID NOs given in the Figure refer to the oligonucleotide sequence of the antisense and sense (5' -> 3') strands; typically, siRNAs will be administered as dsRNAs, so siRNAs will include both the antisense strand and its complementary sense strand. SEQ ID NO. 52 to SEQ ID NO. 102 are antisense strands of siRNAs targeting SEQ ID NO. 1 to SEQ ID NO. 51, respectively. SEQ ID NO. 103 to SEQ ID NO. 153 correspond respectively to the complementary sense strands of siRNAs having antisense strands SEQ ID NO. 52 to SEQ ID NO. 102. SEQ ID NO. 1174 to SEQ ID NO. 1180 are nucleotide strands of short dsRNA complementary (antisense) to the nucleotide sequences SEQ ID NO:1168 to SEQ ID NO:1173 of human EEF1A gene paralogs, respectively. SEQ ID NO. 1181 to SEQ ID NO. 1187 correspond respectively to the (sense) complementary strands of dsRNAs having antisense strands SEQ ID NO. 1174 to SEQ ID NO. 1180. Generally, a siRNA or the dsRNA will include the antisense and sense strand, and may also include 3' mononucleotide or dinucleotide overhangs (for example, U, UU, dT or dTdT). However, this is not essential.

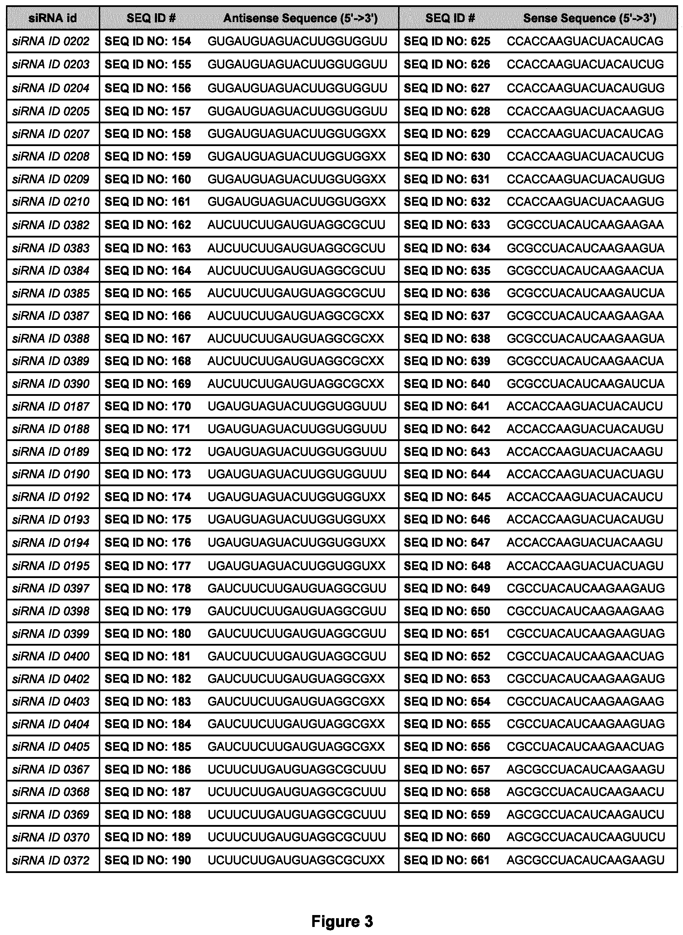

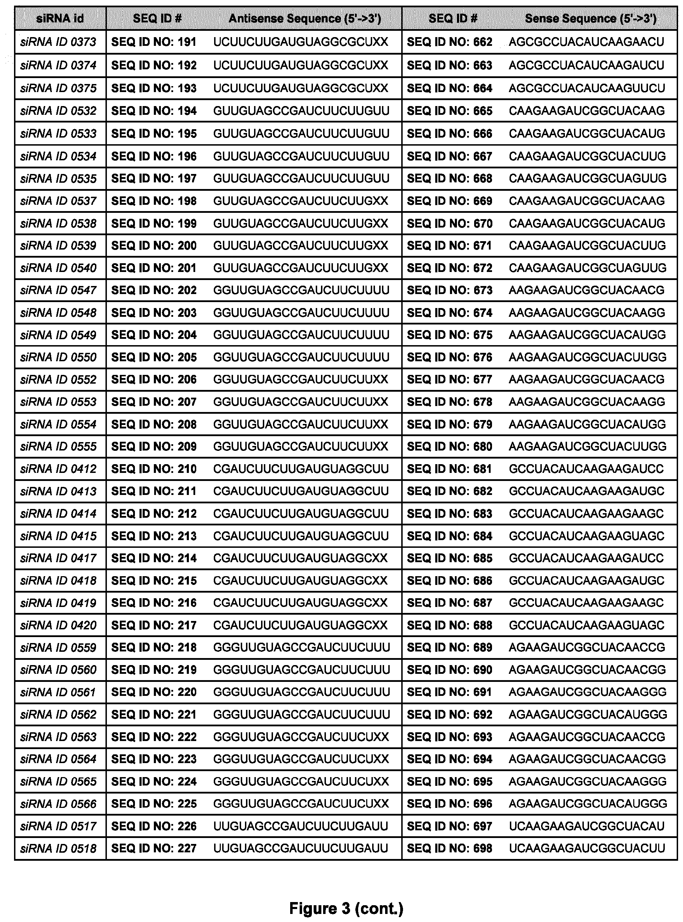

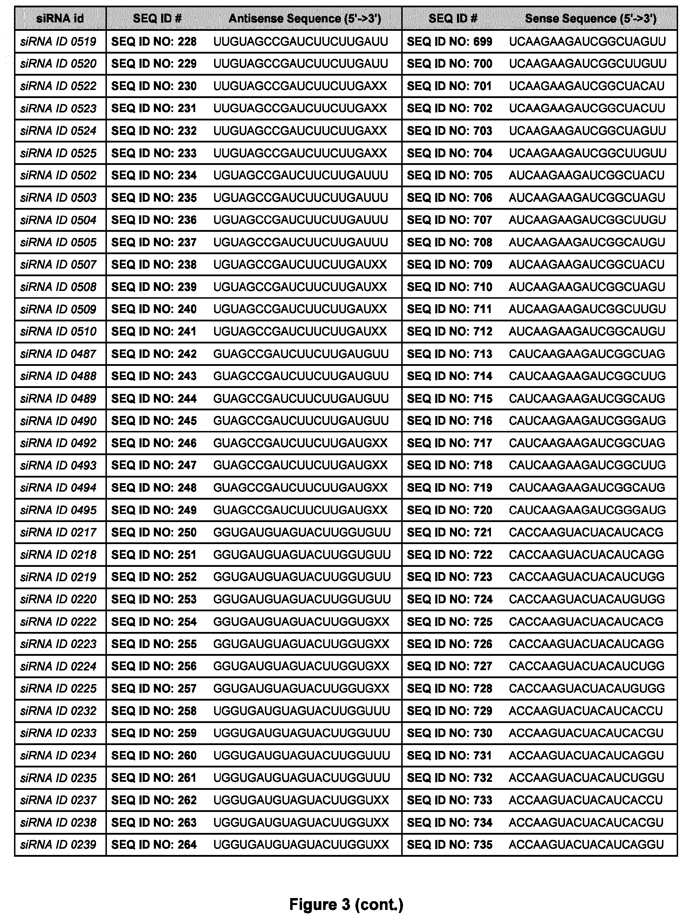

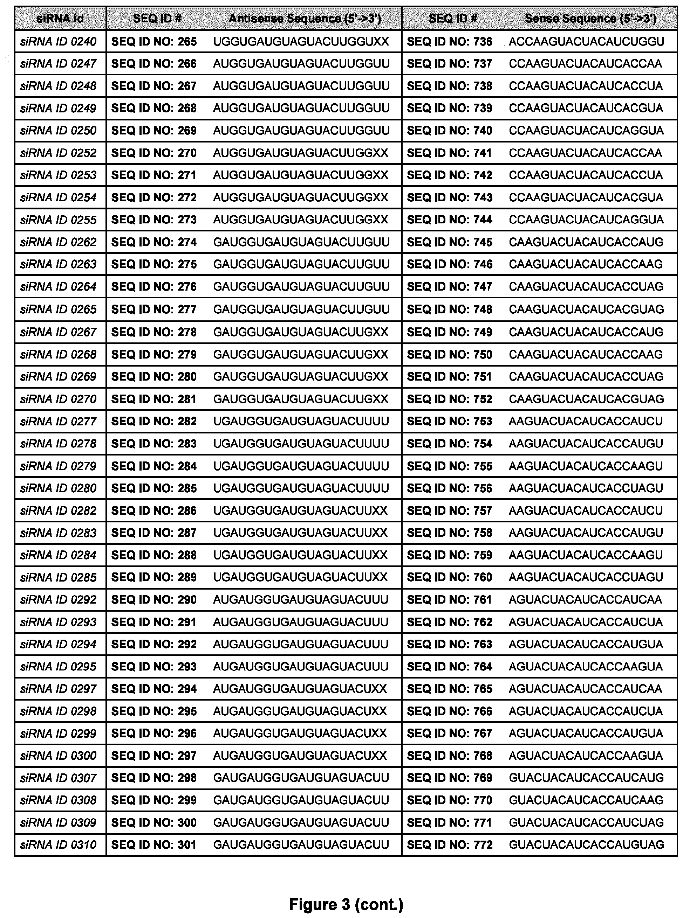

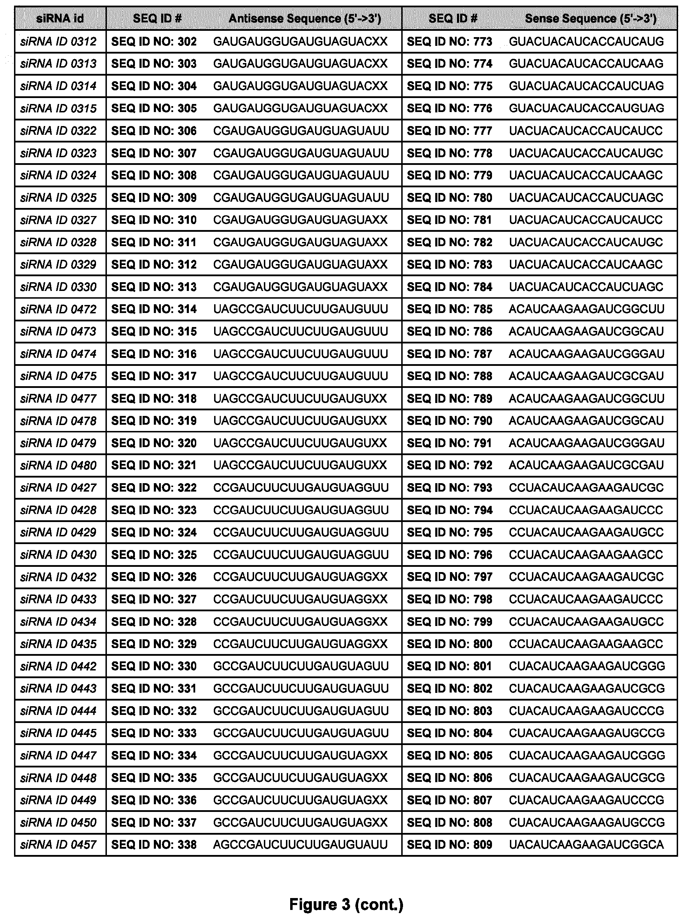

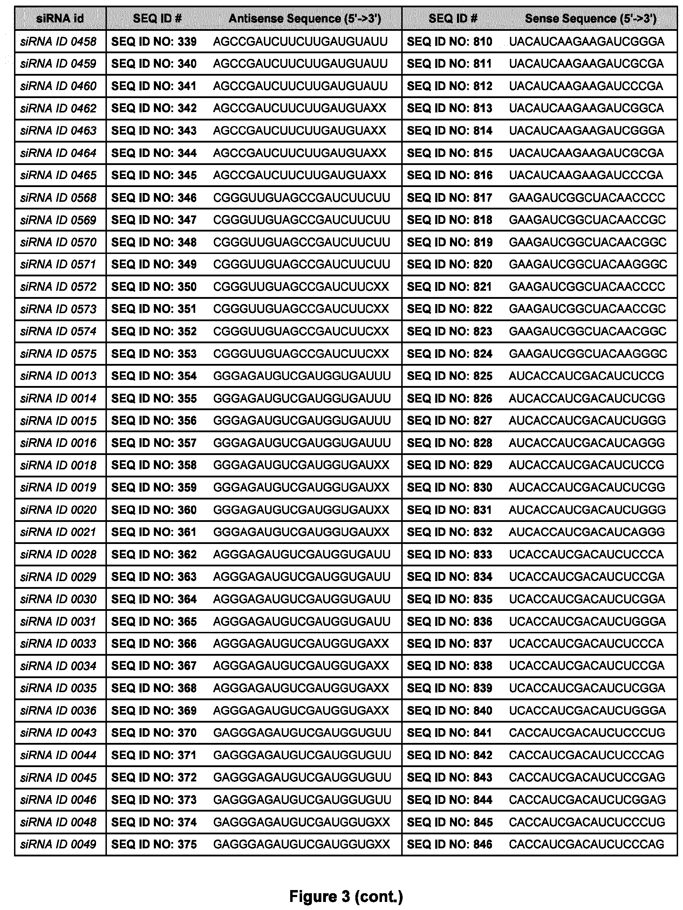

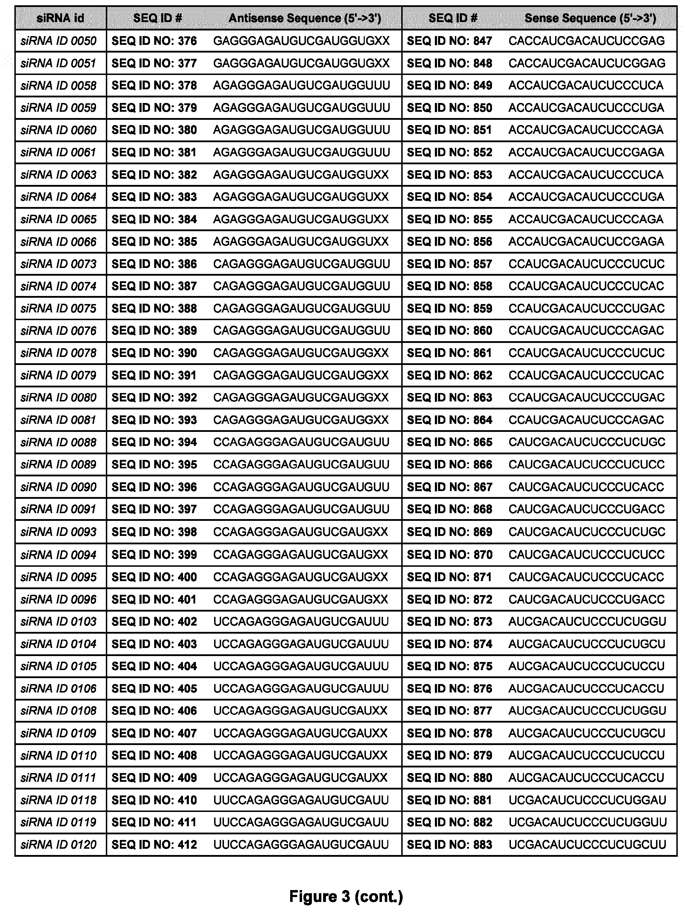

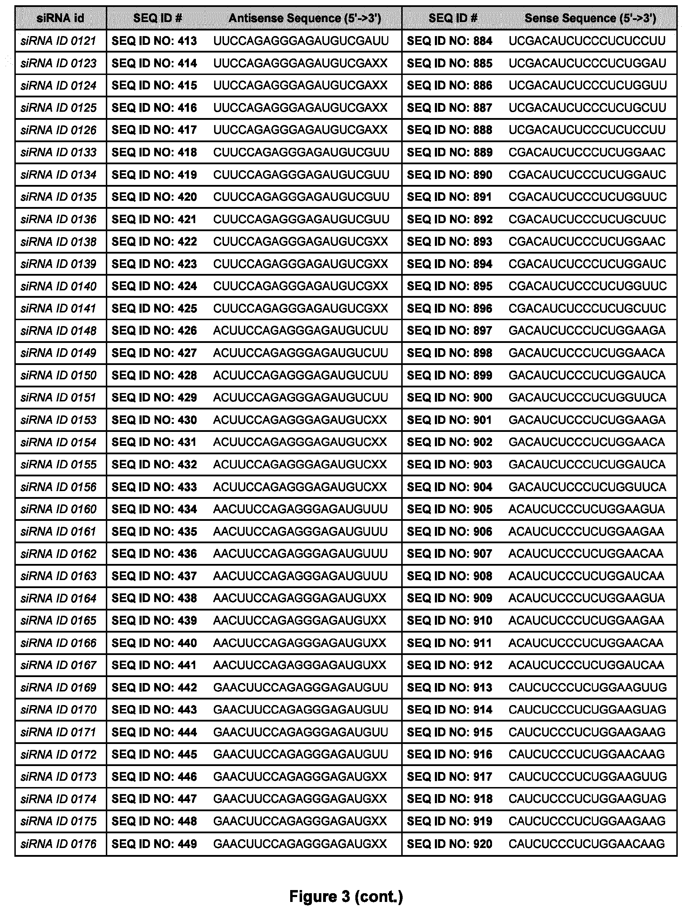

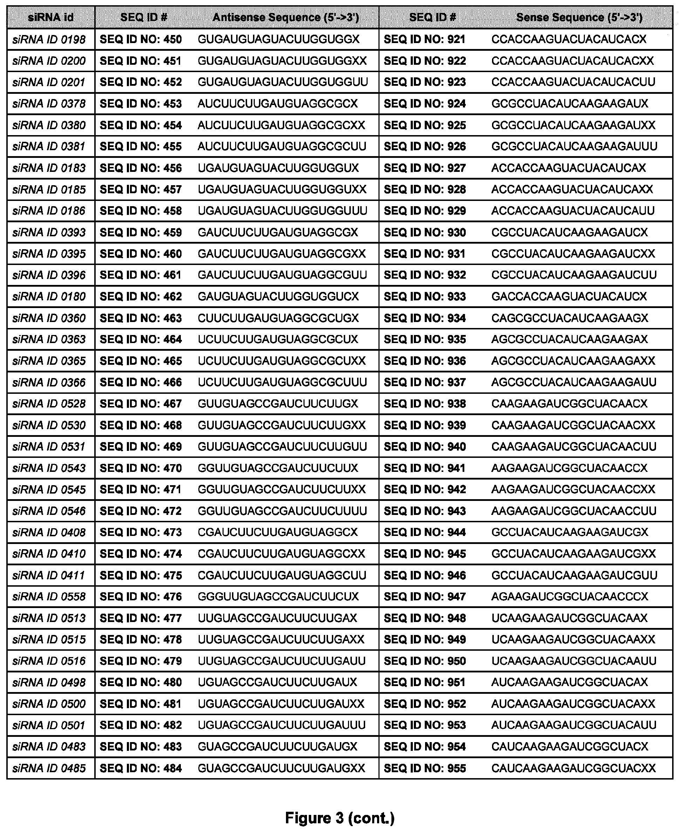

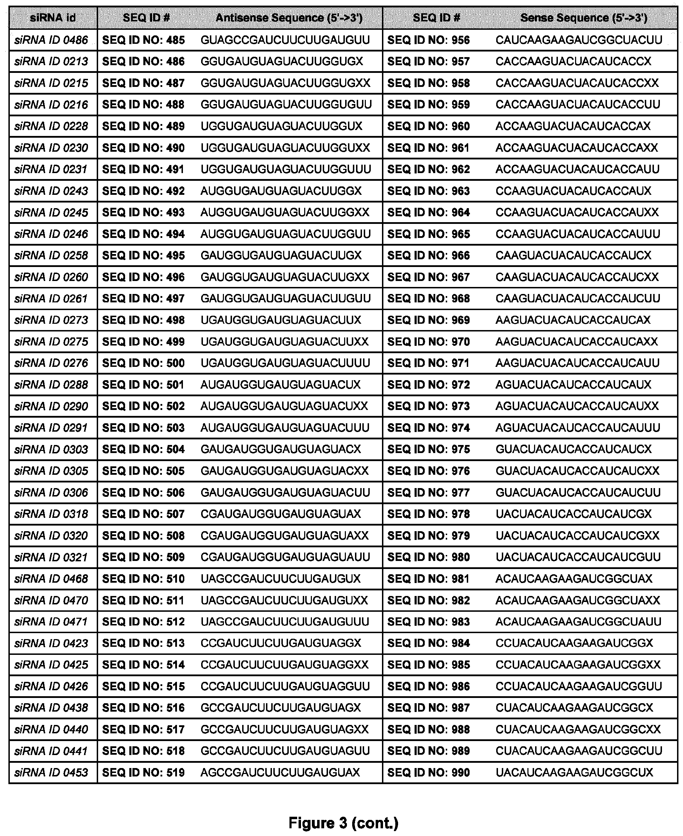

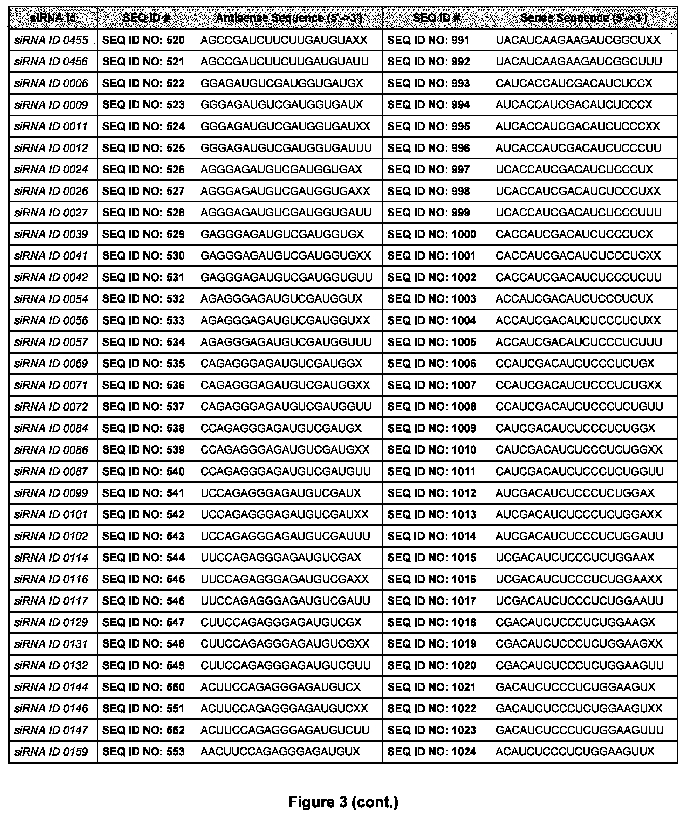

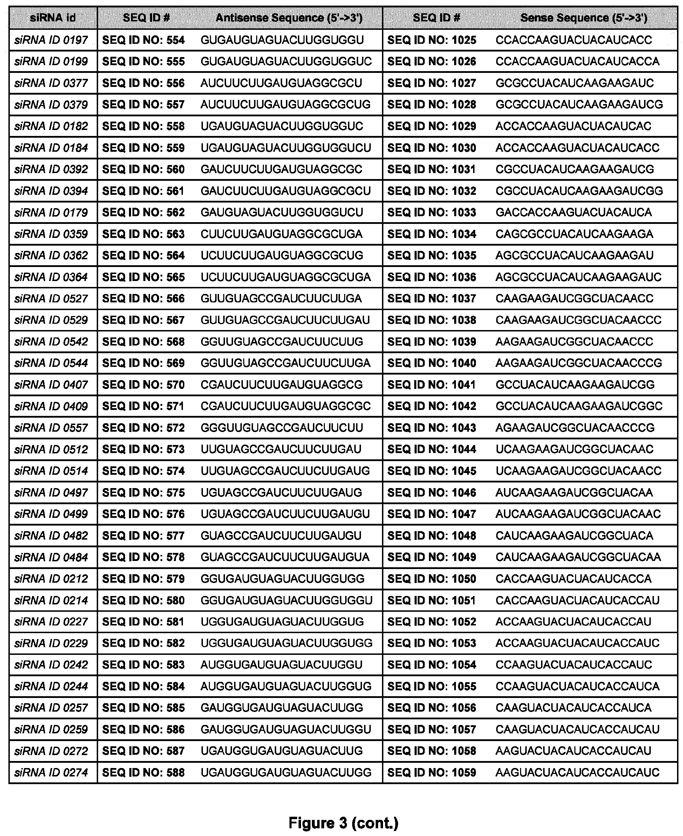

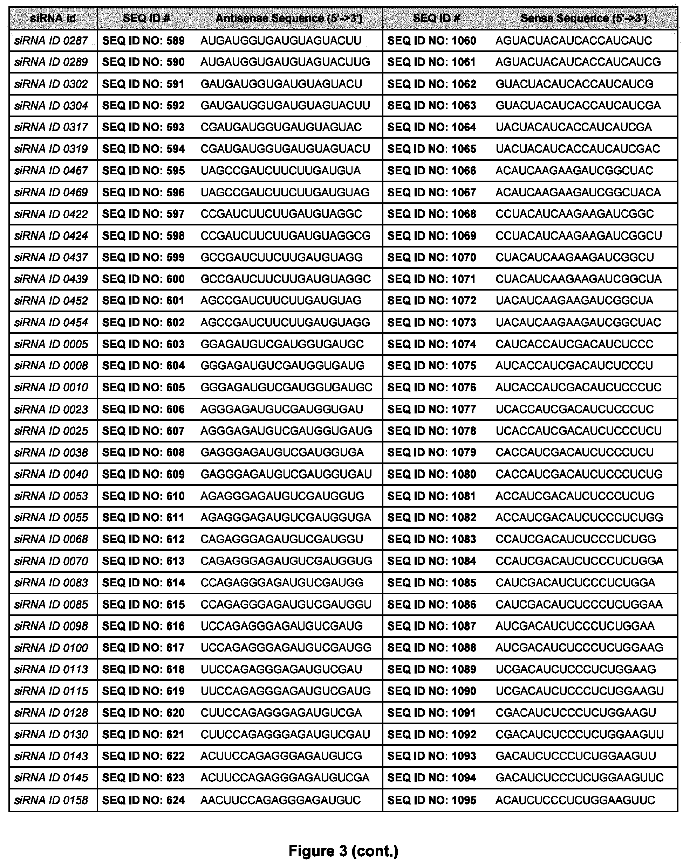

**Figure 3****:** shows the oligonucleotide sequences of the siRNA molecules targeting EEF1A2 with at least one 3'-overhang in any of their strands encompassed by the present invention. The SEQ ID NOs given in the Figure refer to the oligonucleotide sequence of the antisense and sense (5' -> 3') strands; typically, siRNAs will be administered as dsRNAs, so siRNAs will include both the antisense strand and its complementary sense strand. SEQ ID NO. 154 to SEQ ID NO. 624 are antisense strands of siRNAs targeting any of sequences SEQ ID NO. 1 to SEQ ID NO. 51, and SEQ ID NO. 625 to SEQ ID NO. 1095 respectively correspond to complementary sense strands of siRNAs having antisense strands SEQ ID NO. 154 to SEQ ID NO. 624. X is dT (deoxythymidine).
**Figure 4****:** modified 19 nucleotides blunt-ended siRNAs targeting EEF1A2. SEQ ID NO 1096 to SEQ ID NO 1109 and SEQ ID NO: 1110 to SEQ ID NO: 1123 refer respectively to the modified antisense (5' -> 3') strands and modified sense strands (5' -> 3') of different siRNAs which targets sequences SEQ ID NO 1 to SEQ ID NO 51 of the EEF1A2 gene. Legend: sense strand (S), antisense strand (AS), lower case (2'OMe ribonucleotides), * (PS or phosphothioate bond), UPPER CASE UNDERLINED (2'F ribonucleotides), dT (deoxythymidine).
**Figure 5****:** shows oligonucleotide sequences comprising short fragments of 15-21 consecutive nucleotides corresponding to the nucleotide sequences of the sense (SEQ ID NO: 1133 - SEQ ID NO: 1141, and SEQ ID NO: 1191 - SEQ ID NO: 1193) and antisense (SEQ ID NO: 1124 - SEQ ID NO: 1132, and SEQ ID NO: 1188 - SEQ ID NO: 1190) strands for the siRNAs of the present disclosure. "N" is preferably a nucleotide of U, but can be T or dT(deoxythymidine).
**Figure 6****:** shows EEF1A2 expression levels after transfection of siRNAs targeting EEF1A2 in human lung A549 cells.
**Figure 7****:** shows cell viability levels after transfection of siRNAs targeting EEF1A2 in human lung A549 cells.
**Figure** 8: shows EEF1A1 expression levels after transfection of siRNAs targeting EEF1A2 in human lung A549 cells.
**Figure 9****:** shows EEF1A2 expression levels after transfection of siRNAs targeting EEF1A2 in African Green Monkey VeroE6 cells.
**Figure 10****:** shows cell viability levels after transfection of siRNAs targeting EEF1A2 in African Green Monkey VeroE6 cells.
**Figure 11****:** shows EEF1A2 expression levels after transfection of siRNAs targeting EEF1A2 in mouse C2C12 cells.
**Figure 12****:** shows cell viability levels after transfection of siRNAs targeting EEF1A2 in mouse C2C12 cells.
**Figure 13****:** shows EEF1A2 expression levels after transfection of siRNAs targeting EEF1A2 during 6 hours at different collection times in human hepatic Huh-7 cells (a) Lipofectamine 2000 (b) Viromer Green.
**Figure 14****:** shows eEF1A2 protein levels after transfection of siRNAs targeting EEF1A2 with Lipofectamine 2000 during 6 hours at different collection times in human hepatic HuH-7 cells. Protein levels vs. non-transfected cells (NTC) normalized against vinculin (72 hours) and against total protein (96 h).
**Figure 15****:** shows EEFA1 gene expression levels after transfection for 6 hours of siRNAs targeting EEF1A2.
**Figure 16****:** shows EEF1A2 expression levels after transfection of siRNAs targeting EEF1A2 during 6 hours and different collection times in human lung A549 cells.
**Figure 17****:** shows eEF1A2 protein levels after transfection of siRNAs targeting EEF1A2 with Lipofectamine 2000 during 6 hours at different collection times in human lung A549 cells. Protein levels vs. non-transfected cells (NTC) normalized against vinculin.
**Figure 18****:** shows EEFA1 gene expression levels after transfection for 6 hours of siRNAs targeting EEF1A2 in A549 cells.

### DETAILED DESCRIPTION OF THE INVENTION

The present invention relates to the provision and use of siRNA molecules targeting Eukaryotic Translation Elongation Factor 1 Alpha 2 (EEF1A2) gene and/or the eukaryotic translation elongation factor 1 alpha 1 (EEF1A1) gene, encoding an abundant, multifunctional host protein which seems to be involved in viral replication. eEF1A2 protein is an isoform of the alpha subunit of the elongation factor-1 complex, which is responsible for the enzymatic delivery of aminoacyl tRNAs to the ribosome, and which is involved in the viral cycle of some virus, impairing the viral replication, for example of RNA viruses, such as *Coronavirus,* including SARS-CoV-2, SARS-CoV and MERS-CoV or *Influenzavirus,* among others.

Apart from its canonical role in delivering aminoacyl-tRNAs to ribosomes for cellular protein synthesis, eEF1A proteins interact with proteins of different DNA and RNA viruses and seem to be involved in the replication of several viruses by distinct mechanisms. Thus, reducing the expression levels or the activity of EEF1A2 could reduce the replication of DNA or RNA viruses. Besides, in the case of RNA virus with positive single stranded RNA genome, which can function both as a genome and as messenger RNA (mRNA), such reduction in the expression or activity of EEF1A2 could even prevent or diminish the biosynthesis of the first proteins to be expressed after infection by direct translation of genomic RNA that serve genome replication functions, as for instance, the viral RNA-dependent RNA polymerase (RdRP). RNA-dependent RNA polymerase (RdRP) or RNA replicase is an essential enzyme of RNA-containing viruses with no DNA stage, i.e. of all RNA viruses except retroviruses and *Birnaviridae,* that is encoded by all single-stranded RNA (ssRNA) viruses and catalyses the replication of RNA from an RNA template. RdRP can be directly translated from the RNA genome in positive ssRNA viruses, but in case of negative-sense ssRNA viruses whose genome cannot be translated directly into viral proteins, this protein can be found in the virus core together with the genomic RNA to carry out the first mRNA synthesis.

Different aspects of the present invention refer to an siRNA molecule, compositions thereof and its uses as an antiviral therapeutic, as a medicament, or in the prophylactic or therapeutic treatment of a virus infection, preferably of an RNA virus, involving or requiring an Eukaryotic Translation Elongation Factor 1 Alpha protein (eEF1A), preferably the Eukaryotic Translation Elongation Factor 1 Alpha 2 (eEF1A2) and/or the eukaryotic translation elongation factor 1 alpha 1 (eEF1A1), or the expression of its related gene, in any step of the virus cycle of replication. Other aspects of the present invention refer to an siRNA molecule for use as a medicament or in the prophylactic or therapeutic treatment of a virus infection involving or requiring the Eukaryotic Translation Elongation Factor 1 Alpha (eEF1A), preferably eEF1A2 and/or eEF1A1, or the expression of its related gene, in any step of the virus cycle of replication.

In embodiments of the different aspects of the invention, the virus is an RNA virus, mainly RNA virus having a single-stranded RNA (ssRNA) genome, and preferably the RNA strand having positive sense. In preferred embodiments the virus is a positive sense single-stranded RNA virus, and more preferably a human pathogen, which is selected from a *Coronavirus,* preferably a severe acute respiratory syndrome coronavirus 2 (SARS-CoV-2), a severe acute respiratory syndrome coronavirus (SARS-CoV) or a Middle East respiratory syndrome coronavirus (MERS-CoV), a *Flavivirus,* preferably West Nile virus (WNV), Dengue virus (DENV), Zika virus (ZIKV) or yellow fever virus (YFV), an *Hepatovirus,* preferably hepatovirus A or hepatitis A virus (HAV), an hepatitis C virus (HCV), an hepatitis D virus (HDV), an hepatitis E virus (HEV), an *Influenzavirus,* preferably influenzavirus A, influenzavirus B, influenzavirus C, or influenzavirus D, an *Orthopneumovirus,* preferably respiratory syncytial virus (RSV), and an *Arenavirus,* preferably Guanarito virus, Junin virus (Argentinian mammaarenavirus), Lassa virus, Lujo virus, Machupo virus, Sabia virus (Brazilian mammarenavirus) or Whitewater Arroyo virus. In other preferred embodiments, the virus is a positive sense single-stranded RNA virus which is a plant pathogen and is selected from a *Tobamovirus,* including preferably Tomato brown rugose fruit virus, but also others such as Bell pepper mottle virus, Brugmansia mild mottle virus, Cactus mild mottle virus, Clitoria yellow mottle virus, Cucumber fruit mottle mosaic virus, Cucumber green mottle mosaic virus, Cucumber mottle virus, Frangipani mosaic virus, Hibiscus latent Fort Pierce virus, Hibiscus latent Singapore virus, Kyuri green mottle mosaic virus, Maracuja mosaic virus, Obuda pepper virus, Odontoglossum ringspot virus, Opuntia chlorotic ringspot virus, Paprika mild mottle virus, Passion fruit mosaic virus, Pepper mild mottle virus, Plumeria mosaic virus, Rattail cactus necrosis-associated virus, Rehmannia mosaic virus, Ribgrass mosaic virus, Streptocarpus flower break virus, Sunn-hemp mosaic virus, Tobacco latent virus, Tobacco mild green mosaic virus, Tobacco mosaic virus, Tomato mosaic virus, Tomato mottle mosaic virus, Tropical soda apple mosaic virus, Turnip vein-clearing virus, Ullucus mild mottle virus, Wasabi mottle virus, Yellow tailflower mild mottle virus, Youcai mosaic virus, and Zucchini green mottle mosaic virus. A list of virus species identified and that would be within the scope of the present invention can be obtained, for example, from the current ICTV Master Species List published by the International Committee on Taxonomy of Viruses (ICTV) and accessible from its website (https://talk.ictvonline.org/).

In certain aspects of the invention, said siRNA molecule specifically targets a nucleotide sequence of an EEF1A gene paralog, in particular of the EEF1A2 gene, preferably selected from the group comprising or consisting of: SEQ ID NO. 1 to SEQ ID NO. 51 and SEQ ID NO. 1168 to SEQ ID NO. 1173, and reduces expression of one or both gene paralogs, i.e. the EEF1A2 gene and/or EEF1A1 gene, when introduced in a cell. Preferably, the siRNA molecule specifically targets the nucleotide sequence of the EEF1A2 gene and reduces the expression and/or activity of the EEF1A2 gene, although in case of targeting certain specific target sequences, the siRNA molecule may also reduce the expression of the EEF1A1 gene, although potentially at least to a lesser extent. More preferably, the target sequence of the siRNA molecule comprises or consists of any of the nucleotide sequences SEQ ID NO. 1 to SEQ ID NO. 8. In more preferred embodiments of certain aspects, the target sequence comprises or consists of SEQ ID NO. 1. In other preferred embodiments, the target sequence comprises or consists of SEQ ID NO. 3 or SEQ ID NO. 5. Similarly, the siRNA molecule can specifically target the nucleotide sequence of the EEF1A1 gene and reduce the expression of the EEF1A1 gene, and in case of targeting certain specific target sequences of this gene paralog, the siRNA molecule may also reduce, potentially to a lesser extent, the expression of the EEF1A2 gene.

A gene is "targeted" by a siRNA according to the present invention when, for example, the siRNA molecule selectively decreases or inhibits the expression of the gene. The phrase "selectively decrease or inhibit" as used herein encompasses siRNAs that affect expression of one gene, in this case an EEF1A gene, preferably EEF1A2 and/or EEF1A1. Alternatively, a siRNA (or dsRNA, or shRNA) targets a gene when (one strand of) the siRNA (or dsRNA, or shRNA) hybridizes under stringent conditions to the gene transcript, i.e. its mRNA. Hybridizing "under stringent conditions" means annealing to the target mRNA region under standard conditions, e.g., high temperature and/or low salt content which tend to disfavour hybridization. A suitable protocol (involving 0.1×SSC, 68 °C for 2 hours) is described in Maniatis, T., et al., Molecular Cloning: A Laboratory Manual, Cold Spring Harbor Laboratory, 1982, on pages 387-389. However, given that the EEF1A2 gene shares high sequence identity with the paralogous human translation elongation factor 1 alpha-1 (EEF1A1), EEF1A1 gene expression might be also decreased when targeting EEF1A2 gene with specific EEF1A2 siRNAs - for example, those siRNAs targeting certain nucleotide regions of the EEF1A2 gene that also could hybridize with the EEF1A1 mRNA and trigger the mechanism of RNA interference (RNAi) to degrade this mRNA and reduce the EEF1A1 mRNA levels. For example, target sequences SEQ ID NO: 3 and SEQ ID NO: 5 of the EEF1A2 gene have 4 and 5 mismatches, respectively, in relation to the equivalent sequence of the paralogous EEF1A1 gene (i.e. 79% and 74% identity, respectively), but only one mismatch would be within the mRNA region corresponding to the seed sequence of the siRNA molecule which is relevant for its silencing capability. Analogously, when targeting EEF1A1 gene at specific nucleotide sequences with specific EEF1A1 siRNAs that can hybridize and triggering RNAi on EEF1A2 mRNA, the levels of EEF1A2 mRNA might diminish at least in part decreasing also the expression of the EEF1A2 paralog gene.

Nucleic acid sequences cited herein are written in a 5' to 3' direction unless otherwise indicated. The term "nucleic acid" refers to either DNA or RNA or a modified form thereof comprising the purine or pyrimidine bases present in DNA (adenine "A", cytosine "C", guanine "G", thymine "T") or in RNA (adenine "A", cytosine "C", guanine "G", uracil "U"). siRNAs provided herein may comprise "T" bases, for example at 3' ends, even though "T" bases do not naturally occur in RNA. In some cases, these bases may appear as "dT" to differentiate deoxyribonucleotides present in a chain of ribonucleotides.

The target sequence as defined above is described as a target DNA sequence as used for definition of transcript variants in databases used for the purposes of designing siRNAs, whereas the specific compounds to be used will be RNA sequences defined as such, and of course the molecules directly targeted by the siRNAs will be mRNA molecules.

An expert in the field can access any target gene sequence through public databases. For example, the GenBank Accession Number corresponding to an human transcript of EEF1A2 mRNA (Gene ID: 1917) is NM_001958. Furthermore, ENSEMBL (MBL-EBI/Wellcome Trust Sanger Institute) has the following EEF1A2 human Accession Numbers: ENST00000217182, ENST00000298049, ENST00000642899, ENST00000645357, ENST00000645586 and ENST00000646335. All this information is in the free-access Ensembl data base.

Said target regions identified by the present invention comprise or consist of at least one sequence selected from SEQ ID NO. 1 to SEQ ID NO. 51. These sequences present 100% homology between the following species: *Homo sapiens, Macaca mulatta, Macaca fascicularis, Canis lupus familiaris,* and *Mus musculus* (C57/BL2), including *Mus musculus* C57BL/6NJ. Other target regions identified in the present disclosure comprise or consist of at least one sequence selected from SEQ ID NO. 1168 to SEQ ID NO. 1173. These sequences present 100% identity between both gene sequences of human paralogues EEF1A2 and EEF1A1.

In preferred embodiments of certain aspects of the invention, said target regions comprise or consist of a sequence selected from SEQ ID NO. 1, SEQ ID NO. 2, SEQ ID NO. 3, SEQ ID NO. 4, SEQ ID NO. 5, SEQ ID NO. 6, SEQ ID NO. 7 and SEQ ID NO. 8. In other preferred embodiments, said target regions comprise or consist of a sequence selected from SEQ ID NO. 1168, SEQ ID NO. 1169, SEQ ID NO. 1170, SEQ ID NO. 1171, SEQ ID NO. 1172 and SEQ ID NO. 1173. In more preferred embodiments, the target regions are those which are targeted by any or some of the siRNA molecules of the present invention having a high gene silencing efficiency (both on the mRNA expression levels of the EEF1A2 gene or on protein levels of eEF1A2) in cells of different species, preferably of 60% and above, or 70% and above, or 80% and above, or 90% and above or 95% and above, in cells of at least two of the species above mentioned having 100% homology. More preferably, the target regions are those that are targeted by any or some of the siRNA molecules having gene silencing efficiency higher than 80%, 81%, 82%, 83%, 84%, 85%, 86%, 87%, 88%, 89%, 90%, 91%, 92%, 93%, 94%, 95%, 96%, 97%, 98% or 99% in cells of at least two different species, or in cells of at least three different species, or in cells of four different species having 100% homology in the target region of the EEF1A gene, in particular of the EEF1A2 gene paralog, and even more preferably in cells of all different species above mentioned having 100% homology.

In other preferred embodiments, the target regions are those which are targeted by any or some of the siRNA molecules of the present invention having a long-lasting gene silencing efficiency in different cells of at least one species, preferably of 72 h and above, or 96 h and above, or 120 h and above, in at least two cell types of one, two or more species of the above mentioned having 100% homology. More preferably, the target regions are those that are targeted by any or some of the siRNA molecules having lasting silencing effect of at least 72 hours, 96 hours or 120 hours in at least two different cell types of one or two different species having 100% homology in the target region of the EEF1A gene, in particular of the EEF1A2 gene paralog, and even more preferably in cells of all different species above mentioned having 100% homology.

In the RNAi field, when in vitro studies demonstrate that a human siRNA is not able to induce knock-down of the animal model gene, a surrogate compound (animal-active analogue) is synthetized in order to analyze the efficacy of the siRNA in the relevant animal model. This surrogate is designed against the same region as the human siRNA, thus the two siRNAs have the same sequence except for a few nucleotides, depending on the homology between the human and the animal target gene. This approach has been widely used for development of other oligonucleotides, specifically for toxicology and efficacy studies (Kornbrust D et al 2013).

Consequently, a siRNA according to certain aspects of the present invention will preferably comprise a double stranded RNA molecule, whose antisense strand will comprise an RNA sequence substantially complementary to at least one sequence consisting of SEQ ID NO. 1 to SEQ ID NO. 51 and SEQ ID NO. 1168 to SEQ ID NO. 1173, and whose sense strand will comprise an RNA sequence having at least the first 15 nucleotides from the 5'-end complementary to the antisense strand, wherein both strands are hybridised by standard base pairing between nucleotides. More preferably, a siRNA according to certain aspects of the present invention will preferably comprise a double stranded RNA molecule, whose antisense strand will comprise an RNA sequence substantially complementary to any of sequences SEQ ID NO. 1 to SEQ ID NO. 51 and SEQ ID NO. 1168 to SEQ ID NO. 1173, and even more preferably the antisense strand comprises or consists of an RNA sequence substantially complementary to at least one of the sequences from the group consisting of SEQ ID NO. 1 to SEQ ID NO. 8 or from the group consisting of SEQ ID NO. 1168 to SEQ ID NO. 1173.

Within the meaning of the present invention "substantially complementary" to a target mRNA sequence, may also be understood as having between a 85% to 100% complementarity to said target sequence over the length of the siRNA strand. However, the complementary between sense and antisense strands of a siRNA within the present invention may be range of from 70% to 100%, over the length of the double stranded portion of the siRNA. "Identity" as is known by one of ordinary skill in the art, is the degree of sequence relatedness between nucleotide sequences as determined by matching the order and identity of nucleotides between sequences. In one embodiment the antisense strand of an siRNA having 85%, 86%, 87%, 88%, 89%, 90%, 91%, 92%, 93%, 94%, 95%, 96%, 97%, 98% or 99% complementarity to the target mRNA sequence are considered substantially complementary and may be used in the present invention. The percentage of complementarity describes the percentage of contiguous nucleotides in a first nucleic acid molecule that can base pair in the Watson-Crick sense with a set of contiguous nucleotides in a second nucleic acid molecule. In a preferred embodiment, the antisense siRNA strand is 100% complementary to the target mRNA sequence, and the sense strand is 100% complementary to the antisense strand over the double stranded portion of the siRNA. The siRNA may also include unpaired overhangs, for example, 3' mononucleotide overhangs, preferably U or dT, and 3' dinucleotide overhangs, preferably UU or dTdT.

Preferred siRNA antisense strands that target sequences SEQ ID NO. 1 to SEQ ID NO. 51 of EEF1A2 gene or the corresponding mRNA comprise or consist of oligonucleotide sequences having 19, 20 or 21 consecutive nucleotides from one of the nucleotide sequences defined by SEQ ID NO: 1124 (5'-CGAUGAUGGUGAUGUAGUACUUGGUGGUCU-3'), SEQ ID NO: 1125 (5'-CGGGUUGUAGCCGAUCUUCUUGAUGUAGGCGCUGA-3'), SEQ ID NO: 1126 (5'-GAACUUCCAGAGGGAGAUGUCGAUGGUGAUGC-3'), SEQ ID NO: 1127 (5'-[U/C]GCGCAGUCCGCCUGGGAUG-3'), SEQ ID NO: 1128 (5'-[C/U]UUGUCCAGCACCCAGGCAU-3'), SEQ ID NO: 1129 (5'-[A/G]UUCUUGGAGAUGCCCGCCU-3') or SEQ ID NO: 1030. (5'-GUAGCGCUUCUCGCUGUAGG-3'), which correspond to the RNA sequences complementary to the regions of the EEF1A2 mRNAs accessible to the siRNAs of the invention. Preferred siRNA sense strands which are complementary to these antisense strands comprise or consist of oligonucleotide sequences having 19, 20 or 21 consecutive nucleotides from one of the nucleotide sequences defined by SEQ ID NO: 1133 (5'-AGACCACCAAGUACUACAUCACCAUCAUCGA[C/U]-3'), SEQ ID NO: 1134 (5'-UCAGCGCCUACAUCAAGAAGAUCGGCUACAACCCG-3'), SEQ ID NO: 1135 (5'-GCAUCACCAUCGACAUCUCCCUCUGGAAGUUC-3'), SEQ ID NO: 1136 (5'-CAUCCCAGGCGGACUGCGC[G/A]-3'), SEQ ID NO: 1137 (5'-AUGCCUGGGUGCUGGACAA[A/G]-3'), SEQ ID NO: 1138 (5'-AGGCGGGCAUCUCCAAGAA[C/U]-3') or SEQ ID NO: 1139 (5'-CCUACAGCGAGAAGCGCUAC-3'), respectively. Also preferred siRNA sense strands which are complementary to those antisense strands consist of oligonucleotide sequences of 19 nucleotides in length that comprises at least 15, 16, 17 or 18, and preferably only 15, consecutive nucleotides from one of the nucleotide sequences defined by SEQ ID NO: 1133, SEQ ID NO: 1134, SEQ ID NO: 1135, SEQ ID NO: 1136, SEQ ID NO: 1137, SEQ ID NO: 1138 or SEQ ID NO: 1139, respectively.

More preferred siRNA antisense strands that target sequences SEQ ID NO. 1 to SEQ ID NO. 51 of EEF1A2 gene or the corresponding mRNA consist of oligonucleotide sequences which are selected from one or more of SEQ ID NO: 52 to SEQ ID NO: 102, SEQ ID NO: 154 to SEQ ID NO: 624 and SEQ ID NO: 1096 to SEQ ID NO: 1109. Preferred siRNA sense strands which are complementary to these antisense strands consist of oligonucleotide sequences which are selected from one or more of SEQ ID NO: 103 to SEQ ID NO: 153, SEQ ID NO: 625 to SEQ ID NO: 1095 and SEQ ID NO: 1110 to SEQ ID NO: 1123, respectively. Figures 2, 3 and 4 show some examples of siRNA compounds designed for targeting the nucleotide sequences SEQ ID NO. 1 to SEQ ID NO. 51.

Preferred siRNA antisense strands that target sequences SEQ ID NO. 1 to SEQ ID NO. 8 of EEF1A2 gene or the corresponding mRNA comprise or consist of oligonucleotide sequences having 19, 20 or 21 consecutive nucleotides from one of the nucleotide sequences defined by SEQ ID NO: 1124 (5'-CGAUGAUGGUGAUGUAGUACUUGGUGGUCU-3'), SEQ ID NO: 1125 (5'-CGGGUUGUAGCCGAUCUUCUUGAUGUAGGCGCUGA-3'), SEQ ID NO: 1126 (5'-GAACUUCCAGAGGGAGAUGUCGAUGGUGAUGC-3') or SEQ ID NO: 1127 (5'-[U/C]GCGCAGUCCGCCUGGGAUG-3'). Preferred siRNA sense strands which are complementary to these antisense strands comprise or consist of oligonucleotide sequences having 19, 20 or 21 consecutive nucleotides from one of the nucleotide sequences defined by SEQ ID NO: 1133 (5'-AGACCACCAAGUACUACAUCACCAUCAUCGA[C/U]-3'), SEQ ID NO: 1134 (5'-UCAGCGCCUACAUCAAGAAGAUCGGCUACAACCCG-3'), SEQ ID NO: 1135 (5'-GCAUCACCAUCGACAUCUCCCUCUGGAAGUUC-3') or SEQ ID NO: 1136 (5'-CAUCCCAGGCGGACUGCGC[G/A]-3'), respectively. Also preferred siRNA sense strands which are complementary to these antisense strands consist of oligonucleotide sequences of 19 nucleotides in length that comprises at least 15, 16, 17 or 18, and preferably only 15, consecutive nucleotides from one of the nucleotide sequences defined by SEQ ID NO: 1133, SEQ ID NO: 1134, SEQ ID NO: 1135, or SEQ ID NO: 1136, respectively.

Preferred dsRNA antisense strands that target sequences SEQ ID NO. 1168 to SEQ ID NO. 1173 of human EEF1A paralog genes or their corresponding mRNAs comprise or consist of oligonucleotide sequences having 19, 20 or 21 consecutive nucleotides from one of the nucleotide sequences defined by SEQ ID NO: 1188 (5'-ACCCAGGCAUACUUGAAGGA-3'), SEQ ID NO: 1189 (5'-GUCUGCCCAUUCUUGGAGAU-3') or SEQ ID NO: 1190 (5'-GGCUCCAGCAUGUUGUCACC-3'), which correspond to the RNA sequences complementary to the regions of both human EEF1A mRNAs. Preferred dsRNA sense strands which are complementary to these antisense strands comprise or consist of oligonucleotide sequences having 19, 20 or 21 consecutive nucleotides from one of the nucleotide sequences defined by SEQ ID NO: 1191 (5'-UCCUUCAAGUAUGCCUGGGU-3'), SEQ ID NO: 1192 (5'-AUCUCCAAGAAUGGGCAGAC-3') or SEQ ID NO: 1193 (5'-GG(U/T/dT)GACAACA(U/T/dT)GC(U/T/dT)GGAGCC-3'), respectively. Also preferred dsRNA sense strands which are complementary to those antisense strands consist of oligonucleotide sequences of 19 nucleotides in length that comprises at least 15, 16, 17 or 18, and preferably only 15, consecutive nucleotides from one of the nucleotide sequences defined by SEQ ID NO: 1191, SEQ ID NO: 1192 or SEQ ID NO: 1193, respectively.

More preferred dsRNA antisense strands that target sequences SEQ ID NO. 1168 to SEQ ID NO. 1173 of human EEF1A paralog genes or their corresponding mRNAs consist of oligonucleotide sequences which are selected from one or more of SEQ ID NO: 1174 to SEQ ID NO: 1180. Preferred dsRNA sense strands which are complementary to these antisense strands consist of oligonucleotide sequences which are selected from one or more of SEQ ID NO: 1181 to SEQ ID NO: 1187, respectively. Figure 2 shows some examples of dsRNA compounds designed for targeting the nucleotide sequences SEQ ID NO. 1168 to SEQ ID NO. 1173.

Preferred antisense strands can be those that included in a siRNA or dsRNA molecule produce a silencing effect on at least one EEF1A gene paralog expressed in cells, and reduce the percentage of gene expression or the mRNA levels of the EEF1A2 gene, or of both EEF1A2 and EEF1A1 genes, in one or some cell types from at least one, two, three, four, five or more species, and preferably in cell types from at least two species, including *Homo sapiens.* Additionally or alternatively, preferred antisense strands can be also those that included in a siRNA or dsRNA molecule do not produce any remarkable reduction in the cell viability. Additionally or alternatively, also preferred can be antisense strands that within a siRNA or dsRNA molecule are able to produce RNA interference in cells and reduce the expression of the EEF1A gene, preferably the expression of the EEF1A2 gene in cells, during at least one, two, three, four, five or more days. Also, additionally or alternatively, preferred antisense strands can be also those that in a siRNA or dsRNA molecule show an antiviral effect, for example, on the replication of a virus in an infected cell or an organism or subject infected with such virus, or an effect on the expression and production of pro-inflammatory cytokines by infected cells, or an effect on the expression and production of cytokines and chemokines in immune cells, or an effect on markers of inflammation or progression of the viral disease, or on the spread of infection in the infected organism or subject.

More preferred siRNA antisense strands that target sequences SEQ ID NO. 1 to SEQ ID NO. 8 of EEF1A2 gene or the corresponding mRNA comprise or consist of oligonucleotide sequences which are selected from one or more of SEQ ID NO: 1142 to SEQ ID NO: 1149. Preferred siRNA sense strands which are complementary to these antisense strands consist of oligonucleotide sequences which are selected from SEQ ID NO: 1150 to SEQ ID NO: 1157, respectively. In embodiments, preferred siRNA antisense strands comprise or consist of any of the oligonucleotide sequences selected from SEQ ID NO: 1158, SEQ ID NO: 1143, SEQ ID NO: 1159, SEQ ID NO: 1160, SEQ ID NO: 1161, SEQ ID NO: 1147, SEQ ID NO: 1148 and SEQ ID NO: 1162, and the complementary siRNA sense strands respectively comprise or consist of SEQ ID NO: 1163, SEQ ID NO: 1151, SEQ ID NO: 1164, SEQ ID NO: 1165, SEQ ID NO: 1166, SEQ ID NO: 1155, SEQ ID NO: 1156 and SEQ ID NO: 1167, respectively. Examples of preferred siRNA compounds comprising these antisense and sense strands and their target sequences on the EEF1A2 gene are summarized in below Table 1 and Table 2.

**Table 1.**

| **Target sequence** | **Antisense Sequence (5'->3')** | **Sense Sequence (5'->3')** |
|---|---|---|
| CCACCAAGTACTACATCAC (SEQ ID NO: 1) | GUGAUGUAGUACUUGGUGn | CCACCAAGUACUACAn |
| | n= G, GU, GUC, GUU, GdT or GdTdT | n= UCAC,UCACC, UCACCA, UCACdT, UCACdTdT, UCACUU, UCAG, UCUG, UGUG or AGUG |
| | **SEQ ID NO:1142** | **SEQ ID NO: 1150** |
| AGACCACCAAGTACTACAT (SEQ ID NO: 2) | AUGUAGUACUUGGUGGUCn | AGACCACCAAGUACUn |
| | n= U | n= ACAU |
| | **SEQ ID NO: 1143** | **SEQ ID NO: 1151** |
| GCGCCTACATCAAGAAGAT (SEQ ID NO: 3) | AUCUUCUUGAUGUAGGCGn | GCGCCUACAUCAAGAn |
| | n= C, CU, CUG, CUU, CdT or CdTdT | n= AGAU, AGAUC, AGAUCG, AGAUUU, AGAUdT, AGAUdTdT, AGAA, AGUA, ACUA or UCUA |
| | **SE QID NO:1144** | **SEQ ID NO: 1152** |
| ACCACCAAGTACTACATCA (SEQ ID NO: 4) | UGAUGUAGUACUUGGUGGn | ACCACCAAGUACUACn |
| | n= U, UC, UCU, UUU, UdT or UdTdT | n= AUCA, AUCAC, AUCACC, AUCAdT, AUCAdTdT, AUCAUU, AUCU, AUGU, AAGU or UAGU |
| | **SEQ ID NO: 1145** | **SEQ ID NO: 1153** |
| CGCCTACATCAAGAAGATC (SEQ ID NO: 5) | GAUCUUCUUGAUGUAGGCn | CGCCUACAUCAAGAAn |
| | n= G, GC, GCU, GUU, GdT or GdTdT | n= GAUC, GAUCG, GAUCGG, GAUCUU, GAUCdT, GAUCdTdT, GAUG, GAAG, GUAG or CUAG |
| | **SEQ ID NO: 1146** | **SEQ ID NO: 1154** |
| GCATCACCATCGACATCTC (SEQ ID NO: 6) | GAGAUGUCGAUGGUGAUGn | GCAUCACCAUCGACAn |
| | n= C | n= UCUC |
| | **SEQ ID NO: 1147** | **SEQ ID NO: 1155** |
| CATCCCAGGCGGACTGCGC (SEQ ID NO: 7) | GCGCAGUCCGCCUGGGAUn | CAUCCCAGGCGGACUn |
| | n= G | n= GCGC |
| | **SEQ ID NO: 1148** | **SEQ ID NO: 1156** |
| GACCACCAAGTACTACATC (SEQ ID NO: 8) | GAUGUAGUACUUGGUGGUn | GACCACCAAGUACUAn |
| | n= C, CU or CdT | n= CAUC, CAUCA or CAUCdT |
| | **SEQ ID NO: 1149** | **SEQ ID NO: 1157** |
| TCCTTCAAGTATGCCTGGG (SEQ ID NO: 1168) | n₁CCAGGCAUACUUGAAGn₂ , with: | n₁CUUCAAGUAUGCCUGGn₂ , with: |
| | n₁= AC, and n₂= G or GA; or n₂= GA, and n₁= C or AC | n₁= TC, and n₂= G or GT; or n₂= GT, and n₁= TC or C |
| CCTTCAAGTATGCCTGGGT (SEQ ID NO: 1169) | **SEQ ID NO: 1196** | **SEQ ID NO: 1199** |
| ATCTCCAAGAATGGGCAGA (SEQ ID NO: 1170) | n₁CUGCCCAUUCUUGGAGn₂ , with: | n₁CUCCAAGAAUGGGCAGn₂ , with: |
| | n₁= GU, and n₂= A or AU; or n₂= AU, and n₁= U or GU | n₁= AT, and n₂= A or AC; or n₂= AC, and n₁= AT or T |
| TCTCCAAGAATGGGCAGAC (SEQ ID NO: 1171) | **SEQ ID NO: 1197** | **SEQ ID NO: 1200** |
| GGTGACAACATGCTGGAGC (SEQ ID NO: 1172) | n₁CUCCAGCAUGUUGUCAn₂, with | n₁NGACAACANGCNGGAGn₂, with: |
| | n₁= GG, and n₂= C or CC; or n₂= CC, and n₁= GG or G | n₁= GG, and n₂= C or CC; or n₂= CC, and n₁= GG or G; |
| GTGACAACATGCTGGAGCC (SEQID NO:1173) | | being N= U, T or dT |
| | **SEQ ID NO: 1198** | **SEQ ID NO: 1201** |

**Table 2.**

| **Target sequence** | **Antisense Sequence (5'->3')** | **Sense Sequence (5'->3')** |
|---|---|---|
| CCACCAAGTACTACATCAC (SEQ ID NO: 1) | GUGAUGUAGUACUUGGUGn | CCACCAAGUACUACAn |
| | n= G or GUU | n= UCAC, UCAG, UCUG or UGUG |
| | **SEQ ID NO: 1158** | **SEQ ID NO: 1163** |
| AGACCACCAAGTACTACAT (SEQ ID NO: 2) | AUGUAGUACUUGGUGGUCn | AGACCACCAAGUACUn |
| | n= U | n= ACAU |
| | **SEQ ID NO: 1143** | **SEQ ID NO: 1151** |
| GCGCCTACATCAAGAAGAT (SEQ ID NO: 3) | AUCUUCUUGAUGUAGGCGn | GCGCCUACAUCAAGAn |
| | n= C | n= AGAU |
| | **SEQ ID NO: 1159** | **SEQ ID NO: 1164** |
| ACCACCAAGTACTACATCA (SEQ ID NO: 4) | UGAUGUAGUACUUGGUGGn | ACCACCAAGUACUACn |
| | n= U or UUU | n= AUCA, AUGU, AAGU or UAGU |
| | **SEQ ID NO: 1160** | **SEQ ID NO: 1165** |
| CGCCTACATCAAGAAGATC (SEQ ID NO: 5) | GAUCUUCUUGAUGUAGGCn | CGCCUACAUCAAGAAn |
| | n= G | n= GAUC |
| | **SEQ ID NO: 1161** | **SEQ ID NO: 1166** |
| GCATCACCATCGACATCTC (SEQ ID NO: 6) | GAGAUGUCGAUGGUGAUGn | GCAUCACCAUCGACAn |
| | n= C | n= UCUC |
| | **SEQ ID NO: 1147** | **SEQ ID NO: 1155** |
| CATCCCAGGCGGACTGCGC (SEQ ID NO: 7) | GCGCAGUCCGCCUGGGAUn | CAUCCCAGGCGGACUn |
| | n= G | n= GCGC |
| | **SEQ ID NO: 1148** | **SEQ ID NO: 1156** |
| GACCACCAAGTACTACATC (SEQ ID NO: 8) | GAUGUAGUACUUGGUGGUn | GACCACCAAGUACUAn |
| | n= C or CU | n= CAUC or CAUCA |
| | **SEQ ID NO: 1162** | **SEQ ID NO: 1167** |

Also preferred dsRNA antisense strands that target sequences SEQ ID NO. 1168 to SEQ ID NO. 1173 of both human EEF1A gene paralogs or their corresponding mRNAs comprise or consist of oligonucleotide sequences which are selected from one or more of SEQ ID NO: 1196 to SEQ ID NO: 1198. Preferred dsRNA sense strands which are complementary to these antisense strands consist of oligonucleotide sequences which are selected from SEQ ID NO: 1199 to SEQ ID NO: 1201, respectively. Table 1 includes examples of dsRNA compounds comprising these sense and antisense strands targeting both EEF1A paralogs. An even more preferred dsRNA antisense strand that targets sequence SEQ ID NO. 1173 of both human EEF1A gene paralogs is any defined by nucleotide sequence SEQ ID NO. 1194 (5'-GGCUCCAGCAUGUUGUCAC-3'), and the corresponding complementary sense strand is any defined by the nucleotide sequence SEQ ID NO. 1195 (5'-GNGACAACANGCNGGAGCC-3', being N = U, T or deoxythymidine (dT)). In embodiments, preferred dsRNA antisense strands comprise or consist of any of the oligonucleotide sequences selected from SEQ ID NO: 1174, SEQ ID NO: 1175, SEQ ID NO: 1176, SEQ ID NO: 1177, SEQ ID NO: 1178, SEQ ID NO: 1179 and SEQ ID NO: 1180, and the complementary dsRNA sense strands respectively comprise or consist of SEQ ID NO: 1181, SEQ ID NO: 1182, SEQ ID NO: 1183, SEQ ID NO: 1184, SEQ ID NO: 1185, SEQ ID NO: 1186 and SEQ ID NO: 1187 (Figure 2).

Even more preferred siRNA antisense strands that target sequences SEQ ID NO. 1 to SEQ ID NO. 8 of EEF1A2 gene or mRNA consist of oligonucleotide sequences which are selected from one or more of SEQ ID NO: 52 to SEQ ID NO: 59, SEQ ID NO: 154 to SEQ ID NO: 185, SEQ ID NO: 450 to SEQ ID NO: 461, SEQ ID NO: 554 to SEQ ID NO: 562 and SEQ ID NO: 1096 to SEQ ID NO: 1109. Preferred siRNA sense strands which are complementary to these antisense strands consist of oligonucleotide sequences which are selected from one or more of SEQ ID NO: 103 to SEQ ID NO: 110, SEQ ID NO: 625 to SEQ ID NO: 656, SEQ ID NO: 921 to SEQ ID NO: 932, SEQ ID NO: 1025 to SEQ ID NO: 1033 and SEQ ID NO: 1110 to SEQ ID NO: 1123, respectively.

Thus, an aspect of the invention refers to the use of an small interfering RNA (siRNA) molecule which targets the eukaryotic translation elongation factor 1 alpha 2 (EEF1A2) gene and/or the eukaryotic translation elongation factor 1 alpha 1 (EEF1A1) gene, as antiviral compounds against viruses of vegetal and animal organisms (i.e. plants and animals, including humans), preferably of viruses which are pathogens for plants or animals, and more preferably of viruses that cause diseases or conditions in mammals and human subjects. Preferred siRNA compounds are those able to reduce the expression and/or activity of EEF1A2 and/or EEF1A1 in a cell, preferably in a cell of a plant or of an animal, and can reduce the replication of a virus in the cell treated with the siRNA compound and infected by the virus. Reducing the expression and/or activity of EEF1A2 and/or EEF1A1 in the cell with these siRNAs can be advantageous in case of positive-sense single-stranded RNA viruses (ssRNA viruses) that translate their viral genome into viral proteins prior to the replication of the viral genome within the cell carried out by RNA dependent RNA polymerase complexes encoded by the virus RNA. This use as antiviral can be carried out in plants; and another aspect of the invention includes the use of an small interfering RNA (siRNA) molecule which targets the eukaryotic translation elongation factor 1 alpha 2 (EEF1A2) gene and/or the eukaryotic translation elongation factor 1 alpha 1 (EEF1A1) in the prophylactic or therapeutic treatment of a virus infection in a plant, including the use of any of the EEF1A2 siRNAs or dsRNAs and their preferred embodiments described in the present description. Likewise, the use of such siRNAs as antiviral compounds could be carried out in animals, and the invention also refers to an small interfering RNA (siRNA) molecule which targets the eukaryotic translation elongation factor 1 alpha 2 (EEF1A2) gene and/or the eukaryotic translation elongation factor 1 alpha 1 (EEF1A1) for use in the prophylactic or therapeutic treatment of a virus infection in an animal subject, including the siRNAs of the invention and any of the embodiments described herein; the animal subject preferably being a mammalian subject or more preferably a human subject.

Another aspect of the invention refers to a small interfering RNA (siRNA) molecule which targets the eukaryotic translation elongation factor 1 alpha 2 (EEF1A2) gene and/or the eukaryotic translation elongation factor 1 alpha 1 (EEF1A1) gene, preferably against a target sequence of at least 19 nucleotides of the EEF1A2 gene consisting of any of the nucleotides sequences selected from SEQ ID NO: 1 to SEQ ID NO: 51 and SEQ ID NO: 1168 to SEQ ID NO: 1173, preferably for use in medicine. Such siRNA molecules can be used in the medicinal field, and more particularly are for use in the prophylactic or therapeutic treatment of a virus infection in a subject, being especially useful for managing viral infections caused by RNA or DNA viruses which are pathogenic and/or infectious to humans or any other mammal. Typically, the siRNA molecule comprises an antisense strand targeting any of the nucleotides sequences SEQ ID NO: 1 to SEQ ID NO: 51 or SEQ ID NO: 1168 to SEQ ID NO: 1173, and a sense strand complementary to the antisense strand, wherein the antisense strand comprises or consists of at least 19 consecutive nucleotides of any of the nucleotide sequences SEQ ID NO: 1124 to SEQ ID NO: 1130 or SEQ ID NO: 1188 to SEQ ID NO: 1190, and both strands are base paired to form a double stranded RNA (dsRNA) structure comprising at least 15 base pairs (bp), wherein the 3' end of the antisense strand is a blunt end or has a one nucleotide or dinucleotide 3'-overhang, and wherein:
i. the antisense strand consists of 19 nucleotides and the sense strand is an oligonucleotide consisting of a sequence of 19 nucleotides complementary to the antisense strand, and the siRNA is a blunt ended dsRNA structure;
   or
ii. the antisense strand consists of 19 consecutive nucleotides of any of the nucleotide sequences SEQ ID NO: 1124 to SEQ ID NO: 1130 or SEQ ID NO: 1188 to SEQ ID NO: 1190, and further comprises an additional nucleotide or dinucleotide of U or dT at the 3'-end, said additional nucleotide or dinucleotide constituting a 3'-overhang of the antisense strand, and the sense strand is an oligonucleotide having a sequence selected from:
   a. a nucleotide sequence consisting of a sequence of 19 nucleotides in which at least the first 15 consecutive nucleotides from the 5'-end are complementary to the corresponding nucleotides from the 3'-end of the antisense strand, or
   b. a nucleotide sequence consisting of a sequence of 20 or 21 nucleotides in which the first 19 consecutive nucleotides from the 5'-end are complementary to the antisense strand, and are followed by an additional nucleotide or dinucleotide of U or dT at the 3'-end, to form a dsRNA structure of 19 bp with 3'-overhangs of U, dT, UU or dTdT on both strands;
      or
iii. the antisense strand consists of 20 or 21 consecutive nucleotides of any of the nucleotide sequences of SEQ ID NO: 1124 to SEQ ID NO: 1130 or SEQ ID NO: 1188 to SEQ ID NO: 1190, and the sense strand is an oligonucleotide consisting of a sequence of 20 or 21 consecutive nucleotides of any of the nucleotide sequences SEQ ID NO: 1133 to SEQ ID NO: 1139 or SEQ ID NO: 1191 to SEQ ID NO: 1193, respectively, and the siRNA forms a dsRNA structure of 19 bp with 3'-overhangs on both strands.

In preferred embodiments, the antisense strand of the siRNA molecule comprises or consists of at least 19 consecutive nucleotides of any of the nucleotide sequences SEQ ID NO: 1124, SEQ ID NO: 1125, SEQ ID NO: 1126 or SEQ ID NO: 1127, and the sense strand comprises or consists of between 15 to 21 consecutive nucleotides of any of the nucleotide sequences SEQ ID NO: 1133, SEQ ID NO: 1134, SEQ ID NO: 1135 or SEQ ID NO: 1136, respectively. More preferably, the antisense strand of the siRNA molecule comprises or consists of at least 19 consecutive nucleotides of any of the nucleotide sequences SEQ ID NO: 1131, SEQ ID NO: 1132, SEQ ID NO: 1126 or SEQ ID NO: 1127, and the sense strand comprises or consists of between 15 to 21 consecutive nucleotides of any of the nucleotide sequences SEQ ID NO: 1140, SEQ ID NO: 1141, SEQ ID NO: 1135 or SEQ ID NO: 1136, respectively. These siRNAs are targeted to any of the nucleotide sequences from SEQ ID NO: 1 to SEQ ID NO: 8 of the EEF1A2 gene. In particular embodiments, the siRNA comprises or consists of any of the following pairs of antisense and sense strands: an antisense strand of nucleotide sequence SEQ ID NO: 1142 and a sense strand of nucleotide sequence SEQ ID NO: 1150; an antisense strand of nucleotide sequence SEQ ID NO: 1143 and a sense strand of nucleotide sequence SEQ ID NO: 1151; an antisense strand of nucleotide sequence SEQ ID NO: 1145 and a sense strand of nucleotide sequence SEQ ID NO: 1153; an antisense strand of nucleotide sequence SEQ ID NO: 1149 and a sense strand of nucleotide sequence SEQ ID NO: 1157; an antisense strand of nucleotide sequence SEQ ID NO: 1144 and a sense strand of nucleotide sequence SEQ ID NO: 1152; an antisense strand of nucleotide sequence SEQ ID NO: 1146 and a sense strand of nucleotide sequence SEQ ID NO: 1154; an antisense strand of nucleotide sequence SEQ ID NO: 1147 and a sense strand of nucleotide sequence SEQ ID NO: 1155; or an antisense strand of nucleotide sequence SEQ ID NO: 1148 and a sense strand of nucleotide sequence SEQ ID NO: 1156. More preferred siRNAs of these particular embodiments are siRNAs comprising or consisting of any of the following pairs of antisense and sense strands: an antisense strand of nucleotide sequence SEQ ID NO: 1158 and a sense strand of nucleotide sequence SEQ ID NO: 1163; an antisense strand of nucleotide sequence SEQ ID NO: 1143 and a sense strand of nucleotide sequence SEQ ID NO: 1151; an antisense strand of nucleotide sequence SEQ ID NO: 1160 and a sense strand of nucleotide sequence SEQ ID NO: 1165; an antisense strand of nucleotide sequence SEQ ID NO: 1162 and a sense strand of nucleotide sequence SEQ ID NO: 1167; an antisense strand of nucleotide sequence SEQ ID NO: 1159 and a sense strand of nucleotide sequence SEQ ID NO: 1164; an antisense strand of nucleotide sequence SEQ ID NO: 1161 and a sense strand of nucleotide sequence SEQ ID NO: 1166; an antisense strand of nucleotide sequence SEQ ID NO: 1147 and a sense strand of nucleotide sequence SEQ ID NO: 1155; or an antisense strand of nucleotide sequence SEQ ID NO: 1148 and a sense strand of nucleotide sequence SEQ ID NO: 1156.

In other preferred embodiments, the antisense strand of the siRNA molecule comprises or consists of at least 19 consecutive nucleotides of the nucleotide sequence SEQ ID NO: 1124, and the sense strand comprises or consists of between 15 to 21 consecutive nucleotides of the nucleotide sequence SEQ ID NO: 1133. More preferably, the antisense strand of the siRNA molecule comprises or consists of at least 19 consecutive nucleotides of the nucleotide sequence SEQ ID NO: 1131 and the sense strand comprises or consists of between 15 to 21 consecutive nucleotides of the nucleotide sequence SEQ ID NO: 1140. These siRNAs are targeted to any of the nucleotide sequences from SEQ ID NO: 1, SEQ ID NO: 2, SEQ ID NO: 4 or SEQ ID NO: 8 of the EEF1A2 gene. In particular embodiments, the siRNA comprises or consists of any of the following pairs of antisense and sense strands: an antisense strand of nucleotide sequence SEQ ID NO: 1142 and a sense strand of nucleotide sequence SEQ ID NO: 1150; an antisense strand of nucleotide sequence SEQ ID NO: 1143 and a sense strand of nucleotide sequence SEQ ID NO: 1151; an antisense strand of nucleotide sequence SEQ ID NO: 1145 and a sense strand of nucleotide sequence SEQ ID NO: 1153; or an antisense strand of nucleotide sequence SEQ ID NO: 1149 and a sense strand of nucleotide sequence SEQ ID NO: 1157. More preferred siRNAs of these particular embodiments are siRNAs comprising or consisting of any of the following pairs of antisense and sense strands: an antisense strand of nucleotide sequence SEQ ID NO: 1158 and a sense strand of nucleotide sequence SEQ ID NO: 1163; an antisense strand of nucleotide sequence SEQ ID NO: 1143 and a sense strand of nucleotide sequence SEQ ID NO: 1151; an antisense strand of nucleotide sequence SEQ ID NO: 1160 and a sense strand of nucleotide sequence SEQ ID NO: 1165; or an antisense strand of nucleotide sequence SEQ ID NO: 1162 and a sense strand of nucleotide sequence SEQ ID NO: 1167. Even more preferred are siRNAs targeted to the nucleotide sequence SEQ ID NO: 1 of the EEF1A2 gene, which include siRNAs having an antisense strand comprising or consisting of a nucleotide sequence of SEQ ID NO: 1142 or SEQ ID NO: 1158, and a sense strand comprising or consisting of a nucleotide sequence of SEQ ID NO: 1150 or SEQ ID NO: 1163.

In other preferred embodiments, the antisense strand of the siRNA molecule comprises or consists of at least 19 consecutive nucleotides of the nucleotide sequence SEQ ID NO: 1125, and the sense strand comprises or consists of between 15 to 21 consecutive nucleotides of the nucleotide sequence SEQ ID NO: 1134. More preferably, the antisense strand of the siRNA molecule comprises or consists of at least 19 consecutive nucleotides of the nucleotide sequence SEQ ID NO: 1132 and the sense strand comprises or consists of between 15 to 21 consecutive nucleotides of the nucleotide sequence SEQ ID NO: 1141. These siRNAs are targeted to any of the nucleotide sequences from SEQ ID NO: 3 or SEQ ID NO: 5 of the EEF1A2 gene. In particular embodiments, the siRNA comprises or consists of any of the following pairs of antisense and sense strands: an antisense strand of nucleotide sequence SEQ ID NO: 1144 and a sense strand of nucleotide sequence SEQ ID NO: 1152; or an antisense strand of nucleotide sequence SEQ ID NO: 1146 and a sense strand of nucleotide sequence SEQ ID NO: 1154. More preferred siRNAs of these particular embodiments are siRNAs comprising or consisting of any of the following pairs of antisense and sense strands: an antisense strand of nucleotide sequence SEQ ID NO: 1159 and a sense strand of nucleotide sequence SEQ ID NO: 1164; or an antisense strand of nucleotide sequence SEQ ID NO: 1161 and a sense strand of nucleotide sequence SEQ ID NO: 1167. Preferred siRNAs targeted to the nucleotide sequence SEQ ID NO: 3 of the EEF1A2 gene include siRNAs having an antisense strand consisting of a nucleotide sequence of SEQ ID NO: 1144 or SEQ ID NO: 1159, and a sense strand consisting of a nucleotide sequence of SEQ ID NO: 1152 or SEQ ID NO: 1164. Preferred siRNAs targeted to the nucleotide sequence SEQ ID NO: 3 of the EEF1A2 gene include siRNAs having an antisense strand consisting of a nucleotide sequence of SEQ ID NO: 1146 or SEQ ID NO: 1161, and a sense strand consisting of a nucleotide sequence of SEQ ID NO: 1154 or SEQ ID NO: 1166.

In other preferred embodiments, the siRNA molecule is an siRNA duplex comprising or consisting of any or some of the following pairs of nucleotide sequences:
- an antisense strand of nucleotide sequence SEQ ID NO: 52 and a sense strand of nucleotide sequence SEQ ID NO: 103;
- an antisense strand of nucleotide sequence SEQ ID NO: 53 and a sense strand of nucleotide sequence SEQ ID NO: 104;
- an antisense strand of nucleotide sequence SEQ ID NO: 54 and a sense strand of nucleotide sequence SEQ ID NO: 105;
- an antisense strand of nucleotide sequence SEQ ID NO: 55 and a sense strand of nucleotide sequence SEQ ID NO: 106;
- an antisense strand of nucleotide sequence SEQ ID NO: 56 and a sense strand of nucleotide sequence SEQ ID NO: 107;
- an antisense strand of nucleotide sequence SEQ ID NO: 57 and a sense strand of nucleotide sequence SEQ ID NO: 108;
- an antisense strand of nucleotide sequence SEQ ID NO: 58 and a sense strand of nucleotide sequence SEQ ID NO: 109;
- an antisense strand of nucleotide sequence SEQ ID NO: 59 and a sense strand of nucleotide sequence SEQ ID NO: 110;
- an antisense strand of nucleotide sequence SEQ ID NO: 154 and a sense strand of nucleotide sequence SEQ ID NO: 625;
- an antisense strand of nucleotide sequence SEQ ID NO: 155 and a sense strand of nucleotide sequence SEQ ID NO: 626;
- an antisense strand of nucleotide sequence SEQ ID NO: 156 and a sense strand of nucleotide sequence SEQ ID NO: 627;
- an antisense strand of nucleotide sequence SEQ ID NO: 171 and a sense strand of nucleotide sequence SEQ ID NO: 642;
- an antisense strand of nucleotide sequence SEQ ID NO: 172 and a sense strand of nucleotide sequence SEQ ID NO: 643;
- an antisense strand of nucleotide sequence SEQ ID NO: 173 and a sense strand of nucleotide sequence SEQ ID NO: 644; or
- an antisense strand of nucleotide sequence SEQ ID NO: 562 and a sense strand of nucleotide sequence SEQ ID NO: 1033.

In other preferred embodiments, the siRNA molecule has a blunt ended dsRNA structure, and preferably comprises or consists of any of the naked siRNA compounds or siRNA duplexes defined in Figure 2 and their related chemically modified derivative that can be obtained to improve stability and nuclease resistance or modify their biological activity and properties to achieve a better in vivo performance. Chemically modified derivatives preferably are those comprising at least one chemical modification selected from the group consisting of 2'-fluoro modification, 2'-O-methyl modification and a phosphorothioate linkage in the antisense strand, in the sense strand or in both strands. In particular embodiments, the siRNA molecule is an siRNA duplex comprising or consisting of any or some of the following pairs of nucleotide sequences: an antisense strand of nucleotide sequence SEQ ID NO: 52 and a sense strand of nucleotide sequence SEQ ID NO: 103; an antisense strand of nucleotide sequence SEQ ID NO: 53 and a sense strand of nucleotide sequence SEQ ID NO: 104; an antisense strand of nucleotide sequence SEQ ID NO: 54 and a sense strand of nucleotide sequence SEQ ID NO: 105; an antisense strand of nucleotide sequence SEQ ID NO: 55 and a sense strand of nucleotide sequence SEQ ID NO: 106; an antisense strand of nucleotide sequence SEQ ID NO: 56 and a sense strand of nucleotide sequence SEQ ID NO: 107; an antisense strand of nucleotide sequence SEQ ID NO: 57 and a sense strand of nucleotide sequence SEQ ID NO: 108; an antisense strand of nucleotide sequence SEQ ID NO: 58 and a sense strand of nucleotide sequence SEQ ID NO: 109; or an antisense strand of nucleotide sequence SEQ ID NO: 59 and a sense strand of nucleotide sequence SEQ ID NO: 110.

In other preferred embodiments, the siRNA molecule has a 3'-overhang in the antisense strand consisting of an additional nucleotide or dinucleotide of U or dT at the 3'-end, and preferably consisting of a dinucleotide UU. Preferred examples of such siRNA molecules are described in Figure 3, which are naked siRNA molecules that can be chemically modified to improve stability and/or nuclease resistance or modify their biological activity and properties. In particular, these siRNA molecules or duplexes are composed of an antisense strand selected from of any of the nucleotide sequences SEQ ID NO: 154 to SEQ ID NO: 553, and a complementary sense strand selected from of any of the nucleotide sequences SEQ ID NO: 625 to SEQ ID NO: 1024, respectively. In more preferred embodiments, the siRNA molecule is an siRNA duplex comprising or consisting of any or some of the following pairs of nucleotide sequences: an antisense strand of nucleotide sequence SEQ ID NO: 154 and a sense strand of nucleotide sequence SEQ ID NO: 625; an antisense strand of nucleotide sequence SEQ ID NO: 155 and a sense strand of nucleotide sequence SEQ ID NO: 626; an antisense strand of nucleotide sequence SEQ ID NO: 156 and a sense strand of nucleotide sequence SEQ ID NO: 627; an antisense strand of nucleotide sequence SEQ ID NO: 171 and a sense strand of nucleotide sequence SEQ ID NO: 642; an antisense strand of nucleotide sequence SEQ ID NO: 172 and a sense strand of nucleotide sequence SEQ ID NO: 643; an antisense strand of nucleotide sequence SEQ ID NO: 173 and a sense strand of nucleotide sequence SEQ ID NO: 644; or an antisense strand of nucleotide sequence SEQ ID NO: 562 and a sense strand of nucleotide sequence SEQ ID NO: 1033.

An important issue with siRNA molecules is their instability in biological fluids due to the ubiquitous nature of Ribonucleases (RNases). Consequently, the use of many different chemical modifications to nucleotides has been described with the purpose of enhancing compound stability.

Another inherent problem of siRNA molecules is their immunogenicity, whereby siRNAs have been found to induce nonspecific activation of the innate immune system, including up-regulation of certain cytokines, e.g. type I and/or type II interferon as well as IL-12, IL-6 and/or TNF-alpha production. The origin of these effects is thought to be activation of Toll-like receptors such as TLR7, TLR8 and/or TLR3 by siRNA.

Both of these effects, recognition by RNases and immunogenicity, have also been described to be sequence-dependent.

Some of the chemical modifications which enhance compound stability by decreasing susceptibility to RNases are also able to reduce induction of immune recognition and consequently reduce the subsequent immune response. However, insertion of chemically modified nucleotides in a siRNA may also result in decreased silencing efficacy, and hence must be approached with caution.

Consequently, in a preferred embodiment of the various aspects of the present invention, the siRNA further comprises at least one nucleotide with a chemical modification. and/or at least one chemical modification in the ribonucleotide backbone.

Preferred chemical modifications which enhance stability and reduce immunogenic effects include 2'-O-methyl nucleotides, 2'-fluoro nucleotides, 2'-amino nucleotides, 2'-deoxy nucleotides (2'-dA, 2'-dC, 2'-dG or 2'-dT), or nucleotides containing 2'-O or 4'-C methylene bridges. Also other preferred chemical modifications include 5'-methylcytosine (5-mC), 1'-methyladenosine (1'-mA), 7-deaza-7-methyl-2'-dG, N-6 methyl adenine (N6-Me-dA), propandiol, 2',5'-bridged ribonucleotides, 2',5'-bridged deoxyribonucleotides, and backbone linkages 5'-5' or 3'-3'. Other preferred chemical modifications for exonuclease protection include the ExoEndoLight pattern of modification (EEL): modification of all pyrimidines in the sense strand to 2'-O-methyl residues, and modification of all pyrimidines in a 5'-UA-3' or 5'-CA-3' motif in the antisense strand to 2'-O-methyl residues. In addition, position 1 of the sense strand can also be changed to 2'-O-methyl to prevent 5'-phosphorylation of the sense strand and thus increase strand-specificity of the siRNA. In addition, the sense strand can also include a 2'-O-methyl modification in position 14, because 2'-O-Me residues at this position inactivate the sense strand and therefore increase strand-specificity of the siRNAs. In addition, other preferred chemical modifications for nuclease protection include Methyl-Fluoro modification pattern (MEF): alternating 2'-fluoro and 2'-O-methyl modifications starting (5'-end) with a 2'-F on the sense strand and starting with 2'-O-Me on the antisense strand. In addition, position 1 of the sense strand can also be changed to 2'-O-Me and position 1 of the antisense strand to 2'-F (as 2'-F residues are compatible with 5'-phosphorylation whereas 2'O-Me residues are bulky and generally impair phosphorylation). This modification pattern not only stabilizes the molecule but also disables the ability of the RISC to use the sense strand thus promoting strand-specificity. Also, modification of the ribonucleotide backbone can be performed by binding the nucleotides by using phosphorothioate bonds instead of phosphodiester links. A further preferred chemical modification within the meaning of the present invention relates to: 4'-thioribose, 5-propynyluracil 3', 5'-methyluridine or the substitution of uracyl ribonucleotides with deoxythymidine (deoxyribonucleotides). In another preferred embodiment of the present invention, the at least one chemically modified nucleotide and/or the at least one chemical modification in the ribonucleotide backbone is on the sense strand, on the antisense strand or on both strands of the siRNA. Where more than one chemical modification is present, these may be independently selected.

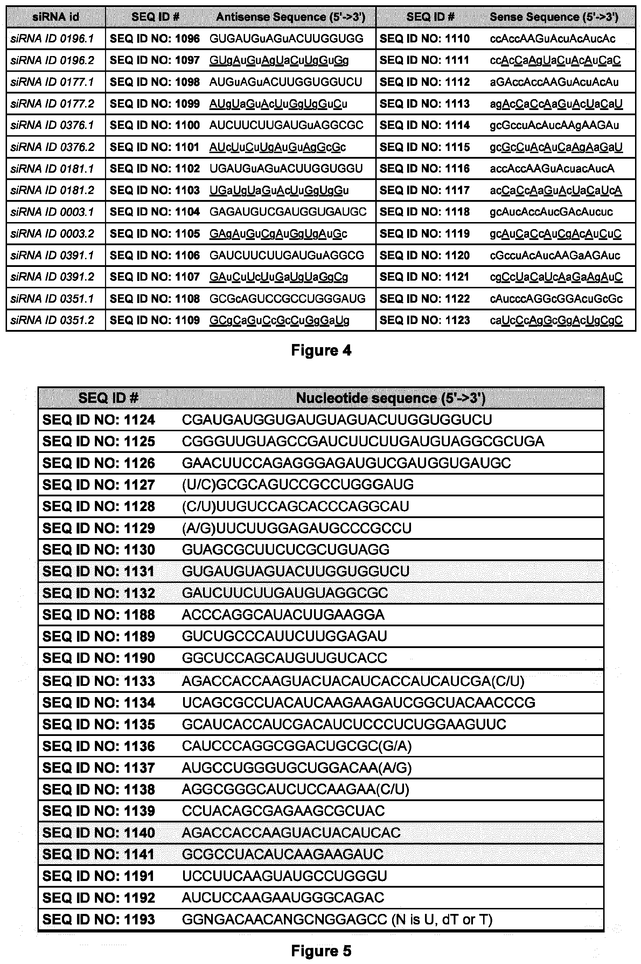

In other preferred embodiments, the siRNA molecule comprises at least one chemical modification in the antisense strand, in the sense strand or in both strands, and the chemical modification is selected from the group consisting of 2'-fluoro modification, 2'-O-methyl modification and a phosphorothioate linkage. Where more than one chemical modification is present, these may be independently selected. In more preferred embodiments, both strands have at least one chemical modification, preferably a 2'-fluoro modification and/or 2'-O-methyl modification. Preferred siRNA molecules comprising chemical modifications are siRNAs that target any of the nucleotide sequences SEQ ID NO: 1, SEQ ID NO:3 or SEQ ID NO:5. Preferably, the siRNA molecule chemically modified targets SEQ ID NO: 1 and comprises or consists of an antisense strand selected from any of the nucleotide sequence SEQ ID NO: 1096 to SEQ ID NO: 1109, and a complementary sense strand selected from any of the nucleotide sequence SEQ ID NO: 1110 to SEQ ID NO: 1123, and more preferably the siRNA molecule is any of the siRNA molecules or duplexes as described in Figure 4. In other embodiments, the siRNA molecule chemically modified targets SEQ ID NO: 3 or SEQ ID NO: 5.

The above described siRNA molecules, also referred to as siRNA compounds or siRNA duplexes, both naked or chemically modified, and any of their embodiments can be referred in the present description as siRNA molecules of the invention, siRNA compounds of the invention, siRNA duplexes of the invention or just as siRNAs of the invention.

The siRNA molecules of the invention can be conjugated to ligands, such as lipids (for example, cholesterol, docosahexanoic acid, sphingolipids), carbohydrates (for example, N-acetylgalactosamine (GalNAc)), glycoproteins, peptides or peptide derivatives (for example, transferrin, tat peptide, GLP1), folate and vitamin E, conjugated to antibodies or aptamers (e.g. anti-Aquaporin 5 (Aqp-5), anti-Caveolins (Cav-1, -2, and -3), anti-CD44v6, anti-CD208, anti-RAGE, anti-Surfactant protein A, anti-Tomsen-Friedenreich marker, anti-TTD1, anti-KL6 and/or anti-CD11b, anti-CD206, anti-CD169, anti-CD44v6, anti-CD208, anti-Cx43), or formulated in nanoparticles, which may be optionally conjugated to antibodies or aptamers, with the purpose of improving their delivery to intended targeted cells and tissues and being internalized into the specific cells. In another aspect, the invention relates to any of these siRNAs conjugated to ligand, antibody or aptamer which can be referred in the present description as siRNA conjugates of the invention.

The siRNAs and siRNA conjugates of the invention when introduced in a cell can reduce the expression levels and/or activity of EEF1A2 and/or EEF1A1 gene, and can be useful for managing cell states as well as diseases and conditions, in which there is an overexpression or an increased activity of EEF1A2 and/or EEF1A1, or there is an abnormal, impaired, or altered function or expression of any of these proteins, preferably of eEF1A2, that compromises or is prejudicial for the normal cell function and may play a role in triggering certain diseases or conditions, such as occur in case of cells infected by certain viruses that seem to usurp the function of these proteins or interact with them during the viral cycle, and that can lead to a condition or disease state in plants or animals after infection with the virus.

Consequently, another aspect of the invention refers to the use of a small interfering RNA (siRNA) molecule which targets the eukaryotic translation elongation factor 1 alpha 2 (EEF1A2) gene and/or the eukaryotic translation elongation factor 1 alpha 1 (EEF1A1), as an antiviral compound against viruses of vegetal and animal organisms (i.e. plants and animals, including humans), preferably of viruses which are pathogens for plants or animals, and more preferably of viruses that cause diseases or conditions in mammals and/or human subjects. In preferred embodiments, the siRNA molecule for use as an antiviral compound is any of the siRNA molecules of the present invention or their embodiments described above. Preferred siRNA compounds are those able to reduce the expression and/or activity of EEF1A2 and/or EEF1A1 in a cell, preferably in a cell of a plant or of an animal, and which can reduce the replication of a virus in the cell treated with the siRNA compound and infected by the virus. Reducing the expression and/or activity of EEF1A2 and/or EEF1A1 in the cell with these siRNAs can be advantageous in case of positive-sense single-stranded RNA viruses (ssRNA viruses) that translate their viral genome into viral proteins prior to the replication of the viral genome within the cell carried out by RNA dependent RNA polymerase complexes encoded by the virus RNA, although it could also reduce the viral gene expression by blocking the protein translation in other RNA viruses (positive-sense single-stranded RNA viruses or double-stranded RNA viruses) or even in DNA viruses. Likewise, the invention also refers to an small interfering RNA (siRNA) molecule which targets the eukaryotic translation elongation factor 1 alpha 2 (EEF1A2) gene and/or the eukaryotic translation elongation factor 1 alpha 1 (EEF1A1) for use in the prophylactic or therapeutic treatment of a virus infection in a subject, including the siRNAs of the invention and any of the embodiments described above. In preferred embodiments, the siRNA molecule for the referred use is any of the siRNA molecules of the present invention or their embodiments described above.

In other embodiments, the use of the siRNA molecule as an antiviral or the siRNA molecule for use, is for the prophylactic or therapeutic treatment of a virus infestation or infection, preferably caused by an RNA virus, in a plant or in an animal subject which is infested, infected or is at risk of exposure to said virus. In more preferred embodiments, the virus is a single stranded RNA (ssRNA) virus, including viruses with a positive ssRNA genome which can act as an mRNA and be directly translated into viral proteins by the host machinery, and viruses with a negative ssRNA genome that requires prior mRNA transcription dependent on an RNA genome RNA dependent RNA polymerase to be translated within the host cell. Preferred positive sense ssRNA viruses are selected from a *Coronavirus,* including severe acute respiratory syndrome coronavirus 2 (SARS-CoV-2), severe acute respiratory syndrome coronavirus (SARS-CoV) or Middle East respiratory syndrome coronavirus (MERS-CoV), a *Flavivirus,* including West Nile virus (WNV), Dengue virus (DENV), Zika virus (ZIKV) and yellow fever virus (YFV), an *Hepatovirus,* including hepatovirus A or hepatitis A virus (HAV), an hepatitis C virus (HCV), an hepatitis E virus (HEV), or a *Tobamovirus,* including tomato brown rugose fruit virus (ToBRFV), tomato mosaic virus (ToMV), tomato mottle mosaic virus (ToMMV), tobacco latent virus (TLV), tobacco mild green mosaic virus (TMGMV), tobacco mosaic virus (TMV), among others, and more preferably is a *Coronavirus.* Preferred negative sense ssRNA viruses are selected from an *Influenzavirus,* including influenzavirus A, influenzavirus B, influenzavirus C, or influenzavirus D, an hepatitis D virus (HDV), or an *Orthopneumovirus,* including respiratory syncytial virus (RSV), among others, and more preferably is an *Influenzavirus.*

In case of positive-sense ssRNA viruses, control of translation elongation by reducing the expression and/or activity of EEF1A2 and/or EEF1A1 in the cell with a siRNA can be advantageous to reduce or prevent reducing the translation of a positive sense single-stranded viral RNA genome into viral proteins prior to its replication within the cell.

### Formulation and Pharmaceutical Compositions

Another aspect of the invention relates to a pharmaceutical composition that includes or comprises an siRNA compound of the invention, e.g., a double-stranded siRNA compound as any of the described above. In embodiments, the pharmaceutical composition can be a solution, an emulsion, microemulsion, cream, jelly, or liposome.

The siRNA compounds of the invention described herein can be formulated for administration to a subject, preferably for administration locally to the lungs and nasal passage (respiratory tissues) via inhalation or intranasal administration, or simultaneous administration to the mouth and nose or to the mouth, nose and eyes, for example as an aerosol to be administered using a face mask covering mouth and nose and optionally also covering the eye, or topically, or systemically, such as parenterally, e.g., via injection.

For purposes of exposition, the formulations, compositions, and methods in this section are discussed with regard to unmodified siRNA compounds. It should be understood, however, that these formulations, compositions, and methods can be practiced with other siRNA compounds, e.g., modified siRNA compounds, and such practice is also described herein.

A formulated siRNA composition can assume a variety of states. In some examples, the composition is at least partially crystalline, uniformly crystalline, and/or anhydrous (for example, less than 90, 80, 70, 60, 50, 40, 30, 20, or 10% water). In another example, the siRNA compound is in an aqueous phase, for example, in a solution that includes water, this form being the preferred form for administration via inhalation.

The aqueous phase or the crystalline compositions can be incorporated into a delivery vehicle, for example, a liposome (particularly for the aqueous phase), or a particle (for example, a microparticle as can be appropriate for a crystalline composition). Generally, the siRNA composition is formulated in a manner that is compatible with the intended method of administration.

An siRNA compound preparation can be formulated in combination with another agent, for example, another therapeutic agent or an agent that stabilizes an siRNA compound, for example, a protein that complexes with the siRNA compound to form an RNA-induced silencing complex. Still other agents include chelators, for example, EDTA (for example, to remove divalent cations such as Mg²⁺), salts, RNase inhibitors and so forth.

The term therapeutic agent include therapies which are currently approved or are being experimentally tested for treatment of the virus disease. In case of treating or preventing a *Coronavirus* disease, such as COVID-19, therapeutic agents can include for example: antibodies, such as bevacizumab, nivolumab, leronlimab (PRO 140), camrelizumab, sarilumab, tocilizumab, gimsilumab, TJM2 (TJ003234, an anti-granulocyte-macrophage colony stimulating factor antibody), lenzilumab, siltuximab, eculizumab, canakinumab, emapalumab, meplazumab, LY3127804 (an anti-Angiopoietin 2 (Ang2) antibody), IFX-1 (an anti-C5a antibody), otilimab or antibodies from recovered SARS patients or COVID-19 patients (for example, VIR-7831 or VIR-7832); antivirals, such as lopinavir/ritonavir, remdesivir, galidesivir, emtricitabine/tenofovir, umifenovir, oseltamivir, EIDD-2801 (an oral ribonucleoside analog), ribavirin, clevudine, recombinant sialidase DAS181, emetine hydrochloride or AT-527 (an oral purine nucleotide prodrug); other RNA-based treatments, such as TGF-beta antisense drug candidate OT-101 or siRNA candidate VIR-2703; and other active compounds and small molecules of approved therapies for other indications, such as chloroquine/ hydroxychloroquine, camostat mesylate, ruxolitinib, fingolimod, interferon beta-1a, nafamostat, losartan, valsartan, telmisartan, alteplase, anakinra, plitidepsin, dipyridamole, baricitinib, tofacitinib, heparin, selinexor, acalabrutinib, ivermectin, nitazoxanide, dantrolene sodium, dapagliflozin, recombinant human C1 esterase inhibitor (Ruconest^{®}), famotidine, apremilast, tranexamic acid, fluvoxamine, imatinib or nafamostat. In case of treating or preventing an infection or disease caused by an influenza virus, the therapeutic agent can be, for example, any of the four FDA-approved influenza antiviral drugs recommended by Centers for Disease Control and Prevention for use against recently circulating influenza viruses, such as Rapivab^{™} (peramivir), Relenza^{™} (zanamivir), Tamiflu^{™} (oseltamivir phosphate, also available as generic), or Xofluza^{™} (baloxavir marboxil), any of the two older drugs historically approved for treatment and prevention of influenza A virus infection, such as amantadine (generic) or rimantadine (Flumadine^{™} and generic), or others as for example, rintatolimod (Ampligen^{®}), umifenovir (Arbidol^{™}), moroxydine. Other therapeutic agents can be antivirals for hepatitis C viruses, such as boceprevir, daclatasvir (Daklinza^{™}), delavirdine, ribavirin, simeprevir (Olysio^{™}), sofosbuvir, talaprevir and taribavirin; antivirals for hepatitis D viruses such as bulevirtide; antivirals for yellow fever viruses such as taribavirin (Viramidine^{™}); or even a broad-spectrum antiviral drug such as nitazoxanide.

The siRNA compound preparation may include another siRNA compound, e.g., a second siRNA compound or RNAi agent that can mediate RNAi with respect to a second gene or with respect to a different target region of the same gene. The siRNA compounds or RNAi agents may be directed to the same virus but different target sequences, or each siRNA compound or RNAi agent may be directed to a different virus; or one or more siRNA compounds may be directed to a target sequence in a host (e.g., patient, e.g., human patient) gene, and one or more siRNA compounds may be directed to a target sequence in a virus (e.g., in a *Coronavirus,* including MERS-CoV, SARS-CoV and SARS-CoV-2, or in an influenzavirus, including *Alphainfluenzavirus* that infects humans, other mammals, and birds, *Betainfluenzavirus* that infects humans and seals, *Deltainfluenzavirus* that infects pigs and cattle, and *Gammainfluenzavirus* that infects humans, pigs, and dogs); or each siRNA compound may be directed to a different gene in a host.

In one embodiment, the pharmaceutical composition includes or comprises an siRNA compound of the invention mixed with a topical delivery agent. The topical delivery agent can be a plurality of microscopic vesicles. The microscopic vesicles can be liposomes. In some embodiments the liposomes are cationic liposomes. Cationic lipids or liposomes can spontaneously form complexes with negatively charged siRNA or oligonucleotides through electrostatic interactions with the positively charged lipids. Examples of cationic lipids include 1,2-dioleoyl-3-trimethylammonium-propane (DOTAP), dimethyldioctadecylammonium (DDAB), 1,2-di-O-octadecenyl-3-trimethylammonium-propane (DOTMA), 3β-[N-(N',N'-dimethylaminoethane)-carbamoyl]cholesterol hydrochloride (DC- CHOL), 1,2-dioleoyl-3-dimethylammonium-propane (DODAP), cetyl trimethylammonium bromide (CTAB), 1,3-dioleoyloxy-2-(6-carboxy-spermyl)-propylamide (DOSPER), or 1,2-dioleoyl-sn-glycero-3-[(N- (5-amino-1-carboxypentyl) iminodiacetic acid)succinyl] (DOGS). Cationic lipids with a hydrophilic head and at least one hydrophobic tail are disclosed in any of the WO 1995/026356 A1, WO 2000/027795 A1, WO 2002/066012 A2, WO 2004/002454 A1, WO 2004/002468 A1, WO 2007/107304 A2, WO 2007/130073 A2, WO 2010/008582 A2, WO 2011/119901 A1 and WO 2012/068176 A1. Optionally, hydrophilic polymers such as polyethylene glycol (PEG) can be also used to shield the positive charge of the cationic lipids and reduce inflammatory response.

In another embodiment, the pharmaceutical composition comprising the siRNA compound of the invention, e.g., a double-stranded siRNA compound, is admixed with a topical penetration enhancer. In one embodiment, the topical penetration enhancer is a fatty acid. The fatty acid can be arachidonic acid, oleic acid, lauric acid, caprylic acid, capric acid, myristic acid, palmitic acid, stearic acid, linoleic acid, linolenic acid, dicaprate, tricaprate, monolein, dilaurin, glyceryl 1-monocaprate, 1-dodecylazacycloheptan-2-one, an acylcarnitine, an acylcholine, or a C1-10 alkyl ester, monoglyceride, diglyceride or pharmaceutically acceptable salt thereof. In another embodiment, the topical penetration enhancer is a bile salt. The bile salt can be cholic acid, dehydrocholic acid, deoxycholic acid, glucholic acid, glycholic acid, glycodeoxycholic acid, taurocholic acid, taurodeoxycholic acid, chenodeoxycholic acid, ursodeoxycholic acid, sodium tauro-24,25-dihydro-fusidate, sodium glycodihydrofusidate, polyoxyethylene-9-lauryl ether or a pharmaceutically acceptable salt thereof. In another embodiment, the penetration enhancer is a chelating agent. The chelating agent can be EDTA, citric acid, a salicyclate, a N-acyl derivative of collagen, laureth-9, an N-amino acyl derivative of a beta-diketone or a mixture thereof. In another embodiment, the penetration enhancer is a surfactant, e.g., an ionic or nonionic surfactant. The surfactant can be sodium lauryl sulfate, polyoxyethylene-9-lauryl ether, polyoxyethylene-20-cetyl ether, a perfluorchemical emulsion or mixture thereof. In another embodiment, the penetration enhancer can be selected from a group consisting of unsaturated cyclic ureas, 1-alkyl-alkones, 1-alkenylazacyclo-alakanones, steroidal anti-inflammatory agents and mixtures thereof. In yet another embodiment the penetration enhancer can be a glycol, a pyrrol, an azone, or a terpene.

In another embodiment, the pharmaceutical composition including or comprising the siRNA compound is in a form suitable for oral delivery, and/or is in an oral dosage form. In one embodiment, oral delivery can be used to deliver an siRNA compound composition to a cell or a region of the throat, the gastro-intestinal tract, e.g., small intestine, colon, and so forth. The oral delivery form can be tablets, capsules or gel capsules. In another embodiment, the pharmaceutical composition includes an enteric material that substantially prevents dissolution of the tablets, capsules or gel capsules in a mammalian stomach. In some embodiments the enteric material is a coating. The coating can be acetate phthalate, propylene glycol, sorbitan monoleate, cellulose acetate trimellitate, hydroxy propyl methylcellulose phthalate or cellulose acetate phthalate. In another embodiment, the oral dosage form of the pharmaceutical composition includes a penetration enhancer. The penetration enhancer can be a bile salt or a fatty acid. The bile salt can be ursodeoxycholic acid, chenodeoxycholic acid, and salts thereof. The fatty acid can be capric acid, lauric acid, and salts thereof. In another embodiment, the oral dosage form of the pharmaceutical composition includes an excipient, which can be for example polyethyleneglycol or precirol. In another embodiment, the oral dosage form of the pharmaceutical composition includes a plasticizer. The plasticizer can be diethyl phthalate, triacetin dibutyl sebacate, dibutyl phthalate or triethyl citrate.

In another embodiment, the pharmaceutical composition includes or comprises the siRNA compound of the invention and a delivery vehicle. In one embodiment, the delivery vehicle can deliver the siRNA compound to a cell by a topical route of administration. The delivery vehicle can be microscopic vesicles. In one example the microscopic vesicles are liposomes. In some embodiments the liposomes are cationic liposomes. Examples of cationic lipids that form cationic liposomes with negatively charged siRNA or oligonucleotides are described above. In another example the microscopic vesicles are micelles. For biomedical applications, micelle structures are of particular interest because of their small size, good biocompatibility, high stability both in vitro and in vivo, and the ability to transport pharmaceuticals. Amphiphilic compounds spontaneously self-associate into micelles when dispersed in water at a concentration above their critical micelle concentration (CMC). For example, polymeric micelles for siRNA delivery can be designed by direct conjugation of hydrophilic (Polyethylene glycol -PEG-) or hydrophobic (lipid) moieties to siRNA via degradable (e.g., disulfide) or non-degradable linkages, followed by their condensation with polycationic ions to form micellar structures called polyion complex micelles (PICs) or polyelectrolyte complex micelles (PECs). In PIC micelles, the polyion segments are usually made of poly(amino acids) like poly(aspartic acid) or poly(L-lysine) or polyethylenimine (PEI). In an alternative approach to design polymeric micelles, siRNA can be complexed with an amphiphilic block copolymer containing polycation (or lipid) segment followed by micellization of the block copolymer-siRNA complex. Useful block copolymers can be PEG-b-poly(L-lysine) (PEG-b-PLL) containing lysine amines modified with 2-iminothiolane (2IT), which can be further modified with cyclo-Arg-Gly-Asp (cRGD) peptide at the PEG terminus. Another useful copolymer can be the triblock copolymer poly(BMA-co-PAA-co-DMAEMA)-b-poly(DMAEMA)-b-poly(AzEMA) resulting of micelle blocks consisting of a core-forming terpolymer of butyl methacrylate-co-2-propyl acrylic acid-co-2-dimethylaminoethyl methacrylate) (BMA-co-PAA-co-DMAEMA), a cationic block for condensing siRNA (DMAEMA) and an azide-presenting corona-forming block for the attachment of alkyne-functionalized mannose (2-azidoethyl methacrylate (AzEMA), which can be further mannosylated by reaction with alkyne functionalized mannose. Cationic micelles can be also formed from diblock copolymers of dimethylaminoethyl methacrylate (pDMAEMA) and butyl methacrylate (BMA), from diblock copolymers of linear PEI and PCL (PEI-PCL) (Jhaveri et al. 2014).

In another embodiment, the siRNA compounds of the invention can be formulated in a pharmaceutical composition with a pharmaceutically acceptable vehicle or carrier that comprises a polymeric nanoparticle. In one embodiment, the polymeric nanoparticle is a nanoparticle comprising poly(beta-amino ester)s (PBAEs), preferably modified with at least one oligopeptide. End-modified PBAEs and related nanoparticles useful for the delivery of polynucleotides in medical applications, as well as methods for their preparation have been described for example in WO2014136100 A1, Dosta et al. 2018 and Segovia et al. 2014. Oligopeptide-modified pBAEs can be products obtained by end-modification of an acrylate-terminated polymer (for example, a C32, C6 or C16 polymers resulting from polymeric addition reaction of primary amines to 1,4-butanediol diacrylate) with thiol-terminated oligopeptides (for example, H-CysArgArgArg-NH₂, H-CysLysLysLys-NH₂, H-CysHisHisHis-NH₂, H-CysGluGluGlu-NH₂, or H-CysAspAspAsp-NH₂). C32 polymers can be obtained by polymerisation of 5-amino-pentanol and 1,4-butanediol diacrylate. C6 polymers may be synthesized by conjugate addition of different ratios of hexylamine/5-amino-1-pentanol (for example, 1:0, 1:1 or 1:3 for C6-100, C6-50 or C6-25 polymers, respectively) to 1,4-butanediol. Similarly, C16 polymers may be synthesized by conjugate addition of different ratios of hexadecylamine/5-amino-1-pentanol (for example, 1:1 or 1:3 for C16-50, C6-50 or C16-25 polymers, respectively) to 1,4-butanediol. C32, C6 or C16 polymers may be also optionally modified with cholesterol by different degrees of esterification (for example, 50%, 25%, 12.5%) of the polymer hydroxyl groups with carboxylic acid-modified cholesterol (cholesterol-COOH).

In another embodiment, the pharmaceutical composition comprising a siRNA compound of the invention is in a pulmonary or nasal dosage form. In one embodiment, the siRNA compound is incorporated into a particle, e.g., a macroparticle, e.g., a microsphere. The particle can be produced by spray drying, lyophilisation, evaporation, fluid bed drying, vacuum drying, or a combination thereof. The microsphere can be formulated as a suspension, a powder, or an implantable solid.

In another embodiment, the pharmaceutical composition comprising the siRNA compound is in an injectable dosage form. In one embodiment, the injectable dosage form of the pharmaceutical composition includes sterile aqueous solutions or dispersions and sterile powders. In some embodiments the sterile solution can include a diluent such as water; saline solution; fixed oils, polyethylene glycols, glycerine, or propylene glycol.

### Treatment Methods and Routes of Delivery

Another aspect of the invention relates to a method of reducing the expression of an EEF1A gene (EEF1A2 and/or EEF1A1) in a cell, comprising contacting said cell with a siRNA compound of the invention. In one embodiment, the cell is a cell from the higher or lower respiratory tract. In another embodiment, the cell is a cell from the gastrointestinal tract, or from the central nervous system, or from the eye or from the endothelium tissue.

Another aspect of the invention relates to a method of reducing the expression of an EEF1A gene (EEF1A2 and/or EEF1A1) in a subject, comprising administering to the subject siRNA compound of the invention.

Another aspect of the invention relates to a method for prophylactic and/or therapeutic treatment of a virus infection or its related diseases in a subject, comprising administering to the subject a therapeutically effective amount of the siRNA compound of the invention, thereby treating the subject. Preferably the virus infection is caused by a virus that involves a host eEF1A protein (i.e. eEF1A2 and/or eEF1A1) during the viral cycle, such as several types of known virus, including coronavirus (SARS-CoV-2, SARS-CoV or MERS-CoV) and influenza virus (influenza A virus, influenza B virus, influenza C virus or influenza D virus), or their related diseases. Exemplary related diseases that can be prevented or treated by the method of the invention include COVID-19, SARS, MERS and influenza.

The siRNA or compositions of the invention can be delivered to a subject by a variety of routes, being preferred the topical or the pulmonary routes, and especially for viral respiratory diseases. The siRNA and pharmaceutical compositions described herein may be administered in a number of ways depending upon whether local or systemic treatment is desired and upon the area to be treated. Administration may be topical (including intranasal, intratracheal, intrapulmonary or onto the corneal surface of the eye), oral or parenteral. Exemplary routes include inhalation, nasal, oral, ophthalmic or intravenous delivery.

In general, the delivery of the siRNA compounds described herein is done to achieve delivery into the subject to the site of infection. This objective can be achieved through local (i.e., topical) administration to the lungs, nasopharynx, pharynx or nasal passage, e.g., into the respiratory tissues via inhalation, nebulization or intranasal administration, local (i.e., topical) administration to the eye, e.g., administration to the corneal surface of the eye through eye drops, ocular spray or nebulization, local administration to the gastrointestinal tract, e.g., via oral, sublingual or rectal, or via systemic administration, e.g., parental administration. Parenteral administration includes intravenous drip, subcutaneous, intraperitoneal or intramuscular injection. The preferred means of administering the siRNA compounds described herein is through direct topical administration to the lungs, nasopharynx, pharynx and/or nasal passage by inhalation of an aerosolized liquid such as a nebulized mist or a nasal spray.

An siRNA compound can be incorporated into pharmaceutical compositions suitable for administration. For example, compositions can include one or more siRNA compounds and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

Formulations for inhalation, intranasal, or parenteral administration are well known in the art. Such formulations may include sterile aqueous solutions which may also contain buffers, diluents and other suitable additives, an example being Phosphate-buffered saline (PBS) or Dextrose 5% in water. For intravenous use, the total concentration of solutes should be controlled to render the preparation isotonic.

The active compounds disclosed herein are preferably administered to the lung(s), nasopharynx, pharynx or nasal passage of a subject by any suitable means. Active compounds may be administered by administering an aerosol suspension of respirable particles comprised of the active compound or active compounds, which the subject inhales. The active compound can be aerosolized in a variety of forms, such as, but not limited to, dry powder inhalants, metered dose inhalants (including pressured metered dose inhalants), or liquid/liquid suspensions. The respirable particles may be liquid or solid. The particles may optionally contain other therapeutic ingredients such as amiloride, benzamil or phenamil, with the selected compound included in an amount effective to inhibit the reabsorption of water from airway mucous secretions (for example, as described in U.S. Pat. No. 4,501,729).

The particulate pharmaceutical composition may optionally be combined with a carrier to aid in dispersion or transport. A suitable carrier such as a sugar (e.g., dextrose, lactose, sucrose, trehalose, mannitol) may be blended with the active compound or compounds in any suitable ratio (e.g., a 1 to 1 ratio by weight).

An active compound may be topically administered by inhalation. As used in this specification, administration by "inhalation" generally refers to the inspiration of particles comprised of the active compound that are of respirable size, that is, particles of a size sufficiently small to pass through the mouth or nose and larynx upon inhalation and into the bronchi and alveoli of the lungs. In general, particles ranging from about 1 to 10 microns in size (more particularly, less than about 5 microns in size) are respirable and suitable for administration by inhalation.

"Topical administration" refers to the delivery to a subject by contacting the formulation directly to a surface of the subject. The most common form of topical delivery is to the skin, but a composition disclosed herein can also be directly applied to other surfaces of the body, e.g., to the eye, a mucous membrane, to surfaces of a body cavity or to an internal surface.

An active compound may be topically delivered by intranasal administration. As used in this specification, "intranasal" administration refers to administration of a dosage form formulated and delivered to topically treat the nasal epithelium. Particles or droplets used for intranasal administration generally have a diameter that is larger than those used for administration by inhalation. For intranasal administration, a particle size in the range of 10-500 microns is preferred to ensure retention in the nasal cavity. Particles of non-respirable size which are included in the aerosol tend to deposit in the throat and be swallowed, and the quantity of non-respirable particles in the aerosol is preferably minimized.

Liquid pharmaceutical compositions of active compound for producing an aerosol can be prepared by combining the active compound with a suitable vehicle, such as sterile pyrogen free water. In certain embodiments hypertonic saline solutions are used to carry out the present invention. These are preferably sterile, pyrogen free solutions, comprising from one to fifteen percent (by weight) of a physiologically acceptable salt, and more preferably from three to seven percent by weight of the physiologically acceptable salt.

Aerosols of liquid particles comprising the active compound may be produced by any suitable means, such as with a pressure-driven jet nebulizer or an ultrasonic nebulizer. See, e.g., U.S. Pat. No. 4,501,729. Nebulizers are commercially available devices which transform solutions or suspensions of the active ingredient into a therapeutic aerosol mist either by means of acceleration of compressed gas, typically air or oxygen, through a narrow venturi orifice or by means of ultrasonic agitation.

Suitable formulations for use in nebulizers may consist of the active ingredient in a liquid carrier, the active ingredient comprising up to 40% w/w of the formulation, but preferably less than 20% w/w. The carrier is typically water (and most preferably sterile, pyrogen-free water) or a dilute aqueous-alcoholic solution, preferably made isotonic but may be hypertonic with body fluids by the addition of, for example, sodium chloride. Optional additives include preservatives if the formulation is not made sterile, for example, methyl hydroxybenzoate, antioxidants, flavouring agents, volatile oils, buffering agents and surfactants.

Aerosols of solid particles comprising the active compound may likewise be produced with any solid particulate therapeutic aerosol generator. Aerosol generators for administering solid particulate therapeutics to a subject produce particles which are respirable and generate a volume of aerosol containing a predetermined metered dose of a therapeutic at a rate suitable for human administration. One illustrative type of solid particulate aerosol generator is an insufflator. Suitable formulations for administration by insufflation include finely comminuted powders which may be delivered using an insufflator or taken into the nasal cavity in the manner of a snuff. In the insufflator, the powder (e.g., a metered dose thereof effective to carry out the treatments described herein) is contained in capsules or cartridges, typically made of gelatin or plastic, which are either pierced or opened in situ and the powder delivered by air drawn through the device upon inhalation or employing a manually-operated pump. The powder employed in the insufflator consists either solely of the active ingredient or of a powder blend comprising the active ingredient, a suitable powder diluent, such as lactose, and an optional surfactant. The active ingredient typically comprises from 0.1 to 100 w/w of the formulation.

A second type of illustrative aerosol generator comprises a metered dose inhaler. Metered dose inhalers are pressurized aerosol dispensers, typically containing a suspension or solution formulation of the active ingredient in a liquefied propellant. During use these devices discharge the formulation through a valve adapted to deliver a metered volume, typically from 10 µl to 200 µl, to produce a fine particle spray containing the active ingredient. Suitable propellants include certain chlorofluorocarbon compounds, for example, dichlorodifluoromethane, trichlorofluoromethane, dichlorotetrafluoroethane and mixtures thereof. The formulation may additionally contain one or more co-solvents, for example, ethanol, surfactants, such as oleic acid or sorbitan trioleate, antioxidant and suitable flavouring agents.

An active siRNA compound may also be administered to the buccal cavity of a human being by spraying into the cavity, without inhalation, from a metered dose spray dispenser, a mixed micellar pharmaceutical formulation as described above and a propellant. In one embodiment, the dispenser is first shaken prior to spraying the pharmaceutical formulation and propellant into the buccal cavity. For example, the medication can be sprayed into the buccal cavity or applied directly, e.g., in a liquid, solid, or gel form to a surface in the buccal cavity. This administration is particularly desirable for the treatment of inflammations of the buccal cavity, e.g., the gums or tongue, e.g., in one embodiment, the buccal administration is by spraying into the cavity, e.g., without inhalation, from a dispenser, e.g., a metered dose spray dispenser that dispenses the pharmaceutical composition and a propellant.

An active siRNA compound may be also administered for oral use. Formulations and compositions for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

An active siRNA compound may be also administered in the form of suppositories, e. g., for rectal administration of the drug. These compositions can be prepared by mixing the drug with a suitable nonirritating excipient that is solid at ordinary temperatures but liquid at the rectal temperature and will therefore melt in the rectum to release the drug. Such materials include cocoa butter and polyethylene glycols.

Administration can be provided by the subject or by another person, e.g., a caregiver. A caregiver can be any entity involved with providing care to the human: for example, a hospital, hospice, doctor's office, outpatient clinic; a healthcare worker such as a doctor, nurse, or other practitioner; or a spouse or guardian, such as a parent. The medication can be provided in measured doses or in a dispenser which delivers a metered dose.

Preferably, the prophylactic and therapeutic treatments are used in human subjects, although the treatments can be also applied to other mammals, including EEF1A2 and/or EEF1A1 humanized mice, dogs, monkeys and macaques, or others expressing proteins eEF1A2 and/or eEF1A1 in tissues accessible to the virus which can be bound to viral proteins and being susceptible of virus infection or of viral replication into their cells, preferably of a virus causing disease in humans, such as any of the coronaviruses, SARS-CoV-2, SARS-CoV-1 or MERS-CoV, or an influenzavirus related to influenza or 'flu' in humans, for example an *Alphainfluenzavirus* (i.e. an influenza A virus, including subtypes H1N1, H2N2, H3N2, H5N1, H7N9, H7N7, H1N2, H9N2, H7N2, H7N3, H5N2 and H10N7), a *Betainfluenzavirus* (i.e. an influenza B virus), or a *Gammainfluenzavirus* (i.e. an influenza C virus).

According to the present invention, virus infestations or infections that can be treated or prevented are those caused by any virus involving or requiring at least an Eukaryotic Translation Elongation Factor 1 Alpha protein (eEF1A), preferably the Eukaryotic Translation Elongation Factor 1 Alpha 2 (eEF1A2) and/or the eukaryotic translation elongation factor 1 alpha 1 (eEF1A1), in any step of the virus cycle within the cell, including viruses with single-stranded (ss) or double-stranded (ds), RNA or DNA genomes, that may or may not use reverse transcriptase (RT). In addition, ssRNA viruses may be either sense (i.e. RNA of positive (+) sense) or antisense (i.e. RNA of negative (-) sense). Examples of these viruses include, among others, animal viruses such as bovine viral diarrhea virus (BVDV), vesicular stomatitis virus (VSV), cutaneous human papillomavirus (HPV) type 38, human immunodeficiency virus 1 (HIV-1), vaccinia virus (Poxviridae), cytomegalovirus (Herpesviridae), hepatitis B virus (HBV), hepatitis C virus (HCV), West Nile virus (WNV), and coronavirus causing respiratory syndromes, including severe acute respiratory syndrome coronavirus SARS-CoV-1 and SARS-CoV-2 (Coronaviridae), but also plant viruses such as turnip yellow mosaic virus (TYMV), tobacco mosaic virus (TMV), brome mosaic virus (BMV), and tomato bushy stunt virus (TBSV). These viruses can be pathogenic for plants in crops, or can cause diseases in livestock animals and humans that can be lead to important economic losses in crops and animal husbandry worldwide, but also to health crisis due to epidemics and pandemics caused by these viruses with the subsequent economic and humanitarian impact that can have on the public health and medical care systems, the health and the economy of the countries. Other viruses of interest to be treated or prevented include the tomato brown rugose fruit virus (ToBRV), a plant virus in the genus Tobamovirus, and any of the influenza viruses that infect vertebrates, including alphainfluenzavirus, betainfluenzavirus, gammainfluenzavirus and deltainfluenzavirus. For example, tomato brown rugose fruit virus (ToBRV) causes symptoms including mosaic and distortion of leaves and brown, wrinkly spots (rugose) on fruits, mainly from tomato and peppers, whose outbreaks can be severe and leave fruit unmarketable.

Influenza A virus (influenzavirus A), the only species of alphainfluenzavirus infects and causes influenza in humans, other mammals, and birds, and may occasionally cause an outbreak or give rise to human influenza pandemics. These are negative-sense, single-stranded, segmented RNA viruses, and include several subtypes which are labelled according to an H number (for the type of hemagglutinin) and an N number (for the type of neuraminidase). There are 18 different known H antigens (H1 to H18) and 11 different known N antigens (N1 to N11). H1N1, H1N2, and H3N2 are influenza A virus subtypes currently circulating among humans, although others such as H2N2, H5N1, H7N9, H7N7, H9N2, H7N2, H7N3, H5N2 and H10N7 have been also confirmed in humans. Human flu symptoms usually include fever, cough, sore throat, muscle aches, conjunctivitis and, in severe cases, breathing problems and pneumonia that may be fatal. The severity of the infection will depend in large part on the state of the infected person's immune system and if the victim has been exposed to the strain before, and is therefore partially immune.

Coronavirus disease 2019 or COVID-19 refers to an infectious disease caused by the new coronavirus discovered at the end of year 2019, SARS-CoV-2, which is responsible for the coronavirus pandemic that affects more than 51.5 million people and have caused more than 1.27 million deaths to date. COVID-19 affects different people in different ways. While understanding of the disease is still developing, most people infected with the COVID-19 virus experience mild to moderate respiratory illness and recover without requiring special treatment. Older people, and those with underlying medical problems like cardiovascular disease, diabetes, chronic respiratory disease, and cancer are more likely to develop serious illness. Most common symptoms are fever or chills, dry cough and tiredness, although less common symptoms include aches and pains, sore throat, diarrhoea, conjunctivitis, headache, loss of taste or smell, congestion or runny nose, nausea or vomiting, and a rash on skin, or discolouration of fingers or toes. Serious symptoms of COVID-19 are difficulty breathing or shortness of breath, chest pain or pressure, and loss of speech or movement, which normally require to seek immediate medical attention or even other collateral symptoms such as cardiovascular alterations as well as neurological and cognitive diseases. People with mild symptoms who are otherwise healthy should manage their symptoms at home. On average it takes 5-6 days from when someone is infected with the virus for symptoms to show, however it can take up to 14 days. Currently, there are many ongoing clinical trials evaluating potential treatments, but there are no specific vaccines or treatments for COVID-19. Thus, the best way to prevent and slow down transmission is to be well informed about the COVID-19 virus, the disease it causes and how it spreads. The COVID-19 virus spreads primarily through droplets of saliva or discharge from the nose when an infected person coughs or sneezes, so it's important to practice respiratory etiquette (for example, by coughing into a flexed elbow). To prevent infection and to slow transmission of COVID-19 and the virus spread, some prevention measurements have been recommended worldwide which include among others people protecting themselves and others from infection by hand washing with soap and water or using an alcohol based rub frequently, avoiding touching the face, covering the mouth and nose when coughing or sneezing, maintaining at least 1 metre distance with people coughing or sneezing, staying home when feeling unwell, refraining from smoking and other activities that weaken the lungs, using face-masks, and practicing physical distancing by avoiding unnecessary travel and staying away from large groups of people. Rapid collection and testing of appropriate specimens from subjects meeting the suspected case definition for COVID-19 is a priority for clinical management and outbreak control and should be guided by a laboratory expert. Suspected cases should be screened for the virus with nucleic acid amplification tests (NAAT), such as RT-PCR.

A "suspected case" is considered a patient or subject with at least one sign and /or symptom characteristic of a particular virus disease, who has travelled to or residence in a location reporting community transmission of the virus disease during some days prior to symptom onset (typically a number of days corresponding to the incubation period of the virus), or has been in contact with a confirmed or probable case of such virus disease, but also a patient or subject who has severe signs/symptoms compatible with the virus disease and requiring hospitalization, and in the absence of an alternative diagnosis that fully explains the clinical presentation. A suspect case for whom testing for the virus disease is inconclusive ("inconclusive" being the result of the test reported by the laboratory) or a suspect case for whom testing could not be performed for any reason can be considered as a "probable case", for which a confirmation of infection with such virus could not be determined. For example, according to the World Health Organization and based on the current available information on COVID-19, a "suspected case" would include: (a) a patient or subject with acute respiratory illness (fever and at least one sign/symptom of respiratory disease, e.g., cough, shortness of breath), and a history of travel to or residence in a location reporting community transmission of COVID-19 disease during the 14 days prior to symptom onset; or (b) a patient or subject with any acute respiratory illness and having been in contact with a confirmed or probable COVID-19 case (see definition of contact below) in the last 14 days prior to symptom onset; or (c) a patient or subject with severe acute respiratory illness (fever and at least one sign/symptom of respiratory disease, e.g., cough, shortness of breath; and requiring hospitalization) and in the absence of an alternative diagnosis that fully explains the clinical presentation. A suspect case for whom testing for the COVID-19 virus is inconclusive ("inconclusive" being the result of the test reported by the laboratory) or a suspect case for whom testing could not be performed for any reason can be considered as a "probable case", for which a confirmation of infection with SARS-CoV-2 or COVID-19 virus could not be determined.

The term "suspect case", including also probable cases, can be also referred to in the present document as "subject suspected of being infected with a/the (particular) virus", or "subject suspected of having an infection with a/the (particular) virus". For example, in case of a suspect case of COVID-19, the suspect case would include a subject suspected of being infected with SARS-CoV-2, and a subject suspected of having an infection with SARS-CoV-2.

A "contact" is a person or a subject who experienced an exposure with a probable or confirmed case of a virus disease during the specific days before and after the onset of symptoms of the probable or confirmed case, which vary depending on the incubation period (i.e. the time between exposure to the virus and symptom onset, also known as the "presymptomatic" period) specific for such virus disease. Generally, the incubation period is expressed as a range of days within a lower and a higher limit in which almost symptomatic individuals will have developed the signs or symptoms of the disease. The incubation period varies depending on the virus type and the strain, and can range of from hours to days, of from a few days to some days or weeks, or from weeks to months or longer. In viruses causing common cold, influenza, dengue, SARS, MERS or COVID-19 typically ranges of between 1 day to 14 days. For example, the incubation period ranges between 1 and 3 days for common cold virus, between 3 and 14 for dengue virus, or between 1 and 14 for some pathogenic human coronaviruses, including SARS-CoV-2 which has an incubation period of between 2 and 14 days, SARS-CoV-1 which has an incubation period of between 1 and 10 days, and MERS-CoV which has an incubation period of between 2 and 14 days. In the above definition of contact, the specific days before the onset of symptoms correspond to the lower limit in days of the incubation period range, while the specific days after the onset of symptoms correspond to the higher limit in days of the incubation period range. For confirmed asymptomatic cases, the period of contact is measured as the number of days corresponding to the lower limit of the incubation period range before through the duration of the incubation period in days after the date on which the sample was taken which led to confirmation.

In the particular case of COVID-19, a "contact" can be defined as a person or a subject who experienced any one of the following exposures during the 2 days before and the 14 days after the onset of symptoms of a probable or confirmed case: (i) face-to-face contact with a probable or confirmed case within 2 meter and for more than 15 minutes; (ii) direct physical contact with a probable or confirmed case; (iii) direct care for a patient or a subject with probable or confirmed COVID-19 disease without using proper personal protective equipment; or (iv) other situations as indicated by local risk assessments. For confirmed asymptomatic cases, the period of contact is measured as the 2 days before through the 14 days after the date on which the sample was taken which led to confirmation. In the present specification, a contact who experienced any one of the above exposures can be also understood as a person who has been exposed to a close contact with a confirmed case, probable case or suspect case of COVID-19 or SARS-CoV-2 infection.

The terms "subject diagnosed with a virus infection", "subject infected by a virus" or "subject confirmed with a virus disease" refers to a person or subject with laboratory confirmation of such virus infection, irrespective of clinical signs and symptoms. For example, in case of virus SARS-CoV-2, the terms "subject diagnosed with an infection with SARS-CoV-2", "subject infected by SARS-CoV-2" or "subject confirmed with COVID-19" would refer to a person or subject with laboratory confirmation of COVID-19 infection, irrespective of clinical signs and symptoms, similarly as for the definition of "confirmed case" established by the WHO. Preferably, the current diagnostic strategy recommended to identify or diagnose patients with COVID-19 or with a SARS-CoV-2 infection is to test samples taken from the respiratory tract of the subject to assess for the presence of one or several nucleic acid targets specific to SARS-CoV-2. However, serum samples in the acute or the convalescent phase could support diagnosis based on validated serologic tests available for SARS-CoV-2, and especially by diagnosing from paired serum samples in the acute and convalescent phase, the initial sample having been collected preferably in the first week of illness, and the second ideally collected 2-4 weeks later. World Health Organization (WHO) has available some information and documents providing interim guidance to laboratories and stakeholders involved in COVID-19 virus laboratory testing of patients, examples of protocols to be used and some in-house and commercial molecular assays available to diagnose and detect the SARS-CoV-2 virus, which can be found and accessed through the website of WHO (for example, on https://www.who.int/emergencies/diseases/novel-coronavirus-2019/technical-guidance/laboratory-guidance) or on web links referred therein (for example, on https://www.finddx.org/covid-19/pipeline/, which collects some of the SARS-CoV-2 tests commercially available or in development for the diagnosis of COVID-19). Regarding the specimens collected for SARS-CoV-2 testing, upper respiratory specimens, such as nasopharyngeal and oropharyngeal swab or wash in ambulatory patients, and/or lower respiratory specimens such as sputum (if produced) and/or endotracheal aspirate or bronchoalveolar lavage in patients with more severe respiratory are the preferred choice. Although additional clinical specimens may be collected as COVID-19 virus has been detected in blood and stool. In surviving patients, serum samples (acute and/or convalescent) can be also useful, and in case of deceased patients autopsy material, including lung tissue. After specimen collection, samples undergo RNA extraction followed by qualitative RT-PCR for target detection. Routine confirmation of cases of SARS-CoV-2 or COVID-19 is based on detection of unique sequences of virus RNA by NAAT such as real-time reverse-transcription polymerase chain reaction (rRT-PCR) with confirmation by nucleic acid sequencing when necessary. The viral genes targeted so far include the N, E, S and RdRP genes. To consider a case as laboratory-confirmed by NAAT, a positive NAAT result of a single discriminatory target can be enough in areas with established SARS-CoV-2 or COVID-19 virus circulation, although in some situations, a positive NAAT result for at least two different targets on the COVID-19 virus genome, of which at least one target is preferably specific for COVID-19 virus using a validated assay; or one positive NAAT result for the presence of betacoronavirus and COVID-19 virus further identified by sequencing partial or whole genome of the virus could be more reliable and convenient, such in case of confirmation in areas with no known COVID-19 virus circulation. One or more negative results by NAAT do not rule out the possibility of SARS-CoV-2 or COVID-19 virus infection. In cases where NAAT assays are negative and there is a strong epidemiological link to SARS-CoV-2 or COVID-19 infection, serum samples in the acute and/or convalescent phase could support diagnosis by serological testing. The terms "subject at risk of an infection with SARS-CoV-2 " or "subject at risk of exposure to SARS-CoV-2" refer to a subject potentially exposed to the SARS-CoV-2 virus or COVID-19, for example, a subject who is in contact with, has been in contact with, cares for, or has cared for a subject/patient diagnosed (i.e. confirmed case) or suspected (i.e. probable case or suspect case) of an infection with virus SARS-CoV-2 or with COVID-19, or a subject/patient having COVID-19, but also a subject who works, has visited or has been in an environment contaminated, suspected of being contaminated, or highly exposed to a contamination with SARS-CoV-2 virus, such as for example a medical centre, a hospital, a laboratory for testing biological samples, or research centres and laboratories for virus research. Subjects at a higher risk of being exposed to SARS-CoV-2 virus, being infected with SARS-CoV-2 virus or developing a SARS-CoV-2 infection or COVID-19, include healthcare workers (including practitioners, nurses, emergency services, assistants and non-clinical staff), pharmacists and subjects or people working at healthcare facilities; subjects or people living in the same household (for example, relatives, flatmates, roommates, domestic workers and caregivers) as a person who has lab-confirmed COVID-19 or who was diagnosed with COVID-19; subjects or people providing care in a household (for example, caregivers) for a person who has lab-confirmed COVID-19 or who was diagnosed with COVID-19; subjects or people being within 2 meters (or 6 feet) of a person who has lab-confirmed COVID-19 or who was diagnosed with COVID-19 for at least 15 minutes; subjects or people in direct contact with secretions from a person who has lab-confirmed COVID-19 or who was diagnosed with COVID-19 (e.g., being coughed or sneezed on, kissing, sharing utensils, etc.).

The term "therapeutically effective amount" is the amount present in the composition that is needed to provide the desired level of drug in the subject to be treated to give the anticipated physiological response.

The term "physiologically effective amount" is that amount delivered to a subject to give the desired palliative or curative effect.

The term "pharmaceutically acceptable carrier" means that the carrier can be taken into the tissues or organs susceptible of being affected under a virus infection or disease with no significant adverse toxicological effects on such tissues or organs. For instance, the carrier can be taken into the lungs, the upper or lower airways, the gastrointestinal tract, the central nervous system, the liver, the skin, or the eye with no significant adverse toxicological effects on the lungs, the upper or lower airways, the gastrointestinal tract, the central nervous system, the liver, the skin, or the eye, respectively.

The term "co-administration" refers to administering to a subject two or more agents (e.g. a siRNA compound of the invention with another active principle), and in particular two or more siRNA compounds. The agents can be contained in a single pharmaceutical composition and be administered at the same time, or the agents can be contained in separate formulations and administered serially to a subject. So long as the two agents can be detected in the subject at the same time, the two agents are said to be co-administered.

The types of pharmaceutical excipients that are useful as carrier include stabilizers such as human serum albumin (HSA), bulking agents such as carbohydrates, amino acids and polypeptides; pH adjusters or buffers; salts such as sodium chloride; and the like. These carriers may be in a crystalline or amorphous form or may be a mixture of the two.

Bulking agents that are particularly valuable include compatible carbohydrates, polypeptides, amino acids or combinations thereof. Suitable carbohydrates include monosaccharides such as galactose, D-mannose, sorbose, and the like; disaccharides, such as lactose, trehalose, and the like; cyclodextrins, such as 2-hydroxypropyl-beta-cyclodextrin; and polysaccharides, such as raffinose, maltodextrins, dextrans, and the like; alditols, such as mannitol, xylitol, and the like. A preferred group of carbohydrates includes lactose, threhalose, raffinose, maltodextrins, and mannitol. Suitable polypeptides include aspartame. Amino acids include alanine and glycine, with glycine being preferred.

Suitable pH adjusters or buffers include organic salts prepared from organic acids and bases, such as sodium citrate, sodium ascorbate, and the like; sodium citrate is preferred.

### Kits

In certain other aspects, the invention provides kits that include a suitable container containing a pharmaceutical formulation of an siRNA compound. In certain embodiments the individual components of the pharmaceutical formulation may be provided in one container. Alternatively, it may be desirable to provide the components of the pharmaceutical formulation separately in two or more containers, e.g., one container for an siRNA compound preparation, and at least another for a carrier compound. The kit may be packaged in a number of different configurations such as one or more containers in a single box. The different components can be combined, e.g., according to instructions provided with the kit. The components can be combined according to a method described herein, e.g., to prepare and administer a pharmaceutical composition. The kit can also include a delivery device.

### Examples

### Obtaining siRNA duplexes

RNAs are preferably chemically synthesized using appropriately protected ribonucleoside phosphoramidites and a conventional DNA/RNA synthesizer. Substitution of one or both strands of a siRNA duplex by 2'-deoxy or 2'-O-methyl oligoribonucleotides abolished silencing in fly extract (Elbashir *et al.* 2001). In mammalian cells, however, it seems possible to substitute the sense siRNA by a 2'-O-methyl oligoribonucleotide (Ge *et al.* 2003).

Most conveniently, siRNAs were obtained from commercial RNA oligo synthesis suppliers, which sell RNA-synthesis products of different quality; for example, intended for target validation. In general, 19, 20 and 21-nt RNAs are not too difficult to synthesize and are readily provided in a quality suitable for RNAi. A number of commercial custom RNA synthesis companies exist; in the present examples, siRNA may be obtained from Biospring (Germany), unless otherwise specified.

### In vitro and animal studies.

In order to evaluate the antiviral potential of the siRNAs identified against an EEF1A gene (EEF1A2 and/or EEF1A1) or any of their combinations with any other siRNA against a different target gene or a small molecule inhibitor as disclosed in the present invention, both *in vitro* and *in vivo* experiments in functional models that reproduce the mechanism of infection of the SARS-CoV-2 virus or of an influenza A virus are carried out. For *in vivo* experiments, siRNAs targeting EEF1A2 and/or EEF1A1 are preferably delivered as a part of a bioconjugate and/or delivered formulated alone/or in conjugation in nanoparticles (NPs) directed to the lung.

For the development of the polymeric nanoparticles, we will use a poly-(β-aminoester) (pBAE) polymer already used in previous studies (Dosta et al. 2018). Based on these polymers, new ones will be generated. New polymers will be synthesized and, optionally will include an antiviral molecule, such as hydroxychloroquine and/or plitidepsin, or an anti-inflammatory molecule, such as a corticosteroid or interferon y (IFN-y). These polymeric particles can also be coated with hyaluronic acid that would guarantee their adhesion in the lung and they have a hydrophobic nucleus that could house other drugs of interest. The newly synthesized polymers will be analyzed through 1H-NMR, IR and HPLC. Said polymers may be vectorized with ligands and/or small peptides and/or molecules directed to surface proteins of lung epithelial cells and alveolar macrophages such as antibodies or aptamers (e.g. anti-Aquaporin 5 (Aqp-5), anti-Caveolins (Cav-1, -2, and -3), anti-CD44v6, anti-CD208, anti-RAGE, anti-Surfactant protein A, anti-Tomsen-Friedenreich marker, anti-TTD1, anti-KL6 and/or anti-CD11b, anti-CD206, anti-CD169, anti-CD44v6, anti-CD208, anti-Cx43. Nanoparticles (NPs) will be synthesized by electrostatic interaction between positive charges of polymer with negative charges of RNA. Different polymer/RNA ratios will be used to obtain the NPs with the characteristics that ensure successful delivery in the target tissues (for example, size less than 250 nm, slightly positive charge and stability to lyophilization). The NPs will be characterized by size and surface charge (by dynamic light scattering), both in the formulation medium and in contact with blood serum to determine their stability under physiological conditions. Also, NPs will be lyophilized and re-characterized to see their stability.

### 1. In vitro experiments

### 1.1. In vitro validation of the efficacy for the different siRNA candidates of the invention

To validate the efficacy *in vitro* of a siRNA targeting EEF1A2 and/or EEF1A1 mRNA, culture cells are transfected with the selected EEF1A2 siRNA (i.e. a siRNA which targets any of the nucleotide sequences defined by SEQ ID NO: 1-8) and the silencing capacity of siRNAs in cells that express good levels of EEF1A2 and/or EEF1A1, but also susceptible to SARS-CoV-2 infection, are evaluated. Transfections are performed with different transfection agents at different times (for example, at 6, 24, 48, 72, 96 and 120 hours) and testing different doses of the siRNA compound (for example, 50 and 100 nM). Different doses of siRNA will be administered for the calculation of IC50 values (dose at which 50% of the messenger RNA disappears at a certain time-point). Cells from different species are used in these experiments which include human cells, and at least one or several cell types from different animal species necessary for further development of the selected compounds, which include murine, macaque and/or dog cells. For the *in vitro* validations of siRNAs, primary lung cells and established lung cell lines are used. Among the cell lines, preferably human cells from the upper (nasopharynx, Detroit 562 ATCC^{®} CCL-138^{™}) and lower respiratory tract (lung, A549 (ATCC^{®} CCL-185^{™}) and/or BEAS-2B (ATCC^{®} CRL-9609^{™}), human broncho-epithelial primary cells (HBEpC), human Primary Bronchial/Tracheal human cells and human Primary Lobar Epithelial Cells are used. Likewise, siRNAs are also validated in mouse cells from the higher respiratory tract, such as Mouse Primary Tracheal Epithelial Cells, and from the lower respiratory tract, such as IMLg [Mlg 2908] cells and/or MLE 12 (ATCC^{®} CRL-2110). siRNAs are also validated in Rhesus macaque cells such as 4MBr-5 (Lung) (ATCC^{®} CCL-208^{™}) and/or RF/6A (Retina) ATCC^{®} CRL-1780, LLC-MK2 and/or FRhK-4 (Kidney) cells (ATCC^{®} CRL-1688^{™}) and in Crab-eating Macaque cells such as (CYNOM-K1) (ECACC, 90071809). Finally, siRNAs are also validated in CF41.Mg (ATCC^{®} CRL-6232^{™}) and/or MDCK dog cells (ATCC ^{®} CCL 34^{™}). IC50 values are calculated for those siRNA candidates having a better performance in the different selected cell models.

### 1.2. Gene expression levels and protein levels of eEF1A after transfection of siRNAs targeting EEF1A2

To determine the efficacy and silencing effect of the siRNAs of the invention on the expression of EEF1A2 and/or EEF1A1, studies on the changes in mRNA expression levels of these genes and on their protein levels are carried out.

Different cell line models were previously selected based on their basal EEF1A1 and EEF1A2 expression levels and based on their cellular and tissue origin (respiratory tract), as well as on their suitability as in-vitro models for infection studies of SARS-CoV-2 or influenza A virus. The selected cell lines expressed sufficient amount of the target gene to allow detection of silencing effects mediated by the candidates and or allowed for successful replication and isolation of the SARS-CoV-2 or influenza A virus. Finally, A549 (human lung cells) [ATCC, # CCL-185^{™}] VeroE6 cells (Kidney epithelial cells of an African green monkey) [ATCC, # CCL-185] and in C2C12 (muscle mouse cells) [C2C12, # CRL-1772^{™}] and ^{™}], HuH-7 (hepatocyte-derived cellular carcinoma cells) were selected as in vitro cell models. Cell cultures were transfected with 100 nM of each compound, 24 hours after transfection cell medium was changed and samples collected and analyzed for gene expression. Sample collection was performed immediately after medium change and 48 and 72 hours after transfection of siRNAs. The studies were carried out with different transfection agents and those were chosen that allowed a clearer evaluation of the potency of each sequence. A549 cells were successfully transfected with Lipofectamine 2000, VeroE6 cells with siPORT, C2C12 with Mirus Transit-X2 and HuH-7 cells with Lipofectamine 2000. Total RNA was collected and extracted for all experimental conditions at different time points (6, 24, 48, 72, 96 and 120 hours). After transfection gene expression levels of EEF1A2 and EEF1A1 were determined by qPCR and protein levels were also determined and quantified by western blot analysis. In parallel, cell viability studies were carried out using MTS assays.

### 1.2.1. Gene expression levels of EEF1A2 and EEF1A1 after transfection of siRNAs targeting EEF1A2

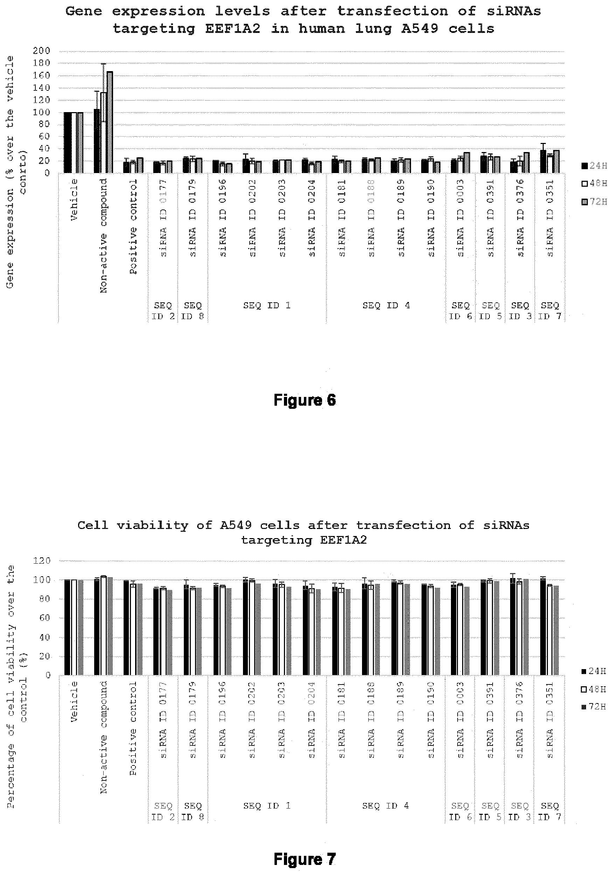

In total, we have tested fourteen candidate siRNAs acting on eight different regions of the EEF1A2 messenger RNA, which are identified by their corresponding siRNA ID number. We also transfected a positive control, and non-active siRNA. In addition to studying the levels of EEF1A2, we also studied the EEF1A1 levels in different transfected cells with the siRNAs targeting EEF1A2. As shown in Figure 6, all siRNAs directed against EEF1A2 produced a very potent reduction of mRNA levels (80%) in the A549 lung cells. This reduction of mRNA was sustained over time, thus, 72 hours after the transfection the basal levels of expression were not recovered. The non-active compound, however, did not produce any reduction in EEF1A2 gene expression levels. This marked reduction did not translate into changes in viability at any of the times studied (Figure 7).

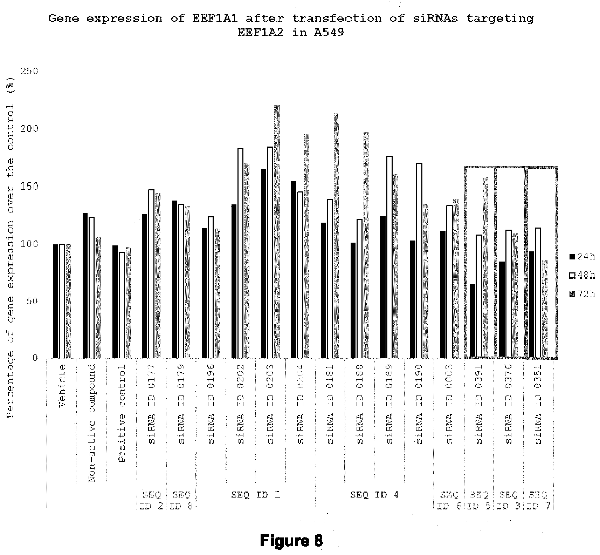

In A549 cells transfected with siRNAs targeting EEF1A2, we also studied the expression levels of the EEF1A1 paralogue (Figure 8). All cells that were transfected with siRNAs directed to EEF1A2 suffered an over-expression to a greater or lesser extent 24, 48 and 72 hours after the transfection. This was especially marked for siRNAs corresponding to SEQ ID 1 and 4. Of special interest were observed for siRNAs ID 0391, ID 0376 which reduced EEF1A1 expression levels, 50% and 20% respectively, 24 hours after transfection and ID 0351 which reduced EEF1A1 expression levels by 20-30% 24h. That is of particular interest if we consider that the region of the EEF1A2 mRNA on which these candidates are acting preserves a high degree of homology with the EEF1A1 messenger, presenting five and four mismatches respectively. These candidates could have a dual-action so that they could act on the two messengers at the same time.

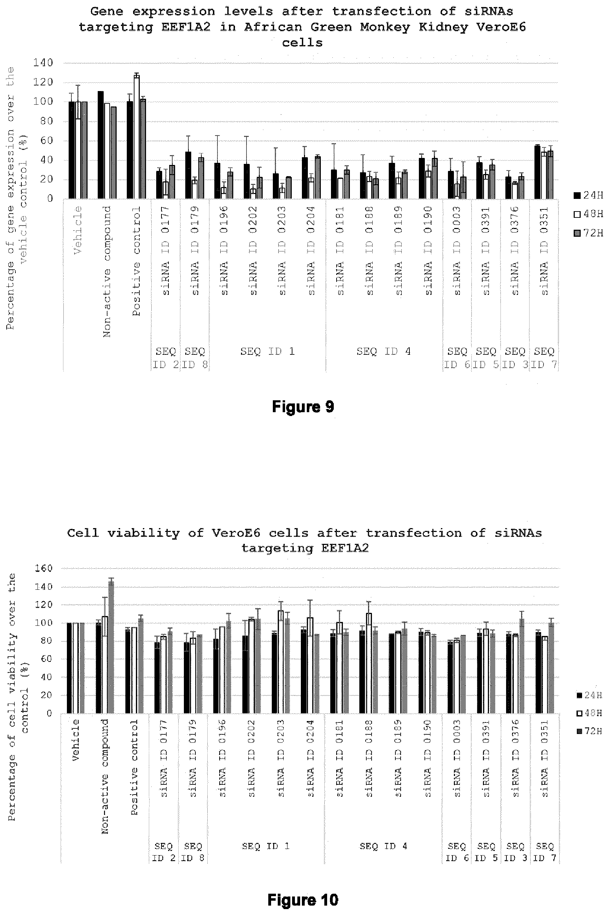

We also studied the levels of expression of EEF1A2 in VeroE6 cells transfected with the different candidates (Figure 9). Similar results were found as with the A549 cells. Again, all siRNAs targeting EEF1A2 effectively reduced EEF1A2 expression levels, except for the positive control which produced no changes from the control. All siRNAs reduced basal levels by 60 - 80% at all three time points studied. Most of the sequences studied achieved maximum values of reduction 48 hours after transfection and started a slight recovery 72 hours after transfection. This was observed for all sequences except for siRNA ID 0188, siRNA ID 0376 and siRNA ID 0351 which achieved sustained decreases over time.

In VeroE6 cells we also found no changes in cell viability, except for siRNAs ID 0177, siRNA ID 0179, siRNA ID 0189, siRNA ID 0190 siRNA ID 0003 and siRNA ID 0376 which produced a 10-20% drop at all three times studied (Figure 10).

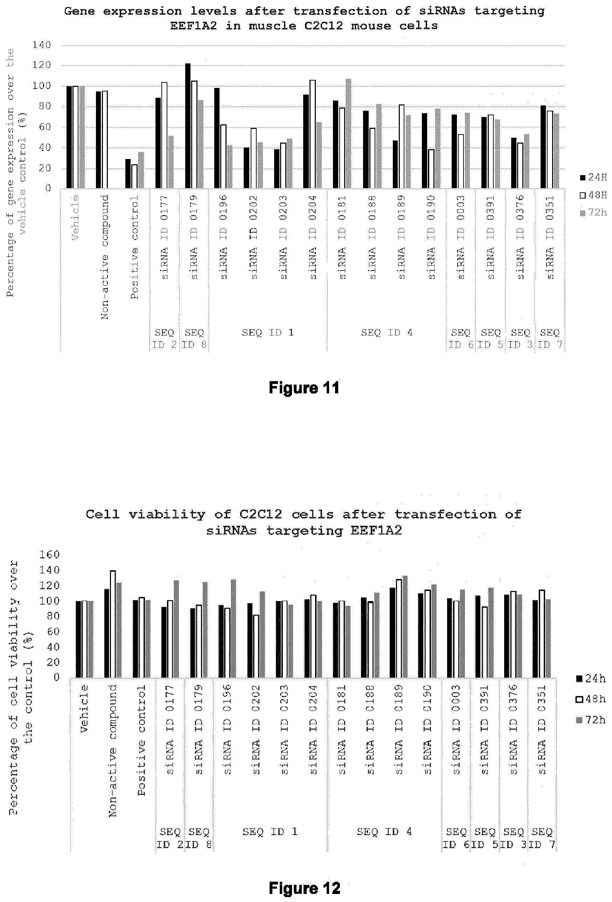

Unlike the previously described cell lines, the effectiveness of siRNAs was lower in C2C12 cells (Figure 11). The siRNAs ID 0177, ID 0179, ID 0204 did not produce an effective reduction of expression levels 24 and 48 hours after the transfection, however 72 hours after the transfection reduced expression levels by 20-60%. Other candidates such as siRNA ID 0190 and siRNA ID 0003 produced their maximum drop point 48 hours after transfection, 60% and 50% respectively. The most effective siRNAs were siRNA ID 0196, siRNA ID 0202 and siRNA ID 0203 with maximum reductions of 60% over the basal levels. While siRNA ID 0196 reduced levels gradually, obtaining the maximum drop 72 hours after transfection, siRNA ID 0202 and siRNA ID 0203 did so in a sustained manner at the three time points considered in the study. The positive control reduced expression levels by 70% over the basal levels. None of the candidates produced changes in cell viability except siRNA ID 0177, siRNA ID 0179 and siRNA ID 0196 which increased the number of viable cells by 40% 72 hours after transfection (Figure 12).

Taken together, these results indicate that the siRNAs were able to reduce the mRNA levels of EEF1A2 with little or no change in the cell viability in cells from different species, most of them showing percentages of reduction similar to, or even better than, those obtained with the positive control siRNA designed to specifically target and silence the EEF1A2 gene of each particular cell species. Remarkably, siRNAs targeting SEQ ID NO. 1 seem to have a better performance in different cell species, while siRNAs targeting SEQ ID NO. 3 or SEQ ID NO. 5 could also reduce, at least in part, the mRNA levels of the paralog gene EEF1A1.

Based on the results, everything points to siRNA ID 0202, siRNA 0203 and siRNA 0204 (corresponding to the region SEQ ID NO. 1) and siRNA ID 0376 from target sequence SEQ ID NO. 3 as candidates for further development.

### 1.2.2. Gene expression and protein levels of eEF1A2 after transfection with selected siRNA candidates Once the selected candidates are chosen, infection studies in different cellular models of infection are carried out to determine the duration of the silencing effect on the mRNA expression and on the protein levels expressed in the cell.

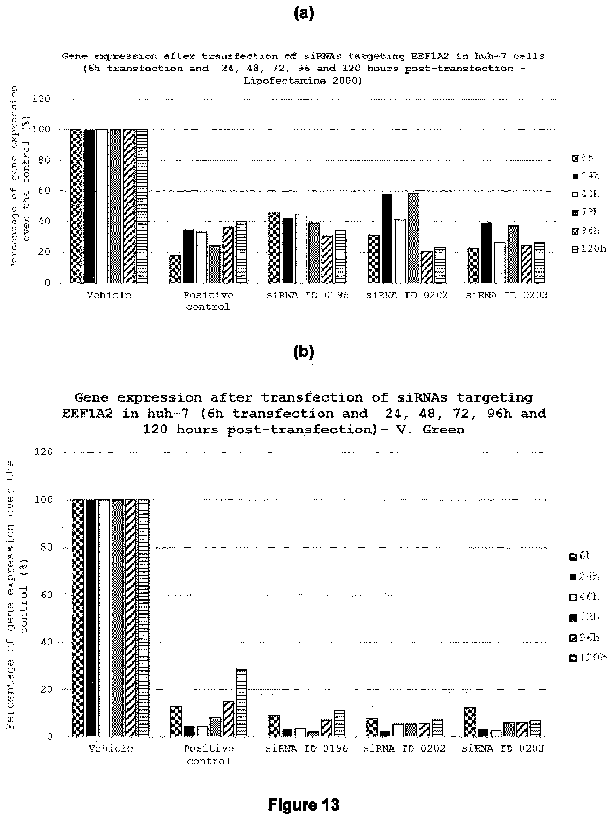

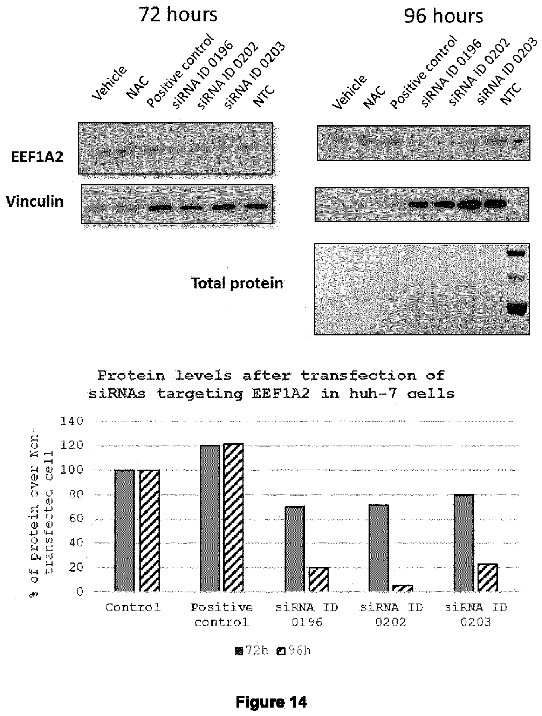

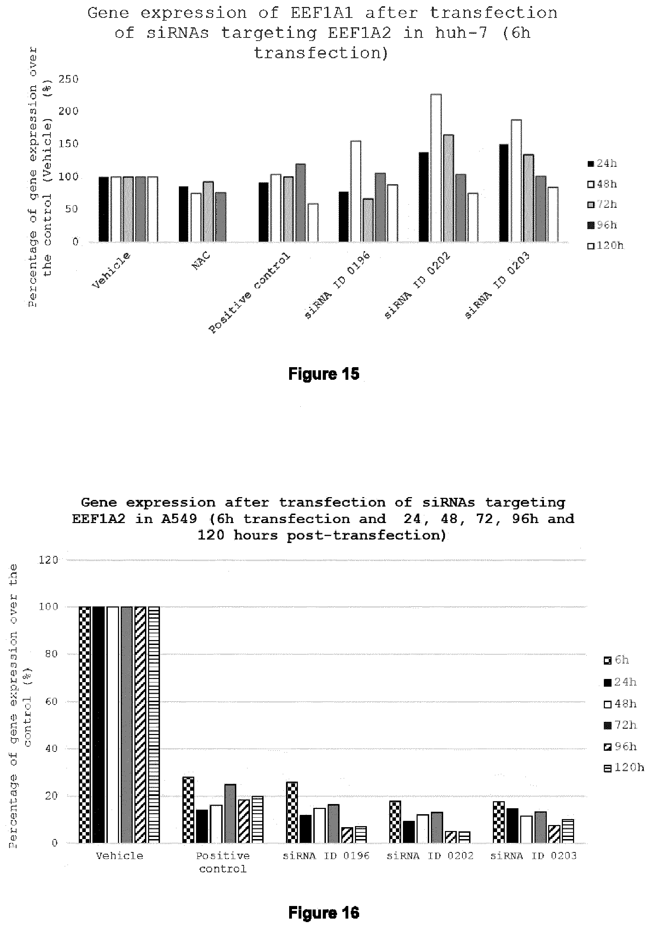

We will perform infection studies in different cellular models of infection. We will use HuH-7 and/or VeroE6 for SARS-CoV-2 studies, and A549, Primary human bronchial/tracheal epithelial and/or U937 for influenza and for other respiratory virus. We performed a validation of the selected candidates at short (6 hours) and long-time points (24, 48, 72, 96 and 120 hours) in some of these models to study the maintained effect of the RNA interference and protein levels for the different selected candidates. We conducted a previous validation for candidates corresponding to the SEQ ID NO. 1 target sequence in both human HuH-7 (Figure 13) and A549 cells (Figure 16). For the temporal expression studies we transfected 100 nM from each candidate for 6 hours and collected the total RNA to study the expression levels of EEF1A2 6, 24, 48, 72 and 96 hours after the transfection. HuH-7 cells were transfected with Lipofectamine 2000 and Viromer green, while A549 cells were transfected with Lipofectamine 2000. As shown in Figure 13, 6 hours after transfection, siRNAs ID 0196, ID 0202 and ID 0203 effectively reduced EEF1A2 levels, achieving decreases of 60-80 % over basal levels that were sustained over time in HuH-7 cells when transfected with Lipofectamine (Figure 13a) and of 80-96 % when transfected with Viromer Green (Figure 13b). None of the candidate siRNAs recovered basal levels five days after transfection. The positive control showed similar behaviour, but in its case, we observed a gradual recovery of the basal levels of expression over time. siRNA ID 0196, siRNA ID 0202 and siRNA ID 0203 maintained decreases by 80% five days after transfection, while the positive control maintained the reduction of 60% over basal levels. siRNA ID 0196 reduced EEF1A2 levels by 60-70%, siRNA ID by 70-80% and siRNA ID 0203 by 80%. When we used Viromer Green as a transfection agent, the three candidates obtained maximum efficiency values of 96% in HuH-7 cells. In this case, while the positive control suffered a gradual recovery of mRNA levels, the siRNAs ID 0196, ID 0202 and ID 0203 maintained the reduction of basal levels by 90% five days after transfection. We also studied the levels of ef1A2 protein to study the dynamics of protein disappearance related to mRNA in Lipofectamine 2000 HuH-7 transfected cells. We transfected HuH-7 cells with 100 nM from each of the candidates for 6 hours. 6 hours after transfection we collected the samples to extract the RNA at different post-transfection times (6, 24, 48, 72, 96 and 120 hours) and for protein analysis (48, 72, 96 and 120 hours) in order to determine the RNA-protein dynamics for ef1A2. The maximum reduction of the protein (80%) was 96 hours after transfection (Figure 14). Everything seems to suggest that the protein is highly stable, and at least five days of interference on the messenger RNA with interference values higher than 60% are needed to achieve a sharp reduction in the protein. We evaluated the protein levels 72 hours and 96 hours post-transfection, as these are the times in which we would predictably obtain the maximum infection values in subsequent studies of viral infection with the HCoV-229E and SARS-CoV-2 coronavirus with infection multiplicity values (MOI) between 0.01- 0.001.

Besides studying the expression levels of EEF1A2 in the transfected cells with the different candidates, we studied those of its EEF1A1 paralogue. As figure 15 shows the positive control produced no appreciable changes in the basal levels of EEF1A1 except 120 hours after transfection (50%). The siRNA ID 0196 produced an over-expression of EEF1A1 (50% above basal levels) 24 hours after transfection and 48 hours after transfection a repression around 40% that gradually recovered over time. On the other hand, siRNA ID 0202 and siRNA ID 0203 increased EEF1A1 levels 24 hours after transfection by 180-200% above basal levels and then gradually reduced levels over time, obtaining a maximum reduction of 40-50% of basal levels when compared to the control, 120 hours after transfection.

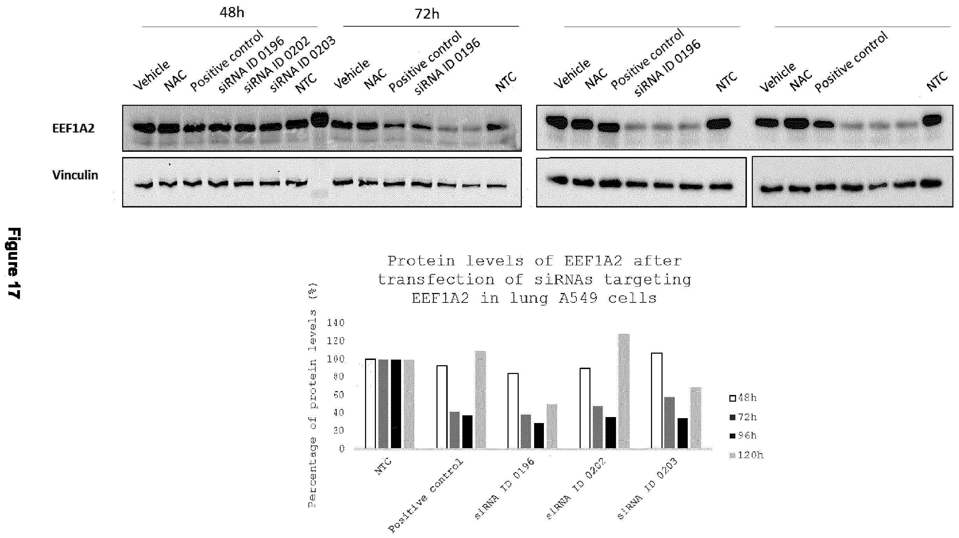

Similarly, we evaluate the efficacy of the selected candidates at short and long times in lung cells A549. Again, we found that the three selected siRNA ID 0196, siRNA ID 0202 and siRNA ID 0203 produced sharp reductions between 80-96% over basal levels. The maximum reductions (by 96-98%) were obtained 96 and 120 hours after transfection. There was no recovery of expression levels at any of the times considered in the study (Figure 16). As for the HuH-7 cells, we also studied the levels of protein eF1A2 in human A549 lung cells, to study the dynamics of the protein. We transfected the A549 cells with 100 nM from each of the candidates for 6 hours. 6 hours after transfection we collected the samples to extract the RNA at different post-transfection times (6, 24, 48, 72, 96 and 120 hours) and for protein analysis (48, 72, 96 and 120 hours) in order to determine the RNA-protein dynamics for eEF1A2. As happened for the HuH-7 cells the maximum reduction of the ef1A2 protein (70%) was 96 hours after transfection (Figure 17). Everything seems to indicate that the protein is highly stable, and at least four days of interference on the mRNA with interference values higher than 60% are needed to achieve a sharp reduction in the protein 96 hours after transfection. The maximum reduction described for the protein is observed in all candidates similarly, however, while the positive control and ID 0202 siRNA fully recover eEF1A2 protein levels, the siRNA ID 0196 and siRNA ID 0202 siRNAs retained a 50% and 30% drop respectively over basal levels.

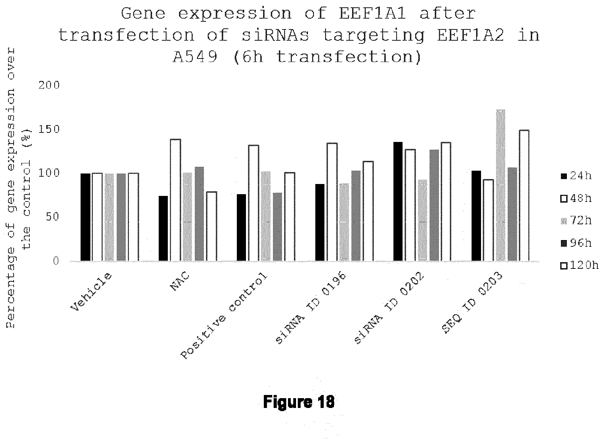

Again, as for the HuH-7 cells we also studied the levels of EEF1A1 after the transfection for 6 hours of siRNAs targeting EEF1A2. Again, as for the HuH-7 cells, we also studied the levels of EEF1A1 after the transfection of siRNAs targeting EEF1A2 for 6 hours in A549 cells. As figure 18 shows, the positive control and siRNA ID 0196 siRNA produced an over-expression of EEF1A1, 50% over basal levels 24 hours after transfection, and 48 hours after transfection the basal levels completely recovered. siRNA ID 0202, similarly, showed an over-expression of 50% above basal levels which was maintained 48 hours after transfection, but first drastically dropped 72 hours after transfection until basal levels were recovered and the overexpressed again, both 96 and 120 hours. The same behaviour was found for siRNA ID 0203 but time shifted. 72 hours after the transfection EEF1A1 levels increased by 80% above basal levels and 96 hours after the transfection fell dramatically until basal levels recovered. 120 hours after the transfection, EEF1A1 levels were again 50% above basal level.

These data show that the siRNA of the present invention, and in particular those targeting SEQ ID NO:1, have a long lasting silencing effect, reducing the expression levels of EEF1A2 gene by a 90% or more at 96 h and even 120 h post-transfection, and that these siRNAs can reduce the eEF1A2 protein levels in different cell types.

### 1.3. In vitro studies of virus infection. Prophylactic and therapeutic antiviral capacity of siRNA compounds.

As the first model for the infection studies, we will use the coronavirus HCoV-229E. Therefore, we will characterize the expression levels of the two targets EEF1A1 and EEF1A2 in HuH-7 cells infected by HCoV-229E with different multiplicities of infection (MOI: 0.001 and 0.01). Once the infection is completed, the RNA will be collected 48 and 72 hours after the infection (post-infection, p.i.). Changes in the expression levels of EEF1A2 and EEF1A1 and other viral genes related to viral pathogenesis, virus -host cell surface receptor interactions, production of viral nucleic acids and proteins within the cell, and host cell viability and metabolism will be evaluated at 48 hours and 72 hours after infection with these two multiplicities of infection, to see if the virus infection reduces or increases the expression of such genes, and in case we will evaluate the levels of expression of EEF1A2 and/or EEF1A1 at longer post-infection times to see if the recovery of mRNA levels occurs or if it continues to reduce or to increase its levels over time. After evaluating the best infection conditions and the target gene expression profile, we will perform the transfection of the different candidates at a concentration of 100 nM and at different transfection times of 6 and 24 hours. Once the transfection has been carried out, we will proceed with the infection with the virus at the selected MOI (0.001 or 0.01) 24, 48 or 72 hours after the transfection to evaluate the antiviral capacity of the different candidates. After the different times of infection, the RNA and/or the protein levels of EF1A1 and EF1A2, as well as other viral genes involved in the infection processes will be quantified and the viral load titrated.

Similarly, as a second model for the infection studies, we will use an influenza A virus (PR8, X31 and/or Victoria strains). The first tests will use the influenza Strain A/PR8/1934. Therefore, we will characterize the expression levels of the two targets EEF1A1 and EEF1A2 in A549 cells infected by with different multiplicities of infection (MOI: 0.001 and 0.01). Once the infection is completed, the RNA will be collected 8, 24, 48 and 72 hours after the infection (p.i.) and changes in EEF1A1 and EEF1A2, and other viral genes related to the progress of infection will be evaluated with these two MOI, to see if the virus infection reduces or increases the expression of such genes, and in case we will evaluate the levels of expression of EEF1A2 and/or EEF1A1 at longer post-infection times to see if the recovery of mRNA levels occurs or if it continues to reduce or to increase its levels over time. Once the transfection has been carried out, we will proceed with the infection with the virus at the selected MOI (0.001 or 0.01) 24, 48 or 72 hours after the transfection to evaluate the antiviral capacity of the different candidates. After the different times of infection, the RNA and/or the protein levels of eEF1A1 and eEF1A2, as well as other viral genes involved in the infection processes will be quantified and the viral load titrated.

### 1.4. In vitro studies of virus infection. Prophylactic and therapeutic antiviral capacity of siRNA compounds.

### 1.4.1. In vitro EEF1A2 siRNA silencing effects on the viral replication of SARS-CoV-2 and Influenza A virus and on the production of pro-inflammatory cytokines of infected cells

Infection studies with SARS-CoV-2 or an Influenza A virus are carried out using *in vitro* antiviral testing by classical virological methods (cytopathic effect (CPE) assays, viral titer reduction assays, plaque reduction assays according to the protocol described by Herzog et al 2008 and/or Xia et al 2020, through CKK-8 and/or Luminiscent and/or MTS cytotoxic assays) optimized to be performed in African green monkey kidney VeroE6 cells (ATCC-1586), and/or human Caco2 and/or Huh7 and/or A549 cells.

Initially, we will titrate the response of the assay to the host cell to determine the linear response range in ATP production by testing different cell densities calculated based on the doubling rate of each cell line used. Once the cell density of the response in the host cell and the linear response range of the assay has been determined, we establish for each line the response time/contact with the virus for each cell line to evaluate the optimal time that allows obtaining an optimal cytopathic effect that allows evaluating each assay in a suitable dynamic range. Once infection models are optimized, the relative titers of infectious viruses can be determined. For this step, SARS-CoV-2 virus or influenza A virus is serially diluted in half-log increments and applied to their respective host cells. A virus-free control can be used, in which the medium only is applied to the host cell. Depending on the kinetics of the CPE (determined during the development of the model), the viral titers are incubated for 72-144 hours. The detection reagents (ATP and/or MTS) are prepared and applied in equal volumes to the assay plates. Luminescence or formazan are measured and correlated with viral dilutions. Dilutions containing infectious virus demonstrate low cell viability and, therefore, low luminescence values due to generalized CPE. In contrast, dilutions that do not contain or limit virus show higher luminescence values. TCID50 values are then obtained for each virus by determining the virus dilution that reduced the untreated signal by 50%.

Optionally, infection studies are also carried out similarly in at least any other cell line which is successfully used to grow SARS-CoV-2, such as African green monkey kidney VeroE6/TMPRSS2 cells, rhesus monkey kidney LLC-MK2 cells, human Calu-3 cells, and/or human HEK293T cells; and/or to grow Influenza A virus such as MDCK (Madin-Darby canine kidney) cells, HEp-2 (human laryngeal carcinoma), HEK293T, and/or HEL (human embryonic lung); and/or to grow any of these viruses, such as Vero (embryonic African green monkey kidney) cells.

A preliminary study is performed in VeroE6 cells to determine the multiplicity of infection (MOI) ratio and the replication kinetics of SARS-CoV-2 virus, and in A549 cells for Influenza A virus Puerto Rico/8/1934 and/or A/X-31 (H3N2) and/or for Influenza B /Victoria/2/87. Then, other respiratory system cells susceptible to virus infection are also used with the selected MOI ratio and are infected to study the (prophylactic and therapeutic) antiviral capacity of siRNAs targeting EEF1A2. Different respiratory system cells are transfected with the EEF1A2 siRNA candidates and then infected with SARS-CoV-2 or influenza A virus for studying viral replication in airway epithelium in vitro and to study the antiviral activity of the selected siRNAs. Different cell line models are used for this purpose: human Tracheal/bronchial epithelial cultures (ATCC^{®} PCS-300-010 ^{™}), human Primary Bronchial/Tracheal human cells and Primary Lobar Epithelial Cells, and lung A549 cells. Antiviral studies are also performed in murine MLg [Mlg 2908] cells and/or MLE 12 lung cells and Rhesus macaque lung 4MBr-5 cells. SARS-CoV-2 or Influenza A viral replication kinetics and in vitro CPE are fully characterized. The antiviral SARS-CoV-2 activity of siRNAs targeting EEF1A2 (alone or in a combination of other drugs) are investigated in vitro through two different approaches: a prophylactic study (inhibition of EEF1A2 and/or EEF1A1, and SARS-CoV-2 infection) and a therapeutic study (Infection with SARS-Cov-2-infection and inhibition with siRNAs candidates targeting EEF1A2). The supernatant of cultured cells is removed and the RNA extracted and analysed by relative quantification using RT-qPCR to determine the amount of virus RNA in controls and treatments by the study of viral structural genes to assess the (%) of inhibition. EC50 values are obtained from dose-response curves of the selected EEF1A2 siRNAs against SARS-CoV-2 or Influenzavirus type A for viral RNA and for target mRNA (EEF1A2). EC50 values are determined at the MOIs selected previously by quantification of viral RNA copy numbers in the cell supernatant 24 or 48 h after the viral infection. The pro-inflammatory cytokines related to the cytokine storm associated with SARS-CoV-2 or Influenza A virus infection are also evaluated. Levels of interleukin (IL)-1β, TNF-α, IL-6, IL-18 and interferon-γ, are determined both at the mRNA level and at the protein level by detecting them in solution via ELISA assays.

Viral RNA is extracted from the supernatant of infected cells using the viral RNA mini kit (QIAGEN, Hilden, Germany) and RNAEasy Kit (QIAGEN, Hilden, Germany and automated nucleic) with the nucleic acid extraction system QIACUBE (QIAGEN, Hilden, Germany) and following the manufacturer's instructions. Detection of the SARS-CoV-2 virus, the target blocked by EEF1A2 siRNA candidates and pro-inflammatory biomarkers are performed using the Real-time PCR on the StepOne Plus and Quant Studio 12K Flex real-time PCR systems (Applied Biosystem, USA). Real-time PCR analysis are performed with universal primers and probes targeting SARS-CoV-2 that amplify structural viral proteins encoded by RdRp, E and N genes, or in case of influenza A virus, with primers and probes that amplify the Polymerase acidic protein (PA) and /or the PB1, PB2 polymerase subunits. Pro-inflammatory cytokines are determined by ELISA in a Microplate reader. All experiments are conducted in triplicates. The relative expression can be determined using the 2-ΔΔCt method. The dose-response effect is determined by a sigmoidal concentration-response function, Y= Bottom + (Top-Bottom)/ (1+10 ^ ((Log EC50-X) *Hill Slope)), fitting to the data using non-linear regression.

### 1.4.2. In vitro EEF1A2 siRNA silencing effect on the expression and production of cytokines and chemokines of immune cells.

The levels of mRNA and/or protein ef1a2 and/or ef1 a1 using QPCR and Western Blot, respectively, and/or the levels of pro-inflammatory cytokines and interleukins IL6, IL7, TNF, CCL2, CCL3, CXCL10 alpha chain of IL2R, IL1 beta and IL18, using QPCR and ELISA will be evaluated in lymphocytes kt-3 Cells (94070705, ECACC), T lymphocytes (Primary CD8 + Cytotoxic T Cells (ATCC^{®} PCS-800-017 ^{™}), Primary CD4 + Helper T Cells (ATCC^{®} PCS-800-016 ^{™}), B lymphocytes (Primary CD19 + B Cells (ATCC^{®} PCS-800-018 ^{™}), HAA1 (ATCC^{®} HB-8534 ^{™}), and Natural Killer (NK) NK92 (NK-92 ^{®} ATCC ^{®} CRL-2407 ^{™}), Primary CD56 + NK Cells (ATCC^{®} PCS-800-019 ^{™}), after transfection of the different siRNAs candidates of the present invention to an specific siRNA concentration, for example, 100 nM).

### 1.5. In vivo antiviral capacity of EEF1A2 siRNAs for preventing the spread of infection of SARS-CoV-2

The siRNA compound of the invention bioconjugated or formulated in nanoparticles can be also evaluated in vivo to study their antiviral capacity against SARS-CoV-2 and to prevent the spread of infection in the host. The efficacy can be studied for the most effective candidate obtained from the in vitro studies alone/or in combination with other drugs in an animal model that reproduces the signs and symptoms of SARS-CoV-2 infection (COVID-19).

For the development of these experiments, two different approaches will be taken into account. The first approach is based on two different mouse models: a mouse model that expresses the human ACE2 receptor (K18-hACE2) and/or the MERS-CoV mouse model. The second approach is based on a Rhesus macaque model of SARS-CoV-2 infection.

In the MERS-CoV mouse model, mice are infected with a mouse-adapted strain of the beta-coronavirus (e.g., BtCoV HKU5-SE), which replicates efficiently in mice and induces alveolar damage. Humanized K18-hACE2 mice are infected with SARS-CoV-2 virus. Humanized ACE2 mice are intranasally infected with SARS-CoV-2 10⁵ (TCID50) pfu in PBS. 24 h previous to infection or after infection, a group of animals are treated with 3 different doses of the treatments.

On their side, macaques are infected with SARS-CoV-2 intranasally with a dosage of 10⁵ times the half-maximal culture infectious dose (TCID50) in 1 ml PBS solution through intranasal instillation. We also administer the siRNA candidate targeting EEF1A2 alone or in combination with other drugs, through the same route at 30 mg per dose in 3 ml D5W solution, with different dosing regimens. Drug administration can also be through aerosol delivery, inhalation, intravenous and subcutaneous injection. The infection can be performed both in epithelial cells of the upper respiratory tract (nasopharynx and trachea) as well as the lower respiratory tract (bronchia and lung) to analyse the efficacy of the treatments developed both in the preventive and therapeutic regimes.

Animals (mice and macaques) are observed daily to record body weight, a murine sepsis score, and death for 14 days. In parallel, a group of animals will be sacrificed at 2, 4 and 8 days respectively to collect different tissues and quantify virus replication and analyse histopathological, serological changes. Complete blood count (CBC), White blood count (WBC), Lymphocyte subsets in peripheral blood can be analysed to study Th1 and Th2 immune response type markers. The perturbations in immune cell subsets and the frequencies of T/NK cells and lymphocyte subsets in peripheral blood can be also analysed. Lymphocyte subsets in peripheral blood will be analysed to study Th1 and Th2 immune response type markers. The perturbations in immune cell subsets and the frequencies of T/NK cells and lymphocyte subsets in peripheral blood can be also analysed. The measurement of an effective SARS-CoV-2 specific antibody response will be performed and IgG, IgM levels will be determined in mice sera samples. Likewise, the degree of activation of alveolar macrophages (M1 and M2 macrophages) and blood ferritin levels will be determined as well as molecular determinations (ef1a2 and ef1a1 tissue levels, proinflammatory cytokines [(IL) -1β, IL-6, IL-18 and interferon-γ] levels will be determined by RT-qPCR (mRNA) and ELISA (protein). Also, specific TLRs, RNA-sensors and downstream pathways of type I/ III IFN and/or the inflammasome pathways will be determined, as well as molecules involved in autophagy processes and endosomal trafficking.

Detection of the SARS-CoV-2 virus, the target blocked by EEF1A2 siRNA candidates and pro-inflammatory biomarkers are performed using the Real-time PCR on the StepOne Plus and Quant Studio 12K Flex real-time PCR systems (Applied Biosystem, USA). Real-time PCR analysis are performed with universal primers and probes targeting SARS-CoV-2 that amplify structural viral proteins encoded by RdRp, E and N genes. Pro-inflammatory cytokines are determined by ELISA in a Microplate reader. All experiments will be conducted in triplicates. The relative expression will be determined using the 2-ΔΔCt method. The dose-response effect will be determined by a sigmoidal concentration-response function, Y= Bottom + (Top-Bottom)/ (1+10 ^ ((Log EC50-X) *Hill Slope)), fitting to the data using non-linear regression.

### REFERENCES

Abbas W, Kumar A and Herbein G (2015). "The eEF1A proteins: at the crossroads of oncogenesis, apoptosis, and viral infections." Front. Oncol. 5: 75. doi: 10.3389/fonc.2015.00075
Amiri A., Noei F., Jeganathan S., Kulkarni G., Pinke D.E. and Lee J.M. (2007). "eEF1A2 activates Akt and stimulates Akt-dependent actin remodeling, invasion and migration." Oncogene 26, 3027-3040 (2007). doi.org/10.1038/sj.onc.1210101.
Angaji S.A, Hedayati S.S, Poor R.H, et al. "Application of RNA interference in treating human diseases" J Genet. 2010. Vol. 89. 4. 527-37.
Asha K., Kumar P., Sanicas M., Meseko C.A., Khanna M. and Kumar B. (2019). Advancements in Nucleic Acid Based Therapeutics against Respiratory Viral Infections. J. Clin. Med. 2019, 8, 6.
Barik S., Lu P. (2015) Therapy of Respiratory Viral Infections with Intranasal siRNAs. In: Sioud M. (eds) RNA Interference. Methods in Molecular Biology (Methods and Protocols), vol 1218, pp 251-262. Humana Press, New York, NY
Cerutti L., Mian, N. et al. (2000). "Domains in gene silencing and cell differentiation proteins: the novel PAZ domain and redefinition of the Piwi domain." Trends Biochem Sci 25(10): 481-2.
Chang C.I, Kim H.A, Dua P, et al. (2011) "Structural Diversity Repertoire of Gene Silencing Small Interfering RNAs" Nucleic Acid Ther. 2011. Vol. 21. 3. 125-31.
Collins R.E. and Cheng X. (2005). "Structural domains in RNAi." FEBS Lett 579(26): 5841-9.
Corman V.M., Lienau J., and Witzenrath M. (2019). "Coronaviren als Ursache respiratorischer Infektionen." Internist 60, 1136-1145. https://doi.org/10.1007/s00108-019-00671-5.
Deleavey G.F and Damha M.J. (2012) "Designing chemically modified oligonucleotides for targeted gene silencing". Chem Biol. 2012 Vol.19.8. 937-54.
Doench J.G., Sharp P.A. (2004) "Specificity of microRNA target selection in translational repression" Genes Dev. 18, 504-511.
Dosta P., Ramos V., Borrós S. (2018). "Stable and efficient generation of poly(β-aminoester)s for RNAi delivery." Mol Systems Design Eng 2018, 3, 677-689.
Dow D, Revol R, Östbye H, Wang H and Daniels R (2018). "Influenza A Virus Cell Entry, Replication, Virion Assembly and Movement." Front. Immunol. 9:1581. doi: 10.3389/fimmu.2018.01581
Elbashir, S. M., W. Lendeckel, et al. (2001). "RNA interference is mediated by 21 - and 22-nucleotide RNAs." Genes Dev 15(2): 188-200.
Fehr A.R., Channappanavar R., and Perlman S. (2017). Middle East Respiratory Syndrome: Emergence of a Pathogenic Human Coronavirus. Annu. Rev. Med. 68, 387-399.
Fire, A., S. Xu, et al. "Potent and specific genetic interference by double-stranded RNA in Caenorhabditis elegans." Nature. 1998 391(6669): 806-11.
Ge Q., McManus M. T., Nguyen T., Shen C. H., Sharp P. A., Eisen H. N., Chen J. (2003). RNA interference of influenza virus production by directly targeting mRNA for degradation and indirectly inhibiting all viral RNA transcription. Proc. Natl. Acad. Sci. U. S. A. 100: 2718-23.
Godinho B.M.D.C., Khvorova A. (2019). The era of RNA interference medicines: the clinical landscape of synthetic gene silencing drugs. Saúde & Tecnologia, 21, p. 05-17.
GuzikT.J., Mohiddin S.A., Dimarco A., Patel V., Savvatis K., Marelli-Berg F.M., Madhur M.S., Tomaszewski M., Maffia P., D'Acquisto F., Nicklin S.A., Marian A.J., Nosalski R., Murray E.C., Guzik B., Berry C., Touyz R.M., Kreutz R., Wang D.W., Bhella D., Sagliocco O., Crea F., Thomson E.C., McInnes I. B. (2020). "COVID-19 and the cardiovascular system: implications for risk assessment, diagnosis, and treatment options." Cardiovas Res 116(10), 1666-1687. https://doi.org/10.1093/cvr/cvaa106
Hamming I., Timens W., Bulthuis M.L.C., Lely A.T., Navis G.J., van Goor H. (2004). Tissue distribution of ACE2 protein, the functional receptor for SARS coronavirus. A first step in understanding SARS pathogenesis. J. Pathology, 203 (2), 631-637.
Harmer D, Gilbert M, Borman R, Clark KL (2002) Quantitative mRNA expression profiling of ACE 2, a novel homologue of angiotensin converting enzyme. FEBS Lett 532:107-110.
Herzog P., Drosten C., Muller M.A. (2008). "Plaque assay for human coronavirus NL63 using human colon carcinoma cells". Virology Journal 2008, 5, 138. doi:10.1186/1743-422X-5-138.
Hoffmann M., Kleine-Weber H., Schroeder S., Krüger N., HerrlerT., Erichsen S., Schiergens T.S., Herrler G., Wu N.-H., Nitsche A., Muller M.A., Drosten C., and Pohlmann S. (2020). SARS-CoV-2 Cell Entry Depends on ACE2 and TMPRSS2 and Is Blocked by a Clinically Proven Protease Inhibitor. Cell 181, 271-280 https://doi.org/10.1016/j.cell.2020.02.052.
Hutvagner, G. and Zamore P.D. (2002). "A microRNA in a multiple-turnover RNAi enzyme complex." Science 297(5589): 2056-60.
Hwang J, Lee S, Lee J-H, Kang W-H, Kang J-H, Kang M-Y, et al. (2015) Plant Translation Elongation Factor 1Bβ Facilitates Potato Virus X (PVX) Infection and Interacts with PVX Triple Gene Block Protein 1. PLoS ONE 10(5): e0128014. https://doi.org/10.1371/journal.pone.0128014
Jhaveri A.M., Torchilin V.P. (2014). "Multifunctional polymeric micelles for delivery of drugs and siRNA." Front. Pharmacol. 2014, 5, 77, 1.
Knipe D M and Howley P M (Eds.), Fields Virology; 6th edition; 2013 Philadelphia: Lippincott Williams and Wilkins, Inc (Wolters Kluwer Health). ISBN 1469830663, 9781469830667.
Kornbrust D, Cavagnaro J, Levin A, et al. "Oligo safety working group exaggerated pharmacology subcommittee consensus document" Nucleic Acid Ther 2013 Vol. 23, 1, Pag: 21-8.
Lewis, B.P., Shih I. et al. (2003) "Prediction of mammalian micro RNA targets" Cell 115:787-798.
Li, D., Rawle D.J., Wu Z., Jin H., Lin M.H., Lor M., Abbott C.M., and Harrich D. (2019). "eEF1Ademonstrates paralog specific effects on HIV-1 reverse transcription efficiency." Virology 530: 65-74.
Li D., Wei T., Abbott C. M. and Harrich D. (2013). "The unexpected roles of eukaryotic translation elongation factors in RNA virus replication and pathogenesis." Microbiol Mol Biol Rev 77(2): 253-266.
Liu J., Carmell M.A., et al. (2004). "Argonaute2 is the catalytic engine of mammalian RNAi." Science 305(5689): 1437-41.
Losada A., Muñoz-Alonso M.J., García C., Sánchez-Murcia P.A., Martínez-Leal J.F., Domínguez J.M., Lillo M.P., Gago F., Galmarini C.M. (2016). "Translation Elongation Factor eEF1A2 is a Novel Anticancer Target for the Marine Natural Product Plitidepsin." Sci. Rep. 6, 35100; doi: 10.1038/srep35100
Ma, J. B., Yuan Y.R., et al. (2005). "Structural basis for 5'-end-specific recognition of guide RNA by the A. fulgidus Piwi protein." Nature 434(7033): 666-70.
Maniatis, T., et al., "Molecular Cloning: A Laboratory Manual". Cold Spring Harbor Laboratory, 1982, at pages 387-389.
Merkel O.M. and Kissel T. (2012). "Nonviral Pulmonary Delivery of siRNA." Acc. Chem. Res. 2012, 45, 7, 961-970.
Moschos SA, Jones SW, Perry MM, Williams AE, Erjefalt JS, Turner JJ, Barnes PJ, Sproat BS, Gait MJ, Lindsay MA (2007). "Lung delivery studies using siRNA conjugated to TAT(48-60) and penetratin reveal peptide induced reduction in gene expression and induction of innate immunity." Bioconjugate Chemistry, 18(5): 1450-1459.
Nykanen A., Haley B., et al. (2001). "ATP requirements and small interfering RNA structure in the RNA interference pathway." Cell 107(3): 309-21.
Orban, T. I. and Izaurralde E. (2005). "Decay of mRNAs targeted by RISC requires XRN1, the Ski complex, and the exosome." Rna 11(4): 459-69.
Parrish, S., J. Fleenor, et al. (2000) "Functional anatomy of a dsRNA trigger: differential requirement for the two trigger strands in RNA interference." Mol Cell. 2000 6(5): 1077-87.
Qiu FN, Huang Y, Chen DY, Li F, Wu YA, Wu WB, Huang XL (2016). "Eukaryotic elongation factor-1α 2 knockdown inhibits hepatocarcinogenesis by suppressing PI3K/Akt/NF-κB signaling." World J Gastroenterol. 22(16):4226-37. doi: 10.3748/wjg.v22.i16.4226.
Rand, T. A., Petersen S., et al. (2005). "Argonaute2 cleaves the anti-guide strand of siRNA during RISC activation." Cell 123(4): 621-9.
Rubio L, Galipienso L and Ferriol I (2020) "Detection of Plant Viruses and Disease Management: Relevance of Genetic Diversity and Evolution." Front. Plant Sci. 11:1092. doi: 10.3389/fpls.2020.01092
Sanghvi Y.S. (2011) "A status update of modified oligonucleotides for chemotherapeutics applications" Curr Protoc Nucleic Acid Chem. 2011 Vol. 4. 4 1 1-22.
Sasikumar A.N., Perez W.B., and Kinzy T.G. (2012) "The Many Roles of the Eukaryotic Elongation Factor 1 Complex." Wiley Interdiscip Rev RNA 3(4): 543-555. doi:10.1002/wrna.1118.
Segovia N., Pont M., Oliva N., Ramos V., Borrós S., Artzi N. (2014). "Hydrogel Doped with Nanoparticles for Local Sustained Release of siRNA in Breast Cancer." Adv Healthc Mater. 2015;4(2):271-280. doi:10.1002/adhm.201400235.
Snape N., Li D., Wei T., Jin H., Lor M., Rawle D. J., Spann K. M. and Harrich D. (2018). "The eukaryotic translation elongation factor 1A regulation of actin stress fibers is important for infectious RSV production." Virol J 15(1): 182.
Soares DC, Barlow PN, Newbery HJ, Porteous DJ, Abbott CM (2009). "Structural Models of Human eEF1A1 and eEF1A2 Reveal Two Distinct Surface Clusters of Sequence Variation and Potential Differences in Phosphorylation." PLoS ONE 4(7): e6315. doi:10.1371/journal.pone.0006315
Song, J. J., Smith S.K., et al. (2004). "Crystal structure of Argonaute and its implications for RISC slicer activity." Science 305(5689): 1434-7.
Sun K., Gu L., Ma L., Duan Y. (2020). Atlas of ACE2 gene expression in mammals reveals novel insights in transmisson of SARS-Cov-2. DOI: 10.1101/2020.03.30.015644
Walton S.P, Wu M, Gredell J.A and Chan C. (2010) "Designing highly active siRNAs for therapeutic applications" FEBS J. 2010. Vol. 277. 23. 4806-13.
Warren K, Wei T, Li D, Qin F, Warrilow D, Lin M-H, Sivakumaran H, Apolloni A, Abbott CM, Jones A, Anderson JL, Harrich D (2012). "Eukaryotic elongation factor 1 complex subunits are critical HIV-1 reverse transcription cofactors." Proc Natl Acad Sci USA 109, 24, 9587-9592.
Wei T., Li D., Marcial D., Khan M., Lin M. H., Snape N., Ghildyal R., Harrich D. and Spann K. (2014). "The eukaryotic elongation factor 1A is critical for genome replication of the paramyxovirus respiratory syncytial virus." PLoS One 9(12): e114447.
Wu C. et al. (2020), "Analysis of therapeutic targets for SARS-CoV-2 and discovery of potential drugs by computational methods", Acta Pharmaceutica Sinica B, Volume 10, Issue 5, 2020, Pages 766-788, doi.org/10.1016/j.apsb.2020.02.008.
Xia S., Liu M., Wang C. et al. (2020). "Inhibition of SARS-CoV-2 (previously 2019-nCoV) infection by a highly potent pan-coronavirus fusion inhibitor targeting its spike protein that harbors a high capacity to mediate membrane fusion." Cell Res 30, 343-355. https://doi.org/10.1038/s41422-020-0305-x.

Further features and aspects of the invention are defined in the following numbered clauses:
**1.** An small interfering RNA (siRNA) molecule which targets the eukaryotic translation elongation factor 1 alpha 2 (EEF1A2) gene, and reduces the expression and/or activity of EEF1A2 in a cell, for use in the prophylactic or therapeutic treatment of a virus infection, the siRNA targeting a nucleotide sequence selected from any of SEQ ID NO: 1 to SEQ ID NO: 51 and SEQ ID NO: 1168 to SEQ ID NO: 1173.
**2.** An siRNA molecule for use according to clause 1, wherein the siRNA molecule comprises an antisense strand which comprises or consists of at least 19 consecutive nucleotides of any of the nucleotide sequences SEQ ID NO: 1124 - SEQ ID NO: 1130 and SEQ ID NO: 1188 - SEQ ID NO: 1190, and a sense strand complementary to the antisense strand, and wherein both strands are base paired to form a double stranded RNA (dsRNA) structure comprising at least 15 base pairs (bp), wherein the 3' end of the antisense strand is a blunt end or has a one nucleotide or dinucleotide 3'-overhang, and wherein:
   i. the antisense strand consists of 19 nucleotides and the sense strand is an oligonucleotide consisting of a sequence of 19 nucleotides complementary to the antisense strand, and the siRNA is a blunt ended dsRNA structure;
      or
   ii. the antisense strand consists of 19 consecutive nucleotides of any of the nucleotide sequences SEQ ID NO: 1124 - SEQ ID NO: 1130 and SEQ ID NO: 1188 - SEQ ID NO: 1190, and further comprises an additional nucleotide or dinucleotide of U or dT at the 3'-end, said additional nucleotide or dinucleotide constituting a 3'-overhang of the antisense strand, and the sense strand is an oligonucleotide having a sequence selected from:
      a. a nucleotide sequence consisting of a sequence of 19 nucleotides in which at least the first 15 consecutive nucleotides from the 5'-end are complementary to the corresponding nucleotides from the 3'-end of the antisense strand, or
      b. a nucleotide sequence consisting of a sequence of 20 or 21 nucleotides in which the first 19 consecutive nucleotides from the 5'-end are complementary to the antisense strand, and are followed by an additional nucleotide or dinucleotide of U or dT at the 3'-end, to form a dsRNA structure of 19 bp with 3'-overhangs of U, dT, UU or dTdT on both strands;
         or
   iii. the antisense strand consists of 20 or 21 consecutive nucleotides of any of the nucleotide sequences SEQ ID NO: 1124 - SEQ ID NO: 1130 and SEQ ID NO: 1188 - SEQ ID NO: 1190, and the sense strand is an oligonucleotide consisting of a sequence of 20 or 21 consecutive nucleotides of any of the nucleotide sequences SEQ ID NO: 1133 - SEQ ID NO: 1139 and SEQ ID NO: 1191 - SEQ ID NO: 1193, respectively, and the siRNA forms a dsRNA structure of 19 bp with 3'-overhangs on both strands.
**3.** An siRNA molecule for use according to clause 1 or 2, wherein the siRNA molecule additionally targets or reduces the expression and/or activity of the eukaryotic translation elongation factor 1 alpha 1 (EEF1A1) gene.
**4.** An siRNA molecule forthe use according to any of clauses 1 to 3, wherein the antisense strand comprises or consists of at least 19 consecutive nucleotides of any of the nucleotide sequences SEQ ID NO: 1124, SEQ ID NO: 1125, SEQ ID NO: 1126 or SEQ ID NO: 1127, and the sense strand is an oligonucleotide consisting of a sequence of between 15 to 21 consecutive nucleotides of any of the nucleotide sequences SEQ ID NO: 1133, SEQ ID NO: 1134, SEQ ID NO: 1135 or SEQ ID NO: 1136, respectively.
**5.** An siRNA molecule for the use according to any of clauses 1 to 4, wherein the siRNA comprises or consists of:
   - an antisense strand of nucleotide sequence SEQ ID NO: 1142 and a sense strand of nucleotide sequence SEQ ID NO: 1150;
   - an antisense strand of nucleotide sequence SEQ ID NO: 1143 and a sense strand of nucleotide sequence SEQ ID NO: 1151;
   - an antisense strand of nucleotide sequence SEQ ID NO: 1145 and a sense strand of nucleotide sequence SEQ ID NO: 1153;
   - an antisense strand of nucleotide sequence SEQ ID NO: 1149 and a sense strand of nucleotide sequence SEQ ID NO: 1157;
   - an antisense strand of nucleotide sequence SEQ ID NO: 1144 and a sense strand of nucleotide sequence SEQ ID NO: 1152;
   - an antisense strand of nucleotide sequence SEQ ID NO: 1146 and a sense strand of nucleotide sequence SEQ ID NO: 1154;
   - an antisense strand of nucleotide sequence SEQ ID NO: 1147 and a sense strand of nucleotide sequence SEQ ID NO: 1155; or
   - an antisense strand of nucleotide sequence SEQ ID NO: 1148 and a sense strand of nucleotide sequence SEQ ID NO: 1156.
**6.** An siRNA molecule for the use according to any of clauses 1 to 5, wherein the siRNA comprises or consists of:
   - an antisense strand of nucleotide sequence SEQ ID NO: 1158 and a sense strand of nucleotide sequence SEQ ID NO: 1163;
   - an antisense strand of nucleotide sequence SEQ ID NO: 1143 and a sense strand of nucleotide sequence SEQ ID NO: 1151;
   - an antisense strand of nucleotide sequence SEQ ID NO: 1160 and a sense strand of nucleotide sequence SEQ ID NO: 1165;
   - an antisense strand of nucleotide sequence SEQ ID NO: 1162 and a sense strand of nucleotide sequence SEQ ID NO: 1167;
   - an antisense strand of nucleotide sequence SEQ ID NO: 1159 and a sense strand of nucleotide sequence SEQ ID NO: 1164;
   - an antisense strand of nucleotide sequence SEQ ID NO: 1161 and a sense strand of nucleotide sequence SEQ ID NO: 1166;
   - an antisense strand of nucleotide sequence SEQ ID NO: 1147 and a sense strand of nucleotide sequence SEQ ID NO: 1155; or
   - an antisense strand of nucleotide sequence SEQ ID NO: 1148 and a sense strand of nucleotide sequence SEQ ID NO: 1156.
**7.** An siRNA molecule for the use according to any of clauses 1 to 6, wherein the siRNA is an siRNA duplex comprising or consisting of:
   - an antisense strand of nucleotide sequence SEQ ID NO: 52 and a sense strand of nucleotide sequence SEQ ID NO: 103;
   - an antisense strand of nucleotide sequence SEQ ID NO: 53 and a sense strand of nucleotide sequence SEQ ID NO: 104;
   - an antisense strand of nucleotide sequence SEQ ID NO: 54 and a sense strand of nucleotide sequence SEQ ID NO: 105;
   - an antisense strand of nucleotide sequence SEQ ID NO: 55 and a sense strand of nucleotide sequence SEQ ID NO: 106;
   - an antisense strand of nucleotide sequence SEQ ID NO: 56 and a sense strand of nucleotide sequence SEQ ID NO: 107;
   - an antisense strand of nucleotide sequence SEQ ID NO: 57 and a sense strand of nucleotide sequence SEQ ID NO: 108;
   - an antisense strand of nucleotide sequence SEQ ID NO: 58 and a sense strand of nucleotide sequence SEQ ID NO: 109;
   - an antisense strand of nucleotide sequence SEQ ID NO: 154 and a sense strand of nucleotide sequence SEQ ID NO: 625;
   - an antisense strand of nucleotide sequence SEQ ID NO: 155 and a sense strand of nucleotide sequence SEQ ID NO: 626;
   - an antisense strand of nucleotide sequence SEQ ID NO: 156 and a sense strand of nucleotide sequence SEQ ID NO: 627;
   - an antisense strand of nucleotide sequence SEQ ID NO: 171 and a sense strand of nucleotide sequence SEQ ID NO: 642;
   - an antisense strand of nucleotide sequence SEQ ID NO: 172 and a sense strand of nucleotide sequence SEQ ID NO: 643;
   - an antisense strand of nucleotide sequence SEQ ID NO: 173 and a sense strand of nucleotide sequence SEQ ID NO: 644; or
   - an antisense strand of nucleotide sequence SEQ ID NO: 562 and a sense strand of nucleotide sequence SEQ ID NO: 1033.
**8.** An siRNA molecule for the use according to any of clauses 1 to 7, wherein the siRNA targets SEQ ID NO: 1.
**9.** An siRNA molecule for the use according to any of clauses 1 to 7, wherein the siRNA targets SEQ ID NO: 3 or SEQ ID NO: 5.
**10.** An siRNA molecule for the use according to any of clauses 1 to 9, wherein the siRNA is a blunt ended dsRNA structure or has a 3'-overhang in the antisense strand consisting of an additional dinucleotide of UU.
**11.** An siRNA molecule for the use according to any of clauses 1 to 10, wherein the siRNA molecule comprises at least one chemical modification in the antisense strand, in the sense strand or in both strands, and the or each chemical modification is independently selected from the group consisting of 2'-fluoro modification, 2'-O-methyl modification and a phosphorothioate linkage.
**12.** An siRNA molecule for the use according to clause 11, wherein the siRNA has an antisense sequence selected from any of nucleotide sequences SEQ ID NO: 1096 to SEQ ID NO: 1109 and a complementary sense sequence which is selected from any of nucleotide sequences SEQ ID NO: 1110 to SEQ ID NO: 1123.
**13.** An siRNA molecule for the use according to any of clauses 1 to 12, wherein the virus infection is in a subject which is an animal, preferably a mammal, more preferably a human.
**14.** An siRNA molecule for the use according to clause 13, wherein the subject has been diagnosed with a virus infection caused by a RNA virus selected from a *Coronavirus*, preferably severe acute respiratory syndrome coronavirus 2 (SARS-CoV-2), severe acute respiratory syndrome coronavirus (SARS-CoV) or Middle East respiratory syndrome coronavirus (MERS-CoV); a *Flavivirus*, preferably West Nile virus (WNV), Dengue virus (DENV), Zika virus (ZIKV) or yellow fever virus (YFV); an *Hepatovirus*, preferably hepatovirus
   A or hepatitis A virus (HAV); an hepatitis C virus (HCV); an hepatitis E virus (HEV); an *Influenzavirus*, preferably influenzavirus A, influenzavirus B, influenzavirus C, or influenzavirus D; an *Orthopneumovirus*, preferably respiratory syncytial virus (RSV); or an *Arenavirus*, preferably Guanarito virus, Junin virus (Argentinian mammaarenavirus), Lassa virus, Lujo virus, Machupo virus, Sabia virus (Brazilian mammarenavirus) or WhitewaterArroyo virus, or is at risk of exposure to any of said viruses.
**15.** An siRNA molecule for the use according to any of clauses 13 to 14, wherein the subject to be treated has COVID-19 or has been exposed to a close contact with another subject suffering from COVID-19 or diagnosed as having an infection with a SARS-CoV-2 virus.
**16.** An siRNA molecule for the use according to any of clauses 1 to 15, wherein the siRNA is formulated for delivery to a tissue of the respiratory tract, the lung, the eye or the skin.
**17.** An siRNA molecule for the use according to any of clauses 1 to 16, wherein the siRNA is formulated for administration as an aerosol.
**18.** An siRNA molecule for the use according to any of clauses 1 to 12, wherein the virus infection is in a plant, preferably wherein the virus infection is caused by tomato brown rugose fruit virus (ToBRV), turnip yellow mosaic virus (TYMV), tobacco mosaic virus (TMV), brome mosaic virus (BMV), or tomato bushy stunt virus (TBSV).
**19.** An small interfering RNA (siRNA) molecule which targets the eukaryotic translation elongation factor 1 alpha 2 (EEF1A2) gene, the siRNA targeting a nucleotide sequence selected from any of SEQ ID NO: 1 to SEQ ID NO: 51 and SEQ ID NO: 1168 to SEQ ID NO: 1173.
**20.** A small interfering RNA (siRNA) molecule which targets the eukaryotic translation elongation factor 1 alpha 2 (EEF1A2) gene, the siRNA comprising an antisense strand which comprises or consists of at least 19 consecutive nucleotides of any of the nucleotide sequences SEQ ID NO: 1124 - SEQ ID NO: 1130 and SEQ ID NO: 1188 - SEQ ID NO: 1190, and a sense strand complementary to the antisense strand, and both strands are base paired to form a double stranded RNA (dsRNA) structure comprising at least 15 base pairs (bp), wherein the 3' end of the antisense strand is a blunt end or has a one nucleotide or dinucleotide 3'-overhang, and wherein:
   i. the antisense strand consists of 19 nucleotides and the sense strand is an oligonucleotide consisting of a sequence of 19 nucleotides complementary to the antisense strand, and the siRNA is a blunt ended dsRNA structure;
      or
   ii. the antisense strand consists of 19 consecutive nucleotides of any of the nucleotide sequences SEQ ID NO: 1124 - SEQ ID NO: 1130 and SEQ ID NO: 1188 - SEQ ID NO: 1190, and further comprises an additional nucleotide or dinucleotide of U or dT at the 3'-end, said additional nucleotide or dinucleotide constituting a 3'-overhang of the antisense strand, and the sense strand is an oligonucleotide having a sequence selected from:
      a. a nucleotide sequence consisting of a sequence of 19 nucleotides in which at least the first 15 consecutive nucleotides from the 5'-end are complementary to the corresponding nucleotides from the 3'-end of the antisense strand, or
      b. a nucleotide sequence consisting of a sequence of 20 or 21 nucleotides in which the first 19 consecutive nucleotides from the 5'-end are complementary to the antisense strand, and are followed by an additional nucleotide or dinucleotide of U or dT at the 3'-end, to form a dsRNA structure of 19 bp with 3'-overhangs of U, dT, UU or dTdT on both strands;
         or
   iii. the antisense strand consists of 20 or 21 consecutive nucleotides of any of the nucleotide sequences SEQ ID NO: 1124- SEQ ID NO: 1130 and SEQ ID NO: 1188 - SEQ ID NO: 1190, and the sense strand is an oligonucleotide consisting of a sequence of 20 or 21 consecutive nucleotides of any of the nucleotide sequences SEQ ID NO: 1133 - SEQ ID NO: 1139 and SEQ ID NO: 1191 - SEQ ID NO: 1193, respectively, and the siRNA forms a dsRNA structure of 19 bp with 3'-overhangs on both strands.
**21.** A siRNA molecule according to clause 19 or 20, wherein the siRNA comprises or consists of:
   - an antisense strand of nucleotide sequence SEQ ID NO: 1142 and a sense strand of nucleotide sequence SEQ ID NO: 1150;
   - an antisense strand of nucleotide sequence SEQ ID NO: 1143 and a sense strand of nucleotide sequence SEQ ID NO: 1151;
   - an antisense strand of nucleotide sequence SEQ ID NO: 1145 and a sense strand of nucleotide sequence SEQ ID NO: 1153;
   - an antisense strand of nucleotide sequence SEQ ID NO: 1149 and a sense strand of nucleotide sequence SEQ ID NO: 1157;
   - an antisense strand of nucleotide sequence SEQ ID NO: 1144 and a sense strand of nucleotide sequence SEQ ID NO: 1152;
   - an antisense strand of nucleotide sequence SEQ ID NO: 1146 and a sense strand of nucleotide sequence SEQ ID NO: 1154;
   - an antisense strand of nucleotide sequence SEQ ID NO: 1147 and a sense strand of nucleotide sequence SEQ ID NO: 1155; or
   - an antisense strand of nucleotide sequence SEQ ID NO: 1148 and a sense strand of nucleotide sequence SEQ ID NO: 1156.
**22.** An siRNA molecule according to any of clauses 19 to 21, wherein the siRNA is an siRNA duplex comprising or consisting of:
   - an antisense strand of nucleotide sequence SEQ ID NO: 52 and a sense strand of nucleotide sequence SEQ ID NO: 103;
   - an antisense strand of nucleotide sequence SEQ ID NO: 53 and a sense strand of nucleotide sequence SEQ ID NO: 104;
   - an antisense strand of nucleotide sequence SEQ ID NO: 54 and a sense strand of nucleotide sequence SEQ ID NO: 105;
   - an antisense strand of nucleotide sequence SEQ ID NO: 55 and a sense strand of nucleotide sequence SEQ ID NO: 106;
   - an antisense strand of nucleotide sequence SEQ ID NO: 56 and a sense strand of nucleotide sequence SEQ ID NO: 107;
   - an antisense strand of nucleotide sequence SEQ ID NO: 57 and a sense strand of nucleotide sequence SEQ ID NO: 108;
   - an antisense strand of nucleotide sequence SEQ ID NO: 58 and a sense strand of nucleotide sequence SEQ ID NO: 109;
   - an antisense strand of nucleotide sequence SEQ ID NO: 154 and a sense strand of nucleotide sequence SEQ ID NO: 625;
   - an antisense strand of nucleotide sequence SEQ ID NO: 155 and a sense strand of nucleotide sequence SEQ ID NO: 626;
   - an antisense strand of nucleotide sequence SEQ ID NO: 156 and a sense strand of nucleotide sequence SEQ ID NO: 627;
   - an antisense strand of nucleotide sequence SEQ ID NO: 171 and a sense strand of nucleotide sequence SEQ ID NO: 642;
   - an antisense strand of nucleotide sequence SEQ ID NO: 172 and a sense strand of nucleotide sequence SEQ ID NO: 643;
   - an antisense strand of nucleotide sequence SEQ ID NO: 173 and a sense strand of nucleotide sequence SEQ ID NO: 644; or
   - an antisense strand of nucleotide sequence SEQ ID NO: 562 and a sense strand of nucleotide sequence SEQ ID NO: 1033.
**23.** Use of an siRNA molecule according to any of clauses 19-22 in the manufacture of a medicament for the prophylactic or therapeutic treatment of a virus infection in a subject.
**24.** An small interfering RNA (siRNA) molecule which targets the eukaryotic translation elongation factor 1 alpha 2 (EEF1A2) gene, and which reduces the expression and/or activity of EEF1A2 in a cell, for use in the prophylactic or therapeutic treatment of a virus infection in a subject.
**25.** A pharmaceutical composition comprising an siRNA molecule defined in any of clauses 19 to 22 and an acceptable carrier.
**26.** A pharmaceutical composition according to clause 25, wherein said composition is formulated for topical administration.
**27.** A pharmaceutical composition according to any of clauses 25 and 26, wherein said composition is formulated for topical administration via the intranasal or intrapulmonary routes.
**28.** A pharmaceutical composition according to any of clauses 25 to 27, wherein the topical administration is by inhalation of said composition.
**29.** A pharmaceutical composition according to any of clauses 25 to 28, wherein the topical administration is by administration of an aerosolized liquid, preferably a nasal spray.
**30.** A method of preventing or treating a virus infection in a subject caused by an RNA virus, comprising administering to said subject an siRNA defined in any of clauses 19-22 or the pharmaceutical composition of any of clauses 25 to 29.
**31.** A method according to clause 30, wherein the RNA virus is selected from a *Coronavirus*, preferably severe acute respiratory syndrome coronavirus 2 (SARS-CoV-2), severe acute respiratory syndrome coronavirus (SARS-CoV) or Middle East respiratory syndrome coronavirus (MERS-CoV), a *Flavivirus*, preferably West Nile virus (WNV), Dengue virus (DENV), Zika virus (ZIKV) or yellow fever virus (YFV), an *Hepatovirus*, preferably hepatovirus A or hepatitis A virus (HAV), an hepatitis C virus (HCV), an hepatitis E virus (HEV), an *Influenzavirus*, preferably influenzavirus A, influenzavirus B, influenzavirus C, or influenzavirus D, an *Orthopneumovirus*, preferably respiratory syncytial virus (RSV), and an *Arenavirus*, preferably Guanarito virus, Junin virus (Argentinian mammaarenavirus), Lassa virus, Lujo virus, Machupo virus, Sabia virus (Brazilian mammarenavirus) or Whitewater Arroyo virus.
**32.** A method according to any of clauses 30 and 31, wherein the siRNA administered or comprised in the composition is in a therapeutically effective amount.
**33.** A method according to any of clauses 30 to 32, wherein the siRNA or the composition is topically administered.
**34.** A method according to any of clauses 30 to 33, wherein the siRNA or the composition is topically administered via the intranasal or intrapulmonary routes.
**35.** A method according to any of clauses 30 to 34, wherein the siRNA or the composition is topically administered by inhalation of said siRNA or composition.
**36.** A method according to any of clauses 30 to 35, wherein the siRNA or the composition is administered as an aerosolized liquid, preferably a nasal spray or produced by a nebulizer.
**37.** A method according to any of clauses 30 to 36, wherein the subject is a human subject.
**38.** A method according to any of clauses 30 to 37, wherein the subject has been diagnosed with, or is at risk of, an infection with SARS-CoV-2.
**39.** A method according to clause 38, wherein the subject at risk of being infected is a subject who has been exposed or is exposed to another subject infected or suspected of being infected by SARS-CoV-2.
**40.** A method according to any of clauses 30 to 39, wherein the subject has been diagnosed with an infection with SARS-CoV-2 and suffers from the related disease COVID-19.
**41.** A method of preventing or treating a virus infection in a plant caused by an RNA virus, comprising applying to said plant an siRNA defined in any of clauses 19-22.
**42.** An antiviral composition comprising at least an siRNA molecule defined in any of clauses 19 to 22 and an acceptable carrier.

## Claims

1. An small interfering RNA (siRNA) molecule which targets the eukaryotic translation elongation factor 1 alpha 2 (EEF1A2) gene, and reduces the expression and/or activity of EEF1A2 in a cell, for use in the prophylactic or therapeutic treatment of a virus infection, the siRNA targeting a nucleotide sequence selected from any of SEQ ID NO: 1 to SEQ ID NO: 51 and SEQ ID NO: 1168 to SEQ ID NO: 1173.

2. An siRNA molecule for use according to claim 1, wherein the siRNA molecule comprises an antisense strand which comprises or consists of at least 19 consecutive nucleotides of any of the nucleotide sequences SEQ ID NO: 1124- SEQ ID NO: 1130 and SEQ ID NO: 1188- SEQ ID NO: 1190, and a sense strand complementary to the antisense strand, and wherein both strands are base paired to form a double stranded RNA (dsRNA) structure comprising at least 15 base pairs (bp), wherein the 3' end of the antisense strand is a blunt end or has a one nucleotide or dinucleotide 3'-overhang, and wherein:
i. the antisense strand consists of 19 nucleotides and the sense strand is an oligonucleotide consisting of a sequence of 19 nucleotides complementary to the antisense strand, and the siRNA is a blunt ended dsRNA structure;
or
ii. the antisense strand consists of 19 consecutive nucleotides of any of the nucleotide sequences SEQ ID NO: 1124- SEQ ID NO: 1130 and SEQ ID NO: 1188 - SEQ ID NO: 1190, and further comprises an additional nucleotide or dinucleotide of U or dT at the 3'-end, said additional nucleotide or dinucleotide constituting a 3'-overhang of the antisense strand, and the sense strand is an oligonucleotide having a sequence selected from:
a. a nucleotide sequence consisting of a sequence of 19 nucleotides in which at least the first 15 consecutive nucleotides from the 5'-end are complementary to the corresponding nucleotides from the 3'-end of the antisense strand, or
b. a nucleotide sequence consisting of a sequence of 20 or 21 nucleotides in which the first 19 consecutive nucleotides from the 5'-end are complementary to the antisense strand, and are followed by an additional nucleotide or dinucleotide of U or dT at the 3'-end, to form a dsRNA structure of 19 bp with 3'-overhangs of U, dT, UU or dTdT on both strands;
or
iii. the antisense strand consists of 20 or 21 consecutive nucleotides of any of the nucleotide sequences SEQ ID NO: 1124- SEQ ID NO: 1130 and SEQ ID NO: 1188 - SEQ ID NO: 1190, and the sense strand is an oligonucleotide consisting of a sequence of 20 or 21 consecutive nucleotides of any of the nucleotide sequences SEQ ID NO: 1133 - SEQ ID NO: 1139 and SEQ ID NO: 1191 - SEQ ID NO: 1193, respectively, and the siRNA forms a dsRNA structure of 19 bp with 3'-overhangs on both strands.

3. An siRNA molecule for use according to claim 1 or 2, wherein the siRNA molecule additionally targets or reduces the expression and/or activity of the eukaryotic translation elongation factor 1 alpha 1 (EEF1A1) gene.

4. An siRNA molecule for the use according to any of claims 1 to 3, wherein the antisense strand comprises or consists of at least 19 consecutive nucleotides of any of the nucleotide sequences SEQ ID NO: 1124, SEQ ID NO: 1125, SEQ ID NO: 1126 or SEQ ID NO: 1127, and the sense strand is an oligonucleotide consisting of a sequence of between 15 to 21 consecutive nucleotides of any of the nucleotide sequences SEQ ID NO: 1133, SEQ ID NO: 1134, SEQ ID NO: 1135 or SEQ ID NO: 1136, respectively.

5. An siRNA molecule forthe use according to any of claims 1 to 4, wherein the siRNA comprises or consists of:
- an antisense strand of nucleotide sequence SEQ ID NO: 1142 and a sense strand of nucleotide sequence SEQ ID NO: 1150;
- an antisense strand of nucleotide sequence SEQ ID NO: 1143 and a sense strand of nucleotide sequence SEQ ID NO: 1151;
- an antisense strand of nucleotide sequence SEQ ID NO: 1145 and a sense strand of nucleotide sequence SEQ ID NO: 1153;
- an antisense strand of nucleotide sequence SEQ ID NO: 1149 and a sense strand of nucleotide sequence SEQ ID NO: 1157;
- an antisense strand of nucleotide sequence SEQ ID NO: 1144 and a sense strand of nucleotide sequence SEQ ID NO: 1152;
- an antisense strand of nucleotide sequence SEQ ID NO: 1146 and a sense strand of nucleotide sequence SEQ ID NO: 1154;
- an antisense strand of nucleotide sequence SEQ ID NO: 1147 and a sense strand of nucleotide sequence SEQ ID NO: 1155; or
- an antisense strand of nucleotide sequence SEQ ID NO: 1148 and a sense strand of nucleotide sequence SEQ ID NO: 1156.

6. An siRNA molecule forthe use according to any of claims 1 to 5, wherein the siRNA comprises or consists of:
- an antisense strand of nucleotide sequence SEQ ID NO: 1158 and a sense strand of nucleotide sequence SEQ ID NO: 1163;
- an antisense strand of nucleotide sequence SEQ ID NO: 1143 and a sense strand of nucleotide sequence SEQ ID NO: 1151;
- an antisense strand of nucleotide sequence SEQ ID NO: 1160 and a sense strand of nucleotide sequence SEQ ID NO: 1165;
- an antisense strand of nucleotide sequence SEQ ID NO: 1162 and a sense strand of nucleotide sequence SEQ ID NO: 1167;
- an antisense strand of nucleotide sequence SEQ ID NO: 1159 and a sense strand of nucleotide sequence SEQ ID NO: 1164;
- an antisense strand of nucleotide sequence SEQ ID NO: 1161 and a sense strand of nucleotide sequence SEQ ID NO: 1166;
- an antisense strand of nucleotide sequence SEQ ID NO: 1147 and a sense strand of nucleotide sequence SEQ ID NO: 1155; or
- an antisense strand of nucleotide sequence SEQ ID NO: 1148 and a sense strand of nucleotide sequence SEQ ID NO: 1156.

7. An siRNA molecule for the use according to any of claims 1 to 6, wherein the siRNA is an siRNA duplex comprising or consisting of:
- an antisense strand of nucleotide sequence SEQ ID NO: 52 and a sense strand of nucleotide sequence SEQ ID NO: 103;
- an antisense strand of nucleotide sequence SEQ ID NO: 53 and a sense strand of nucleotide sequence SEQ ID NO: 104;
- an antisense strand of nucleotide sequence SEQ ID NO: 54 and a sense strand of nucleotide sequence SEQ ID NO: 105;
- an antisense strand of nucleotide sequence SEQ ID NO: 55 and a sense strand of nucleotide sequence SEQ ID NO: 106;
- an antisense strand of nucleotide sequence SEQ ID NO: 56 and a sense strand of nucleotide sequence SEQ ID NO: 107;
- an antisense strand of nucleotide sequence SEQ ID NO: 57 and a sense strand of nucleotide sequence SEQ ID NO: 108;
- an antisense strand of nucleotide sequence SEQ ID NO: 58 and a sense strand of nucleotide sequence SEQ ID NO: 109;
- an antisense strand of nucleotide sequence SEQ ID NO: 154 and a sense strand of nucleotide sequence SEQ ID NO: 625;
- an antisense strand of nucleotide sequence SEQ ID NO: 155 and a sense strand of nucleotide sequence SEQ ID NO: 626;
- an antisense strand of nucleotide sequence SEQ ID NO: 156 and a sense strand of nucleotide sequence SEQ ID NO: 627;
- an antisense strand of nucleotide sequence SEQ ID NO: 171 and a sense strand of nucleotide sequence SEQ ID NO: 642;
- an antisense strand of nucleotide sequence SEQ ID NO: 172 and a sense strand of nucleotide sequence SEQ ID NO: 643;
- an antisense strand of nucleotide sequence SEQ ID NO: 173 and a sense strand of nucleotide sequence SEQ ID NO: 644; or
- an antisense strand of nucleotide sequence SEQ ID NO: 562 and a sense strand of nucleotide sequence SEQ ID NO: 1033.

8. An siRNA molecule for the use according to any of claims 1 to 7, wherein the siRNA targets SEQ ID NO: 1.

9. An siRNA molecule for the use according to any of claims 1 to 7, wherein the siRNA targets SEQ ID NO: 3 or SEQ ID NO: 5.

10. An siRNA molecule for the use according to any of claims 1 to 9, wherein the virus infection is in a subject which is an animal, preferably a mammal, more preferably a human.

11. An siRNA molecule for the use according to claim 10, wherein the subject has been diagnosed with a virus infection caused by a RNA virus selected from a *Coronavirus*, preferably severe acute respiratory syndrome coronavirus 2 (SARS-CoV-2), severe acute respiratory syndrome coronavirus (SARS-CoV) or Middle East respiratory syndrome coronavirus (MERS-CoV); a *Flavivirus*, preferably West Nile virus (WNV), Dengue virus (DENV), Zika virus (ZIKV) or yellow fever virus (YFV); an *Hepatovirus*, preferably hepatovirus A or hepatitis A virus (HAV); an hepatitis C virus (HCV); an hepatitis E virus (HEV); an *Influenzavirus*, preferably influenzavirus A, influenzavirus B, influenzavirus C, or influenzavirus D; an *Orthopneumovirus*, preferably respiratory syncytial virus (RSV); or an *Arenavirus*, preferably Guanarito virus, Junin virus (Argentinian mammaarenavirus), Lassa virus, Lujo virus, Machupo virus, Sabia virus (Brazilian mammarenavirus) or Whitewater Arroyo virus, or is at risk of exposure to any of said viruses.

12. An siRNA molecule for the use according to any of claims 1 to 9, wherein the virus infection is in a plant, preferably wherein the virus infection is caused by tomato brown rugose fruit virus (ToBRV), turnip yellow mosaic virus (TYMV), tobacco mosaic virus (TMV), brome mosaic virus (BMV), or tomato bushy stunt virus (TBSV).

13. An small interfering RNA (siRNA) molecule which targets the eukaryotic translation elongation factor 1 alpha 2 (EEF1A2) gene, the siRNA targeting a nucleotide sequence selected from any of SEQ ID NO: 1 to SEQ ID NO: 51 and SEQ ID NO: 1168 to SEQ ID NO: 1173.

14. A siRNA molecule according to claim 13, wherein the siRNA comprises or consists of:
- an antisense strand of nucleotide sequence SEQ ID NO: 1142 and a sense strand of nucleotide sequence SEQ ID NO: 1150;
- an antisense strand of nucleotide sequence SEQ ID NO: 1143 and a sense strand of nucleotide sequence SEQ ID NO: 1151;
- an antisense strand of nucleotide sequence SEQ ID NO: 1145 and a sense strand of nucleotide sequence SEQ ID NO: 1153;
- an antisense strand of nucleotide sequence SEQ ID NO: 1149 and a sense strand of nucleotide sequence SEQ ID NO: 1157;
- an antisense strand of nucleotide sequence SEQ ID NO: 1144 and a sense strand of nucleotide sequence SEQ ID NO: 1152;
- an antisense strand of nucleotide sequence SEQ ID NO: 1146 and a sense strand of nucleotide sequence SEQ ID NO: 1154;
- an antisense strand of nucleotide sequence SEQ ID NO: 1147 and a sense strand of nucleotide sequence SEQ ID NO: 1155; or
- an antisense strand of nucleotide sequence SEQ ID NO: 1148 and a sense strand of nucleotide sequence SEQ ID NO: 1156.

15. An siRNA molecule according to any of claims 13 or 14, wherein the siRNA is an siRNA duplex comprising or consisting of:
- an antisense strand of nucleotide sequence SEQ ID NO: 52 and a sense strand of nucleotide sequence SEQ ID NO: 103;
- an antisense strand of nucleotide sequence SEQ ID NO: 53 and a sense strand of nucleotide sequence SEQ ID NO: 104;
- an antisense strand of nucleotide sequence SEQ ID NO: 54 and a sense strand of nucleotide sequence SEQ ID NO: 105;
- an antisense strand of nucleotide sequence SEQ ID NO: 55 and a sense strand of nucleotide sequence SEQ ID NO: 106;
- an antisense strand of nucleotide sequence SEQ ID NO: 56 and a sense strand of nucleotide sequence SEQ ID NO: 107;
- an antisense strand of nucleotide sequence SEQ ID NO: 57 and a sense strand of nucleotide sequence SEQ ID NO: 108;
- an antisense strand of nucleotide sequence SEQ ID NO: 58 and a sense strand of nucleotide sequence SEQ ID NO: 109;
- an antisense strand of nucleotide sequence SEQ ID NO: 154 and a sense strand of nucleotide sequence SEQ ID NO: 625;
- an antisense strand of nucleotide sequence SEQ ID NO: 155 and a sense strand of nucleotide sequence SEQ ID NO: 626;
- an antisense strand of nucleotide sequence SEQ ID NO: 156 and a sense strand of nucleotide sequence SEQ ID NO: 627;
- an antisense strand of nucleotide sequence SEQ ID NO: 171 and a sense strand of nucleotide sequence SEQ ID NO: 642;
- an antisense strand of nucleotide sequence SEQ ID NO: 172 and a sense strand of nucleotide sequence SEQ ID NO: 643;
- an antisense strand of nucleotide sequence SEQ ID NO: 173 and a sense strand of nucleotide sequence SEQ ID NO: 644; or
- an antisense strand of nucleotide sequence SEQ ID NO: 562 and a sense strand of nucleotide sequence SEQ ID NO: 1033.

16. An small interfering RNA (siRNA) molecule which targets the eukaryotic translation elongation factor 1 alpha 2 (EEF1A2) gene, and which reduces the expression and/or activity of EEF1A2 in a cell, for use in the prophylactic or therapeutic treatment of a virus infection in a subject.

17. An antiviral composition comprising at least an siRNA molecule defined in any of claims 13 to 15 and an acceptable carrier.
